(19) Europäisches Patentamt / European Patent Office / Office européen des brevets

(11) **EP 2 527 454 B1**

(12) **EUROPEAN PATENT SPECIFICATION**

(45) Date of publication and mention of the grant of the patent:
**03.05.2017 Bulletin 2017/18**

(51) Int Cl.:
***C12N 15/82*** *(2006.01)* ***A01H 5/00*** *(2006.01)*
***C12N 9/10*** *(2006.01)*

(21) Application number: **12181577.3**

(22) Date of filing: **03.11.2009**

(54) **Vegetabile material, plants and a method of producing a plant having altered lignin properties**

Vegetabiles Material, Pflanzen und Verfahren zur Herstellung einer Pflanze mit veränderten Lignin-Eigenschaften

Matière végétale, plantes et procédé de production d'une plante dont les propriétés de la lignine sont modifiées

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **03.11.2008 SE 0850065**
**03.11.2008 US 110614 P**

(43) Date of publication of application:
**28.11.2012 Bulletin 2012/48**

(62) Document number(s) of the earlier application(s) in accordance with Art. 76 EPC:
**09829400.2 / 2 350 291**

(73) Proprietor: **Swetree Technologies AB**
**904 03 Umeå (SE)**

(72) Inventors:
- **Hertzberg, Magnus**
  **903 39 Umeå (SE)**
- **Sundberg, Björn**
  **907 36 Umeå (SE)**
- **Sandberg, Göran**
  **905 82 Umeå (SE)**
- **Schrader, Jarmo**
  **31162 Bad Salzdetfurth (DE)**
- **Teeri, Tuula**
  **186 56 Täby (SE)**
- **Aspeborg, Henrik**
  **141 70 Segeltorp (SE)**
- **Wallbäcks, Lars**
  **905 91 Umeå (SE)**
- **Bhalerao, Rishikeshi**
  **907 41 Umeå (SE)**
- **Trygg, Johan**
  **904 35 Umeå (SE)**
- **Johansson, Karin**
  **904 40 Röbäck (SE)**
- **Karlsson, Ann**
  **905 88 Umeå (SE)**
- **Jonsson, Pär**
  **903 60 UMEÅ (SE)**

(74) Representative: **Zacco Sweden AB**
**P.O. Box 5581**
**114 85 Stockholm (SE)**

(56) References cited:
**EP-A2- 1 033 405** **WO-A2-2007/067525**
**US-A1- 2004 031 072** **US-B1- 7 317 136**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

SEQUENCE LISTING

<110> Swetree Technologies AB

<120> A method for modulation of lignin content in plants

<130> 213225

<160> 195

<170> PatentIn version 3.4

<210> 1
<211> 42
<212> DNA
<213> Artificial

<220>
<223> Primer

<220>
<221> misc_feature
<222> (42)..(42)
<223> n is a, c, g, or t

<400> 1
gggacaagtt tgtacaaaaa agcaggcttt tttttttttt vn 42

<210> 2
<211> 34
<212> DNA
<213> Artificial

<220>
<223> Primer

<400> 2
agaaagctgg gtattgaata gccaaatatc tctc 34

<210> 3
<211> 31
<212> DNA
<213> Artificial

<220>
<223> Primer

<400> 3
agaaagctgg gttccttaac ttgtccgaga g 31

<210> 4
<211> 34
<212> DNA
<213> Artificial

<220>
<223> Primer

<400> 4
agaaagctgg gttagagttc gagaggtcat cttc 34

<210> 5
<211> 32
<212> DNA
<213> Artificial

<220>
<223> Primer

<400> 5
agaaagctgg gtgcttagtg gtctgggttt ag 32

<210> 6
<211> 34
<212> DNA
<213> Artificial

<220>
<223> Primer

<400> 6
agaaagctgg gtgaaggtgg tggttcaatt atac 34

<210> 7
<211> 49
<212> DNA

<213> Artificial

<220>
<223> Primer

<400> 7
ggggaccact ttgtacaaga aagctgggtc gctgacacca ccactttcc 49

<210> 8
<211> 50
<212> DNA
<213> Artificial

<220>
<223> Primer

<400> 8
ggggaccact ttgtacaaga aagctgggta acgtgatttc aaagggtcgc 50

<210> 9
<211> 54
<212> DNA
<213> Artificial

<220>
<223> Primer

<400> 9
ggggaccact ttgtacaaga aagctgggtg taatacgact cactataggg cgaa 54

<210> 10
<211> 31
<212> DNA
<213> Artificial

<220>
<223> Primer

<400> 10
aaaaagcagg ctttgttgtt gtttcgatct g 31

<210> 11
<211> 32
<212> DNA
<213> Artificial

<220>
<223> Primer

<400> 11
aaaaagcagg ctcgagaaag aagaacctag tg 32

<210> 12
<211> 33
<212> DNA
<213> Artificial

<220>

<223> Primer

<400> 12
aaaaagcagg ctatttgggt cattcttgct cac 33

<210> 13
<211> 33
<212> DNA
<213> Artificial

<220>
<223> Primer

<400> 13
aaaaagcagg ctaatcagtt gcttctcatt gtc 33

<210> 14
<211> 33
<212> DNA
<213> Artificial

<220>
<223> Primer

<400> 14
aaaaagcagg ctccatccct catcactaaa tcc 33

<210> 15
<211> 49
<212> DNA
<213> Artificial

<220>
<223> Primer

<400> 15
ggggacaagt ttgtacaaaa aagcaggcta gaaagccagg ggagaatgc 49

<210> 16
<211> 53
<212> DNA
<213> Artificial

<220>
<223> Primer

<400> 16
ggggacaagt ttgtacaaaa aagcaggctg ctctgttgtt actctggaca tgc 53

<210> 17
<211> 54
<212> DNA
<213> Artificial

<220>
<223> Primer

<400> 17

ggggacaagt ttgtacaaaa aagcaggcta ttgattaacc atggagaaag caca 54

<210> 18
<211> 54
<212> DNA
<213> Artificial

<220>
<223> Primer

<400> 18
ggggacaagt ttgtacaaaa aagcaggcta agctgatgat caaatgagga catc 54

<210> 19
<211> 54
<212> DNA
<213> Artificial

<220>
<223> Primer

<400> 19
ggggacaagt ttgtacaaaa aagcaggcta ttaatcatgt caggtgatca aagg 54

<210> 20
<211> 52
<212> DNA
<213> Artificial

<220>
<223> Primer

<400> 20
ggggaccact ttgtacaaga aagctgggtc ctttttcccc tacaaatcat gg 52

<210> 21
<211> 54
<212> DNA
<213> Artificial

<220>
<223> Primer

<400> 21
ggggaccact ttgtacaaga aagctgggtc aaggatgtat gcttggttct atga 54

<210> 22
<211> 54
<212> DNA
<213> Artificial

<220>
<223> Primer

<400> 22
ggggaccact ttgtacaaga aagctgggta agaagccaaa ggacttaagc tgtc 54

<210> 23

<211> 1351
<212> DNA
<213> Artificial

<220>
<223> Construct sequence

<400> 23

```
cgcacagaga ggatttcagg aagcgctggg gatgtgttgg atgatgatcc agtgggaagg      60
ttgaaggtct atgtttatga gcttccgagt aaatacaaca agaaactgct gcagaaggat     120
cccagatgtc tcacccatat gtttgctgca gaaatcttca tgcatagatt tctcttatcc     180
agcccagttc gaacccttaa cccagatgaa gcagattggt tttattcccc tatataccc c    240
acttgtgatc tcacacccat gggcttgccg ttgcccttca aatctcctag gatgatgaga     300
agtgcgatac agctaatctc ctccaattgg ccttactgga atcgtacgga aggggctgat     360
cacttttttg ttgtgcccca tgactttgga gcctgcttcc actatcagga agagaaagcc     420
gttgagcggg gcattcttcc gctactccag cgttctactt tggttcaaac ttttgggcaa     480
cgaaatcatg tgtgcttgaa tgaggggtca attacaattc ctccttttgc tcctcctcaa     540
aagatgcagg cccaccagat tccccctgac attccacggt ccatttttgt ctatttccgt     600
ggattgtttt atgatgtaaa taatgatcca gaaggtggtt attatgcaag aggagcaagg     660
gctgcagttt gggagaattt caagaacaat ccactcttcg acatctccac tgaccatcca     720
acaacatatt atgaagacat gcagcgagct atattttgct tgtgcccgct cggttgggct     780
ccatggagcc ctagattagt tgaagcagtg gtatttggat gcattcctgt catcatagca     840
gatgatattg ttttgccatt tgccgatgct atcccatggg aggaaattgg ggtgtttgta     900
gcagaggaag atgtccctaa cctggacaca atcctaacat ccataccacc agaggtgatt     960
ttaaggaaac aaaggcttct tgcaaatcct tctatgaaac gtgcaatgtt attcccacaa    1020

cctgcacaac caggtgatgc tttccaccaa atcctaaacg ggctggctcg taagttgccg    1080
cacgacagga gtgtttactt gaagtctggt cagaatattt tgaattggac agcaggacca    1140
gttggggacc tgaaaccttg gtaagagaag tgtcatttcc tcaacagaca tggctgtggt    1200
gtatctactg tttcatctaa tcgcaaggac tgtaaacttc tctttgaagt tttgattttt    1260
gtatagattc ttgaatttca cgtgtacatc tgggacatag ttcgatgctt taaataactt    1320
cgcatttttc ttggtatcgg caaaaaaaaa a                                    1351
```

<210> 24
<211> 813
<212> DNA
<213> Artificial

<220>
<223> Construct sequence

<400> 24

```
tgcagacttt gggaggtatt ctccaaatat agcaaatgtt ggcaaagatg tgattgcacc     60
atacaaacat gtaattaaaa gctatgccaa cgactcctct aactttgaca gccgcccgac    120
tttgctgtat tttcaaggag ccatatacag gaaagatggt ggctttgctc ggcaagaatt    180
attttatgct ctaaaagatg aaaaagacgt gcattttcag ttcggtagtg tacaaaagga    240
tggagtgagc aaagcttctc aaggaatgca ctcctccaaa ttctgcctta acatagcagg    300
tgacacaccc tcatcaaacc gcctctttga tgccattgcc agccactgcg ttccggtcat    360
catcagtgac gatattgagc tcccttatga agatgtcctc gactactctc agttctgcat    420
atttgtccgc acttcagatg ctgtcagaga aaagttcctc ataaatcttg tcaggagtat    480
taagaaggat gaatggacta gaatgtggca gaggttgaaa gaggtagaaa acttctttga    540
gttccaatat ccatcaaagg aaggagatgc agttcaaatg atatggcagg ctgtagcaag    600
aaaagtccct gcaattaggt tgaaggtaaa taagtttagg cgattttctc gttttgggac    660
ccctaaagat ggagtgttga gaaaaatacc atcacctagt aatttttggt gaaacattca    720
gtgtttcttt atagtgaact ttgaggttct gtttatatat aaaggtgaga aattcaattt    780
attctcactg cagattttgg caaaaaaaaa aaa                                 813
```

<210> 25
<211> 807
<212> DNA
<213> Artificial

<220>
<223> Construct sequence

<400> 25

```
attagcaatg aaagaaccta tggcgaaaac agtccttgag aatggttctc agggacccaa     60
cgtggcagaa tctgatatta tgaaatttcc acgggaccga aggttgaata tagatgatga    120
ccataacttc tgtaatattc cgtgtggcca aaatggtgat ggaaatctca tgtctgctgc    180
```

tggaacatat gatgtatcac acttgaatgg atttgcagta ggcagccaga tgtctcttgc 240

attgggattg caaagtaacg acagtgattc atttcccacg tttgatgggg cccatacgag 300

aggtaacacc atatcagctt ccccggttgg gcataatgag gtggattatc actgtatgga 360

tacaggaaag caacaagaca ggattgccag ttcccatcgg ttacatgatt ttgtggtttg 420

agagaatcct tctagcttta cgagggagtt tcatcaatcc tgccaagaga aggattgatg 480

gagttttgga taacataaag cagaagaaaa caagctcagt gatactggtt tgttattcat 540

tcaagccccg tgctcttctc ttggtttgaa tagaagaaaa ccaacaccct tacagccaca 600

gacatagatg taacataagg gcatggtgcc tgcttgtaat atacgtacat attacaggga 660

ttgtagatat atcgatctgt gcatatacaa taagattttt gtgatctttt gtactcttaa 720

gcaatttcta caagctctcc ctaattgtga cactgatgta tcatttcatt cctaattctg 780

aaataaatgt gatttgctaa aaaaaaa 807

<210> 26
<211> 849
<212> DNA
<213> Artificial

<220>
<223> Construct sequence

<400> 26

```
agcagtcaga gcttataact agctaattga ttaaccatgg agaaagcaca cacaaaatct      60
gctcttaaga agcttgttaa ggctagctca cagtctgcat cgtggagcaa tgctgctaga     120
ggaatggcaa aagatgatct caaggatccg ctttatgaca aatccaaggt tgccccaaaa     180
cctcttgcaa aagaaaacac taagccccag gaattcaaac tccacactgg acaaagagct     240
ctcaaacgtg ccatgttcaa ctattctgtg gcaaccaaga tatatatgaa tgagcaacag     300
aagaggcaaa tagagaggat acaaaagatc atagaagaag aagaggttcg tatgatgagg     360
aaggagatgg ttccaagagc tcaattgatg ccttactttg acagaccttt ctttccccaa     420
agatcaagca ggccattgac agttcctaga gagccaagtt tccacatggt gaacagcaag     480
tgttggagct gcatccctga ggatgaactt tactactact ttgaacatgc tcatccccat     540
gatcatgcct ggaggcctgt taattaaata attgccatga tttgtagggg aaaaaggaga     600
gtggagtatt ctctctgtgc tgctaatttc ttcatgctgt ccaagttagt cttgtataat     660
ttggactttt ggagtgttga tagtgtttac tgtttagatc atgtggtagg tcttgtagtg     720
ggtgtaagta gtcgcttttt catagccgaa agtccttttc aatgggggat tttgcttacc     780
aataaggcgg atattgtact tcgttttgta ctattaaaaa gcaagtcacc ttcttccaaa     840
aaaaaaaaa                                                             849
```

<210> 27
<211> 1139
<212> DNA
<213> Artificial

<220>
<223> Construct sequence

<400> 27

ggaagaccta ctcaccgatg caggcatggt tctcactgca ccctctggct ccggcaataa 60
ttgaggtcaa ggcattgcat catttcgatt ggttcgcaaa ggggaaggtg ccagtcatgg 120
aagcaatgga aaaggatcag agggtgaggt cacagtttag aggggttcg tctgccattg 180
tagcaaataa cactgagaag cctcatgtta tcgcagcaaa actacaaaca cttagtccca 240
aatacaattc agtgatgaat cacatccgaa ttcatctacc tgagctgttc ccaagcctaa 300
acaaggttgt gttcctagac gacgacatag tggtgcagtc cgatctttcg cctctatggg 360
acgtcgacat ggatggaaag gttaacggtg cagtagaaac ctgtcgagga gaagacaagt 420
ccgtcatgtc aaagaagttg aagagctatt tgaacttctc tcatcctttg atatcagaaa 480
atttcaaacc caacgaatgt gcctgggctt atggaatgaa catatttgat ttagaggctt 540
ggaggaaaac cacataagca caacatacca tcactgggtt gaagagaact tgaaatcaga 600
cctgagcttg tggcagctag gaacactgcc tccaggtctg atagcattcc atggccacgt 660
ccacgttatc gatccctttt ggcacatgtt gggtcttggc taccaagaaa atacaagttt 720
ggcagatgct gagactgccg gagtcatcca tttcaacggt cgagcaaagc cttggctgga 780
tatagcattc cctcagctcc gccccctgtg ggcgaagtac atcaactttt ccgacaagtt 840
cataaagggc tgtcatatta ggccatctta acttgagttt tgagatgaaa atgttggctg 900
cgatcgtgat taatatatgt ctatatataa ggagaaaaaa agcagagaag agagagttct 960
tcacaaatcc aaggccttga atccagagag taaaatattc ataaccaagc atttgtttgc 1020
ttggtttttt tagaattcat cctctgtggg cgaacaaccc ttccctatta cttcaaatct 1080
tgattcaatt ttaatgagaa attaagcatg ttatggattt tcaacgggca aaaaaaaaa 1139

<210> 28
<211> 898
<212> DNA
<213> Artificial

<220>
<223> Construct sequence

<400> 28

cgaaaaacga gatacacaag attaaccaaa ttgtagtaga tcccagcttg catcttcaaa 60
agaggagcca cctttattac gctgttgaga ctcgaacaac acctgatgct ttcaagatat 120
ttggaggttc gccctggctg attcttacaa gagctttcat ggagtactgt gtccaaggat 180
gggacaacct tccaagaaaa ctactaatgt acttcagcaa cacggcatcc ccacttgaat 240
cgtatttcca ctctgtcctc tgcaactctc ctgagtttca aaacacaaca gtaagcgatg 300

```
atttaaggta caatattctg gaaactacta cagatgggga atcaccttat gacaaaatgc     360
taaatggtgg agcagcattt gcaaggccat ttaaagaaga tgctgctgct ctaaacatga     420
tagatgagaa cgtcttgaac cgtgaaccca acggattggt gcctggaaaa tggtgcttag     480
accagggtat gaacaagagc tcagaggcat caaagcctcc aggggaggat ttgtgttcaa     540
cctggggtaa cattaatgat gtcaagccag gatcttatgg tatcaagctt gcatttttat     600
tgtctaagat tgccagtgaa gagaaattga caactagtca atgccttcaa gctacaaaaa     660
tggggtcatc atagaaccaa gcatacaacc tagtgtggat aattttttc ttttaatcga      720
aaactatagg ttaaatcaag cttcttagcc atataaatcc tttgctaaat gcactacaaa     780
tgcatacttg cttgttaaaa caaaagctag aagcacccag agatatattg tacatacatt     840
cacattttat cgaggatttc tacaataaat ttattgtttt tcccccaaaa aaaaaaaa       898
```

<210> 29
<211> 1278
<212> DNA
<213> Artificial

<220>
<223> Construct sequence

<400> 29

```
aatcactctt tcaacttcaa gctgagcgaa ttttgtgtgt ccaaagacta gcagcccaag      60
ctttaagtga aggttctgga agctcagttt caatacattg gtacataatg accagcccgt     120
ttactcatga ttccacacga ttttttttg aaaatcacaa gtactttggc cttgaagcag     180
atcaggttac cttcttccag caaggcacca taccttgtgt ttccaaggat ggcagattta     240
tcatggagac tccatttagg gtagctaagg ctccgggtgg gaatggagga gtatattcag     300
cgctgaaata ttcaaaatta ttagaggata tggcctcaag agggatcaag tacgtggact     360
gctatggggt tgacaacgca ctggtacgtg tagctgatcc agcttttttg ggatatttca     420
ttgataaagg tgtagcagct gcagcaaaag ttgttcgtaa ggcatacccc caagaaaagg     480
ttggtgtttt tgtaaggcaa ggtaaaggtg gacctcttac cgtggttgaa tacagtgagc     540
tggatcagtc actggcttct gcagtcaatc aacaaactgg acgccttcgt ttttgttgga     600
gtaatgtgtg cttgcacatg ttctctttgg attttctaaa ccaagtggca aatggccttg     660
agaaagacag catttaccat ctagctgaga aaagaattcc ttctattcat ggtgatacga     720
tgggattaaa actagagcaa ttcatattcg atgcattccc atatgctcct tcaactgctc     780
tttttgaggt accacgtgaa gaagaatttg cacctgtaaa aaatgccaat gggtcaaatt     840
ttgacactcc tgagagtgct cggctgcttg ttcttcgact gcatactcgc tgggtggttg     900
cagcaggtgg cttcttaaca cattcggtgc ctttatatgc aactggtgtg gaggtatcac     960
cactttgctc ctatgctggc gaaaatctag aagccatatg ccggggaaga acatttcacg    1020
caccttgtga gattacattc tagttaaatt tgtatccata tattcttgat ttgtacggct    1080
cccattggaa aggggaaagg ggttatccat cccacgcatg tactgttgtg gtagattagc    1140
gagagatatg aatctgtaag agttccaggt tttgttacat cctgggtgtg atgtcaagag    1200
tgatactatt tgattaatgt atatcattca agttattgtt gagcaatgat aatcctctct    1260
tcttcctaaa aaaaaaaa                                                  1278
```

<210> 30
<211> 491
<212> DNA
<213> Artificial

<220>
<223> Construct sequence

<400> 30

```
gaaaagggtt gcaattatta caccaagaat ttccatggat cagttgaggc tttccatgta     60
gacaccgttg ataccacagg tgctggtgat gcatttgttg gtgctctcct ttgcaagatt    120
gttgatgacc aatctgtgct tgaggacgag ccaaggttga gagagatact aagatttgca    180
aatgcttgtg gagccattac aaccaccaaa aagggagcaa tccctgctct tcctactccg    240
gccgatgccc tcaaactggt gaacgaagga aaatgaactc tttttttta atccgcactg    300
catttttccc tttccttttc agctcctggt tctcaattca atcgaagagc tgttgcaatc    360
tcaaattttc gcctttttct ctcgagtgta atgatttgaa agacttgcgc tttaaggtct    420
tgccaaataa attttttct tttgtgttag ctttctccaa atattatatt tgtttaaagc    480
aaaaaaaaaa a                                                          491
```

<210> 31
<211> 979
<212> DNA
<213> Artificial

<220>
<223> Construct sequence

<400> 31

```
caaggaggta tattgagtgg agagggcggt gatgaaacca aagatattct tttattggac     60
gtggctcccc tcaccctggg tattgaaact gttggtggag tgatgaccaa gttgattcca    120
aggaacactg ctattccaac caagaaatct caagttttca ccacttacca ggatcagcag    180
actactgtct ccattcaggt ctttgaaggt gaaaggagtc tcacaaagga ttgcaggctg    240
cttggtaaat ttgatttgac tagcattcct ccagctccaa ggggaacccc tcaaattgag    300
gtcacgtttg aggttgatgc caatggtatc ctgaatgtca aggctgaaga caagggcact    360
ggtaaatcag aaaagatcac catcacgaat gacaagggtc gtttgagcca ggaggaaatc    420
gagcgcatgg ttcgtgaggc agaggagttt gccgaggagg acaagaaggt gaaggagagg    480
attgatgctc gtaacagttt ggagacctat gtatataaca tgaaaaacca gattaacgac    540
```

aaagataagc ttgctgacaa gctcgagtct gatgagaagg agaaaataga aactgctacc 600

aaggaggccc ttgaatggct ggatgacaac cagaatgccg agaaagagga ttatgaagag 660

aagctcaagg aggtggaagc tgtttgcaac ccaatcatca ctgctgttta ccagagatct 720

ggtggagctg caggtggtgg atcagctgaa gattccgagg acgactctca agatgaactc 780

tgagaatctt tctgcttcct tctacccctc ctggaaagca agggcaagcg atgaggatgt 840

aggctagatg aagtagagga tattgtaatt ttgttgatcc tagaagctaa agggttaaaa 900

taacatatcc ccttttttttg gtttttttgag gttaaaaata catggaatag tttgtttcca 960

aaaaaaaaaa aaaaaaaaa 979

<210> 32
<211> 701
<212> DNA
<213> Artificial

<220>
<223> Construct sequence

<400> 32

ggaatggaga gctctaccgt tacatttgtg ataccaaggg agcttttgtg cagcctgctt 60

tgtatgaggc ttttggattg actgttgtgg aggccatgac atgtggtttg ccaacctttg 120

ctacttgcaa tggcggtcct gctgagatca ttgtgcatgg aaaatccgga ttccatattg 180

atccttacca tggagtacag gctgctgaac tccttgttga cttctttgag aagtgcaagg 240

ctgatcccac ttactgggac aaaatctccc agggaggcct gcagcgaatc caagagaagt 300

atacctggaa aatttactct caaaggctcc tgactctcac aggagtttat ggcttctgga 360

agcacgtttc caaccttgat caccgtgaga gccgtcgcta tctggaaatg ttctatgcac 420

tgaaatatcg caaattggct gattctgttc ctttgactat cgagtaaatg gaactggaga 480

aatcgaggaa acatgggttg gtttgagtcg ggttccgggt ccagaataat ggtcatttca 540

cgatagtgat tggacaagaa aggctttgat cttcttttttg ttttttttgtt tttcctttct 600

gtgtgcatct tttttgtttt cccttttctt ttcatcatgc agcctcttca atttcagtaa 660

agattgtgtc tgttgagcta aaaaaaaaaa aaaaaaaaaa a 701

<210> 33
<211> 1439
<212> DNA
<213> Artificial

<220>
<223> Construct sequence

<400> 33

```
ctccaatcac gcgtccgctc tacacaggtc gatccaaagc aattttctcc attacaagag      60
aagccaagaa tttgtgacga acttggtata aattagtttt gttttgacaa gagaaagact     120
tgtttgttta atttccatgt gtctgaggag ggaacgaaga acaaccccag ggtgcttttc     180
attgttcgga cacctcttag attctcagac aacttgaaca caaaatattt cccgagttcc     240
aggatcattt tgaaggatac aaggaaagag gaataaaaag aaagaacaat ggcattcacg     300
ggaaccctgg ataagtgcaa ggcttgtgat aagactgttt attttgttga tatgatgtct     360
cttgaagggg tgccttatca taaatcctgc ttcaaatgca gccattgcaa aggaactctt     420
gtgatgagca actactcatc catggatgga gtcctctact gcaagactca ttttgagcaa     480
cctttcaagg agggtggaga tttcagcagg aattttcaga aagggaagcc cgagaggact     540
catgacctgt ctaggatccc cagcaaacta tctccagttg ttctgtggaa cccaggacaa     600
atgctccact tgcggcaaaa cagtgtaccc tttggagaag gtttgttttc agctctaaac     660
ttaaataaat ccattaggtc ctaaagacct gtgtgctatt cataccccta catttatcag     720
ataaatgacg aacatatttt aggtacatct tctctatggt agatggatct atctgagaaa     780
tgttttcaag tctctgttat ttaatagcca tgcctgtgat aatctggaat aagcacttaa     840
gtgctataac cattgatgac ataggttacc atggagggag aatgttacca caagacatgc     900
ttcaggtgtg ctcatggtgg atgtccactc acacactcat cttatgctgc tcttgacgga     960
gtcctctact gcaaggtcca cttcgcccag ctcttcatgg aaaagggaac ctacagccat    1020
gtccttgcat cagctgcaca taagagatca aattcgacac ctccggaact ggccgggtca    1080
aacccagagg aaggagctgc tgcggaggag gaaaagtcag aagagcaatc ttagacaaag    1140
cacatcatgt tttcttgcat tgctatcttt tcaagttgcc aggactgctt cgagttttca    1200
tgttgttgaa tattattgcc ggattgtgtt caaagtttgt ttttggattt gactatctat    1260
gttacaatgc cttgctacag gatattgaat tgcactagtt tttacaaatt cctcctcttg    1320
caatttaata ttcttggcaa caggatacaa atgaaaaacc caaatcctag gaaccagtat    1380
caacattcat tgatctcagc tctttgctca gatggattgc gttttccaaa aaaaaaaaa     1439
```

<210> 34
<211> 792
<212> DNA
<213> Artificial

<220>
<223> Construct sequence

<400> 34

```
cttgttcgat ccctgcacta atcctgatta atgcctgcat tccctttag attttgtgtt      60
tgattttgtc ctgtaaggag ctggatatgc gggatcatat ggagagattt gtagttcttc    120
cattctccat cgcctgtgct tctcactcca gtgttgatgt ggcctccagt gaatcctcca    180
agaaaccaag acccgaaacc aaatcacatg catcaagagg acaagaaggg gaggaaagct    240
cttgtaaaga aaagacgaag aacagtacac ttggtttcct gctggctctt ccaaagcctt    300
gcatatccag tagcttgcac aaattgatta gaggcatcaa gactctctcc caagtatttg    360

tgtacaagga agaagacgag gagctaatgg aaagagagat ggaaatcgga tatccaactg    420
atgtgaagca tgtaacacac ataggattgg atggaactac gatgacaaat cctataaagg    480
gctgggattg tctgaaatct ccagaaataa ttccattccc ttcatttact ttaaggcagt    540
tcgagcttgc aatggctgca caagctcatg gacctcttgt tggggtcgat cattccaagc    600
ttgtttgatt cattgatttt tcttttcatt tcctgatctt gtttctttga cactagatga    660
ctgatgtgat gaagattgat caatgttttt gatggaggca ctggttgcag tgatgtgttt    720
ttggtgtttg tgtgggacct tgacaatgtt tttctgggtg gccattgaaa ttgttcttgc    780
aaaaaaaaaa aa                                                        792
```

<210> 35
<211> 823
<212> DNA
<213> Artificial

<220>
<223> Construct sequence

<400> 35

```
gctagctagc tttgcagagt tctgtcgatt caatggcgac caaagtgtat attgtgtact     60
attccatgta tggacatgta gaggaactgg cagaagagat taagaaaggg gcttcatctg    120
ttgaaggtgt tgaggcccaa ttatggcagg ttcctgagac actgccagaa gaggtgcttg    180
gaaagatgag tgcaccacca aagagtgatg taccagtcat tacacctagt gaactcgctg    240
aagctgatgg ctttgtgctt ggattcccga caagatttgg aatgatggct gcccaattca    300
aagcttttct ggatgcaact ggaagtctat ggagaacaca gcaacttgct ggcaagcccg    360
ctggaatctt ctttagcact ggatcgcaag gtggtggcca agagaccaca gcgttgactg    420
ctattaccca acttgttcac catgggatga tatttgttcc cattggctac acatttggtg    480
ctggcatgtt tgagatggag aaggtgaaag gtggaagtcc ttacggcgca ggaactttcg    540
ctggggatgg gtcgagacag ccaaccgagc ttgaattgga gcaggctttc caccagggca    600
agtacattgc tgccatcaca aagaagctca aaggagctgc ttaagtttac tcaattacaa    660
gaatacggtc tggtcttcca ttttttcacc ctatagaata tgcatttgca aagaataatt    720
cgtattccac cgtttattat atgcttttca tgtgtgtaac atgatagtga ttattgtctc    780
aatgatatca aggttggttg tgttttcatt taaaaaaaaa aaa                      823
```

<210> 36
<211> 1113
<212> DNA
<213> Artificial

<220>
<223> Construct sequence

<220>
<221> misc_feature
<222> (579)..(579)
<223> n is a, c, g, or t

<220>
<221> misc_feature
<222> (639)..(639)
<223> n is a, c, g, or t

<400> 36

gcggacgcgt gggctctttt ctttctttct ccatcaccca cgcgttttct tagtttcaat 60

tcttgtcaaa atagtcttga tcattttctc tgcaaaagaa attaatcatg tcaggtgatc 120

aaaggcctaa agattctgct gaaggctctt cgcgatctgg aggcgatcac caccagcttc 180

aatcacctcc tttgagccgg tatgagtcgc agaaacggcg agactggaac actttcgggc 240

agtacttgaa gaatcagaga cccccagttt cattatctca gtgtaattgc aatcatgtgc 300

ttgatttcct tcggtatctt gatcagtttg gcaagactaa ggttcatcac catgggtgcg 360

tcttctttgg acaacctgat cctcctgctc cttgtacatg ccctctaagg caagcttggg 420

ggagccttga cgccctaatc ggacgccttc gagcagcttt tgaggagcat ggaggatcag 480

cggagactaa ccccttttgga aatggagcta ttcgggttta tttgcgtgaa gtgaaagagt 540

gtcaagctaa ggcaagaggg attccttaca agaagaagna gaagaagaag actcaaatag 600

gcccagagac gaagcaaagc cttcgatgca gacagcttng tcctttggct tcttccgata 660

aatggatcac aaaataggac aaatggaggt gaagttccag aaaggcaaat gttgggggcc 720

tagcctctgc ctcagtactt aatatatagc tagctagctt ctctctttgc agatagtgtt 780

acatcacagc ctacttctaa ataatcttgt cctcgcaggt tggtctaaga agctattcct 840

agcttatagg aattaaaact gtagtcgtaa tgttcatcga cttcttaaat agtaagacgt 900

tagcactata tgtccagctc tgttgtttct ttgctgcaga agaaaactag tgtcttcagc 960

taatggtgaa aatggctttt agcttctgcc caaatgtagc tttgggcctg cctctcatta 1020

catgagctgt gctttctagt aatccttcaa ttgtccaaag taccatgttt ttcttcatgt 1080

ttaatctcta gttaatttcc taaaaaaaaa aaa 1113

<210> 37
<211> 991
<212> DNA
<213> Artificial

<220>
<223> Construct sequence

<400> 37

cgcaatccat cctatggtgc tttatctgac tatcagttta tgcgaccccg cgcacgtggc 60

aagaaacttc ttgaagagtg ggctgccccc ttgaaaagtg ttgaggatat atacgagaaa 120

ttcaaggtat actgccttgg gaagttgaaa agtagtcctt ggtcagaact agatggcctt 180

cagccagaga ctaaaatcat taatgaacag ctgggtaaga tcaacttgaa gggactcctg 240
actatcaata gtcaaccagc tgtcaatggg gaaaaatctg attctccatc tgttggttgg 300
ggtgggccag gagggtatgt atatcagaag gcttatctag agtttttctg ctccaaggac 360
aagctgaatg ctcttgtgga gaagtgcaag tctttccctt ttgtaactta cattgctgtg 420
aacaaaggag ggagttggat ttctaatgtt gccctgactg atgtgaatgc tgtaacctgg 480
ggagtcttcc cagcaaagga gattatccaa ccaactgttg tggatcccac cagcttcagt 540
gtgtggaagg atgaggcatt tgaaatctgg tcaagaggat gggcttcctt gtacccagag 600
ggtgacccat ccagaaccct acttgaagag gtgaagagca gctacttttt ggtcagcttg 660
gtagataatg attacatcca tggggatatt ttcgctgtct tcgccgattt atgatgcaag 720
gggctctttg caacccctgc atttgctaag agtatccaag tccccagaaa aatggtggtg 780
ctttacccta agttgaatcc cagctttaat catcttatcg gcatacattg tttttgaaat 840
tatttccgta ttgaaatgta ataattcatt gtgattttgc tactcccctc tccattatat 900
gctgcaaatt tgtatttgga gtggcaacta attctaagct ttaagaaaat atgattgcct 960
ctttttcttg gtacttccga aaaaaaaaaa a 991

<210> 38
<211> 1273
<212> DNA
<213> Artificial

<220>
<223> Construct sequence

<400> 38

```
ctctgctttc tctatctaga aaacatatct ctctttttcg tccctcacgt tcccttgcaa      60
tggcttccag tggagtgaac gacaagtcac tgatcgtcag cttcggggag atgctgatag     120
acttcgtacc aacggtctct ggcgtctccc tcgctgaagc tccggggttt gtgaaggcac     180
cgggtggtgc tccggcgaac gtggcgatag cggtggcgag gctgggagga aaggcggcat     240
ttgttggaaa acttggtgat gatgagtttg gcaacatgct cgccggaatt ttgaaggaaa     300
acggcgtgat cgctacaggg ataaattttg acacaggtgc taggactgct ctcgcgtttg     360
ttactctacg cgctgatgga gagcgtgagt ttatgtttta tagaaatcca agtgctgata     420
tgctactaag accagaagag ttaaatcttg agttaattag atctgctaag gttttccact     480
atggatcaat aagtttgatt gtggagccat gcagatcagc acatttacag gcaatgaggg     540
tggcgaagga tgcaggtgca ttgctttcgt atgacccaaa tctgaggctg ccattgtggc     600
catcagaaga ggaggcgcgt gagcagatat tgagcatctg ggacgaggca gatgtgatca     660
aagtcagtga taatgagctt gagttcctca ctggaagtga caaagttgat gatgaaactg     720
catgtcactt tggcgcccta actttaagtt gctcttggtc acccttggtg aaaagggttg     780
caattattac accaagaatt tccatggatc agttgaggct ttccatgtag acaccgttga     840

taccacaggt gctggtgatg catttgttgg tgctctcctt tgcaagattg ttgatgacca     900
atctgtgctt gaggacgagc caaggttgag agagatacta agatttgcaa atgcttgtgg     960
agccattaca accaccaaaa agggagcaat ccctgctctt cctactccgg ccgatgccct    1020
caaactggtg aacgaaggaa aatgaactct ttttttttaa tccgcactgc atttttccct    1080
ttccttttca gctcctggtt ctcaattcaa tcgaagagct gttgcaatct caaattttcg    1140
cctttttctc tcgagtgtaa tgatttgaaa gacttgcgct ttaaggtctt gccaaataaa    1200
tttttttctt ttgtgttagc tttctccaaa tattatattt gtttaaagca aaaatttctg    1260
caaaaaaaaa aaa                                                       1273
```

<210> 39
<211> 803
<212> DNA
<213> Artificial

<220>
<223> Construct sequence

<400> 39

```
cttcgtcatg gggagaagg agcccaagaa agctgaatct gagggagcct ctctgcctac      60
acaagttcaa gaacatggac ctgttaagga agagaaagaa gtccctttaa atgattctgc     120
taatgagaag agctcggtcc tagttactga aaaggttgca gatccacctg ctactgcaaa     180
aaactccagg gggtcaaatg acagagatgc tgtgcttgcg agggttgaag cggagaaaag     240
atgtgctcta cttaaagcat gggaagaaaa tgagaaagct aaagcggaga acaaggctca     300
caagaaactc tctgccattg gatcatggga gacaatcaag agagaatctg tggaggcaaa     360
aataaagaag tatgaggaaa aagtggaaaa gaagaaggct gaatatgcag agaaaatgaa     420
gagcaaagta gccgaactcc acaaggcagc cgaggagaag aaagcaatga ttgaagcaaa     480
aaaaggggag gaccgtctca aggtagagga aactgcagcg aaattccgag caactggtta     540
tacaccaagg aagtgtctaa gatgttttgg caaattctaa tgaacaatag tggggtaaac     600
ggaagagaaa ggctgctaga attggatttg cttgattttt tggcttgtta ttttatgctg     660
tgctcaaata ttgttggttc agctgtaaat gtcttttgat atttgtaaaa accaataaat     720
gggagtgcat gtgttacatt acccaagttc tgacaagacc cgagtttgaa tttgatattg     780
tcttttgtgc gaaaaaaaaa aaa                                             803
```

<210> 40
<211> 1116
<212> DNA
<213> Artificial

<220>
<223> Construct sequence

<400> 40

```
caaacaagca ctggcactgg caatggcaat gccacttctt cttcaactac caccaccact      60
tcttcttctt caacatcttc aagttccatg agcagtaata acagtaatgg caacaatggt     120
tgtcagcaga accaaagtaa ccagcttgga gagacaagat caagtcttta ttattcaaca     180
aatgcaatgt cttttgttac caagtctttg ttgcctacta gaagaagact caggcttgat     240
cctcctaaca agctcttctt cccttatgaa ccaggaaaac aggttaggag tgccattggc     300
ataaaaaaca ctagcaagtc tcatgtagct ttcaagttcc aaacaactgc accaaagagc     360
tgttacatgc gtcctcccgg ggctatactt gcacctggtg aaagtcttat tgcaactgta     420
ttcaagtttg tggagcctcc agagaacaac gagagactat ttgatcagaa gagcagggta     480
aagttcaaga tcatgagctt gaaagtgaaa ggagaaatgg aatatgttcc tgagatgttt     540
gatgagcaga aggatcaagt agcagttgag caaattttac gagttgtttt tcttgatcca     600
gaatgcccta gccctgcact ggaaaagctt aaaagacaac tggctgaggc tgaagctgag     660
ctcgaagcac gcaagaagcc tccagaagat gcaggccccc gagttgttgg cgaaggactt     720
gttatagatg agtggaaaga gcggagggaa agatacctgg cacgccagca ggttgaagtt     780
gattctgtgt gaagatcctg ttttccttgt aagcacttgt tttttcttga gctgaggttt     840
ccttcatgca atgttgaatg ctttgcctct cggtccgttg gtggaggcgg atgtgtgata     900
agtccggtcc agttaggggt gcagacataa gatttggaaa gcaggattta tcagcaaagc     960
ctgtaaaaaa aaggtaaaat caaggcattt gtcatgtcat attaagctgt aaggcactta    1020
attatgtact tgtgccctat ttattgtaga tatgggattt tgattaacga tgaattagtt    1080
aaatttgtgt tgtgaaaaaa aaaaaaaaaa aaaaaa                              1116
```

<210> 41
<211> 868
<212> DNA
<213> Artificial

<220>
<223> Construct sequence

<220>
<221> misc_feature
<222> (586)..(586)
<223> n is a, c, g, or t

<400> 41

gggccttata gacaaagatg tggtgaggac tgatagagca ctgtctttct atgatgggga 60
tgataatcca aatgtgaata tcttacgtga tattctgttg acgtactcct tctataactt 120
tgatcttggt tactgtcagg gtatgagtga tttgctatcc ccaattttgt ttgtgatgga 180
ggatgaatca gaatcgtttt ggtgttttgt ggcactgatg gaacgtcttg gacccaattt 240
taatcgtgat caaaatggca tgcactctca gctttttgca ttatccaagc tggtggagtt 300
gcttgattgc ccattacata actatttcaa gcagaatgac tgcttgaatt acttcttttg 360

tttccgctgg gttctaatac aatttaaaag ggaatttgaa tacaagaaaa caatgcgatt 420
gtgggaagtg ttgtggaccc attatttgtc tgagcatctg caactgtatg tatgtgttgc 480
aatcttgaag cgataccgca acaaaataat gggggagcgc atggactttg acacactctt 540
gaaatttatc aacgagctga gtggccatat tgaccttgac gcaatnctta gagatgcaga 600
ggctctgtgc atatgtgctg gtgagaatgg tgctgctcgc atcccaccag gaactccacc 660
ttcattgcct actgagaatg agaatgcttt attatatgct caagatgatg aagtactgta 720
atacagttcg tctcattggt acgatataat tggctttata ttttgttttc ttcatgaaat 780
agctgtgtat aattgctgtt ttttcgaaat aaaatgttaa taatcaagca atcaactcca 840
tttgctgacc tccttgaaaa aaaaaaaa 868

<210> 42
<211> 752
<212> DNA
<213> Artificial

<220>
<223> Construct sequence

<400> 42

```
gttcaataag ataagatcat caccacctcc aaaattcaag ttcttacagg atgcggatga      60
gaaactacat agaaggaaat tgatgcaaga agctggggag aaggtccaaa ggcatgatgt     120
ttttgctcag gatcatacca aaattcctgc tagttcaaat tcccacaaag acgaggatga     180
tgggcctttt atcaccatca ttattgacag gaacaaagaa agagagctta acgaacaaaa     240
tcatcaacta ccagactatc agtcaagcac ttcacaggta cttcctcttg ctactgcacc     300
ttcaacatca aaatcagcag ccaagaaaag ttccttcttt cattagatct ccgtgatcga     360
gtaaaacgag atagtttttt tttttttttt tcctatgtat ttctattaac caactttagt     420
cttcttttta ggagttcttt ttctttaatc tttctatggg atggtgttag aaggttaacc     480
ttgccaaacc cttttgcaat gtctgaaaaa tcttcgggtg ggtggggagt tggacacact     540
ctctgcagta taatgttaaa agttggacaa gtgcaggtgg gggtcttgag ggagtaatct     600
gttgtattca ctcttcatat atctctgagc tgtctgatat ctgtaatcat tctggtgagt     660
ggtggtacta ctgatgtttt tgtccaaaag gcactgtaag aattctttca gaccatgcac     720
ttctttattt atttttttcta aaaaaaaaaa aa                                  752
```

<210> 43
<211> 939
<212> DNA
<213> Artificial

<220>
<223> Construct sequence

<400> 43

```
tcgtacgccc acgcgtccgc acctaggcgc gcaccaaaga gggttaaatc aacactgaag      60
atcgataagt tggacgatat tcgcaatgaa tggattgatt ctgatattct aatatttaat     120
tcagggcatt ggtggacacc gagtaaactc tttgaaatgg gctgttattt tctggtaggc     180
agatcgctga agctaggaat gcctatcact gctgcctttg aaagggcact gcatacatgg     240
gcatcatggc ttaacactac cataaatgca aatagaacaa gcgtgttctt ccgcactttt     300
gaatcttccc attggagtgg ccggaaccgc ctttcttgca aagtgactcc gcagccttca     360
tcaagaactg gagggaggga tcgtagccca atatctgaca ccataatcaa ggttgtgaag     420
gcaatggccg ttcccgtaac agttttgcat gtgacaccca tgggtgcatt ccggagtgat     480
gctcatgttg gcacttggag tgacaatcca tctgtccctg attgcagcca ctggtgctta     540
cctggtgtac ctgatatgtg gaacgagatt ctcttgtcaa atatgttgtc caggaattaa     600
gaggccattc tttccataag ctagaaaatg caactgtgta gtgtgttcca tattcttccc     660
tcttcaattg accccattca atttccaaac cttctactca actactttca gtacatacga     720
agatcttctc tgctgagaag ttgtgtgcat ttgtcacaaa tattgtttgt tgtgtagttc     780
agggatccaa aaattacttg atttgtctgt tctagttgca cttgagttca catgtaatgg     840
taatgatttc tctccagcgt tagctgaaat ctggaagctc ttctcttcag atgccaccga     900
gctgttgtac aataaaagcg ttattgcaaa aaaaaaaaa                            939
```

<210> 44
<211> 556
<212> DNA
<213> Artificial

<220>
<223> Construct sequence

<400> 44

```
ggggaagttt tagaagctgt cataatgaag gatcgggcca cgggtcgtgc tcgaggattt      60
ggttttgttg ttttcgcaga ccctgctgtt gctgagagag ttgtgatgga aaaacacctt     120
atagatggta gaaatgttga ggcaaagaaa gcagttccta gagaggatca gaacaccctg     180
aacaaaaaca gtagcagcgt taatggttca cctggccctg cccgaacaaa gaagatattt     240
gtaggaggtt tagcatctac agttacagag agtgacttta ggaagtactt tgatcagttt     300
ggagtgatta cagatgtggt agttatgtat gatcacaaca ctcaaaggcc aagaggtttt     360
ggattcatca cctatgattc agaggaagca gtggatagag tattgcacaa aacctttcat     420
gaactcaacg gtaaaatggt tgaggtcaag cgggctgtcc ccaaagagtt atctccaggg     480
ccaactagga accagttagg ggggtttaat tatagtccta gtagagtcag tagcttcctc     540
aatggttaca ctcagg                                                     556
```

<210> 45
<211> 580
<212> DNA
<213> Artificial

<220>
<223> Construct sequence

<400> 45

```
tggtgctgga gattttgata ttggtctagg aggggtaggg tatgggagga acaataggac      60
agctgttgca ccagcatcat ctcatgctgc atctaatggt ggttatgatg gggcttatgc     120
agacttttat gagaagggct cattatacgg ggataatacc tggcaatctt caccttcgga     180
actggatgtg tctggctcat ttggttttgg gcttggaaat gcaacttctg atgttatgac     240
taaaaattct gctggttatg ttggtggtta tagtgttgct aatagacaat caaatagagg     300
aattgctgca taggatgatt atgatatagg tgacggtgaa gtgaatctcg tacatgtttc     360
ttgttcagat gatcaagttt caactttgct catcatatag agcagtcaca aggaaatctg     420
tgtatggttt gagcttttga gatttttcta atataggttt tctttcttgg aattttatta     480
tgtaaaatca cattgtgggg tatactcaac atgattgtat gatgcatctt tggtgacagc     540
tatattaaaa aaggaaacaa ttttttagca aaaaaaaaaa                           580
```

<210> 46
<211> 1441
<212> DNA
<213> Artificial

<220>
<223> Construct sequence

<220>
<221> misc_feature

<222> (967)..(967)
<223> n is a, c, g, or t

<400> 46

```
ctctttactt gtgcacgctt aaagaagaga tcacacaatg gcaaaccctt ctctctgtct     60
cctcttcctc ctctctctcc tgaccccagc cctcgtctcc tcttccccgg ttcaggaccc    120
cgaacttgta gttcaagaag ttcatagggc catcaatgcc tctaggagga agttgggata    180
tctctcttgt ggaaccggca accccattga tgactgctgg agatgcgatc ccaattggga    240
gaagaaccgc cagaggctag ctgattgtgc gattgggttt ggtaagaatg ccattggtgg    300
aagaaatggt aagatttatg tggttacaga atctggtagt gatgatcccg tgaaccctaa    360
gccagggact ctcaggcatg ctgtcattca agaagagcca ttgtggatca tttttgctcg    420
tgacatgaca attcaattga aggaagaact gatcatgaac tcgttcaaga ctatcgatgg    480
tagaggtgcg agtgtccata ttgctggtgg tccatgcatt actatacagt atgttaccaa    540
cattattata catggactaa acatacacga ttgcaagaga ggagggaatg ctatggtgag    600
ggactcccca aaccactttg gatggaggac tgtatcagat ggtgatggtg tgccatcttt    660
ggtggtctca catctgggtg gaccataatt cattgtcaaa ttgtaacgat ggactcgttg    720
atgccatcca tgggcctcag ccatcaccat ttcgaacaat tacatgaccc accatgataa    780
agtcatgctt ctggggcata gtgattccta tactcaagac aagaacatgc aagtcaccat    840
agccttcaat cactttggag agggtcttgt ccagagaatg ccaagatgca gacatggata    900
tttccatgtg gtcaacaatg actacaccca ttgggaaatg tacgccattg gagggagtgc    960
tagcccnacc atcaacagcc aaggcaacag atttgtagca cctgacatca ggttcagtaa   1020
agaggtaaca aaacatgaag atgcaccaga gagtgaatgg aagaattgga attggaggtc   1080
tgaaggagat ctactgttga atggagcgtt tttcgtagca tctggcgcag gcgcttcatc   1140
aagctatgct agggcatcaa gcttgggtgc aagaccatct tcacttgtcg gtccaatcac   1200
gatgggggca ggtgcactta actgcaagaa gggggtcgt tgctagctga ttgtgaaaga    1260
ttacacaata ttttgtgact agtaatttgg actataatta attagctggc tagaaaagta   1320
attaagcaga agtggaatta aaaaatgagg agagtgaaga aaagtccgag cactagtttc   1380
tcctggcttt ttcttccctc caaattttta ttctttttac ccgaaaaaaa aaaaaaaaaa   1440
a                                                                   1441
```

<210> 47
<211> 650
<212> DNA
<213> Artificial

<220>
<223> Construct sequence

<400> 47

```
cgagaccagt ggccttttct atccaagaat caagcatgga taaaacagaa caagatcttg     60
attcttgttc tgttgtcaac accaatgtat catcaccaga gttcttcaac gccttcgagt    120
ttatttcctc gatgtcatcc ggctttgatc tgtctggtct ttttgagact aagaggaaat    180
caagctcaat gttcacttca aaattttcag cgagtgcgat catggaaaaa attgaagggg    240
ttgcaagggg gctgagttat aaggtggcaa aagtcaaaga tttcaaagtg acgttacaag    300
gtccatgtga ggggagaaag gggaagctgg cggtgacggc ggaggtgttc gaggtggcac    360
cggaggttgc ggtggtggag ttctccaagt cttccggaga taccttggag tatactaggt    420
tctgcgagga agatgttagg cctgcactaa aagacatcgt ttggacatgg caaggagaca    480
atgcttgtaa tcaagataac aacaatagtc atgtagaaga tcctgagatt caaatgttgt    540
agaaaggctt tgatcttaat ttggtgagtt ttaggatttt atataatctg taaataagtt    600
tttgttattt aattaatgca tcaagttgag tgttcttggg aaaaaaaaaa               650
```

<210> 48
<211> 957
<212> DNA
<213> Artificial

<220>
<223> Construct sequence

<220>
<221> misc_feature
<222> (214)..(214)
<223> n is a, c, g, or t

<400> 48

```
cccacgcgtc cggtattttc tgtggaggag aaacaaaaag caatctcttg ggtttttgtt      60
cttttaaaga tttggtttgg gttctctctt tgaaagttat caagaacagt aaaggggctc     120
ttgattgact gatgcaagtt taagattttc tattcttttt agagaaagaa agaagttttc     180
ttttgaaatg aatttgatgt gtgaggtttt cttngctgaa gaagaagaga aggttgaagg     240
agagtgggaa aaggagagat agtatttacc aaagccaagg tagctagcag aagtagagag     300
gcagatagca ataaactaca aaataaaatt tcatctaagg ggttttagtt tgttctgctt     360
ctgctgggta attggcaatg tctgctacta taatcagcga ccctatggtg gtctctgcac     420
cggagacaca agcaacagca gcagccgcag ctgcggcaac gacagcaaca caactcattg     480
cacagataga ggttgagtcc gtcagatgtg attgctgtgg cctaatcgaa gagtgcacgc     540
cagaatacat tgaaagagta cgcgaaagat atcatggaaa atggatttgt gggttatgca     600
cggaggctat aaaatatgaa attgtaagga ctgagaggct tattagcact gaagaagcaa     660
tgacaaagca catgaacgtc tgcaagaagt ttgtctcctc agggccacct cctgatccaa     720
caactcattt gatagccgcc atgaggcaaa tcctgagaag aagcttggat tctccgagag     780
gttcaaggtc aaccccaagt agtcctaaca aaacgaacgg agcaatccgt gtcgctgcac     840
ttgctaggtc cgaaagttgc ttccctgctt tgtctggttg atagataatt ctgagcaccg     900
tggaagacgg ggtgattcat aaaatggtga tgaagctatt ggaagcaaaa aaaaaaa        957
```

<210> 49
<211> 1020
<212> DNA
<213> Artificial

<220>
<223> Construct sequence

<400> 49

```
gtcaagataa agtcccattc tttttcctag ccccaagttt gtttgagggg tgataaaaaa      60
tgaaccaaga gatgaatggt gttgacactg agattgatca gaaccaccaa gagaatgtgc     120
aagagaaaat cgattatgtg tttaaggtgg tggtgatcgg tgactctgca gtgggcaaga     180
cgcaaattct ttccaggttt accaagaatg aattctgctt tgattcaaag tctaccatcg     240
gtgtcgagtt cccagactag gactgtcatc attaaagaca aggtcatcaa ggctcagatc     300
tgggatactg ctggccaaga aaggtaccgg gcagtgacaa gcgcatacta tagaggggca     360
```

```
ctaggggcca tgttagtcta cgacattacc aagagaccaa cgtttgatca tgtggctagg    420
tgggtggagg agctccgagc ccatgctgac agctcaattg tgatcatgct gatcggaaac    480
aaggctgatc ttgtggacct cagggcagtt ccaacagaag acgcggtgga atttgcagag    540
gatcaaggcc tctttttttc tgagacatca gcccttagtg gtgacaatgt ggatggtgca    600
tttttcaggc tgctagaaga aatttacggt gtgatttgta agaagtcatt ggaatgtggc    660
aatggaaaac cccatgctgc tgatgccata acgcttagag gttctaagat tgatggcata    720
tcagggacgg atctggggat tagtgagatg aagaaattat ctgcttgctc gtgttgattt    780
gatcatttct cttgtgaatt gtgtactata agacttcacc actcccatgt tcttaattga    840
ttctgtggct ttctttggaa agtggtgatc ggtcgtgtgg tgagggtggc aagttttttc    900
ttttctgtga cctgtcaaga ttttagcagt attgtacttg tcttacagaa cccatgaatt    960
tggttttttt atatgtattg atttggatgg atggttttcc ttttcctctg aaaaaaaaaa   1020
```

<210> 50
<211> 1160
<212> DNA
<213> Artificial

<220>
<223> Construct sequence

<220>
<221> misc_feature
<222> (449)..(449)
<223> n is a, c, g, or t

<220>
<221> misc_feature
<222> (557)..(557)
<223> n is a, c, g, or t

<220>
<221> misc_feature
<222> (585)..(585)
<223> n is a, c, g, or t

<400> 50

```
atgaactaga aattcaaagc ttccaacaca tattttaatg gcttacaatc cttcttcaag     60
gtcttgttct tatgattctt tttgtttcct attcattaca ttcgttcttc catatttctt    120
cctcacaaaa gctcaagctg caagccatgg tagaacctca tgtggtacca ttccaataaa    180
ctacccttttt ggcatcgatg atggctgtgg cagtccatat tacaggcaca tgcttctatg   240
ctccgattcg ggcattctcg agcttcgaac gccttccggg agataccatg ttcgtagcat    300
aagctactca gaccctcaca tgatagtcac cgatccattc atgtggaagt gtcaagatgg    360
tcatcacttt cgtgcaacta gggcatttag ccttgatgca agcacacatt taacactctc    420
ctctcaaaat gactacctct tcttcaatng tagtgaagag aaagtgattg ttgagccaaa    480
acctatcttc tgcgagaggt ttcctgatcg gtgcgactcg acatgtgata gtgctagtta    540
cctttgcagg cacttgncca ggatgtggtg ctgcattagg aggangttct tgctgctctt    600
acttcccaaa agcgaccgaa tctttgaggc tgatgctgaa gtattgtgct agttacacta    660
gtatttattg gagaattaat ggtgcaaatg ctcctgatga tcatgtacct gagtatggta    720
ttagagttga ttttgacatt ccagtgacta cagattgcct tcaatgtcaa gacatgaaga    780
aaggaggtgg aagatgtgga tttgacacaa aatcacagaa tttcttatgt ctgtgcaatc    840
agagatcaaa tgtcacaaca tattgcaatg gtatatcaca gcagcagtag ccgtagcaag    900
gcaggaataa ttgcagggac tgtaactgga gtttcagctg ctggggcctt aggaattggt    960
gctggtctat ggtattggaa gaaagtgaga gcctcagcac cagtaacgtg tggggttcaa   1020
agcaatgaga ataggctctt ttaagaacaa atcaacaaaa gtgaatacta atcactttct   1080
cttttctaat ttccctattt tttgagttgc ctttcgcact ttaataagca atttaatgga   1140
tctgttttct ttgttttaaa                                                1160
```

<210> 51
<211> 896
<212> DNA
<213> Artificial

<220>
<223> Construct sequence

<400> 51

```
cattatccca aaaaacgagc aaggcagata cagcagccaa aaccatgtca cccggtcccg      60
tcgttgaggc tgaacccgcc gccgccgtgg aagatacaca gaagaagaag cctcctcagc     120
aggacgtgga tgaacctgtg gtggaggacg tgaaagaaga tgagaaggaa gaggacgacg     180
acgatgatga tgaagacgac gatgatgaag atgatgacaa ggatgatgat accccaggtg     240
ctaatgggag ttccaagcag agcagaagtg aaaagaagag tcgcaaggca atgttgaagc     300
ttggcatgaa acctgttact ggtgttagca gagtcaccat caagagaacc aaaaatatac     360
tgtttttat ctcaaagcct gatgtcttca agagccaaaa ttctgagacc tatatcatat      420
ttggagaggc aaagatagag gatttgagct ctcagctgca gacacaggct gctcagcagt     480
ttagggtgcc agacatgtca tctatgctac caaaatcaga tgcttctact gcagctgctg     540
ctgcaccagc agatgaagaa gaggaagaag tcgatgagac tggggttgag cctagggaca     600
ttgatcttgt tatgacacag gctggagttt ctaggagcaa ggctgtcaag gctctccaga     660
cgaacaatgg ggacattgtc agtgctatca tggagcttac tacataggtt ggctccctgg     720
ttactctcct attttctgct cacaagttct tggaacaatt taatcatggt agtcacattg     780
gcttgccatc tatgaggtcg ctaattatcc attgtttgtg tcaaatttga gattattacc     840
tattgcggtt tttctttagt agcaagctct tattgtgctc tttgcaaaaa aaaaaa         896
```

<210> 52
<211> 415
<212> DNA
<213> Artificial

<220>
<223> Construct sequence

<400> 52

```
attgaatagc caaatatctc tctctctgga ctagaatgtg aaggagcagt tagcgaagga      60
aagattggag aatgccggag ctggtgtacc aagagcagag ctcgtcgaga tcaggattta     120
gagctcgaga tgcaagtcct gactctgtaa ttttcactct ggagtccaac ttcagtctct     180
tctcttctgc ttctgctagc gtcgatcgct gctcttttgc ttccgatgct catgatcatg     240
actctctcgc ctctgaaatc tctctggtaa aaccatttct ctaacttccc tgcatgcatg     300
cgttttctaa cccatggttt ttgagtgagt tttttgagag tgcaatgcag catttggcag     360
ctggtcatga tcaacaagag aactcttcga gtggtccaga tcgaaacaac aacaa          415
```

<210> 53
<211> 566
<212> DNA
<213> Artificial

<220>
<223> Construct sequence

<400> 53

```
tccttaactt gtccgagaga ggagcaattt ttaacaccgg gcttgcctca catttttcat      60
tggaaaatgc atgcctgaca attccattgg ggcattttat cagaatgttt aaaatattta     120
tattttgcgt cggctctata gcatatgctt ataagcattg aaatatgatt caactgaagt     180
attcgcacat gttttgctgt ttacgtttta tatgattgtt gtgtttagtt ctccacaagc     240
agcctggcag ttattcgatc cggtatcttg acgttatctt atcacatgct gtaaatccta     300
tggctcaaac tggcaattat tcaaggcagt tcttaaagag ttcaaggttt tctttcctgt     360
tgatcaacct ttgtaaagct tatgaagcaa ttgtgcaaat aattggtgct cgacagaagg     420
caatctctta gttcgctcac gacatttcta gccgattttc gggaaaacat tagtggactg     480
gagtcattga ttcaatcaag gtgaaattgg aaatcattta gaaaagcaat tcctgttctt     540
tgacaacact aggttcttct ttctcg                                          566
```

<210> 54
<211> 581
<212> DNA
<213> Artificial

<220>
<223> Construct sequence

<400> 54

```
tagagttcga gaggtcatct tcttgttata aaatcctcaa aagcttgagg tgaaaaaata      60
agcaagggag aggagagagc tagctagcta gtcaagttaa gagtcagaaa acaagaatgt     120
cttccaggaa gaaaaagaag gcagctcttt atgagaagtt acgtgctgct accaattcta     180
atgctatgaa caaaacttca atcatagtag acgcgtcaaa atatattgga gagttgaaga     240
acaaggtgga taggctaaaa aaagaaatcg gaacatcttc aaccccccaa aattcattac     300
ctgcgcaggt cacagtggaa aacctagaaa agggtttcct tattaatgta ttttcaggaa     360
agaattgccc tggattactt gtctccatac ttgaagcctt cgaggaacta ggccttgatg     420
tgctcgatgc tagggtttct tgtgaagaca atttccaact tgaagcgatt ggtggagacc     480
aaaaccaagg ccatgatgct caagtagtga aacaagcagt gctgcaagct atccataact     540
ggaatgaagg cagctagcta gtgagcaaga atgacccaaa t                         581
```

<210> 55
<211> 598
<212> DNA

<213> Artificial

<220>
<223> Construct sequence

<400> 55

```
gcttagtggt ctgggtttag tgagaaatgt gattggacac tgtttgagtg ggcgcggtgg     60
aggattcctc ttcttcgggg atgatcttta tgattcatct cgtgtagctt ggacaccaat    120
gtcacccaat tcgaaacact attcacctgg attcgccgaa ttgacttttg atgggaaaac    180
tactgggttc aaaatcctga tcgtagcttt tgacagcggg gcctcttata cttaccttaa    240
ttctcaggcg tatcaaggtc taatttctct gatgaagaga gaaatacccg cgaagcattt    300
gacagaagcg ctggatgatc agacgcttcc gatttgctgg aaaggacgga aacctttcaa    360
aagcgtacgt gatgtcaaga aatacttcaa gacctttgca ttgagcttta agaatggcgg    420
aaaatctaaa actcaacttg aattcccacc agaagcttat cttatagtat catccaacgg    480
aaacgcttgc ttgggaattc taaatggtac agaagtaggt ctgaatgatc tgaatgtcat    540
tggagacata tcaatgcaag acagagtggt gatttatgac aatgagaagc aactgatt      598
```

<210> 56
<211> 273
<212> DNA
<213> Artificial

<220>
<223> Construct sequence

<400> 56

```
gaaggtggtg gttcaattat acacatagtt gaccacatgg atttggagcc ttggagtgtg     60
cctgaagtac tacggccgct gtatgaatcc tcaactgtac ttgctcaaaa gacaacaatg    120
gtggctctac gccagctgcg gcagatagct caggaagctt ctcagtctaa tgtgaccaac    180
tggggcagac gacctgcagc tctacgagca ctgagccaga ggttgagcag gggttttaat    240

gaggctctca atggatttag tgatgaggga tgg                                 273
```

<210> 57
<211> 274
<212> DNA
<213> Artificial

<220>
<223> Construct sequence

<400> 57

cgctgacacc accactttcc tccttgacag cccctccttc ctcaacttcc ttcaacatct 60

caacaccaat agccattgcg attgtgaaga tgataataca aacgccacta ttgactctat 120

catccccacc ataaaaatca cttcttgcat gctagaaatg gatccaatgc ttgtgtgtgc 180

agtgtgtaaa gatcagttct tgattgatgt tgaggccaag cagctgccct gcagtcacct 240

gtaccatcca ggttgcattc tcccctggct ttct 274

<210> 58
<211> 259
<212> DNA
<213> Artificial

<220>
<223> Construct sequence

<400> 58

aacgtgattt caaagggtcg caaaggaact caaatagagc cccaagatcc cacagaacta 60

gagttattga cagaggttcg aacgagtttc caaaagaagg ggaggaggct taaagacgtt 120

ttgaggagtg gaaactgcat actgaggaag ttccaaaaac atcgagagga cgattctaat 180

caagttcttt acttcttctc tcaagtggat atgaagttag tagcaagggt tctgagcatg 240

tccagagtaa caacagagc 259

<210> 59
<211> 1464
<212> DNA
<213> Populus trichocarpa

<400> 59

caccctgaag ccccatttcg gtttttagtc gaaatcttgg gttgggggct ggaaatatga 60

ggacatgctt gtgggttttt gccgtggttc ttgttttcgg ttttgttgat ggaaaagaaa 120

ttgaaaggtt gcgcacggag aggatttcag gaagcgctgg ggatgtgttg gatgatgatc 180

cagtgggaag gttgaaggtc tatgtttatg agcttccgag taaatacaac aagaaactgc 240

tgcagaagga ccccagatgt ctcacccata tgtttgctgc tgaaatcttc atgcatagat 300

ttctcttatc cagcccagtt cgaaccctta atccagatga agcagattgg ttttattccc 360

ctatataccc cacttgtgat ctcacaccca tgggcttgcc gttgcccttc aaatctccta 420

ggatgatgag aagtgcgata cagctaatct cctccaattg gccttactgg aatcgtacgg 480

```
aaggggctga tcactttttt gttgtgcccc atgactttgg agcctgcttc cactatcagg     540
aagagaaagc cattgagcgg ggcattctcc cgttactcca gcgttctact ttggttcaaa     600
cttttgggca acgaaatcat gtgtgcttga atgaggggtc aattacaatt cctccttttg     660
ctcctcctca aaagatgcag gcccaccaga ttccccctga cattccacgg tccatttttg     720
tctatttccg tggattgttt tatgatgtaa ataatgatcc agaaggtggt tattatgcaa     780
gaggagcaag ggctgcagtt tgggagaatt tcaagaacaa tccactcttc gacatctcca     840
ctgaccatcc aacaacatat tatgaagaca tgcagcgagc tatattttgc ttgtgcccgc     900
tcggttgggc cccgtggagt cctagattag ttgaagcagt ggtatttgga tgcattcctg     960
tcatcatagc agatgatatt gttttgccat ttgccgatgc tatcccatgg gaggaaattg    1020
gggtgtttgt agcagaggaa gatgtcccta acctggacac aatcctaaca tctataccac    1080
cagaagtgat tttaaggaaa caaaggcttc ttgcaaatcc ttctatgaaa cgtgcaatgt    1140
tattcccaca acctgcacaa ccaggtgatg ctttccacca aatactaaac gggctggctc    1200
gtaagttgcc gcacgacagg agtgtttact tgaagtctgg tcaaaatatt ttgaattgga    1260
cagcaggacc agttggggac ctgaaacctt ggtaagagaa gtgtcatttc ctcaacagac    1320
atggctgtgg tgtatccact gtttcatcta atcgcaagga ctgtaaactt ctctttgaag    1380
ttttgatttt tgtatagatt cttgaatttc acatgtacat ctgggacata gttcgatgct    1440
ttaaataact tcgcattttt cttg                                           1464
```

<210> 60
<211> 1495
<212> DNA
<213> Populus trichocarpa

<400> 60

attgcaatgg ctgagaagca ctcctcatca tcccttgggg ttgtttctag gaaatctatg 60
ttttgcttgt tttctttcgt gtctgccttg tttatactgt catggttttt tatgttgcgt 120
tccacggtca gtgccggatt tattgacctt agtttgttac ctcatcctaa acttaattct 180
aaagatgatt taggagatgt gaaatgtaac agttttgata ataattgcaa tcaagttctt 240
aaggttttca tgtatgattt gccttctgag tttcattttg gactcttgga ttggaaacct 300
caagggggta gtgtttggcc tgatcttaga gctaaagtgc ctgcatatcc tggtgggctg 360
aacttgcaac atagtattga atattggctt acaatggatc ttcttgcttc agagattcct 420
ggcataccta gagctggtag tgcagtaaga gttcaaaatt ctagtgaagc tgatgttata 480
tttgtgccat tcttttcttc tataagctat aaccgatatt ccaaggtgaa tccacaccaa 540
aagaagagca agaacaaatc attggaagaa aagttggtca agtttgtgac ttcgcaaaag 600
gaatggaaga ggtctggggg gcgagaccat atcattttgg ctcaccatcc aaatagcatg 660
ttatatgcaa ggatgaagct atggacggca atgttcatac ttgcagactt tgggaggtat 720
tctccaaata tagcaaatgt tggcaaagat gtgattgcac catacaaaca tgtaattaaa 780
agctatgcca acgactcctc taactttgac agccgcccga ctttgctgta ttttcaagga 840
gccatataca ggaaagatgg tggctttgct cggcaagaat tattttatgc tctaaaagat 900
gaaaaagacg tgcattttca gttcggtagt gtacaaaagg atggagtgag caaagcttct 960
caaggaatgc actcctccaa attctgcctt aacatagcag gtgacacgcc ctcatcaaac 1020
cgcctctttg atgccattgc cagccactgc gttccggtca tcatcagtga tgatatcgag 1080
ctcccttatg aggatgtcct tgactactct caattctgca tttttgtccg cacttcagat 1140
gctgtcagag aaaagttcct cataaacctt gtcaggagta ttaagaagga tgaatggact 1200
agaatgtggc agaggttgaa agaggtagaa aacttctttg agttccaata tccatcaaag 1260
gaaggcgatg cagttcaaat gatatggcag gctgtagcaa gaaaagtccc tgcaattagg 1320
ttgaaggtaa ataagtttag gcgatttttct cgttttggga cccatgaaga tggagtgtta 1380
agaaaaatac catcacctag taatttttgg tgaaacattc agtgtttctt tatactttga 1440
ggttctgttt atatataaag gtgagaaatt caatttattc tcactacaga ttttg 1495

<210> 61
<211> 2487
<212> DNA
<213> Populus trichocarpa

<400> 61

```
atggctacct attacccaac ttcaagaagt cagaataaca atttgcacgc tttgcttaca     60
ggggaccaga aacttgcttc ttattctgat ctgccttccg atcttagtag catgaaaagt    120
tatacgaacc acactccagc tgctgggtca tactcggaca tattgtatgg gggttccttg    180
tcttctcaaa atggtgctga attctcatct agtggagcta gaaatgagat agtattcatt    240
ccacctacaa gtgacacgat gaatttacaa tctgttggtg ggcagttgaa tacagcagca    300
ggtaacctgg ttggtgactc tgttagtgga gattcccagg ctgttccgcc aaggatgcac    360
cttggcattc cagactgtga gcagaatttc cagtctcaag gattaccact gaggcttggc    420
atgcaggtgc aatctgcagt ttctatgcct tcacttcagt atcagtatct aaaccaaaat    480
ttcccttcat ctctgagttc acatctgctt gtgccagaga agtggacttt accctgtgaa    540
ggtgatgaaa gcaatcaaag caaggagttg agagaatttg aaggattgcc tggatttgcc    600
ggaagcagcc ataaccctat caaaacagag tcttcacaca atcctcagta catagttggc    660
ctcagagata tgcatgctga aatgaatatg tatggattgt ctggttatgc taacacactc    720
ttgaactcca gatacctcaa gtcagtgcaa cacttgctcg atgaggtggt taatgtaaaa    780
aaggctttga agcagcctca atccaacaaa tgctccgatg acttcaagga gagtgatagg    840
aggccgagta gctgttctat gcttccctca tcaaatgtga aaccaccgga tcctgctgag    900
tctactgccg actccactcc tgagctatcg ccagtagaac ggcaggactt acttgacaaa    960
```

aagactaagc ttctgtccat gctggaagag gtagacagaa aatacaaaca gtattaccat 1020
caaatgcaaa ttgtggtgtt atattttgac acggtggctg gtcatggggc agctaaatca 1080
tatacagcac tcgccctcca aacaatttct cgtcactttc gctgtttgcg cgatgcaatc 1140
agtggccaaa tagaagttat catgaaaagg cttggggagc aaggtacttc accaaatggt 1200
caaggaggta taccacgtct ccgttatgta gatcatcaga ccagacaaca gagggctctc 1260
cagcagcttg gtgtgatgcg acatgcttgg aggcctcaaa gggggctccc tgagagctct 1320
gtttcagtcc ttcgagcttg gctctttgag catttccttc atccctaccc gagtgattca 1380
gagaaaatca tgttagcaag gcaggcaggc ttgaccagaa gccaggtcgc gaactggttt 1440
ataaatgcgc gggtgcgtct ttggaagccc atggtcgagg acatgtacaa agaagaattt 1500
ggtgattcag agacaaactc caaatcttct ttggacgaaa caaccaaagc ccatggagat 1560
aaatcaggca atcacttgac atctgagaat aggctacgtg agttgtatga gagtgtgaca 1620
tctacagctg ctgatatttc ccagccaggg caagcccatg acataaaatc tagtcatatt 1680
cttgaattag aaatgaaaga acctatggcg aaaacagtcc ttgagaatgg ttctcaggga 1740
cccaatgtgg cagaatctga tattatgaaa tttccacggg accgaaggtt gaatatagat 1800
gatgaccata acttctgtcc acatgggaat attccgtgtg gccaaaatgg tgatggaaat 1860
cttatgtctg ctgctgctac atatgatgta tcacacttga atggatttgc agtaggcagc 1920
cagatgtctc ttgcattggg attgcaaagt aacgacagtg attcatttcc cacgtttgat 1980
ggggcccata tgagaggtaa cactatatca gcttcctcgg tggggcataa tgaggtggat 2040
tatcactgta tggatacagg aaagcaacaa gacaggattg ccaattccca tcggttacat 2100
gattttgtgg tttaagagaa tccttctagc tttacgaggg agtttcatca atcctgccaa 2160
gagaaggatt gatggagttt tggataacat aaagcagaag aaaacaagct cagtgatatt 2220
ggtttgctat tcattcaagc cccgtgctct tctcttggtt tgaatagaag aaacccaaca 2280
cccttacagt cacagacata gatgtaacat aagggcatgg tgcctgcttg taatatacgt 2340
acagattaca gggattgtag atatatctgt gcatatacaa taagattttt gtgatctttt 2400
gtactcttaa ggaatttcta caagctctcc ctaattgtga cactgatgta tcatttcatt 2460
cctaattctg aaataaatgt gatttgc 2487

<210> 62
<211> 957
<212> DNA
<213> Populus trichocarpa

<400> 62

attcaattca attgtaccag cagcagcagc agtagtcaga gcttataact agctaattga 60
ttaaccatgg agaaagcaca tacaaaatct gctcttaaga agcttgttaa ggctagctca 120
cagtctgcac catggagcaa tgctgctaga ggaatggcaa aagatgatct caaggatccg 180
ctttatgaca aatccaaggt tgccccaaaa ccttttgcaa aagaaaacac taagccccaa 240
gaattcaaac tccacactgg acaaagagct ctcaaacgtg ccatgttcaa ctattctgtg 300
gcaaccaaga tatatatgaa tgagcaacag aagaggcaaa tagagaggat acaaaagatc 360
atagaagaag aagaggttcg tacgatgagg aaggagatgg ttccaagagc tcaattgatg 420
ccttactttg acagaccttt ctttccccaa agatcaagca ggccattgac agttcctaga 480
gagccaagtt tccacatggt gaacagcaag tgttggagct gcatccctga ggatgaactt 540
tactactact ttgaacatgc tcatccccat gatcatgcct ggaagcctgt taagtaaata 600
aattgccatg atttgtaggg gaaaaaggag agtggagtat tctctctgtg atgttaattt 660
cttcatgctg tccaagttag tcttgtataa tttggagtgt tgatagtgtt tactgtttag 720
atcatgtggt aggtcttgta gtgggtgtaa gtagtcgctt tttcatagcc gaaagtcctt 780
ttcaatgggg ggttttgctt accaataagg cggatattgt acttcgtttt gtactattaa 840
aaagcaagtc accctctttt ctgtctttta gcagtatggt cgtgcatgcc caattaagca 900
cttcatttta ttttgctgga aatgaattat caaagcagac tataattctt ttagcca 957

<210> 63
<211> 2017
<212> DNA
<213> Populus trichocarpa

<400> 63

```
acttgggttt ctcgcatttc tactcatcgc cagttcttct ttgggtgtga ttgccaaata     60
atgacatagc caaccaagag aaagaaaacg aggcacagct ctatttcggg cttcttgttt    120
cttggtttcg ctgtgttgtg tgctcggttt aaaacaaaat gcagcttcat atatcgccga    180
gtttgaggca tgttacagtt tttcctggaa aaggtgtcag agagtttatc aaagtgaggg    240
ttggagctag aagggtttct tatcgaatgc tcttctattc tctcttgttc ttcacgtttc    300
ttctccggtt tgtgttcgtt ttgtccacag ttgactccat tgatggagaa accaagtgct    360
ctacactagg ctgcttgggg aaaagactag ggccaaggat attggggaga aggcttgact    420
cggctgtacc agaggttatg tttcaagtgc tagaacaacc acttggcaat gatgaattaa    480
agggaaggag tgatattcct caaacattag aggaatttat ggacgaagtc aagaacacaa    540
gattagacgc gaaaactttt gctctcaagc tcagagaaat ggtcactcta ctcgaacaaa    600
ggactagaaa tgccaaaatc caagaatatc tgtacaggca tgtagcatca agtagcatac    660
cgaagcagct tcattgcctt gccttgaggc tagccagtga gcactccact aatgcagctg    720
ctcgacttca gctccccttg ccggaactcg tccctgcgct tgttgacaat acttattttc    780
actttgtcct ggcttcagac aatgtgcttg ctgctgctgt tgttgccaat tcacttgtcc    840
aaaatgcgtt gcgcccccag aagtttgtgc tgcacataat aacagatagg aaaacttatt    900
cacctatgca agcatggttc tcactgcacc ctttggctcc ggcaataatt gaggtcaagg    960
```

```
cattgcatca tttcgattgg ttcgcaaagg ggaaagtgcc agtcatggaa gcaatggaaa   1020
aggatcagag ggtgaggtca cagtttagag ggggttcatc tgcaattgta gcaaataaca   1080
ccgagaagcc tcatattatc gcagcaaaac tacaaacact tagtcccaaa tacaattcag   1140
tgatgaatca catccgaatt catctacctg agttgttccc aagcctaaac aaggttgtgt   1200
tcctagacga tgacatagtg gtgcagtccg atctttcgcc tctctgggac attgacatga   1260
atggaaaggt taacggggca gtagaaacct gtcgaggaga agacaagttt gtcatgtcaa   1320
agaagttgaa gagctatttg aacttctctc atcccttgat atcagaaaat ttcaagccga   1380
acgaatgtgc ctgggcttat ggcatgaaca tatttgattt agaggcttgg aggaaaacca   1440
acataagcac aacataccat cactgggttg aagagaactt gaaatcagac ctgagcttgt   1500
ggcagctagg aacactacct ccaggtctga tagcattcca tggccacgtc cacgttatcg   1560
atcccttttg gcacatgttg ggtctaggct accaagaaaa tacaagtttg gcagatgctg   1620
agactgccgg cgtcatccat ttcaatggtc gagcaaagcc ttggctagat atagcattcc   1680
ctcagctccg ccccctgtgg gctaagtaca tcaacttttc tgacaagttc ataaagggct   1740
gtcatattag gccatcttaa cttgaatttt gagatgaaaa tgttgactgc gatcgtgatt   1800
aatatatgtc tatatataag gagaaaaaag cagagaggag agagttcttc acaaatccaa   1860
ggccttgaat ccagaatgta aaatattcat aaccaggcat ttgtctgctt ggttttttta   1920
gaattcatcc tctgtgggtg aacaaccctt ccttattact tcaaatcttg attcaatttt   1980
aatgagaaat taagcatgtt atggattttc aaccgtt                              2017
```

<210> 64
<211> 1496
<212> DNA
<213> Populus trichocarpa

<400> 64

```
aagctgatga tcaaatgagg acatcaaaaa tttcttcagg gaattcaggc tatcatgcat      60
ggattcttgc ttttgcaatg agtctactga tactgattgc cttgtcgaag tcatggtttt     120
atgatcacgc ttctgctact gcaagtgagg atttgcaata cttttcagtg attgtccctt     180
caaaagggcg ggcctatcct cctgttcttg cttattggat atgtggtact agtggagatg     240
ggaagagaat gttaaggcta ttaaaagcaa tttatcatcc aaggaaccaa tatctattgc     300
agcttgatgc tgaatcctca gattatgaaa gggcagagtt agttgtttca gttcaatctg     360
aaagtttgtt tcaagcatac ggtaatgtta atgttgttgg aaaaggttat gctatcaacg     420
aaatgggatc gtctgctctt gctgccatac tcaacgctgc tgcattgctt cttaagctga     480
gtgcagactg ggattggttc atcaatttaa gtgtctcaga ctatcctcta gtgagtcaag     540
atgatctcct ccatgccttc acttccttgc caagagatct caacttcatc aactatacta     600
atgacactgc gaaaaacgag atacacaaga ttaaccaaat tgttgtagac cccagcttac     660
atcttcagaa gagtagccac ctttattacg ctgttgagac tcgaacaaca cctgatgctt     720
tcaagatatt tggaggttca ccctggctga ttcttacaag agctttcatg gagtactgtg     780
tccaaggatg ggacaacctt ccaagaaaac tactaatgta cttcagcaac acggcatccc     840
cacttgaatc gtatttccac tctgtcctct gcaactctcc tgagtttcaa aacacaacag     900
taagcaatga tttaaggtac aatattctgg aaactactac agatggggaa tcaccttatg     960
acaaaatgct aaatggtgga gcagcatttg caaggccatt taaagaagat gctgctgcac    1020
taaacatgat agatgagaat gtcttgaacc gtgaacccaa tggattggtg cctggaaaat    1080
ggtgcttaga ccaaggtctg aacaagagct cagaggcatc aaagcctcca ggggaggatt    1140
tgtgttcaac ttggggtaac attaatgatg tcaagccagg atcttatggt atcaagcttg    1200
catttttatt gtctaagatt gccggtgaag agaaattgac aactagtcaa tgccttcaag    1260
ctacacaaat ggggtcatca tagaaccaag catacatcct tgtgtggata atttgtttct    1320
tttaatcgaa aactataggt taaatcaagc ttcttagcca tatgaatcct ttgctaaatg    1380
cactataaag gcgtacttgc ttgttagaac aaaagctaga agcacccagt gatatattgt    1440
acatacattc acattttatc gaggatttct acaataaact cattgttttt ccctta        1496
```

<210> 65
<211> 1703
<212> DNA
<213> Populus trichocarpa

<400> 65

```
gagagagaga atcttcgtat tgaaatgagg gaacctattg aaaccaacaa cggatcacct      60
ccaccactac ctccacaagc gttgctcgag aggcttaaag attatggcca ggaagacgct     120
tttgccctct gggacgagct ctctactgaa gagcgagaac tccttgtcaa ggacgtcgag     180
agcttagatc ttccaaggtt ggatcggata atccgatgtt cgcttcgatc tcaagggcta     240
ccggcggcgg caattgagcc ggttccggag aataccgtgt cgacggtaga agatagaacg     300
atagaagaaa gagagagatg gtggaagatg ggattgaaag caatctctga tggcaaattg     360
gctgttgtgc tattatctgg tggccagggg acaaggctag gaagttcaga tccaaaagga     420
tgtttcaata tagcgcttcc atctggcaaa tcactctttc aacttcaagc tgagcgaatt     480
ttgtgtgtcc aaagattagc agcccaagct tcaagtgaag gttctggaag ctcagtttcg     540
atacattggt acataatgac cagcccgttt actcatgatt ccacacgatt tttttttgaa     600
aatcacaagt acttcggcct tgaagcagat caggttacct tcttccagca aggcaccata     660
ccttgtgttt ccaaggatgg cagatttatc atggagactc catttagggt agctaaggct     720
ccggatggga atggaggagt atattcagcg ctgaaatatt caaaattatt agaggatatg     780
gcctcaagag ggatcaagca cgtggactgc tatggggttg acaacgcact ggtacgtgta     840
gctgatccag ctttttttggg atatttcatt gataaaggtg tagcagctgc agcaaaagtt     900

gttcgtaagg catacccccca agaaaaggtt ggtgtttttg taaggcaagg taaaggtgga     960
cctcttaccg tggttgaata cagtgagctg gatcagtcac tggcttctgc agttaatcaa    1020
caaactggac gccttcgttt ttgttggagt aatgtgtgct tgcacatgtt ctctttggat    1080
tttctaaacc aagtggcaaa tggccttgag aaagacagca tttaccatct agctgagaaa    1140
agaattcctt ctattcatgg tgatacgatg ggattaaaac tagagcaatt catattcgat    1200
gcattcccat atgctccttc aacagctctt tttgaggtac cacgtgaaga agaatttgca    1260
cctgtaaaaa atgccaatgg gtcaaatttt gacactcctg agagtgctcg gctgcttgtt    1320
cttcgactgc attctcgctg ggtggttgca gcaggtggct tcttaacaca ttcggtgcct    1380
ttatatgcaa ctggtgtgga ggtatcacca ctttgctctt atgctggcga aaatctagag    1440
gccatatgcc ggggaagaac atttcacgca ccttgtgaga ttacattcta gttaaatctg    1500
tatccaaata ttcttgattt gtacggatcc cattggaaag ggggttatcca tcccacgcat    1560
gtgttgtggt agattggcga gagataagag ttccaggttt tgttacatcc tgggtgtgat    1620
gtcaagaatg atacaatttg attaacgtat atcattcaag ttattgttga gcaatgataa    1680
tcctctcttc ttcttttttc caa                                             1703
```

<210> 66
<211> 1312

<212> DNA
<213> Populus trichocarpa

<400> 66

```
ctctctctct ctccctctcc ctctccctgc cattccctcc tttgctttct ctatccagaa     60
aacacacaac tctctttctc gtccctcacg ttctcttgca atggcttcca atggagtgaa    120
cgacaagtca ctgatcgtca gcttcgggga gatgctgata gacttcgtcc cgacggtctc    180
tggcgtctcc ctcgctgaag ctccggggtt tgtgaaggca ccgggtggtg ctccggcgaa    240
cgtggcgata gcggtggcga ggctgggagg aaaggcggca tttgttggaa aacttggtga    300
tgatgagttc ggcaacatgc tcgccggaat tttgaaggaa aacggcgtga tcgctactgg    360
gataaatttt gacacaggtg ctaggactgc tctcgcgttt gttactctac gcgctgatgg    420
agagcgtgag tttatgtttt atagaaatcc aagtgctgat atgttactaa ggccagaaga    480
gttaaatctt gagttaatta gatctgctaa ggttttccac tatggatcaa taagtttgat    540
tgtggagcca tgcagatcag cacatttaca ggcaatgagg gtggcgaagg atgcaggtgc    600
attgctttcg tatgacccaa atctgaggct gccattgtgg ccatcagcag aggaggcgcg    660
tgagcagata ttgagcatct gggacgaggc agatgtggtc aaagtcagtg ataatgagct    720
tgagttcctc actggaagtg acaaaattga tgatgaaact gctatgtcac tctggcgtcc    780
taactttaag ttgctcttgg tcactcttgg tgaaaagggt tgcaattatt acaccaagaa    840
tttccatgga tcagttgagg ctttccatgt ggacactgtt gataccacag gtgctggtga    900
ttcatttgtt ggtgctctcc tttgcaagat tgttgacgac cactctgtgc ttgaggatga    960
gccaaggctg agagagatac ttagatttgc aaatgcttgt ggagccatta caaccaccaa   1020
aaagggagca atccctgctc tgcctactcc ggccgatgcc ctcaaactgg tgaaagaagg   1080
aaaataaact atttttttgt taatccgcac tgcattttat ccctttcctt ttcagctcct   1140
ggttctcaat tcaatcgaag agctgttgca atctcaaatt ttcgcctttt tctcttgagt   1200
gtaatgatgg aaaagacttg cgctttaagg tcttgccaaa taaaattttt cttttaaaca   1260
aattgatctt gggcatcaag cagcaatcat caaagtgtta gctttctcca aa             1312
```

<210> 67
<211> 2353
<212> DNA
<213> Populus trichocarpa

<400> 67

gaaacactga aacaagcaga actcaacatc acacgctctc gggaatatta ttcactctcg 60
aaacaaccag aaactcctcc attgtagatt cagagagaga gggaggcaga cagacagaga 120
gtaggcgatg gcgggcacgt ggagagcacg tggagctgtg gtatccgcga ttctcttatt 180
cgggtgtttg tttgcgattt cgattgcgaa agaggaggca acgaagctag gaacagtaat 240
tggaatagat cttggaacga catattcatg tgttggtgtt tacaagaacg gtcatgtgga 300
aatcatagca aatgaccaag gaaaccgtat taccccatcg tgggtggctt ttactgacag 360
tgagaggttg atcggtgaag cagccaagaa tctggcggct gtcaatcccg agaggaccat 420
ctttgatgtc aagagactta ttggaagaaa gtttgaggat aaagaggtgc agaaggacat 480
gaaacttgtg ccttacaaga ttgtgaataa ggatggaaag ccttatattc aagttaagat 540
taaggatggg gagactaagg tcttcagtcc tgaagagata agtgctatga ttttgaccaa 600
gatgaaggaa acagctgagg cattccttgg aaagaaaatt aaggatgctg ttgtcactgt 660
tcctgcatac ttcaatgatg cccaaaggca agctaccaag gatgctggtg tcatagctgg 720
actcaatgtg gctagaatta ttaatgagcc aactgccgct gctattgcct atggtttgga 780
taagaagggt ggtgaaaaga acattcttgt ctttgatctt ggtggtggta catttgatgt 840
cagtgtcttg acaattgaca acggtgtctt tgaggttctt tctaccaatg gtgacacaca 900
tttgggaggt gaggattttg accagaggat tatggagtac ttcattaagt tgatcaagaa 960
aaagcatgga aaggatatca gcaaggacaa cagggctctt ggaaaactca ggagggaatg 1020
tgagcgtgcc aagagagctc ttagcagcca gcaccaggtc cgtgtagaga ttgaatcact 1080
ccatgatggt atggatttct ctgagccact gaccagagcc cgttttgaag agttgaacaa 1140
cgatttgttc agaaagacca tgggacctgt gaagaaggct atggacgatg ctggtttgga 1200
gaagaatcag attgatgaga ttgttcttgt tggtggaagt accagaattc caaaggttca 1260
acaacttctt aaggattact ttgatggaaa ggagcctaac aagggtgtga accctgacga 1320

ggctgttgct tttggtgctg ctgttcaagg aggtatattg agtggagagg gcggtgatga 1380

aaccaaagat attcttcttt tggacgtggc tcccctcacc ctgggtattg aaactgttgg 1440

tggagtgatg accaagttga ttccaaggaa cactgttatt ccaaccaaga aatctcaagt 1500

tttcaccact taccaggatc agcagactac tgtctccatt caggtctttg aaggtgaaag 1560

gagtctcaca aaggattgca ggctgcttgg taaatttgat ttgactagca ttcctccagc 1620

tccaagggga acccctcaaa ttgaggtcac atttgaggtt gatgccaatg gtatcctgaa 1680

tgtcaaggcc gaagacaagg gcactggtaa atcggagaag atcaccatca ctaatgacag 1740

gggtcgtttg agccaggagg aaatcgagcg catggttcgt gaggcagagg aatttgccga 1800

ggaggacaag aaggtgaagg agaggattga tgctcgtaac agtttggaga cctatgtata 1860

taacatgaaa aaccagatta acgacaaaga taagcttgct gacaagctcg agtctgatga 1920

gaaggagaaa atagaaactg ctaccaagga cgcccttgaa tggctggacg acaaccagaa 1980

tgccgagaaa gaggactatg aagagaagct caaggaggtg gaagctgttt gcaacccaat 2040

catcactgcc gtgtaccaga gatctgctgg agccccaggt ggtggatcag cagaagattc 2100

cgaggacgac tctcaagatg aactctgaga atctttctgc ttccttctac ccctgctgga 2160

aagcaagggc aagggatgag gatgtaggct agatgaagtt ttgctgatcc tagaagctaa 2220

agggttaaaa taacatatcc cccttttttt ggtttttttga ggttaaaaat acatggaata 2280

gtttgttttc tctacatgtt taaaacacct tctacgagtt atagaaggta ggtccgtgag 2340

attgagtagt gtt 2353

<210> 68
<211> 2774
<212> DNA
<213> Populus trichocarpa

<400> 68

```
ggagctatgt ctgtacttac tcgtgtccaa agcattcgtg aacgtttgga tgagaccctc     60
aagactcatc gcaacgagat tgttgcctta ctgaccagga ttgaaggcaa gggaaaagga    120
attcttcaac accaccagat tattgctgag tttgaagcaa tccctgaaga gatcaggaag    180
atattggcgg gtggtgcttt ttctgaagtt ctcagatcca cacaggaagc aattgttttg    240
cctccatggg ttgcccttgc tgtgcgccca aggcctggtg tctgggagta cgtcagagtg    300
aatgtccaag cacttgttgt tgaggagttg cgtgttgctg agtatcttca tttcaaggag    360
gaacttgttg atggaggctc caatggcaac ttcgtgcttg aattggactt tgaaccattc    420
agtgcatctt tccctcgccc aactctttcg aagtacattg gtaacggtgt tgagttcctc    480
aaccgccacc tttcagctaa gttgttccat gacaaggaaa gcctgcaccc cctgcttgca    540
tttctcaagg tgcactgtca caaggggaag aatatgatgc tgaatgacag aattcataac    600
ctagactctc tgcaatatgt tctgaggaag gccgaggaat atctgtcttc tctgaaacct    660
```

gagactccat actctcaatt cgaacacaag ttccaggaga tcggcttaga gagaggatgg 720
ggcaatactg ctgagcgtgt tcttcagatg attcaacttc ttttggatct tcttgaggca 780
ccagatccct gcactcttga aactttcctt ggcagaattc ctatggtctt caatgttgtg 840
atcatgtccc ctcatggata cttcgcccaa gacaatgttt tggggtatcc tgataccgga 900
ggccaggttg tttacatttt ggatcaagtt cgtgccttgg agagtgaaat gcttctgcgt 960
atcaagcagc aaggacttga tatcaccccc cgaattctca ttattactcg actgctccct 1020
gatgcagtag gaaccacttg tggacaacgt ctggagaaag tctatggatc ggagcattgt 1080
gatattcttc gagttccctt cagagatgaa aagggaatgg tccggaaatg gatctctcgc 1140
tttgaagttt ggccatacct agaaacttac actgaggatg ttgctgctga aattgctaag 1200
gagttgcagg gaaagcctga tctgatcatt ggaaattaca gtgatggaaa cgttgttgcc 1260
tccttgctag cacacaaatt aggtgttaca gagtgcacta ttgcacatgc tctagaaaaa 1320
acaaagtacc cagattcaga tatttactgg aagaagtttg atgaaaagta ccacttttca 1380
tgccagttta cagctgatct tttgccatg aaccatacag atttcatcat caccagcaca 1440
ttccaagaga ttgctggaag caaggatact gttggacagt acgagagcca cactgctttc 1500
actctccctg gcctctacag agttgttcat ggtatcgatg tatttgatcc caaattcaac 1560
attgtatccc ctggtgctga cgagagcata tacttccctt acactgagaa gaaacttagg 1620
ttgacttctt tccatgaaga aattgaagaa cttctttaca gctccgttga gaacgacgag 1680
cacttatgtg tgctaaaaga ccgcaacaag ccaattctat tcaccatggc gaggctggac 1740
agagttaaga atttaactgg tcttgtagaa tggtatggaa agaataccaa gctgcgcgaa 1800
ttagctaatc ttgttgtagt tggtggtgat agaagaaagg agtctaaaga tatagaagag 1860
caagctgaga tgaagaaaat gtacagtcat atagagaaat acaaattgaa tggccagttc 1920
agatggattt cttctcagat gaaccgcgtg aggaatggag agctctaccg ttacatttgt 1980
gataccaagg gagctttcgt gcagcctgct ttgtatgagg cttttggatt gactgttgtt 2040
gaggccatga catgtggttt gccaaccttt gctacttgca atggtggtcc tgctgagatc 2100
attgtgcatg gaaaatccgg attccatatt gatccttacc atggagtaca ggctgctgaa 2160
ctccttgttg acttctttga gaagtgcaag gctgatccca gttactggga caaaatctcc 2220
cagggaggcc tgcagcgaat ccaagagaag tatacctgga aaatttactc tcaaaggctc 2280
ctgactctca caggagttta tggcttctgg aagcatgttt ccaaccttga tcatcgtgag 2340
agccgtcgct atctggaaat gttctatgca ctcaaatatc gcaaattggc tgattctgtt 2400
cctttgacta tcgagtaaat ggagctggag aaatcaagga aacatgggtt ggtttgagtc 2460
gggttccggg tccagaataa tggtgtcatt tcacgatagt gattggacaa gaaaggcttt 2520
gatcttcttt ttgttttttt gtttttcctt tctgtgtgta tctttttttgt ctttcctttt 2580
cttttcatca tgctgcctct tgaatttcag taaagattgt gtcttttgag ttcatcatcc 2640

cctctatcca tttcttgcaa attctctgtc tcttgagatt ggaaatcatg ggaaatgatc 2700
cagcatcaat tcatgtattt ctcctcaata ctcgagattg gaagttgatg gctattcttc 2760
atcattatat aaag 2774

<210> 69
<211> 1428
<212> DNA
<213> Populus trichocarpa

<400> 69

ctccaatcac gtgtccgctt tacacaggtc gatccaaagc aattttctcc attgcaagag 60
aagccaagaa tttgtgacga acttggtata aattagtttt gttttgacaa gagagagact 120
tctttgttta atttccatgt gtctgaggag ggaacgaaga acaaccccag ggtgcttttc 180
attgttcgga cacctcttag attctcagac aacttgaaca caaaaatatt tcccgagttc 240
caggatcatt ttgaaggata caaggaaaga ggaataaaaa gaaagaacaa tggcattcac 300
gggaaccctg gataagtgca aggcttgtga taagactgtt tattttgttg atatgatgtc 360
tcttgaaggg gtgccttatc ataaatcctg cttcaaatgc agccactgca aaggaactct 420
tgtgatgagc aactactcat ccatggatgg agtcctctac tgcaagactc attttgagca 480
acttttcaag gagggtggag atttcagcaa gaattttcag aaagggaagc ccgagaggac 540
tcatgacctg tctaggatcc ccagcaagct atcttcagtg ttctgtggaa cccaagacaa 600
atgctccgct tgcggcaaaa cagtgtaccc attggagaag gtttgttttc agctctgaac 660
ttaaataaat ccattaggtc ctaaagacct gtgtgctatt tataccccta catttatcag 720
ataaatgacc aacatattct taggtacatc ttatctatta tagatggatc tatctgagaa 780
atgttttcaa gtctctgtta tttaataggc atgcctatga taatctggaa taagtacaga 840
tgtgctataa ccattgatga cataggttac catggaggga gaatgttacc acaagacatg 900
cttcaggtgt gctcatggtg gatgtccact cacacattca tcttatgctg ctcttgatgg 960
agtcctctac tgcaaggtcc acttcgctca gctcttcatg gaaaagggaa cctacagcca 1020
tgtccttgca tcagctgcac acaagagatc aaattccaca cctcctgaac tggccgggtc 1080
aaacccagag gaaggagctg ctgtggagga ggaaaagcca gaagagcaat cttaaacaaa 1140
gcacatcatg ttttcttgca ttgctatctt ttcaagttgc caggactgct ttgagttttc 1200
atgttgttga atattattgc cggattgtgt tcaaagtttg tttttggatt tgactatctg 1260
tgttacagat gccttgctac aggatattga attgcaatag tttttacaaa ttcctcctct 1320
tgcaatttaa tactcttggc aacaggatac aaatgaaaaa cccaaatcct aggaaccagt 1380
atcaacattt attgatctca gctctttgct cagatggctt gcgttttc 1428

<210> 70
<211> 776
<212> DNA
<213> Populus trichocarpa

<400> 70

```
cttgttcaat cccggcacta ctcctgaatg cctgcattcc cttttagatt ttgtgtttga      60
ttttgtcctg taaggaggat atgcgggatc atatggaaag atttgtagtt cttccattct     120
ccatcgcctg tgcctctcac tccagtgttg atgtggcctc cagtgaatcc tgcaagaaac     180
caaaacccga aaccaaatca catgcaacaa gaggacaaga aggggaggaa agctcttgta     240
aagaaaagac gaagaacaat acatttggtt tcctgctggc tcttccaaag ccttgcatat     300
ccagtagcat tcacaaattg attaggggca ttaagactct ctcccaagta tttgtgtaca     360
aagaagaaga cgaggagcta atggaaagag agatggaaat cggatatcca actgatgtga     420
agcatgtaac acacatagga ttggatggaa ctactatgac aaatcctatt aagggctggg     480
aatgcctgaa atctccagaa ataattccat tcccttcatt tactttaagg cagttcgagc     540
ttgcaatggc tgcacaagct catggacctc ttgttggggt cgatcattcc aagcttgttt     600
gattcattga tttttctttt catttcctga tcttgtttct ttgacactag atgactgatg     660
tgatgaagat tgatcaatgt ttttgatgga ggcactggtt gcagtgatgt gtttatggtg     720
tgtgtgtggg accttgacaa tgtttttctg ggtggccatt gaaattgttc ttgcaa         776
```

<210> 71
<211> 819
<212> DNA
<213> Populus trichocarpa

<400> 71

gagtgtaagc tagctagctt tgcagagttc tgccgattca atggcgacca aagtgtatat 60
tgtgtactat tccatgtatg gacatgtaga gaaactggca gaagagatta agaaaggggc 120
ttcatctgtt gaaggtgttg aggcccaatt atggcaggtt cctgagacac tgccagaaga 180
ggtgcttgga aagatgagtg caccaccaaa gagtgatgta ccaatcatta cacctggtga 240
actcgctgaa gctgatggat ttgtgtttgg attcccgaca agatttggaa tgatggctgc 300
ccaattcaaa gcttttctgg atgcaactgg aggtctatgg aaaacacagc aacttgctgg 360
caagcccgct ggaatcttct ttagcactgg atcgcaaggt ggtggccaag agaccacagc 420
gttgactgct attacccaac ttgttcacca tgggatgata tttgttccca ttggctacac 480
atttggtgct ggcatgtttg agatggagaa ggtgaaaggt ggaagtcctt acggcgcagg 540
aactttcgct ggggatgggt caagacagcc aaccgagctt gaattggagc aggctttcca 600
ccagggcaag tacattgctg ccatcacaaa gaagctcaaa ggagctgctt aagtttactc 660
aattacaaga atacggtctg gtcttccatt ttttcaccct atagaatatg catttgcaaa 720
gaataattct tattctactg tttattatat gcttttcatg tgtgtaacac gatagtgatt 780
attgtctcaa tgatatcaag gttggttgtg ttttcattt 819

<210> 72
<211> 1246
<212> DNA
<213> Populus trichocarpa

<400> 72

```
aaggaaaaga aatcaaaatt gttgctaatt ggacgagagg aagaaaagga gaacaaaagc      60
accttcatag ggcccatttc atgtctcctc atgagaccta agcttccccc cccactgcac     120
aatcacacta ttcttctttc atctcttttc tttccttctt tctccatcac ccacccgttt     180
tcttaatttc aattcttgtc aaaatagtct tgatcatttt ctctgcaaaa gaaattaatc     240
atgtcaggtg atcaaaggcc taaagattct gctgaaggct cttcgcgatc tggaggcgat     300
caccaccagc ttcaatcagc tcctttgagc cggtatgagt cgcagaaacg gcgagactgg     360
aacactttcg ggcagtactt gaagaatcag agacccccag tttcattatc tcagtgtaat     420
tgcaatcatg tgcttgattt ccttcggtat cttgatcagt ttggcaagac taaggttcat     480
ctacatgggt gcgtcttctt tggacaacct gatcctcctg ctccttgtac atgccctcta     540
aggcaagctt gggggagcct tgatgcccta atcggacgcc ttcgagcagc ttttgaggag     600
catggaggat cagcggagac taaccctttt ggaaatggag ctattcgggt ttatttgcgt     660
gaagtgaaag agtgtcaagc taaggcaaga gggattcctt acaagaagaa gaagaagaag     720
aagactcaaa taaggccaag agacgaagca aagccttcga tgcagacagc ttaagtcctt     780
tggcttcttc cgataaatgg atcacaaaat aggacaaatg gagatgaagt tccagaaagg     840
caaatgttgg gggtctagcc tctgcttcag tacttattat atagctagct agcttctctc     900
tttgcagata gtgttacatc acagcctact tctaaataat cttgtcctgt caggttggtc     960
taagaagcta ttcctagctt ataggaatta aaactgtagt cgtaatgttt ctactacatc    1020
ctcgacttct taattagtaa gacgttaaca ctatatgtcc agctctgttg tttctttgct    1080
gcagaagaaa actagtgtct tcagctaatg gtgaaaatgg cttttagctt ctgcctaaat    1140
gtagctttgg gcttgcctct cattagatga gctgtactta ctagtaatcc ttcaattgtc    1200
caaagtacca agtttttctt catgtttaat cttcagttaa tttcct                   1246
```

<210> 73
<211> 2176
<212> DNA
<213> Populus trichocarpa

<400> 73

```
acaactacaa ctgctacttg taggtgtggg tttgctctta actctttcat tctatctgcg      60
atcctctctc cctccccctt ggcacttgtc aagcagcaga gaatgaaggt tattgataag     120
ataagggcgg cagcagcagc aggagattca aatggaggag atagcagcag caataataag     180
gttgttttct ccttcgaatt ctttcctcca aagaccgatg atggagtgga caacctgttc     240
gagagaatgg acaggatggt ctctcacaat ccttccttct gtgacatcac ttggggtgct     300
```

ggtggctcca ccgcagatct gaccttagac atcgccaaca agatgcagaa catcatctgt 360
gtcgagacta tgatgcatct cacctgcacc aacatgcccg ttgagaagat cgatcacgcc 420
ctcgaaacca tcaaatccaa tggaatccag aatgttcttg ctcttcgtgg tgatcctcct 480
cacggtcagg ataaattcgt ccagatccaa ggcggcctcg cttgcgctct cgacctcgtc 540
aaacatatca gatccaaata tggtgattac ttcggcatta ctgtcgctgg ctatccagag 600
gcacatcctg atgtgattgg aagtgatgga tttgctacgc ttgaggatta ccataaagat 660
cttgcttatt tgaagcagaa ggtggatgct ggggctgatc taattgttac tcaactcttt 720
tatgatactg atattttctt gaaatttgtg aatgactgcc gccaaatcgg aatcaattgt 780
cccattgtcc ctggtattat gcctattaat aactacaagg gcttcatacg tatggctggt 840
ttttgcaaaa ccaaggtacc agccgaggtt actgcggcat tggagcctat caaggataat 900
gaagaagccg tcagagccta tgggattcac cttggaactg aaatgtgcaa gaagatttta 960
gctcatggaa tcaagacgtt gcatctttat actctgaaca tggagaaatc tgctttagct 1020
atattaatga atcttggttt gattgaagag tccaagatta caaggccgtt accatggcgc 1080
cccccgacaa atgttttccg tgtaaaagaa gatgtccgtc cgatattttg ggctaatcgt 1140
ccaaagagct acctaacaag gacaataggt tgggaccagt accctcatgg ccgatggggt 1200
gattctcgca atccatccta tggtgcttta tctgactatc agtttatgcg accccgggca 1260
cgtggcaaga aacttcttga agagtgggct gccccttga aaagtgttga ggatatatac 1320
gagaaattca aggtatactg ccttgggaag ttgaaaagta gtccttggtc agaactagat 1380
ggccttcagc cagagactaa aatcattaat gaacagctgg gtaagatcaa cttgaaggga 1440
ttcctgacta tcaatagtca accagctgtc aatggggaaa aatctgattc tccatctgtt 1500
ggttggggtg ggccaggagg gtatgtatat cagaaggctt atctagaatt tttctgctcc 1560
aaggacaagc tgaatgctct tgttgacaag tgcaagtctt tccctttgt aacttacatt 1620
gctgtgaaca aaggagggag ttggatctct aacgttgccc tgactgatgt gaatgctgta 1680
acctggggag tcttcccagg aaaggagatt atccaaccaa ctgttgtgga tcccactagc 1740
ttcagtgtgt ggaaggatga ggcatttgaa atctggtcaa gaggatgggc ttccttgtac 1800
ccagagggtg acccatccag aacgctactt gaagaggtgc agagcagcta ctttttggtc 1860
agcttggtag ataatgatta catccatggg gacatttttg ctgtcttcgc ggatttatga 1920
tgctaggggc cctttgcagc cctgcactt tctaagagtc tatccaagtc cccaaaaaat 1980
ggtgctttat cctaagttga atcccagctt taatcttctt atggcataca ttgtttttga 2040
aattatttcc gtattgaaat gtaataataa tccattgtga ttttgctact cccctctcca 2100
ttatatgctg caaatttgta tttggagtgg caactaattg taagcttaaa gaaaatatga 2160
ttgcctcttt ttcttg 2176

<210> 74
<211> 1304
<212> DNA
<213> Populus trichocarpa

<400> 74

```
ctctctctct ctccctctcc ctctccctgc cattccctcc tttgctttct ctatccagaa     60
aacacacaac tctctttctc gtccctcacg ttctcttgca atggcttcca atggagtgaa    120
cgacaagtca ctgatcgtca gcttcgggga gatgctgata gacttcgtcc cgacggtctc    180
tggcgtctcc ctcgctgaag ctccggggtt tgtgaaggca ccgggtggtg ctccggcgaa    240
cgtggcgata gcggtggcga ggctgggagg aaaggcggca tttgttggaa aacttggtga    300
tgatgagttc ggcaacatgc tcgccggaat tttgaaggaa aacggcgtga tcgctactgg    360
gataaatttt gacacaggtg ctaggactgc tctcgcgttt gttactctac gcgctgatgg    420
agagcgtgag tttatgtttt atagaaatcc aagtgctgat atgttactaa ggccagaaga    480
gttaaatctt gagttaatta gatctgctaa ggttttccac tatggatcaa taagtttgat    540
tgtggagcca tgcagatcag cacatttaca ggcaatgagg gtggcgaagg atgcaggtgc    600
attgctttcg tatgacccaa atctgaggct gccattgtgg ccatcagcag aggaggcgcg    660
tgagcagata ttgagcatct gggacgaggc agatgtggtc aaagtcagtg ataatgagct    720
tgagttcctc actggaagtg acaaaattga tgatgaaact gctatgtcac tctggcgtcc    780
taactttaag ttgctcttgg tcactcttgg tgaaaagggt tgcaattatt acaccaagaa    840
tttccatgga tcagttgagg ctttccatgt ggacactgtt gataccacag gtgctggtga    900
ttcatttgtt ggtgctctcc tttgcaagat tgttgacgac cactctgtgc ttgaggatga    960
gccaaggctg agagagatac ttagatttgc aaatgcttgt ggagccatta caaccaccaa   1020
aaagggagca atccctgctc tgcctactcc ggccgatgcc ctcaaactgg tgaaagaagg   1080
aaaataaact atttttttgt taatccgcac tgcattttat ccctttcctt ttcagctcct   1140
ggttctcaat tcaatcgaag agctgttgca atctcaaatt ttcgcctttt tctcttgagt   1200
gtaatgatgg aaaagacttg cgctttaagg tcttgccaaa taaaattttt cttttgtgtt   1260
agctttctcc aaatattata tttgtttaaa gctaaaattt ctgc                     1304
```

<210> 75
<211> 853
<212> DNA
<213> Populus trichocarpa

<400> 75

```
gaagtatttg gtgacaattg tcttcgtaca tgggtttccg ggtcttggtg gcgaaagtaa      60
aagacttcgt tatgggggag aaggagccca ggaaagctga atctgaggga gcttctctgc     120
ctacacaagc tgaagaacat ggacctgtta aagaagagaa agaagcccct ttaaatgatt     180
atgctaatga gaagagctcg gtcctggtta ctgaaaaggt tgcagatcca cctgctactg     240
caaaaaactc aagggggcca aatgacagag atgctgtgct tgcgagggtt gaagcggaga     300
aaagatgtgc tctaattaaa gcatgggaag aaaatgagaa agctaaagcg gagaacaggc     360
tcacaagaaa ctctctgcca ttggatcatg ggagacaatc aagagagaat ctgtggaggc     420
aaaaataaag aagtatgagg aaaaagtgga aaagaagaag gctgaatatg cagagaaaat     480
gaagaacaaa gtagccgaac tccacaaggc agccgaggag aagaaagcaa tgattgaagc     540
aaaaaaaggt gaggaccgtc tcgaggtaga ggaaactgca gcgaaattcc gagcaactgg     600
gtatacacca aggaagtgtc taagatgttt tggcaaattc taatgaacaa cagtggggta     660
aacgggagag aaaaggctgc tagaattgga tttgcttgat tttttggctt gttagtttat     720
gctgtgctca aatattgttg gttcagctgt aaatgtcttt tgatatttgt aaaaaacagt     780
aaatgggagt gcatgcgtta cattacccaa gttctgacaa gacccgagtt tgaatttgat     840
attttctttt gtg                                                        853
```

<210> 76
<211> 849
<212> DNA
<213> Populus trichocarpa

<400> 76

```
caaacaagca ctggcactgg cactggcaat ggcaatgcca cttcttcttc aactaacacc      60
accacttctt cttcttcaac atcttcaagt tccatgagca gtaataacag taatggcaac     120
aatggttgtc agcagaacca aagtaaccag cttggagagg caagatcaag tctttattat     180
tcaacaaatg caatgtcttt tgttaccaag tctttgttgc ctactagaag aagactcagg     240
ctcgatcctc ctaacaagct cttcttccct tatgaaccag gaaaacaggt taggagtgcc     300
attggcataa aaaacactag caagtctcat gtagctttca agttccaaac aactgcacca     360
aagagctgtt acatgcgtcc tcccggggct atacttgcac ctggtgaaag tcttattgca     420
actgtattca agtttgtgga gcctccagag aacaatgaga gactactgga tcagaagagc     480
agggtaaagt tcaagatcat gagcttgaaa gtgaaaggag aaatggaata tgttcctgag     540
atgtttgatg agcagaagga tcaagtagca gttgagcaaa ttttacgagt tgtttttctt     600
gatccagaat gccctagccc tgcactggaa aagcttaaaa gacaactggc tgaggctgaa     660
gctgagctcg aagcacgcaa gaagcctcca gaagatgcag gcccccgagt tgttggcgaa     720
ggacttgtta tagatgagtg gaaagagcgg agggaaagat acctggcacg ccagcaggtt     780
gaagttgatt ctgtgtgaag atcctgtttc cttgcaagca cttgtttttt cttgagctga     840
ggtttcctt                                                             849
```

<210> 77
<211> 1598
<212> DNA
<213> Populus trichocarpa

<400> 77

atgggtttta accgggtttt gcagaaaacc ttgtcctctt tagagttgcc tgtttatatt 60
gcaagtggac catctgcatc cgtttcagat ggtggtgaat cttcatcttg tgaactccaa 120
gagaggattg gtgactctat ccgtgatgaa attcctaggc cttctcaaaa tcctgggagg 180
cagaagcata agagtcatga tcctgctcga gatcttacga tccatgtatt ggaaaaattc 240
tctcttgtga ccaaatttgc tcgggacaca agttcacaac tatttcgaga aagtaatagc 300
aatggctatg gtgctgtaga aaggaaaagt tctagctact ctctccctga tgtcccccat 360
aagccatcca tggatgcaga gatagctctt gaggaaggtc ctgttccctc agatcctctg 420
gagttcgata aaatgacact cgtatgggga aaaccacgac agccaccctt ggggtcagaa 480
gagtgggcta ctttcttgga ttctgaaggg cgagtcatgg attcaaaagc cttaaaaaag 540
agaatattct atggtggagt tgagcacaca acacgcagag aggtctggcc atttttgctg 600
ggatatcatg cttatgattc aacgtatgcg gagagggaat acctcaagtc ttccaaaaag 660
tcagagtatg agacagtaag acaacaatgg cagagtatct ctactgaaca agcaaagaga 720
tttacaaaat ttagggaaag gaagggcctt atagacaaag atgtggtgag gactgataga 780
gcgctgtctt tctatgatgg ggatgataat ccaaatgtga atatcttacg tgatattctg 840
ttgacgtact ccttctataa ctttgatctt ggttactgtc agggtatgag tgacttacta 900
tccccaattt tgtttgtgat ggaggatgaa tcagaatcgt tttggtgttt tgtggcactg 960
atggaacgtc ttggacccaa ttttaatcgc gatcaaaatg gcatgcactc tcagcttttt 1020
gcattatcca agctggtgga gttgcttgat tgcccattac ataactattt caagcagaat 1080
gactgcttga attacttctt ttgtttccgc tgggttctaa tacaatttaa aagggaattt 1140
gaatacaaga aaacaatgcg attgtgggag gtgttgtgga cccattactt gtctgagcat 1200
ctgcacctgt atgtatgtgt tgcaatcttg aagcgatacc gcaagaagat aatgggggag 1260
cacatggact ttgacacgct cttgaaattt atcaatgagc tgagtggcca tattgacctt 1320
gacgcaatac ttagagatgc agaggctctg tgcatatgtg ctggtgagaa tggtgctgct 1380
cacatcccac caggaactcc accttcattg cctactgaga atgagaatgc tttattatat 1440
gctcaagatg atgaagtact gtaatacagt tcttctcatt ggtacgatat aagcggcttt 1500
attttttgtt ttcttcatca aatagctgtg tataattgct gtttttttcga aataaaatgt 1560
taataatcaa gcaatcaact ccatttgctg tcctcctt 1598

<210> 78
<211> 1222
<212> DNA
<213> Populus trichocarpa

<400> 78

agagattgga gatgattaca gcagcccagc gagagagctc ttctttatgt tctgtttaag 60

aaagccatct tctttaaggc acaatcaaga actatgttca tcagtgagaa taacagatgc 120

acttgtccaa cacgagcaag aatcccagct caatattaat acaagcaaag atatattgct 180

ccggccctgg agtgatgata atgtggagac agagctcatg aggctgcata gtctctcagg 240

tccaccaaga tttctcttta caattgttga agaaacaaag gaggacttag agtctgaaga 300

tggcaggtct agaggtgatc ctaagagtgc gaaaggatca agaagtagaa gcttaagtga 360

tttgcttcgt actgtggaga ctccatatct aacccctctt tcttctccgc catttttcac 420

acctcctctt actcctagct ataatcagat tggattcaat catctctttg aatcgtcaaa 480

agatgcagag ttcaataaga taagatcatc accacctcca aaattcaagt tcttacagga 540

tgcggatgag aaactacata gaaggaaatt gatgcaagaa gcaggggaga aggtccaaag 600

gcatgatgtt tttgctcagg atcataccaa aatacctgct agttcaaatt cacacaaaga 660

tgaggatgat gggcctttta tcaccatcat tattgacagg aacaaagaaa gagagcttaa 720

tgaacaaaat catcaactac cagactacca gtcaagcact tcccaggtac ttcctcttgc 780

tacttcacct tcaacatcaa aatcagcagc caagaaaagt tccttctttc attagatctc 840

cgtgatcaag taaaacgaga tagttttttt ttttccccat gtatttctat taaccaactt 900

tagtcttctt tttaggagtt ctttttcttt aatattttta tgggatcgtg ttagaaggtt 960

aaccttgcca aaccctttg caatgtctga aaaatcttcg ggtgggtggg ggagttggac 1020

acactctctg cagtataatg ttaaaagttg gacaagtgca agtgggggtc ttgagggagt 1080

gatctgttgt cttcactctt catgaatctc tgagctgtct gatatctgta atcattctgg 1140

tgagtggtgg tactactgat gtttttgtcc aaaaggcact gtaagaattc tttcaagacc 1200

attcacttct ttatttattt tt 1222

<210> 79
<211> 1418
<212> DNA
<213> Populus trichocarpa

<400> 79

atggcatatg tatatgtgat tctgtttcca agatttcagc cagttattaa taagacgtat 60
gagactcccc atttcaatag ttctgtaagt gagtgcaatt attttgaggg aaactggata 120
caagatgaga gttacccttt atatgatgca tctcagtgtc ctttcgctga gaatggattt 180
aattgcttgg ctaatgggag aagagacgga ggttatacaa aatggaggtg gaagcctaaa 240
aattgtgaaa ttccgaggtt taatgcgcgt gaaattcttg aaaagatgcg tgggaagcga 300
attgtttttg ttggtgattc attgagtaga acacagtggg agtctttgat atgtatgctc 360
atgacaggtg tggaggacaa gagaagtgtt tatgaaatca atgggaataa aatcaccaag 420
cagattaggt ttttaggtgt acggtttagt tcttttgact tgagaattga tttctataga 480
tcagtttttc tggtgcagcc tggtccggca cctaggcgcg cgccaaagag ggttaaatca 540

acactgaaga tcgataagtt ggacgatatt cgcaatgaat ggattgattc tgatattcta 600
atatttaatt cagggcattg gtggacaccg agtaaacttt ttgaaatggg ctgttatttt 660
ctggtaggcg gatcgctgaa gctaggaatg cctatcactg ctgccttcga aagggcactg 720
catacatggg catcatggct taatactacc ataaatgcaa atagaacaag cgtgttcttc 780
cgcacttttg aatcttccca ttggagtggc cggaaccgcc tttcttgcaa agtgactcgg 840
cggccttcat caagaactgg agggagggat cgtagcctga tatctgacat cataatcaag 900
gttgtgaagg caatggcagt tcccgtaaca gttttgcatg tgacacccat gggtgcattc 960
cggagtgatg ctcatgttgg cacttggagt gataatccat ctgtgcctga ttgcagccac 1020
tggtgcttac ctggtgtacc tgatatgtgg aacgagattc tcttgtcaaa tatgttgtcc 1080
aggaattaat aggccattct ttcccataag ctagaaaatg caactgtgta gtgtgttcca 1140
tattcttccc tcttcaattg accccattca atttccaaac cttctactca actactttca 1200
atacatacga agattttctc tgctgagaag ttgtgtgcat ttgtcacaaa tattgtttgt 1260
tgtgtagttc agggatccaa aaattacttg atttgtcttt tctagttgca cttgagttca 1320
catgtaatgg taatgatttc tttccagcgt tagctgaaat ctggaagctc ttctcttcag 1380
atgccaccga gctgttgtac aataaaagcg ttattgct 1418

<210> 80
<211> 2038
<212> DNA
<213> Populus trichocarpa

<400> 80

```
aggtacccat tttttttgttg ttggaaacaa acccattttc tctctctgtc tttgtgtctt     60
tctttgataa attttaatca tcaacaacaa cgacaacagt aacttgcaaa gagagaaaga    120
gggaggagag agagcaaaaa agaaagggtc tctctcaccc tctcttattg ttatgtctcc    180
tcgcagatct tagattcttt cagtttccta tctgggttct tctttttgtt tgtcaggatt    240
cctggattgc ttttgattga tatttgattg ctcaaaggtt gtttctactt ttggattaaa    300
tccaagagtg accttttgtt gtaaatatcc aagctttgct ttgtattttt gacaagaatg    360
gaaatggagc ttggaaagtt gttcattggt gggatttcat gggacacgaa cgaagaccgt    420
cttaaggagt atttccgggc ttttggggaa gttttagaag ctgtcataat gaaggatcgg    480
gccacgggtc gtgctcgagg atttggtttt gttgttttcg cagaccctgc tgttgctgag    540
agagttgtga tggaaaagca ccttatagat ggtagaaatg ttgaggcaaa gaaggcagtt    600
cctagagagg atcagaacac cctgaacaaa aacagtagca gcgttaatgg ttcacctggc    660
cctgcccgaa caaagaagat atttgtagga ggtttagcat ctacagttac agagagtgac    720
tttaggaagt actttgatca gtttggggtg attacagatg tggtggtcat gtatgatcac    780
aacactcaaa ggccaagagg ttttggattc atcacctacg attcagagga agcagtggat    840
```

```
agagtattgc acaaaacctt tcatgaactc aacggtaaaa tggttgaggt caagcgggct     900
gtccccaaag agttatctcc aggaccagct aggaaccagt taggggggtt taactatagt     960
cctagtagag tcagtagctt cctcaatggt tacactcagg gatacaatcc aagttttgtt    1020
ggagggtatg gcgttagaat ggatggcagg tttagtcctg ttactgctgg gcggagtaac    1080
ttttctccat ttggtagtgg ttatgggatg ggattgaatt ttgagcaggt gttgaaccca    1140
gtttatgggg gaaattcaaa catcagttct aatgctggct atggacgagt aagtccttca    1200
tatagtggaa atgcaagcag gtacagcaac cccattggat ttagtggagg caatggagga    1260
agtagttctg tcttaaatcc aattgcccac tctttatggg gaaatggaag tattaatcat    1320
gcttctaaca ccacaaactc cagttctttt atgagttctg gaactgggag ctcaggaatg    1380
ggttctttta gcagtatggg agcactttgg ggttcctctg ctaattctga acaaggtgga    1440
ggagttcgcg cagtcaatag cagtaatctt agctttggtg gtggagattt tgatattggt    1500
ctagaagggg tagggtatgg aaggaacagt aggacaggtg ttgcaccagc atcatctcat    1560
gctgcatcta atggtggtta tgatggggct tatgcagact tttatgagaa gggctcatta    1620
tacgggggata atacctggca atcttcacct tcggaactgg atgtgtctgg ctcatttggt    1680
tttgggcttg gaaatgcaac ttctgatgtt atgactaaaa attctgctgg ttatgttggt    1740
ggctatagtg ttgctaatag acaatcaaat agaggaattg ctgcatagga tgattatgat    1800
ataggtgacg gtgaagtgaa tttcgtacat gtttcttgtc cagatgatca tgtttcaact    1860
ttgctcatca caaggaaatc tgtgtatggt ttgagctttt gagatttttc taatataggt    1920
tttctttctt ggaattttat taagatgtaa aatcacattg tggggtatac tcaacatgat    1980
tgtatgatgc atctttggtg agagctatat taaaaaagga aacaattttt tagcatta      2038
```

<210> 81
<211> 1611
<212> DNA
<213> Populus trichocarpa

<400> 81

```
acactcttcc ctctctttac ttgtgcacgc ttaaagaaga gatcacacaa tggcaaaccc      60
ttctctctgt ctcctcctcc tcctctctct cctgacccca gccctcgtct cctcttcccc     120
ggttcaggac ccagaatttg tagtacaaga agtacatagg gccatcaatg cctctaggag     180
gaagttggga tatctctctt gtggaaccgg caaccccatt gatgactgct ggagatgcga     240
tcccaattgg gagaagaacc gccagaggct agctgattgt gcaattgggt ttggtaagaa     300
tgccattggt ggaagaaatg gtaagattta tgtggttaca gaatctggta atgatgatcc     360
cgtgaaccct aagccaggga ctctcaggca tgctgtcatt caagaagagc ctttgtggat     420
catttttgct cgtgacatga caattcaatt gaaggaagaa ctgatcatga actcgttcaa     480
gactatcgat ggtagaggtg caagtgtcca tattgccggt ggcccatgca ttactataca     540
gtatgtgacc aacattatta tacatggact aaacatacac gattgcaaga gaggagggaa     600
tgctatggtg agggactccc caaaccactt tggatggagg actgtatcag atggtgatgg     660
tgtgtccatc tttggtggtg ctcacatctg ggtggaccat aattcattgt caaattgtaa     720
cgatggactc gttgatgcca tccatgggtc ctcagccatc accatttcga acaattacat     780
gacccaccat gataaagtca tgcttctggg gcatagtgat tcctacactc aagacaagaa     840
catgcaagtc accatagcct tcaatcactt tggagaaggt cttgtccaga gaatgccaag     900
atgtagacat ggatatttcc atgtggtcaa caatgactac acccattggg aaatgtacgc     960
cattggaggg agtgctagcc caaccatcaa cagccaaggc aacagatttg tagcacctga    1020
catcaggttc agtaaagagg taacaaaaca tgaagatgca ccagagagtg aatggaagaa    1080
ttggaattgg aggtctgaag gagatctact attgaatgga gcatttttcg tagcatctgg    1140
cgcaggcgct tcatcaagct atgctagggc atcaagcttg ggtgcaagac catcttcact    1200
tgtcggtcca atcacgatgg gggcaggtgc acttaactgc aggaaggggg gtcgttgcta    1260
gctgattgtg aaagattaaa caatattttg tgacgagtaa tttggactat aattaattag    1320
ctggctagaa aagtaattaa gcagaaggga attaaaaaat gaggagggtg aagaaaagtc    1380
caagcactag tttcccctgg ctttttcttc cctccaaatt tttattcttt ttacccattg    1440
ttatctgctc ttatctttcc tccccccttct cctttttgtag tcaaaccttg aagattacaa    1500
cctcagcctg ttactctttg aacctaaggc attctccttg agaattcttg gtgggcatag    1560
agaaaacaag tgctcaagtg ttgatatagt atatatcaat tatctattca t             1611
```

<210> 82
<211> 1798
<212> DNA
<213> Populus trichocarpa

<400> 82

```
atataaaaat aatactaggt gctctccaat ggctgcaaaa ccatcaacag cgttcctttt      60
ctcttctata ttttaaagaa aaaacacctt ctttcaacaa ccttttgatg gattccatca     120
gaattagcat ctcttcaatc ctttcttgga ttcccttaga acatgttctt tgatagccaa     180
atcaccaaag ttgaacaaaa agagaaaatc ttgttgagtt cttgagctaa acgagaggag     240
gaggagaatt gtttttcgtt tttcaaatat ggaggagagg catgttattt ttgggaagta     300
tgaaatgggt aggcttttag ggaagggaac ttttgctaaa gtttactatg ggaaacactt     360
ggtgacagga gagagtgtgg caatcaaagt cataagcaaa gatcaagtca agaaagaagg     420
gatgatggag caaatccaga gagaaatctc agtcatgcgt cttgttcgtc atcccaacat     480
tgtagagctc aaggaagtca tggctaccaa gacaaaaatc ttcttcatca tggagtatgt     540
tcgaggagga gagttgtttg ccaaagtagc caaaggaagg ctgaaagaag aagctgctcg     600
aaaatatttc cagcaactaa tcagcgcaat tgattattgt catagcagag gtgtttatca     660
tagagatttg aagccagaga atatgttgct tgatgaagat gaaaacttga aaatctctga     720
ttttggcttg tcagccttgc ctgaacaatt tcggcaagat gggcttttgc atactcagtg     780
tggaactcct gcttatgttg ccccagaagt cttgagaaag aaaggctatg atggatcaaa     840
agctgatata tggtcatgtg gggtgattct ttatgtgctt cttgcaggat tcttgccatt     900
tcaagatgag aatgttatga agatgtacag gaaagtcttc aaggctgaat atcagtttcc     960
gccttggttt tcaacagatt ctaagaggtt gatttcaaga cttcttgttg ctgatcctga    1020
aaagagaatc acaattcctg ccataatgag aaatcattgg tttcttaaag ggttttcaag    1080
accagtggcc ttttctatcc aagaatcaag catggatcaa acaggacaag aacaagatct    1140
tgattcttgt tctgttgtca acaccaaggt atcatcacca gaattcttca atgcattcga    1200
gtttatttcc tcaatgtcat ccggctttga tctgtctagt ctttttgaga ctaagaggaa    1260
atcaggctct atgttcactt caaaattttc agcgagtgct atcatggaaa aaattgaagg    1320
ggttgcaaaa gggctgagtt ataaggtggc aaaagtcaaa gatttcaaag tgacgttaca    1380
aggtccatgc gagggtagaa aggggaagct ggcagtgacg gcggaggtgt tcgaggtggc    1440
accggaggtt gcggtggtgg agttctccaa gtcttccgga gataccttgg agtatactaa    1500
gttctgtgag gaagatgtta ggcctgcact aaaagacatc gtttggacat ggcaaggaga    1560
caatgtttgt aataaagata acaacaatag tcatgtagaa gatcgtgaaa ttcaaatgtt    1620
gtagaaatta aggctttgat cttaatttgg tgagttaatt ttaggatttt atataatctg    1680
taaataagtt ttcgttattt aattaatgca tcaagttgag tgtttttgtt aagctagcac    1740
agtgtgtgtc tatatatata tatatatata tatattatta agcttgaatt caagatta      1798
```

<210> 83

<211> 1178
<212> DNA
<213> Populus trichocarpa

<400> 83

```
gtattttctg tggaggagaa acaaaaagca atctcttggg ttttaaagat ttggtttggg      60
ttctctcttt gaaagttatc aagaacagta aaggggctct tgattgactg atgcaagttt     120
aagattttct attcttttta gagaaagaag gaagttttct tttgaaatga atttgatgtg     180
tgaggttttc tttgctgaag aagaagagaa ggttgaagaa ggagagtggg aaaaggagag     240
atagtattta ccaaagccaa ggtagctagc agaagtagag aggcagatag ctataaacaa     300
catacaaaat aaaatttcat ctaaggggtt ttagtttgtt ctgcttctgc tgggtaattg     360
gcaatgtcta ctactataat cagtgaccct atggtggtct cagcaccaga gacacaagca     420
acagcagcag ctgcagctgc agcaacgaca gcaacacaac tcattgcaca gatagaggtt     480
gagtcagtca aatgtgattg ctgtggccta atcgaagagt gcacaccagc atacattgaa     540
agagtacgcg aaagatatca tggaaaatgg atttgtgggt tatgtgcgga ggctataaaa     600
tatgagattg taaggactga gaggcttatt agcactgaag aagcaatgac aaagcacatg     660
aacttctgca agaagtttgt ttcctcaggg ccacctcctg atccaacaac tcatttgata     720
gccgccatga gacaaatcct gagaagaagc ttggattctc cgagaggttt aaggtcaacc     780
ccaagtagtc caaacaaaac gaacggagca attcgtgccg ctgcacttgc taggtccgaa     840
agttgcttcc ctgctctgtc tggttgatat ataatactga gcatcgtgga agacggggtg     900
attcataaaa tggtgatgaa gctattggaa aaaaaaaaga cagaaaaaaa cacattccat     960
tttcatgcaa ataagaaaga tcaaaggatt gaagttaggt gcctaatcta gctacttata    1020
tagttgttaa atacagaact tcctgtcaca agaaaaatca cggaactccc ttttttcctg    1080
aagctttata tttcagctgt acgtacttgt gtaatctgtc tttttaaaac catggcttcc    1140
ttcctctttt taccaaatta tatttctcaa ttttgata                           1178
```

<210> 84
<211> 1010
<212> DNA
<213> Populus trichocarpa

<400> 84

```
gtcaagataa agtcccattc tttttccttg ccccaagttt gtttgagggg ttataaaaaa      60
tgaaccaaga gatgaatggt gttgatacag agattgatca gaaccaccaa gagaatgtgc     120
aagagaaaat cgattatgtg tttaaggtgg tggtgatcgg tgactctgca gtaggcaaga     180
cgcaaattct ttccaggttt accaagaatg aattctgctt tgattcaaag tctaccatcg     240
gtgtcgagtt ccagactagg actgtcatca ttaaagacaa ggtcatcaag gctcagatct     300
gggatactgc tggccaagaa aggtaccggg cagtgacaag cgcatactat agaggggcat     360
taggggctat gttagtctac gacattacca agagaccaac gtttgatcat gtggctaggt     420
gggtggagga gctccgagcc catgctgaca actcaattgt gatcatgctg attggaaaca     480
aggctgatct tgtggacctc agggcagttc caacagaaga cgcggtggaa tttgcagagg     540
agcaaggcct atttttttct gagacatcag cccttagtgg tgacaatgtg gacggtgcat     600
ttttcaggct gctagaagaa atttacggtg tgatttgtaa gaagtcattg gaatgtggca     660
atggaaatcc ccatgctgct gatgccataa cgcttagagg ttctaagatt gatggcatat     720
cagggactga tctggagatt agtgagatga agaaattatc tgcttgctcg tgttgatttg     780
atcatttttc tagtgaattg tgtactataa gactttacca ctcgatgttc ttaattgatt     840
ctgtggcttt ctttggaaag tggtgatcgt ttcggtagtg tggtaagggt ggcaagtttt     900
ttcttctctg tgacctgtca agattttagc agtattgtac ttgtcttaca gaaaccatga     960
atttggtttt tttatatgta ttgatttgga tggatggttt tccttttcct               1010
```

<210> 85
<211> 1159
<212> DNA
<213> Populus trichocarpa

<400> 85

```
atgaactaga aattcaaagc ttccatcaca tattttaatg gcttacaatc cttcttcaag     60
gtcctgtttt tatgattctt tttgtttcct attcattaca ttcgttcttc catttttctt    120
cctcacaaaa gctcaagctg caagccattg tagaacctca tgtggtacca ttccaataaa    180
ctaccctttt ggcatcgatg atggctgtgg cagtccatat tacaggcaca tgcttttatg    240
ctccgattcg ggcattctcg agcttcgaac gccttccggg agataccaag ttcgtagcat    300
aagctactca gaccctcaca tgatagtcac cgatccattc atgtggaagt gtaaagatgg    360
tcatcacttt cgtgcaacta gggcatttag ccttgataca agcacacatt taacactctc    420
ctctcaaaat gactacctct tcttcaattg cagtgaagag aaagtgattg ttgagccaaa    480
acctatcttc tgcgagaggt ttcctgatcg gtgcgactcg acatgtgata gtgctagtta    540
cctttgcagg cacttgccag gatgtggtgc tgcattagga ggacgttctt gctgctctta    600
cttcccaaaa gcgaccgaat ctttgaggct gatgctgaag tattgtgcta gttacactag    660
tatttattgg agaattaatg gtgcaaatgc tcctgatgat catgtacctg agtatggtat    720
tagagttgat tttgacattc cagtgactac agattgcctt caatgtcaag acatgaagaa    780
aggaggtgga agatgtggat ttgacacaca atcacagaat ttcctatgtc tgtgcaatca    840
gagatcaaat gtcacaacat attgcaatgg tatatcacag cagcagtagc catagcaagg    900
caggaataat tgcagggact gtaactggag tttcagctgc tggggcatta ggaattggtg    960
ctggtctatg gtattggaag aaagtgagag cctcagcacc agtaacatgt ggggttcaaa   1020
gcaatgagaa taggctcttt taagaacaaa tcaacaaaag tgaataccaa tcactttctc   1080
ttttctaatt tccctatttt ttgagttgcc tttcgcactt taataagcaa cttaatggat   1140
ctgttttctt tgttttaaa                                                1159
```

<210> 86
<211> 884
<212> DNA
<213> Populus trichocarpa

<400> 86

```
cattatccca aaaaacgagt aaggcatata cagcagccaa aaccatgtca cccggtcccg      60
tcgttgaggc tgaacccgcc gccgccggtg gaagatacac agcagaagaa gcctcctcac     120
aggacgtgga tgaacctgtg gtggaggacg tgaaagaaga tgagaaggaa gaggacgatg     180
atgatgatga tgaggacgat gatgatgaag atgatgacaa ggatgatgat accccaggtg     240
ctaatgggag ttccaagcag agcagaagtg aaaagaagag tcgcaaggca atgttgaagc     300
ttggcatgaa acctgttact ggtgttagca gggtcaccat caagagaacc aaaaatatac     360
tgttttttat ctcaaagcct gatgtcttca agagcccaaa ttctgagacc tatatcatat     420
ttggagaggc aaagatagag gatttgagct ctcagctgca gacacaggct gctcagcagt     480

ttagggtgcc agacatgtca tctatgttac caaaaccaga tgcttctact gcagctgctg     540
ctgcaccagc agatgaagaa gaggaagaag tcgatgagac aggggttgag cctagggaca     600
ttgatcttgt tatgacacag gctggagttt ctaggagcaa ggctgtcaag gctctccaga     660
cgaataatgg ggacattgtc agtgctatca tggagcttac tacttaggat ggctccctgg     720
ttactctctt attttatgct gacaagttct cggaacaatt taatcatggt agtcaaattg     780
gcttgccatc tatgaggtcg ctaattatcc attgtttgtg tcaaatctga gatttttacc     840
tattgcggtt tttctttagt agcaggctct tattgtgctc tttg                      884
```

<210> 87
<211> 2371
<212> DNA
<213> Populus trichocarpa

<400> 87

```
atgccattga atagccaaat ctctctctct cttagaatgt gaaggagcag ttagcgaagg     60
aaaaattgga gaatgccgga gctggtgtac caagagcaga gctcgtcgag atcaggattt    120
agagctcgag atgcaagtcc tgactctgta attttcactc tagagtccaa cttcagtctc    180
ttctcttctg cttctgctag cgtcgatcgc tgctcttttg cttccgatgc tcatgatcat    240
gactctctcg cctctgaaat ctctctggta aaaccatttc tctaacttcc ctgcatgcat    300
gcgttttcta acccatggtt tttgagtgag ttttttgaga gtgcaatgca gcatttggca    360
gctggtcatg atcaacaaga gaactcttcg agtggtccag atcgaaacaa caacaagcag    420
aagcagcaca ctcacacccg tctctccaga aaagcagaaa aagttaaagc tgttaaaaaa    480
gaagacaaca gaattagttc tgtagaagac gatattcatc tcctagattc tgcaagaagc    540
tccttctctc tcgctctcaa agagtgtcag gagaggagac cgagatctga agcgatcaca    600
aagaaaccag acaggcggag gcctgcgtca ctagatctta acaatgtggt cacatcttct    660
tcgccgagat tgggtaacat gaagaagagt atagcttgtt catctcgaaa atctggtaca    720
tttccgagtc ctgggacccc aaattacctt aatcagtatc attctagtgt tggaatgcag    780
aagggttgga gttcagagag agtgccgttg ccaaataata gtaataggcg gcaagtgatg    840
aatactactg gagctgctgt tttgccttat aataataatg gaaggacatt gccgtccaaa    900
tgggaggatg ctgagaggtg gatatttagt cctgtttcgg gagatggggt tgttaggagt    960
tcgattcagc ctgcacagag gaggcctaag tcaaagagtg gaccacttgg accaccggga   1020
gttgcgtatt attccttgta ttcgcctggg atgcaggtgt tcgatggagg gaatgcgggg   1080
aattttgttg ctggttctcc gttttcagct ggtgttattg ctgctgatgg attgggtatt   1140
aggtcacatg ggagccatgg tgtgtcattt cccatgcgga cagagccctg tatggctcgt   1200
tcggttagtg tgcatggttg ttctgagatg gtagctcagt cttcgttgcc atcacaagat   1260
gaaaagcttg atggtgtcaa ggatgctgca actgatatct cccgtgttgt ttcaagaagg   1320
```

gacatggcca cccaaatgag ccctgtaggt agtaatcatt catctcccac caggaagcca 1380
tcattctcca cctccacccc ctctgtccta cctattgtgg aactgcagag cgtaccttcc 1440
tctagatctg agaccaggga tgtacaggtg gatgagaggg ttactgtcac aaggtggtct 1500
aagaaacaca gagcccgcaa ccatggaaaa agttcacaag ttgttgatga ctggagaaag 1560
aaagctgctg atactctttc atcaggttgg gatgtttcag aggctggaaa gagcatttca 1620
aaggtcaaaa gagaggaagc taaaatcaca gcatgggaga acctgcagaa ggcaaaagct 1680
gaggcagaaa taaggaaact tgaggttctt tttccccacg cgttttcgtc gtacaatttt 1740
gactaacttg aaggattaat tcacttaatg atcagccttg gtgcagcaaa gaagatattt 1800
tctctaatat ttcctttctg ctcacttgaa agctttgcta gtacttaatt ataagatact 1860
ggtcttaact gactagattc attgcagatg aagcttgaga aaaagagatc atcatctatg 1920
gatagaatta tgaataagct gagatcagct caaaagagag cccaggaaat gaggagctcg 1980
gtgctagcaa accaggcaca tcaagtttcc acgaactctc acaaagttat atcattccgc 2040
agaacccgtc agaagggttc cctgagtggt tgtttcacat gccatgcttt ctgatatctc 2100
atccagccac atgttggtac acttcgatag atataaaacc tggtcaaatt gcaggaaaaa 2160
ctgcagcaga atatggaagc gtataggagg gaaaccacca ttgacactat tttggtgtca 2220
atggagttga gttggggcgt gaagaacaag tactactgtt aagatttcag acgggacaca 2280
agaccctgga tggtatcaaa atctccagga aatttgtcta ctaaaccttg tatgcatgta 2340
tgtatgtatg tatatcaaca attattatta t 2371

<210> 88
<211> 2514
<212> DNA
<213> Populus trichocarpa

<400> 88

```
tttttttta aacctcctcc tcggatatta tatacttcgt tttctctctt ctctgccatt      60
aatcatacct cacaccgatc cagattttgt ttgctcataa acagagggag tgggagagac     120
ccccgtttaa cttttctttt tttcctttg aagtaatacg acatctccct ctcctcctcc      180
tcctcctcct cctcctctat ttgttcttga tttcgattat ctgatcggac ggtcacggtc     240
ttatttgtat tcacctgcaa tatcgtacgg ccagggatct ctcttctctt cttactgagc     300
aagttgcatc tcataagcac tggcgatcat ggagctgaat ggtcaaacaa gagtgagaag     360
aaaggaccat tttgctcata caaacggtga tttagcatta ccaagtgttg gtgatgtaga     420
tccctggact gcatgggcat ataagcctcg aactatttcg ttgttactta ttggtgcttg     480
ctttctaata tgggcaagtg gagccctaga tccagagagc tgcacatctg gtgatgttgt     540
tacatctgtg aaaaggggta tatgggcaat gactgcagtt tttcttggtt attgcttgct     600
acaggcccct tcaacggttc taataaggcc acatccagca atttggcgct tagttcatgg     660
```

```
attggccatt gtttatcttg ttgccctcac atttctgctt tttcagaagc gtgatgatgc    720
gcggcaattt atgaagtttc tccatcctga ccttggaatt gaactacctg aaagatcata    780
tggtgctgat tgtcgcattt atgtgcctga aaatcctaca agcaagttta agaacgtttt    840
ggacactctt tttgatgaat ttgttctagc acatatcttt ggatggtggg gcaaggctat    900
attaatccgt aatcagccac ttctatgggt attgtcaatt ggttttgagc tgatggagtt    960
taccttccgc cacatgctac caaacttcaa tgaatgctgg tgggacagta tcattcttga   1020
tattttgata tgcaattggt ttggcatttg ggctggaatg catacagtcg ggtattttga   1080
tggaaaaaca tacgagtggg ttggtataag tcgccaacct aatattatga gcaaggtcaa   1140
acgaacattg gaacaattta cacctgcaca gtgggacaaa gatgaatggc atcccttgct   1200
tggtccatgg cgatttatcc aagttcttag tctctgtatt gtcttcctga ctgtagagct   1260
caacacattc tttttgaagt tttgtctatg ggttcctcct cggaaccctg taatagtgta   1320
caggttgatc ttgtggtggc taattgccat acctacaaca cgtgaataca attcatatct   1380
ccaagaccga aagcctgtga aaaaggtagg cgctttttgt tggctttccc ttgctatttg   1440
catcgtggaa cttctcattt gcatcaagtt tggacatggt ctttatccca aaccaatgcc   1500
tgcatggttg gtcatcttct ggacatctgt tggagttagc cttgtaatat tcctgattat   1560
gtggtcatgg aaaagtttgg gaagaaagag acgatgaatt tgctagcatg gtacactatt   1620
cttttatttc ttcatgttct tatgggtata catcttgtaa atgccctata gcttcatgga   1680
attgtaaatg cttattctct taggttagtg gatcacagtt ttagagtatt caattctttt   1740
atgataggta gttggaaaat gcctctaatg gtaggttcat gtgttgctaa tttgcagcaa   1800
tcatcaaatt ggtaattttc ccttttaact tgtctgagag aggagcaatt tttaacaccg   1860
ggcttgcctc acatttttca ttggaaaatg catgcctgac aattccattg gggcatttta   1920
tcagaatgtt taaaatattt atattttgcg tcggctctat agcatatgct tataagcatt   1980
gaaatatgat tcaactgaag tattcgcaca tgttttgctg tttacgttta tatgattgtt   2040
gtgtttagtt cgccacaagc agcctggcag ttattcgatc cggtatcttg acgttatctt   2100
atcacctgct gtaaatccta tggctcaaac tggcaattat tcaaggcagt tcttgaaaga   2160
gttcaaggtt ttctttcctg ttgatcgacc tttgtaaagc ctatgaagca attgtgcaaa   2220
taattggtgc tcgacagaag gcaatctcta gtttgctcac gacaattcta gccgattttc   2280
gggaaaacat ttgtggactg gagtcattga ttcaatcaag gtgaaattgg aaatcattta   2340
gaaaagcaat tcctgttctt tgacaacact aggttcttct ttctcgatcc gtgcccatgt   2400
cactgtgtgt atatatagaa atagaaaaag tgtatctctc ttaatcatgc aggattttgt   2460
tttttgcagt caatatgccc atgacttata atctcagtct tcttcgaaaa cact         2514
```

<210> 89
<211> 852
<212> DNA
<213> Populus trichocarpa

<400> 89

```
acgaacaagc aatgatagag ttcgagaggt catcttcttg ttataaaatc ctcaaaagct     60
cgaggtgaaa aaataagcaa gggagaggag agagctagct agctagtcaa gttaagaggc    120
agaaaacaag aatgtcttcc aggaagaaaa agaaggcagc tctttatgag aagttacgtg    180
ctgctaccaa ttctaatgct atgaacaaaa cctcaatcat agtagacgcg tcaaaatata    240
ttggagagtt gaagaacaag gtggataggc taaaaaaaga aatcggaaca tcttcaaccc    300
cccaaaattc attacctgcg caggtcacag tggaaaacct agaaaagggt ttccttatta    360
atgtattttc aggaaagaat tgccctggat tacttgtctc catacttgaa gccttcgagg    420
aactaggcct tgatgtgctt gatgctaggg tttcttgtga agacaatttc caacttgaag    480
cgattggtgg agaccaaaac caaggccatg atgctcaagt agtgaaacag gcagtgctgc    540
aagctatcca taactggaat gaaggcagct agctagtgag caagaatgac ccaaatattt    600
tcttccccta tcttcctgta cgctactgag aaattgggaa tttgtatatt gaatttcaag    660
cacactaggc agtcctaatt ccaagttagt agtcagtcaa aatgtgcagc aatgggattc    720
tggtaattaa ttttcagttt ttaattaagc aacttttacc ttcgatgatc tatacatata    780
tttgagcata aaatgcttaa ttaaaatccg tatgattggt ttcagctaat taaggagctc    840
tttggcaaga tc                                                        852
```

<210> 90
<211> 1320
<212> DNA
<213> Populus trichocarpa

<400> 90

```
agagttccat catccattgt cttaccactt catgggaatg tctatcctac tgggttctat      60
aatgtgaccc tcaacattgg ccaaccatca aaaccttact ttctcgatgt agatactgga     120
agtgatctta catggctcca atgtgatgtt ccccgtgccc aatgcacaga ggcaccgcat     180
ccatattaca aacccagtaa caatctagtg gcttgtaagg accccatttg tcaatccttg     240
cacaccggtg gtgaccaaag atgtgaaaac ccaggacagt gtgactatga ggttgagtat     300
gctgatggtg gatcatccct tggcgtcctt gtcaaggatg cctttaatct caactttaca     360
agtgaaaagc gccagagtcc tcttctggcc cttgggttgt gtggatatga tcagcttcct     420
ggtgggactt atcatcccat agacggagtg cttggtcttg gccggggaaa acctagcatt     480
gtatcacagc ttagtggtct gggtttagtg agaaatgtga ttggacactg tttgagtggg     540
cgcggtggag gattcctctt cttcggggat gatctttatg attcatctcg tgtagcttgg     600
acaccaatgt cacccaatgc gaaacactat tcacctggat ttgctgaatt gacttttgat     660
gggaaaacta ctgggttcaa aaacctgatc gtagcttttg atagtggggc ctcttatact     720
taccttaatt ctcaggtgta tcaaggtcta atttctctga taaagagaga attatccacg     780
aagcctttga gagaagcact ggatgatcag acgcttccga tttgctggaa aggacggaaa     840
cctttcaaaa gcgtacgtga tgtcaagaaa tacttcaaga cctttgcatt gagctttgcg     900
aatgacggaa aatctaaaac tcagcttgaa ttcccaccag aagcttatct tatagtatca     960
tctaagggaa acgcctgctt gggagttcta aatggtacag aagtaggtct gaatgatctg    1020
aatgtcattg gagacatatc aatgcaagac agagtggtga tttatgacaa tgagaagcaa    1080
ctgattggat gggcacctcg aaattgcgac aggattccta aatccagaag cattatcatt    1140
tgatgacagg tgttctaaac gttaaaaaaa acttgtcctg tacagaaagc tgctaagctg    1200
taacattgta ttcttccacc attacagcag attccttgta aagtaatact atgtatatgt    1260
ggtctggtga atcagacccc acaattatac ttgatgggtt atgaaaattt aataaaataa    1320
```

<210> 91
<211> 2811
<212> DNA
<213> Populus trichocarpa

<400> 91

```
atggaaatgt cctgcaagga tggtaagcag cctatcatgg acaatggcaa gtatgtccgg      60
tacacgcctg agcaggtcga agccctggaa aggctctatc atgattgtcc caaacccagc     120
tccattcgcc gccagcagct cattagggag tgtccaattc tctccaatat tgagcccaaa     180
caaatcaaag tttggttcca aaatagaaga tgtcgagaga aacagaggaa agaagcgtct     240
cgcctccagg cggtgaatag gaagctgagt gcaatgaaca agcttctgat ggaagagaat     300
gataggttgc agaagcaggt gtcgcagctg gtgtatgaga atggctactt tcgccaacat     360
acccataaca caccgcttgc aaccaaagat acaagctgtg aatcagtggt gacaagcggt     420
caacaccacc tgacacctca gcatccgcca agggatgcta gtcctgcagg gcttttgtcc     480
attgcagaag agactttaac agagtttctt tcgaaggcta ctggaactgc tgtagagtgg     540
gtccaaatgc ctggaatgaa gcctggtccg gattccagtg gaatcgttgc tatttctcat     600
ggttgtgctg gtgtgggagc acgagcttgt ggcctagtgg gtcttgaacc tacaagggtt     660
gctgaaatcc tcaaggatcg gccatcatgg tttcgtgatt gccgagctgt ggatgtgctt     720
aatgtgctgc ccaccgcaaa tggtggaacc attgagctgc tttatatgca gctctatgcg     780
ccaactacgt tggcacctgg tcgtgacttc tggttgttgc gttatacttc tgttttagaa     840
gatggaagcc ttgtggtttg tgagagatct ctgaaaaata ctcaaaatgg cccgagcatg     900
ccaccagtgc agcattttgt gagagcagaa atgctcccaa gtgggtatct ggtacggcct     960
tgtgaaggtg gtggttcaat catacacata gttgaccaca tggatttgga gccttggagt    1020
gtgcctgaag tactacggcc gctgtatgaa tcctcaactg tacttgctca aaagacaaca    1080
atggtggctc tacgccagct gcggcagata gctcaggaag cttctcagtc caatgtgacc    1140
```

aactggggca gacgacctgc agctctacga gcactgagcc agaggttgag caggggtttt 1200
aatgaggctc tcaatggatt tagtgatgag ggatggtcaa tgatcggaaa tgatggcatg 1260
gatgatgtta ctatcctcgt gaactcatct cctgacaagt tgatgggttc aaatctttcc 1320
ttcactaatg ggtttccagc tgtcagcagt gctgtcctgt gtgctaaagc atcgatgctt 1380
ttacagaatg tccctcctgc aatccttctc agattcttgc gagagcacag gtcagaatgg 1440
gcagataaca atattgatgc ctatgcagct gcagcagtta aagttggtcc ttttagccta 1500
caaggttctc gagttggaag tttcgggggt caagttatac ttccgctggc tcacactatt 1560
gaacatgaag agttcctgga ggtcataaaa ctggaaggtg ttggccattc tcctgaagat 1620
ccaataatgc ccagagatgt gtttctttta caactctgct gtggaatgga cgagaatgct 1680
gttggaacat gtgctgaact tatatttgct cccattgatg ctacttttgc tgatgatgca 1740
ccgcttttac cttctggttt tcgcatcatt ccccttgatt ctgggaagga agcctccagt 1800
ccaaatcgta ccttagatct tgcggctgct cttgaagtcg gaccagctgg aaacagagca 1860
tccagtgatc attctgctaa ttctggttgc acaagatctg taatgacaat cgcatttgaa 1920
tttgcgtttg agagccacat gcaagaacat gtagcatcaa tgactcggca atatatccgc 1980
agcattatat cttcagttca gagggtggca ttagcactat ctcctcatct tggttcacag 2040
gctggtcttc ggtcaccact gggtactcct gaagcacaga cacttgctcg ttggatctgc 2100
cagagctaca ggagctattt gggtgtggag ctactcaaat ccaatggcga aggaagtgaa 2160
tctattctga aaaccttgtg gcatcattca gatgctatta tgtgctgctc actgaaggcg 2220
ttgcccatct ttacttttgc aaaccaagct ggacttgaca tgcttgagac aaccttagtt 2280
gcactgcaag acataacttt ggaaaagata tttgatgatc atggaagaaa aactctctgc 2340
tcagaattct cacagattat gcaacagggt tttacttgtc ttcaaggtgg tatctgtttg 2400
tcaagcatgg gcagaccagt ttcatatgaa agagctgtgg cctggaaagt gttgaatgaa 2460
gaagaaaatg cgcattgcat ctgctttatg tttataaact ggtcttttgt ctgagacttg 2520
aaaccagaat ctagtggagt ggctgattta agagtgagaa ctctatatct gtcttatttt 2580
gctggcttgt atttctttta tgtataacat gttgcaaccg tggagcacct agttcatttg 2640
ctatgttata aatttgtgtg gggttcatta tagtagagat tatggacgag acgatatagt 2700
tgccggggat cattttgttt ctgactggtt tgttcttaag tcctcacctg ggtctatgtt 2760
gattattgca accgaaatct gtcaatcctg gtttttcctg tgttgcttac a 2811

<210> 92
<211> 886
<212> DNA
<213> Populus trichocarpa

<400> 92

gttcagggaa tcagcctagc caagaagaga ttacaacgcc agaagctaag tcacactgat 60

cagagcaaca aaaaaagtac accaacaatg tcttccaccc caacaaatga atggcaaacc 120
tactggtgtc atgaatgtga cctcagcatc caccttctca ccaccaccac tcctctttgt 180
cctcactgcc accatgactt tcttgaactc atggacccca ttcccacctc caccgccgct 240
gacaccacca ctttcctcct tgacagcccc tccttcctca acttccttca acatctcaac 300
accaatagcc attgcgattg tgaagatgat aatataaacg ccactattga ctctatcatc 360
cccaccataa aaatcacttc ttgcatgcta gaaatggatc caatgcttgt gtgtgcagtg 420
tgtaaagatc agttcttgat tgatgttgag gccaagcagc tgccctgcag tcacttgtac 480
catccaggct gcattctccc ctggctttct aaccacaact cttgtcctct ctgtcgcttc 540
cagttacaaa cgccagtagt cagagaggag aatttggaaa attggtctcc tgatcatcct 600
catcatgatg ctaatcatgc tcatgtcggg gttttgtcca cctctcttcc tccacacttt 660
tgaatttgaa tgcaggtaaa taaacgcttc tggggatttt gggtttttgg tttggaattg 720
atcgagtgaa gtgtaaatca acagtgaacg attgtgttac acaaagaact tgtgatggtt 780
gtgcatctcc ttctcccctt cagactttca ctatggatat tgtaaagtaa taccttgttt 840
gggattggat tttggccaga aatacagcgt ggttgaattg attttt 886

<210> 93
<211> 887
<212> DNA
<213> Populus trichocarpa

<400> 93

```
gacccccttc aatcgattgc aaggcatgaa gcgtcagctc cagcattgac atcccttctt     60
ccacagaaat tcctgctaag caagcgcaga aagtccagtt ctgacccgat gatgaacttt    120
gttagggaat atcataatga tttggaagtt gtgtatgttg ggcaactctg cctttcatgg    180
gaaatccttc attggcagta tgaaaaggca ctcgagctat gggattccga cccttatggg    240
atgcgacagt acaatgaagt tgctggtgaa tttcaacagt ttcaagtgat attgcaaaga    300
tttatagaga atgaaccttt tgaaggtcca cgggtgaaaa attacattaa gaatcgatac    360
gtgttgcgta atctccttca agttccggta ataaaagagg acagtatgaa ggataaaaag    420
gcaagacgaa aaggaaggga tgatggttct attacaagtg atatgctagt agagataatg    480
gaagaatcaa taaggatttt ttggcgattt gttcgatctg ataaagatgc acaaaacgtg    540
atttcaaagg gtcgcaaagg aactcaaata gagccccaag atcccactga actagagtta    600
ttgacagagg tgcgaacgaa tttccaaaag aaggagagga ggcttaaaga cgtcttgaga    660
agtggaaact gcatactgaa gaagttccaa aaacatcgag aggacaattc taatcaagtt    720
ctttacttct tctctcaagt ggatatgaag ttagtagcga gggttctgag catgtccaga    780
gtaacaacag accagctact atggtgtcac aataaattaa gcaagataaa ttttgttagc    840
cggaagatac acgtagagcc atcattttg cttttccat catgatg                    887
```

<210> 94
<211> 1471
<212> DNA
<213> Populus tremula x tremuloides

<400> 94

```
ccccatttcg gtttttagtc gaaatcttgg gttgggggct ggaaatatga ggacatgctt      60
gtgggttttt gctttggttc ttgttttcgg ttttgttgat ggaaaaaaaa ttgaaaggtt     120
gcgcacagag aggatttcag gaagcgctgg ggatgtgttg gatgatgatc cagtgggaag     180
gttgaaggtc tatgtttatg agcttccgag taaatacaac aagaaactgc tgcagaagga     240
ccccagatgt ctcacccata tgtttgctgc agaaatcttc atgcatagat ttctcttatc     300
cagcccagtt cgaaccctta atccagatga agcagattgg ttttattccc ctatataccc     360
cacttgtgat ctcacaccca tgggcttgcc gttgcccttc aaatctccta ggatgatgag     420
aagtgcgata cagctaatct cctccaattg gccttactgg aatcgtacgg aaggggctga     480
tcactttttt gttgtgcccc atgactttgg agcctgcttc cactatcagg aagagaaagc     540
cgttgagcgg ggcattcttc cgctactcca gcgttctact ttggttcaaa cttttgggca     600
acgaaatcat gtgtgcttga atgaggggtc aattacaatt cctccttttg ctcctcctca     660
aaagatgcag gcccaccaga tccccccctga cattccacgg tccatttttg tctatttccg     720
tggattgttt tatgatgtaa ataatgatcc agaaggtggt tattatgcaa gaggagcaag     780
ggctgcagtt tgggagaatt tcaagaacaa tccactcttc gacatctcca ctgaccatcc     840
aacaacatat tatgaagaca tgcagcgagc tatattttgc ttgtgcccac tcggttgggc     900
tccatggagc cctagattag ttgaagcagt ggtatttgga tgcattcctg tcatcatagc     960
agatgatatt gttttgccat ttgccgatgc tatcccatgg gaggaaattg gggtgtttgt    1020
agcagaggaa gatgtcccta acctggacac aatcctaaca tccataccac cagaagtgat    1080
tttaaggaaa caaaggcttc ttgcaaatcc ttctatgaaa cgtgcaatgt tattcccaca    1140
acctgcacaa ccaggtgatg ctttccacca aatcctaaac gggctggctc gtaagttgcc    1200
gcacgacagg agtgtttact tgaagtctgg tcagaatatt ttgaattgga cagcaggacc    1260
agttggggac ctgaaacctt ggtaagagaa gtgtcatttc ctcaacagac atggctgtgg    1320
tgtatctact gtttcatcta atcgcaagga ctgtaaactt ctctttgaag ttttgatttt    1380
tgtatagatt cttgaatttc acgtgtacat ctgggacata gttcgatgct ttaaataact    1440
tcgcattttt cttggtaaaa aaaaaaaaaa a                                   1471
```

<210> 95
<211> 1351
<212> DNA
<213> Populus tremula x tremuloides

<400> 95

cagcttcatt gccttgccct gaggctagcc agtgagcact ccaccaatgc agctgctcga 60
ctgcagctcc ccttgccgga actcgtccct gcgctcgttg acaatactta ttttcacttt 120
gtcctggctt ccgacaatgt gctcgccgct gctgttgttg ccaattcact tgtccaaaat 180
gcgctgcgcc cccagaagtt tgtgttacac ataataacag ataggaagac ctactcaccg 240
atgcaggcat ggttctcact gcaccctctg gctccggcaa taattgaggt caaggcattg 300
catcatttcg attggttcgc aaaggggaag gtgccagtca tggaagcaat ggaaaaggat 360
cagagggtga ggtcacagtt tagagggggt tcgtctgcca ttgtagcaaa taacactgag 420
aagcctcatg ttatcgcagc aaaactacaa acacttagtc ccaaatacaa ttcagtgatg 480
aaccacatcc gaattcatct acctgagctg ttcccaagcc taaacaaggt tgtgttccta 540
gacgatgaca tagtggtgca gtccgatctt tcgccgctct gggacatcga catggatggg 600
aaggttaacg gggcagtaga aacctgtcga ggagaagaca agttcgtcat gtcaaagaag 660
ttgaagagct atttgaactt ctctcatcct ttgatatcag aaaatttcaa acccaacgaa 720
tgtgcctggg cttatggaat gaacatattt gatttagagg cttggaggaa aaccaacata 780
agcacaacat accatcactg ggttgaagag aacttgaaat cagacctgag cttgtggcag 840
ctaggaacac tgcctccagg tctgatcgca ttccatggcc acgtccacgt tatcgatccc 900
ttttggcaca tgttgggtct tggctaccaa gaaaatacaa gtttggcaga tgctgagact 960
gccggagtca tccatttcaa cggtcgagca aagccttggc tagatatagc attccctcag 1020
ctccgccccc tgtgggcgaa gtacatcaac ttttccgaca agttcataaa gggctgtcat 1080
attaggccat cttaacttga gttttgagat gaaaatgttg gctgcgatcg tgattaatat 1140
atgtctatat ataaggagaa aaaaagcaga gaagagagag ttcttcacaa atccaaggcc 1200
ttgaatccag agagtaaaat attcataacc aagcatttgt ttgcttggtt tttttagaat 1260
tcatcctctg tgggcgaaca acccttccct attacttcaa atcttgattc aattttaatg 1320
agaaattaag catgttatgg attttcaacg g 1351

<210> 96
<211> 1507
<212> DNA
<213> Populus tremula x tremuloides

<220>
<221> misc_feature
<222> (597)..(597)
<223> n is a, c, g, or t

<400> 96

```
aagctgatga tcaaatgagg acatcaaaaa tttcttcagg gaattcaggc taccatgtat      60
ggattcttgc ttttgcaatg agtctactga tactgattgc cttgtcgaag tcatggtttt     120
atgatcacgc ttctgctgct gcaagtgagg atttgcagta cttttcagtg atcgtccctt     180

caaaagggcg ggactatcct cctgttcttg cttattggat atgtggtacc agtggagatg     240
ggaagagaat gttaaggcta ttaaaagcaa tttatcatcc aaggaaccaa tatctattgc     300
aacttgatgc ggaatcctca gattatgaaa gggcggagtt ggttgtttca gttcaatctg     360
aaagtttgtt tcaagcattc ggtaatgtta atgttgttgg aaaaggtttt gctatcaacg     420
aaatgggatc ctctgctctt gctgccatac tcaacgctgc tgcgttgctt cttaagctga     480
gtacagatgg ggactggttc atcaatttaa gtgtctcaga ctatcctcta gtgagtcaag     540
atgatctcct ccatgccttc acttccttgc caagagatct caacttcatc aactatncta     600
atgacactgc gaaaaacgag atacacaaga ttaaccaaat tgtagtagat cccagcttgc     660
atcttcaaaa gaggagccac ctttattacg ctgttgagac tcgaacaaca cctgatgctt     720
tcaagatatt tggaggttcg ccctggctga ttcttacaag agctttcatg gagtactgtg     780
tccaaggatg ggacaacctt ccaagaaaac tactaatgta cttcagcaac acggcatccc     840
cacttgaatc gtatttccac tctgtcctct gcaactctcc tgagtttcaa aacacaacag     900
taagcgatga tttaaggtac aatattctgg aaactactac agatggggaa tcaccttatg     960
acaaaatgct aaatggtgga gcagcatttg caaggccatt taaagaagat gctgctgctc    1020
taaacatgat agatgagaac gtcttgaacc gtgaacccaa cggattggtg cctggaaaat    1080
ggtgcttaga ccagggtatg aacaagagct cagaggcatc aaagcctcca ggggaggatt    1140
tgtgttcaac ctggggtaac attaatgatg tcaagccagg atcttatggt atcaagcttg    1200
catttttatt gtctaagatt gccagtgaag agaaattgac aactagtcaa tgccttcaag    1260
ctacaaaaat ggggtcatca tagaaccaag catacatcct tgtgtggata attttttttct    1320
tttaatcgaa aactataggt taaatcaagc ttcttagcca tataaatcct ttgctaaatg    1380
cactacaaat gcatacttgc ttgttaaaac aaaagctaga agcacccaga gatatattgt    1440
acatacattc acattttatc gaggatttct acaataaatt tattgttttt cccccaaaaa    1500
aaaaaaa                                                               1507
```

<210> 97
<211> 1903
<212> DNA
<213> Populus tremula x tremuloides

<400> 97

```
ttttggggta tcctgatacc ggaggccagg ttgtttacat tttagatcaa gttcgtgcct      60
tggagaatga aatgcttctg cgtatcaagc agcaaggact tgatatcatc ccccgaattc     120
tcattattac tcgactgctc cctgatgcag taggaaccac ttgtggacaa cgtctggaga     180
aagtctatgg atcggagcat tgtgatattc ttcgagttcc cttcagagat gaaaagggaa     240
tggtccggaa atggatctct cgctttgaag tttggccata cctagaaact tacactgagg     300
atgttgctgc tgaaattgct aaggagttgc agggaaagcc tgatctgatc attggaaatt     360
```

acagtgatgg aaacgttgtt gcctccttgc tagcacacaa attaggtgtt acagagtgca 420
ctattgcaca tgctctagaa aaaacaaagt acccagattc agatatttac tggaagaagt 480
ttgatgaaaa gtaccacttt tcatgccagt ttacagctga tctttttgcc atgaaccata 540
cagatttcat catcaccagc acattccaag agattgctgg aagcaaggat actgttggac 600
agtacgagag ccacacagct ttcactctcc ctggcctcta cagagttgtt catggtatcg 660
atgtatttga tcccaaattc aacattgtat cccctggtgc tgacgagagc atatacttcc 720
cttacactga ggagaaactt aggctgactt ctttccatga agaaattgaa gaacttcttt 780
acagccccgt tgagaacgac gagcacttat gtgtgctaaa agaccgcaac aagccaattc 840
tattcaccat ggcgaggctg gacagagtta agaatttaac tggtcttgta gaatggtatg 900
gaaagaatac caagctgcgc gagttagcta atcttgttgt agttggtggt gatagaagaa 960
aggagtctaa agatatagaa gagcaagctg agatgaagaa gatgtacagt catatagaga 1020
aatacaactt gaatggccag ttcagatgga tttcttctca gatgaaccgc gtgaggaatg 1080
gagagctcta ccgttacatt tgtgatacca agggagcttt tgtgcagcct gctttgtatg 1140
aggcttttgg attgactgtt gtggaggcca tgacatgtgg tttgccaacc tttgctactt 1200
gcaatggcgg tcctgctgag atcattgtgc atggaaaatc cggattccat attgatcctt 1260
accatggagt acaggctgct gaactccttg ttgacttctt tgagaagtgc aaggctgatc 1320
ccacttactg ggacaaaatc tcccagggag gcctgcagcg aatccaagag aagtatacct 1380
ggaaaattta ctctcaaagg ctcctgactc tcacaggagt ttatggcttc tggaagcacg 1440
tttccaacct tgatcaccgt gagagccgtc gctatctgga aatgttctat gcactgaaat 1500
atcgcaaatt ggctgattct gttcctttga ctatcgagta aatggaactg gagaaatcga 1560
ggaaacatgg gttggtttga gtcgggttcc gggtccagaa taatggtcat ttcacgatag 1620
tgattggaca agaaaggctt tgatcttctt tttgtttttt tgtttttcct ttctgtgtgc 1680
atcttttttg ttttcccttt tcttttcatc atgcagcctc ttcaatttca gtaaagattg 1740
tgtctgttga gttcatcatc ccttatctga tctctatcca tttcttgcaa attctctgtc 1800
tcttgagttg gaaatcatgg gaattgatcc aacatcaatt cctgtatttc tcctcagtac 1860
tcgagattgg aagttgatgg ctattcttca tcattatata aag 1903

<210> 98
<211> 2198
<212> DNA
<213> Populus tremula x tremuloides

<400> 98

gacaactaca actgctactt gtcggtgtgg gtttgctctt aactctttca ttctatctgc 60

gatcctctct ccctccccct tggcacttgt caagcagcag agaatgaagg ttattgataa 120

gataagggcg gcagcagcag caggagattc aaatggagga gatagcagca gcaataataa 180

```
ggttgttttc tccttcgaat tctttcctcc aaagaccgat gatggagtgg acaacctgtt    240
cgagagaatg gacaggatgg tctctcacaa tccttccttc tgtgacatca cttggggtgc    300
tggtggctcc accgcagatc tgaccttaga catcgccaac aagatgcaga acatcatctg    360
tgtcgagact atgatgcatc tcacctgcac caacatgccc gttgagaaga tcgatcacgc    420
cctcgaaacc atcaaatcca atggaatcca gaatgttctt gctcttcgtg gtgatcctcc    480
tcacggtcag gataaattcg tccagatcca aggcggcctc gcttgcgctc tcgacctcgt    540
caaacatatc agatccaaat atggtgatta cttcggcatt actgtcgctg gctatccaga    600
ggcacatcct gatgtgattg gaagtgatgg atttgctacg cctgaggatt accataaaga    660
tcttgcttat ttgaagcaga aggtggatgc tggggctgat ctaattgtta ctcaactgtt    720
ttatgatact gatattttct tgaaatttgt gaatgactgc cgccaaatcg gaatcacttg    780
tcccattgtc cctggtatta tgcccattaa taactacaag ggcttcatac gtatgactgg    840
tttttgcaaa accaaggtac ccgccgaggt tactgcagca ttggagccta tcaaggataa    900
tgaagaagcc gtcagagcct atgggattca ccttggaact gaaatgtgca agaagatttt    960
agctcatgga atcaagacat tgcatctcta tactctgaac atggagaaat ccgctttagc   1020
tatattaatg aatcttggtt tgattgaaga gtccaagatt acaaggccgt taccatggcg   1080
ccccccgaca aatgttttcc gtgtaaaaga agatgtccgt ccgatatttt gggctaatcg   1140
tccaaagagc tacctaacaa ggacaatagg ttgggaccag taccctcatg gccgatgggg   1200
tgattctcgc aatccatcct atggtgcttt atctgactat cagtttatgc gaccccgcgc   1260
acgtggcaag aaacttcttg aagagtgggc tgcccccttg aaaagtgttg aggatatata   1320
cgagaaattc aaggtatact gccttgggaa gttgaaaagt agtccttggt cagaactaga   1380
tggccttcag ccagagacta aaatcattaa tgaacagctg ggtaagatca acttgaaggg   1440
actcctgact atcaatagtc aaccagctgt caatggggaa aaatctgatt ctccatctgt   1500
tggttggggt gggccaggag ggtatgtata tcagaaggct tatctagagt tttctgctc    1560
caaggacaag ctgaatgctc ttgtggagaa gtgcaagtct ttccctttg taacttacat    1620
tgctgtgaac aaaggaggga gttggatttc taatgttgcc ctgactgatg tgaatgctgt   1680
aacctgggga gtcttcccag caaaggagat tatccaacca actgttgtgg atcccaccag   1740
cttcagtgtg tggaaggatg aggcatttga aatctggtca agaggatggg cttccttgta   1800
cccagagggt gacccatcca gaaccctact tgaagaggtg aagagcagct actttttggt   1860
cagcttggta gataatgatt acatccatgg ggatattttc gctgtcttcg ccgatttatg   1920
atgcaagggg ctctttgcaa cccctgcatt tgctaagagt atccaagtcc ccagaaaaat   1980
ggtggtgctt taccctaagt tgaatcccag ctttaatcat cttatcggca tacattgttt   2040
ttgaaattat ttccgtattg aaatgtaata attcattgtg attttgctac tcccctctcc   2100
attatatgct gcaaatttgt atttggagtg gcaactaatt ctaagcttta agaaaatatg   2160
```

attgcctctt tttcttggta cttccgaaaa aaaaaaaa 2198

<210> 99
<211> 1548
<212> DNA
<213> Populus tremula x tremuloides

<220>
<221> misc_feature
<222> (1266)..(1266)
<223> n is a, c, g, or t

<400> 99

```
gtggaccatc tgcatccgtt tcagatggtg gtgaatcttc atcttttgaa ctccaagaga     60
ggattggtga ctctatccgt gatgaaattc ctaggccttc tcaaaatcct ggtaggcaga    120
agcataagag tcatgatcct gctcgagatc ttacgatcca tgtattggaa aaattctctc    180
ttgtgaccaa atttgctcgg gacacaagtt cacaactatt tcgagaaagt aatagcaatg    240
gctatggtgc tgtagaaagg aaaagttcta gctactctct ccctgatgtc ccccataagc    300
catccatgga tgcagagatg gctcccgagg aaggtcctgt tccctcagat cctctggagt    360
tcgataaaat gacactcgta tggggaaaac cacgacagcc acccttgggg tcatgaagag    420
tgggctactt tcttggattc tgaagggcga gtcatggatt caaaagcctt aaaaaagaga    480
atattctatg gtggagttga gcacacaaca cgcagagagg tctggccatt tttgctggga    540
tatcatgctt atgattcaac gtatgcggag agggaatacc tcaagtcttc caaaaagtca    600
gagtatgaga cagtaagaca acaatggcag agtatctcta ctgaacaagc aaagagattt    660
acaaaattta gggaaaggaa gggccttata gacaaagatg tggtgaggac tgatagagca    720
ctgtctttct atgatgggga tgataatcca aatgtgaata tcttacgtga tattctgttg    780
acgtactcct tctataactt tgatcttggt tactgtcagg gtatgagtga tttgctatcc    840
ccaattttgt ttgtgatgga ggatgaatca gaatcgtttt ggtgttttgt ggcactgatg    900
gaacgtcttg gacccaattt taatcgtgat caaaatggca tgcactctca gctttttgca    960
ttatccaagc tggtggagtt gcttgattgc ccattacata actatttcaa gcagaatgac   1020
tgcttgaatt acttcttttg tttccgctgg gttctaatac aatttaaaag ggaatttgaa   1080
tacaagaaaa caatgcgatt gtgggaagtg ttgtggaccc attatttgtc tgagcatctg   1140
caactgtatg tatgtgttgc aatcttgaag cgataccgca acaaaataat gggggagcgc   1200
atggactttg acacactctt gaaatttatc aacgagctga gtggccatat tgaccttgac   1260
gcaatnctta gagatgcaga ggctctgtgc atatgtgctg gtgagaatgg tgctgctcgc   1320
atcccaccag gaactccacc ttcattgcct actgagaatg agaatgcttt attatatgct   1380
caagatgatg aagtactgta atacagttcg tctcattggt acgatataat tggctttata   1440
ttttgttttc ttcatgaaat agctgtgtat aattgctgtt ttttcgaaat aaaatgttaa   1500

taatcaagca atcaactcca tttgctgacc tccttgaaaa aaaaaaa                  1548
```

<210> 100
<211> 648
<212> DNA
<213> Populus tremula x tremuloides

<220>

<221> misc_feature
<222> (579)..(579)
<223> n is a, c, g, or t

<400> 100

```
gacatcctgt gtgctccaag aatataaaaa taatactagg tgctctctaa tggctgcaaa      60
accaacaaca gcgttccttt tctcttctat attttaaaga aaaaacacct tctttcaaca     120
accctttgat ggattccatc agaattagca tctcttcaat cctttcttgg attcccttaa     180
aacatgttct ttgatagcca aatcaccaaa gttgaacaga aaaggaaaat cttgttgagt     240
tcttgagcta aacgagagga ggaggagaat tgtttttcgt ttttcagata tggaggagag     300
gcatgttatt tttgggaagt atgaaatggg gaggctttta gggaagggaa cttttgctaa     360
agtttactat gggaaacact tggtgacagg agagagtgtg gcaatcaaag tcataagcaa     420
agatcaagtc aagaaagaag ggatgatgga gcaaatccag agagaaatct cagtcatgcg     480
tcttgttcgt catcccaaca ttgtagagct caaggaagtc atggctacca agactaaaat     540
cttcttcatc atggagtatg tccgaggagg agagttgtnt gccaaagtag ccaaaggaag     600
gctgaaagaa gaagctgctc gaaaatattt ccagcaacta atcagcgc                  648
```

<210> 101
<211> 1030
<212> DNA
<213> Populus tremula x tremuloides

<400> 101

```
tgatgaagat gagcaacttg aaaatctgct gattttggct tgtcaccctt gccggaacag      60
tctccggcaa cgacgggctt ttgtactact cagtgcggca actcctgctt atgttgcccc     120
ggaagtcttg agaaagaaag gctatgatgg catcaaaagc tgatatatgg tcatgtggag     180
tgattcttta tgttcttctt gcagggttct tgccatttca agacgagaat gttatgaaga     240
tgtacaggaa agttttcaag gctgaatatc agttcccccc ttggtttcaa cagattccaa     300
gaggttgatt tcaagacttc ttgttgctga tcctgaaaag agaatcacaa ttcctgccat     360
aatgagaaat cattggtttc ttaaagggtt ttcgagacca gtggcctttt ctatccaaga     420
atcaagcatg gataaaacag atcaagatct tgattcttgt tctgttgtca acaccaatgt     480
atcatcacca gagttcttca acgccttcga gtttatttcc tcgatgtcat ccggctttga     540
tctgtctggt ctttttgaga ctaagaggaa atcaagctca atgttcactt caaaattttc     600
```

agcgagtgcg atcatggaaa aaattgaagg ggttgcaagg gggctgagtt ataaggtggc 660

aaaagtcaaa gatttcaaag tgacgttaca aggtccatgt gaggggagaa aggggaagct 720

ggcggtgacg gcggaggtgt tcgaggtggc accggaggtt gcggtggtgg agttctccaa 780

gtcttccgga gataccttgg agtatactag gttctgcgag gaagatgtta ggcctgcact 840

aaaagacatc gtttggacat ggcaaggaga caatgtttgt aataaagata acaacaatag 900

tcatgtagaa gatcctgaga ttcaaatgtt gtagaaaggc tttgatctta atttggtgag 960

ttttaggatt ttatataatc tgtaaataag tttttgttat ttaattaatg tatcaagttg 1020

agtgttcttg 1030

<210> 102
<211> 1215
<212> DNA
<213> Populus tremula x tremuloides

<400> 102

```
cccacgcgtc cggtattttc tgtggaggag aaacaaaaag caatctcttg ggtttttgtt      60
cttttaaaga tttggtttgg gttctctctt tgaaagttat caagaacagt aaaggggctc     120
ttgattgact gatgcaagtt taagattttc tattcttttt agagaaagaa agaagttttc     180
ttttgaaatg aatttgatgt gtgaggtttt cttcgctgaa gaagaagaga aggttgaagg     240
agagtgggaa aaggagagat agtatttacc aaagccaagg tagctagcag aagtagagag     300
gcagatagca ataaactaca aaataaaatt tcatctaagg ggttttagtt tgttctgctt     360
ctgctgggta attggcaatg tctgctacta taatcagcga ccctatggtg gtctctgcac     420
cggagacaca agcaacagca gcagccgcag ctgcggcaac gacagcaaca caactcattg     480
cacagataga ggttgagtcc gtcagatgtg attgctgtgg cctaatcgaa gagtgcacgc     540
cagaatacat tgaaagagta cgcgaaagat atcatggaaa atggatttgt gggttatgca     600
cggaggctat aaaatatgaa attgtaagga ctgagaggct tattagcact gaagaagcaa     660
tgacaaagca catgaacgtc tgcaagaagt ttgtctcctc agggccacct cctgatccaa     720
caactcattt gatagccgcc atgaggcaaa tcctgagaag aagcttggat tctccgagag     780
gttcaaggtc aaccccaagt agtcctaaca aaacgaacgg agcaatccgt gtcgctgcac     840
ttgctaggtc cgaaagttgc ttccctgctt tgtctggttg atagataatt ctgagcaccg     900
tggaagacgg ggtgattcat aaaatggtga tgaagctatt ggaagaaaat aaaaaaaaga     960
gagaaaaaaa cacattccat tttcatgcaa ataagaaaga tcaaaggatt gaagttaggt    1020
gcctaatcta gccacttata tagttgttta atacagaact tcctgtcaga agaaaaatca    1080
tggaactccc ttttttcctg aagctttata tttcagctgt acgtacttgt gtaatctgtc    1140
tttttgaaac catggtttcc ttcctctttt taccaagctg tatctctcaa ttttgataaa    1200
aaaaaaaaaa aaaaa                                                      1215
```

<210> 103
<211> 484
<212> DNA
<213> Populus tremula x tremuloides

<400> 103

```
ggatgccatt gaatagccaa atatctctct ctctggacta gaatgtgaag gagcagttag      60
cgaaggaaag attggagaat gccggagctg gtgtaccaag agcagagctc gtcgagatca     120
ggatttagag ctcgagatgc aagtcctgac tctgtaattt tcactctgga gtccaacttc     180
agtctcttct cttctgcttc tgctagcgtc gatcgctgct cttttgcttc cgatgctcat     240
gatcatgact ctctcgcctc tgaaatctct ctggtaaaac catttctcta acttccctgc     300
atgcatgcgt tttctaaccc atggtttttg agtgagtttt ttgagagtgc aatgcagcat     360
ttggcagctg gtcatgatca acaagagaac tcttcgagtg gtccagatcg aaacaacaac     420
aagcagcagc agcagcagca gcacactcac acccgtctct ccagaaaagc agaaaaagtt     480
aaag                                                                  484
```

<210> 104
<211> 812
<212> DNA
<213> Populus tremula x tremuloides

<400> 104

```
tgatactctt ccatcgggtt gggatgtttc agaggctgga aagagcattt caaaggtcaa      60
aagagaggaa gccaaaatca cagcatggga gaacctgcag aaggcaaaag ctgaggcaga     120
aataaggaaa cttgaggttc tttttcccca cgcgtttcgt cgtacaattt tgacacttaa     180
acgattaatt cacttaatga tcagccttgg tgcagcaaag aagatattct atctaatatt     240
tcctttctgc ccacttgaaa gcttagctaa tacttaacta taagatactg gtcttaactg     300
actcgattca tttcagatga agcttgagaa aaagagatcg tcatctatgg atagaattat     360
gaataagctg agatcagctc aaaagagagc ccaggaaatg aggagctcgg tgctagcaaa     420
ccaggcacat caagtttcca cgaactctca caaagtttta tcattccgca gaacccgtca     480
gaagggttcc ctgagtggtt gtttcacatg ccatgctttc tgatatctca tccagccaca     540
tgttggtaca ctttgataga tataatacct ggtcaaattg caggaaaaac tgcagcagaa     600
tgtggaagcg tataggaggg aaaccaccat tgacactatt ttggtgtcaa tggagttgag     660
ttggggcgtg aagaacaagt actactgtta agatttcaga cgggacacaa accctggatg     720
gtatcaaaat ctccaggaaa tttgactgct aaaccttgta tgtatgcatg tatgtatgta     780
tgtatatcaa cgattattat tattattatt at                                   812
```

<210> 105
<211> 2550
<212> DNA
<213> Populus tremula x tremuloides

<220>

<221> misc_feature
<222> (127)..(127)
<223> n is a, c, g, or t

<400> 105

```
gccttccgtt tttttttaa acctcctcct cagatattat atacttcgtt ttctctcttc     60
tctgccatta atcatacttc acaccgatcc aggttttgtt tgctcctaaa cagagagtgg    120
gagaganccg tttaactttt cttttttcc ttttgaagta atactacatc tccctctcct    180
cctcctcctc cacctcctcc tctatttgtt cttgatttcg attatctgat cggacggtca    240
cggtcttatt tgtattcgcc tgcaatatcg tacggcctgg gatctctctt ctcttactga    300
gcaagttgca tctcataagc actggcgatc atggagctga atggtcaaac aagagtgaga    360
agaaaggacc attttgctca tacaaacggt gatttagcat tagcaagtgt tggtgatgta    420
gatccctgga ctgcatgggc atataagcct cgaactattt cgctgttact tattggtgct    480
tgctttctaa tatgggcaag tggagcccta gatccagaga gctgtacatc tggtgatgtt    540
gttacatctg tgaaaagggg tatatgggca atgactgcag tttttcttgg ttattgcttg    600
ctacaagccc cttcaacggt tctaataagg ccacatccag caatttggcg cttagttcat    660
ggattggcca ttgtttatct tgttgccctc acatttctgc tttttcagaa gcgtgatgat    720
gcgcggcaat ttatgaagtt tctccatcct gaccttggaa ttgaactacc tgaaagatca    780
tatggtgctg attgtcgcat ttatgtgcct gaaaatccta caagcaagtt taagaacgtt    840
ttggacactc tttttgatga atttgttcta gcacatatct ttggatggtg gggcaaggct    900
atattaatcc gtaatcagcc acttctatgg gtattgtcaa ttggttttga gctgatggag    960
tttaccttcc gccacatgct accaaacttc aatgaatgct ggtgggacag tatcattctt   1020
gatattttga tatgcaattg gtttggcatt tgggctggaa tgcatacagt cgggtatttt   1080
gatggaaaaa catacgagtg ggttggtata agtcgccaac ctaatattat gagcaaggtc   1140
aaacgaacat tggaacaatt tacacctgca cagtgggaca aagatgaatg gcatcccttg   1200
cttggtccat ggcgatttat ccaagttctt agtctctgta ttgtcttcct gactgtagag   1260
ctcaacacat tctttttgaa gttttgtcta tgggttcctc ctcggaaccc tgtaatagtg   1320
tacaggctga tcttgtggtg gctaattgcc atacctacaa cacgtgaata caattcatat   1380
ctccaagacc gaaagcctgt gaaaaaggtt ggtgcttttt gttggctttc ccttgctatt   1440
tgcattgtgg aacttctcat ttgcatcaag tttggacatg gtctttatcc caaaccaatg   1500
cctgcatggt tggtcatctt ctggacatct gttggagtta gccttgtaat attcctgatt   1560
atgtggtcat ggaaaagttt gggaagaaag agacgatgat tttgctagca tggtacacta   1620
ttcttttatt ccttcatgtt cttatgggta tacatcttgt aaatgcccta tagcttcatg   1680
gaattgtaaa tgcttattct cttaggttag tggatcacag ttttagagta ttcaattctt   1740
```

```
ttatgatagg tagttggaaa atgcttctaa tggtaggttc atgtgttgct aatttgcagc   1800
agtcatcaaa atggtaattt tccttaactt gtccgagaga ggagcaattt ttaacaccgg   1860
gcttgcctca catttttcat tggaaaatgc atgcctgaca attccattgg ggcattttat   1920
cagaatgttt aaaatattta tattttgcgt cggctctata gcatatgctt ataagcattg   1980
aaatatgatt caactgaagt attcgcacat gttttgctgt ttacgtttta tatgattgtt   2040
gtgtttagtt ctccacaagc agcctggcag ttattcgatc cggtatcttg acgttatctt   2100
atcacatgct gtaaatccta tggctcaaac tggcaattat tcaaggcagt tcttaaagag   2160
ttcaaggttt tctttcctgt tgatcaacct ttgtaaagct tatgaagcaa ttgtgcaaat   2220
aattggtgct cgacagaagg caatctctta gttcgctcac gacatttcta gccgattttc   2280
gggaaaacat tagtggactg gagtcattga ttcaatcaag gtgaaattgg aaatcattta   2340
gaaaagcaat tcctgttctt tgacaacact aggttcttct ttctcgatcc gtgcccatgt   2400
cactgtgtgt gtatatatag aaacataaaa agtgtatctc tctcaatcat gcaggatttt   2460
gtattttgca atcaatacgc ccatgacgta tactctcagt cttcttcgaa aacactctta   2520
ccaaaaaaaa aaaaaaaaaa aaaaaaaaaa                                    2550
```

<210> 106
<211> 787
<212> DNA
<213> Populus tremula x tremuloides

<400> 106

```
ggacgaacaa gcaatgatag agttcgagag gtcatcttct tgttataaaa tcctcaaaag     60
cttgaggtga aaaaataagc aagggagagg agagagctag ctagctagtc aagttaagag    120
tcagaaaaca agaatgtctt ccaggaagaa aaagaaggca gctctttatg agaagttacg    180
tgctgctacc aattctaatg ctatgaacaa aacttcaatc atagtagacg cgtcaaaata    240
tattggagag ttgaagaaca aggtggatag gctaaaaaaa gaaatcggaa catcttcaac    300
cccccaaaat tcattacctg cgcaggtcac agtggaaaac ctagaaaagg gtttccttat    360
taatgtattt tcaggaaaga attgccctgg attacttgtc tccatacttg aagccttcga    420
ggaactaggc cttgatgtgc tcgatgctag ggtttcttgt gaagacaatt tccaacttga    480
agcgattggt ggagaccaaa accaaggcca tgatgctcaa gtagtgaaac aagcagtgct    540
gcaagctatc cataactgga atgaaggcag ctagctagtg agcaagaatg acccaaatat    600
tttcttcccc tatcttcctg tacgctactg agaaattggg aatttgtata ttgaatttca    660
agcacactag gcagtcctaa ttccaagtta gtagtcagtc aaaaagtgca gcaatgggat    720
tctggtaatt aattttcagt ttttgattaa ttaagcaact tttaccttcg atgatgtaaa    780
aaaaaaa                                                               787
```

<210> 107
<211> 880
<212> DNA
<213> Populus tremula x tremuloides

<400> 107

gatgggaact ggcatatggc ggggaaaact agcattgtat cacagcttag tggtctgggt 60
ttagtgagaa atgtgattgg acactgtttg agtgggcgcg gtggaggatt cctcttcttc 120
ggggatgatc tttatgattc atctcgtgta gcttggacac caatgtcacc caattcgaaa 180
cactattcac ctggattcgc cgaattgact tttgatggga aaactactgg gttcaaaatc 240
ctgatcgtag cttttgacag cggggcctct tatacttacc ttaattctca ggcgtatcaa 300
ggtctaattt ctctgatgaa gagagaaata cccgcgaagc atttgacaga agcgctggat 360
gatcagacgc ttccgatttg ctggaaagga cggaaacctt tcaaaagcgt acgtgatgtc 420
aagaaatact tcaagacctt tgcattgagc tttaagaatg gcggaaaatc taaaactcaa 480
cttgaattcc caccagaagc ttatcttata gtatcatcca acggaaacgc ttgcttggga 540
attctaaatg gtacagaagt aggtctgaat gatctgaatg tcattggaga catatcaatg 600
caagacagag tggtgattta tgacaatgag aagcaactga ttggatgggc acctggaaat 660
tgcgacaggc ttcctaaatc cagaagcatt atcatttgat gacaggtgtt ctaaacgtta 720
aaaaaaacaa ttgtcctgta cagaaagctg ctaagctgta acattgtatt cttccgccat 780
tacagcagat tccttgtaga gtaaaactat gtatatgtgg tctggtgaat cagaccccac 840
aattatactt gattggttat gaaaatttaa taaaataacg 880

<210> 108
<211> 486
<212> DNA
<213> Populus tremula x tremuloides

<400> 108

agatcactga aaaatactca gaatggcccg agcatgccac cagtgcagca ttttgtgaga 60
gcagaaatgc tcccaagtgg gtatctggta cggccttgtg aaggtggtgg ttcaattata 120
cacatagttg accacatgga tttggagcct tggagtgtgc ctgaagtact acggccgctg 180
tatgaatcct caactgtact tgctcaaaag acaacaatgg tggctctacg ccagctgcgg 240
cagatagctc aggaagcttc tcagtctaat gtgaccaact ggggcagacg accagcagct 300
ctacgagcac tgagccagag gttgagcacg gggttttaat gaggctctca atggatttag 360
tgatgaggga tggtcaatga tcggaaatga tggcatggat gatgttacta tcctcgtgaa 420
ctcatctcct gacaagttga tgggttcaaa tctttccttc actaatgggt atccagctgt 480
cagcag 486

<210> 109
<211> 854
<212> DNA
<213> Populus tremula x tremuloides

<400> 109

```
ggccacatgc aagagcatgt agcatcaatg gctcggcaat atatccgcag cattatatct     60
tcagttcaga gggtggcatt agcactatct ccttctcatc ttggttcaca ggctggtctt    120
cggtcaccac tgggtactcc tgaagcacag acacttgctc gttggatctg ccagagctac    180
aggagctatt tgggtgtgga gctactcaaa tccaatggcg aaggaagtga atctattctg    240
aaaaccttgt ggcatcattc agatgccatt atgtgctgct cactgaaggt gttgcccgtc    300
tttacttttg caaaccaagc tggacttgac atgcttgaga caacattagt tgcactgcaa    360
gacataactt tggaaaagat atttgatgat catggaagaa aaactctctg ctcagaattc    420
ccacagatca tgcaacaggg ttttacttgt cttcaaggtg gtatctgttt gtcaagcatg    480
ggcagaccag tttcatatga aagagctgtg gcctggaaag tgttgaatga agaagaaaat    540
gcgcattgct tctgctttat gtttataaac tggtcttttg tctgagactt gaaaccagaa    600
tctagtggag tggctgattt aagagtgaga actctatacc tgtcttattt tgctggcttg    660
tatttcttct atgtataaca tgttacaacc gtggagcacc cagttcattt gctatgttat    720
aaatttgtgt ggggttcatt atcgtagaga ttgtggacga gacgatctag ttgccgggga    780
tcattttgtt tctgactggt ttgttctgaa gtcctcacct gggtctatgt tgattattgc    840
aacccaaatc tgtc                                                      854
```

<210> 110
<211> 607
<212> DNA
<213> Populus tremula x tremuloides

<400> 110

```
ggaatcagcc tagccaagta gagattacaa cgccaaaagc taggtcacac tgatcagagc     60
aacaacaaaa gtacaccaac aatgtcttcc accccaacaa atgaatggca aacctactgg    120
tgtcatgaat gtgacctcag catccacctt ctcaccacca ccactcctct ttgccctcac    180
tgccaccatg actttcttga actcatggac cccattccca cctccaccac cgctgacacc    240
accactttcc tccttgacag cccctccttc ctcaacttcc ttcaacatct caacaccaat    300
agccattgcg attgtgaagc atgataatac aaacgccact attgactcta tcatccccac    360
cataaaaatc acttcttgca tgctagaaat ggatccaatg cttgtgtgtg cagtgtgtaa    420
agatcagttc ttgattgata gttgaggcca agcagcatgc cctgcagtca cctgtaccat    480
ccaggttgca ttctcccctg gctttctaac cacaactctt gtccctctct gtcgcttcca    540
gttacaaacg ccagtatgtc agagaggaga attacgaaac ttgggtctcc tgatcatcct    600
catcatg                                                              607
```

<210> 111
<211> 259
<212> DNA
<213> Populus tremula x tremuloides

<400> 111

```
gggttttgac cacctttttt cctccacact tttgaatttg aatgcaggta aataaatgct      60
tttggggatt ttgggttttt ggtttggaat tgatcgagtg aagtgtaaat caacagtgaa     120
cgattgtgtt acacaaagaa cttgtgaggg ttgggcatcc ccttcccccc ttcagatttc     180
cactatggat attgtaaagt aacccccttgt ttgggattgg attttggcca aaaaaacagg    240
gtggttgaat tgattttttt                                                259
```

<210> 112
<211> 475
<212> DNA
<213> Populus tremula x tremuloides

<400> 112

```
gcacgggtga aaagttacat taagaatcga tatgtgttgc gcaatctcct tcaagttccg      60
gtaataaaag aggacagtat gaaggataaa aaggcaagac aaaaaggaag ggatgatgat     120
tcaattacaa gtgatatgct agtagagata atggaagaat caataaggat tttttggcga     180
tttgttcgat ctgataaaga tgcacaaaac gtgatttcaa agggtcgcaa aggaactcaa     240
atagagcccc aagatcccac agaactagag ttattgacag aggttcgaac gagtttccaa     300
aagaaggaga ggaggcttaa agacgttttg aggagtggaa actgcatact gaggaagttc     360
caaaaacatc gagaggacga ttctaatcaa gttctttact tcttctctca agtggatatg     420
aagttagtag caagggttct gagcatgtcc agagtaacaa cagagcagct actat          475
```

<210> 113
<211> 572
<212> DNA
<213> Artificial

<220>
<223> Construct sequence

<220>
<221> misc_feature
<222> (77)..(77)
<223> n is a, c, g, or t

<400> 113

```
attgattaac catggagaaa gcacacacaa aatctgctct taagaagctt gttaaggcta      60
gctcacagtc tgcatcntgg agcaatgctg ctagaggaat ggcgaaagat gatctcaagg     120
atccgcttta tgacaaatcc aaggttgccc caaaacccct tgcaaaagaa aacacgaagc     180
cccaagaatt caaactccac actggacaaa gagctctcaa acgtgccatg ttcaactatt     240
ctgtggcaac caagatatat atgaatgagc aacagaagag gcaaatagag aggatacaaa     300
agatcataga agaagaagag gttcgtatga tgaggaagga gatggttcca agagctcaat     360
tgatgcctta ctttgacaga cctttctttc cccaaagatc aagcaggcca ttgacagttc     420
ctagagagcc aagtttccac atggtgaaca gcaagtgttg gagctgcatc cctgaggatg     480
aactttacta ctactttgaa catgctcatc cccatgacca tgcctggaag cctgttaagt     540
aaataattgc catgatttgt aggggaaaaa gg                                   572
```

<210> 114
<211> 1302
<212> DNA
<213> Artificial

<220>
<223> Construct sequence

<220>
<221> misc_feature
<222> (597)..(597)
<223> n is a, c, g, or t

<400> 114

aagctgatga tcaaatgagg acatcaaaaa tttcttcagg gaattcaggc taccatgtat 60
ggattcttgc ttttgcaatg agtctactga tactgattgc cttgtcgaag tcatggtttt 120
atgatcacgc ttctgctgct gcaagtgagg atttgcagta cttttcagtg atcgtccctt 180
caaaagggcg ggactatcct cctgttcttg cttattggat atgtggtacc agtggagatg 240
ggaagagaat gttaaggcta ttaaaagcaa tttatcatcc aaggaaccaa tatctattgc 300
aacttgatgc ggaatcctca gattatgaaa gggcggagtt ggttgtttca gttcaatctg 360
aaagtttgtt tcaagcattc ggtaatgtta atgttgttgg aaaaggtttt gctatcaacg 420
aaatgggatc ctctgctctt gctgccatac tcaacgctgc tgcgttgctt cttaagctga 480
gtacagatgg ggactggttc atcaatttaa gtgtctcaga ctatcctcta gtgagtcaag 540
atgatctcct ccatgccttc acttccttgc caagagatct caacttcatc aactatncta 600
atgacactgc gaaaaacgag atacacaaga ttaaccaaat tgtagtagat cccagcttgc 660
atcttcaaaa gaggagccac ctttattacg ctgttgagac tcgaacaaca cctgatgctt 720
tcaagatatt tggaggttcg ccctggctga ttcttacaag agctttcatg gagtactgtg 780
tccaaggatg ggacaacctt ccaagaaaac tactaatgta cttcagcaac acggcatccc 840
cacttgaatc gtatttccac tctgtcctct gcaactctcc tgagtttcaa aacacaacag 900
taagcgatga tttaaggtac aatattctgg aaactactac agatggggaa tcaccttatg 960
acaaaatgct aaatggtgga gcagcatttg caaggccatt taaagaagat gctgctgctc 1020
taaacatgat agatgagaac gtcttgaacc gtgaacccaa cggattggtg cctggaaaat 1080
ggtgcttaga ccagggtatg aacaagagct cagaggcatc aaagcctcca ggggaggatt 1140
tgtgttcaac ctggggtaac attaatgatg tcaagccagg atcttatggt atcaagcttg 1200
catttttatt gtctaagatt gccagtgaag agaaattgac aactagtcaa tgccttcaag 1260

ctacaaaaat ggggtcatca tagaaccaag catacatcct tg 1302

<210> 115
<211> 556
<212> DNA
<213> Artificial

<220>
<223> Construct sequence

<400> 115

```
attaatcatg tcaggtgatc aaaggcctaa agattctgct gagggctctt cgcgatctgg      60
cggcgaccac taccagcctc aaccggctcc tttgagcagg tatgagtcgc agaaacggcg     120
agactggaac acttttgggc agtacttgaa gaatcagaga cccccagttt cactatctca     180
gagtaattgc aaccacgtgc ttgatttcct tcggtatctc gatcagtttg gcaagactaa     240
ggttcatcta catggctgtg tctttttttgg acaacctaat cctcctgctc cttgtacctg   300
ccctctaagg caagcttggg ggagcctcga tgccctaatc ggacggcttc gagcagctta     360
tgaggagcac ggaggatctg cagagaccaa ccccttttgga aatggagcta ttcgggttta    420
tttgcgtgaa gtgaaagagt gtcaagctaa ggcaaggggg attccataca agaagaaaac     480
gaagaagaag actcaaataa gggcaagaaa tgaagcgaag cctcctatgc agtcagctta     540
agtcctttgg cttctt                                                     556
```

<210> 116
<211> 4470
<212> DNA
<213> Artificial

<220>
<223> Cloning vector pDONR201

<400> 116

```
ctttcctgcg ttatcccctg attctgtgga taaccgtatt accgctagcc aggaagagtt      60
tgtagaaacg caaaaaggcc atccgtcagg atggccttct gcttagtttg atgcctggca     120
gtttatggcg ggcgtcctgc ccgccaccct ccgggccgtt gcttcacaac gttcaaatcc     180
gctcccggcg gatttgtcct actcaggaga gcgttcaccg acaaacaaca gataaaacga     240
aaggcccagt cttccgactg agcctttcgt tttatttgat gcctggcagt tccctactct     300
cgcgttaacg ctagcatgga tctcgggccc caaataatga ttttattttg actgatagtg     360
acctgttcgt tgcaacaaat tgatgagcaa tgctttttta taatgccaag tttgtacaaa     420
aaagcagaac gagaaacgta aaatgatata aatatcaata tattaaatta gattttgcat     480
aaaaaacaga ctacataata ctgtaaaaca caacatatcc agtcactatg aatcaactac     540
ttagatggta ttagtgacct gtagtcgacc gacagccttc caaatgttct tcgggtgatg     600
ctgccaactt agtcgaccga cagccttcca aatgttcttc tcaaacggaa tcgtcgtatc     660
```

cagcctactc gctattgtcc tcaatgccgt attaaatcat aaaaagaaat aagaaaaaga 720
ggtgcgagcc tcttttttgt gtgacaaaat aaaaacatct acctattcat atacgctagt 780
gtcatagtcc tgaaaatcat ctgcatcaag aacaatttca caactcttat acttttctct 840
tacaagtcgt tcggcttcat ctggattttc agcctctata cttactaaac gtgataaagt 900
ttctgtaatt tctactgtat cgacctgcag actggctgtg tataagggag cctgacattt 960
atattcccca gaacatcagg ttaatggcgt ttttgatgtc attttcgcgg tggctgagat 1020
cagccacttc ttccccgata acggagaccg gcacactggc catatcggtg gtcatcatgc 1080
gccagctttc atccccgata tgcaccaccg ggtaaagttc acgggagact ttatctgaca 1140
gcagacgtgc actggccagg gggatcacca tccgtcgccc gggcgtgtca ataatatcac 1200
tctgtacatc cacaaacaga cgataacggc tctctctttt ataggtgtaa accttaaact 1260
gcatttcacc agtccctgtt ctcgtcagca aaagagccgt tcatttcaat aaaccgggcg 1320
acctcagcca tcccttcctg attttccgct ttccagcgtt cggcacgcag acgacgggct 1380
tcattctgca tggttgtgct taccagaccg gagatattga catcatatat gccttgagca 1440
actgatagct gtcgctgtca actgtcactg taatacgctg cttcatagca cacctctttt 1500
tgacatactt cgggtataca tatcagtata tattcttata ccgcaaaaat cagcgcgcaa 1560
atacgcatac tgttatctgg cttttagtaa gccggatcca cgcgattacg ccccgccctg 1620
ccactcatcg cagtactgtt gtaattcatt aagcattctg ccgacatgga agccatcaca 1680
gacggcatga tgaacctgaa tcgccagcgg catcagcacc ttgtcgcctt gcgtataata 1740
tttgcccatg gtgaaaacgg gggcgaagaa gttgtccata ttggccacgt ttaaatcaaa 1800
actggtgaaa ctcacccagg gattggctga gacgaaaaac atattctcaa taaacccttt 1860
agggaaatag gccaggtttt caccgtaaca cgccacatct tgcgaatata tgtgtagaaa 1920
ctgccggaaa tcgtcgtggt attcactcca gagcgatgaa aacgtttcag tttgctcatg 1980
gaaaacggtg taacaagggt gaacactatc ccatatcacc agctcaccgt ctttcattgc 2040
catacggaat tccggatgag cattcatcag gcgggcaaga atgtgaataa aggccggata 2100
aaacttgtgc ttatttttct ttacggtctt taaaaaggcc gtaatatcca gctgaacggt 2160
ctggttatag gtacattgag caactgactg aaatgcctca aaatgttctt tacgatgcca 2220
ttgggatata tcaacggtgg tatatccagt gatttttttc tccattttag cttccttagc 2280
tcctgaaaat ctcgataact caaaaaatac gcccggtagt gatcttattt cattatggtg 2340
aaagttggaa cctcttacgt gccgatcaac gtctcatttt cgccaaaagt tggcccaggg 2400
cttcccggta tcaacaggga caccaggatt tatttattct gcgaagtgat cttccgtcac 2460
aggtatttat tcggcgcaaa gtgcgtcggg tgatgctgcc aacttagtcg actacaggtc 2520
actaatacca tctaagtagt tgattcatag tgactggata tgttgtgttt tacagtatta 2580
tgtagtctgt tttttatgca aaatctaatt taatatattg atatttatat cattttacgt 2640

```
ttctcgttca gctttcttgt acaaagtggg cattataaga aagcattgct tatcaatttg   2700
ttgcaacgaa caggtcacta tcagtcaaaa taaaatcatt atttgccatc cagctgcagc   2760
tctggcccgt gtctcaaaat ctctgatgtt acattgcaca agataaaaat atatcatcat   2820
gaacaataaa actgtctgct tacataaaca gtaatacaag gggtgttatg agccatattc   2880
aacgggaaac gtcgaggccg cgattaaatt ccaacatgga tgctgattta tatgggtata   2940
aatgggctcg cgataatgtc gggcaatcag gtgcgacaat ctatcgcttg tatgggaagc   3000
ccgatgcgcc agagttgttt ctgaaacatg gcaaaggtag cgttgccaat gatgttacag   3060
atgagatggt cagactaaac tggctgacgg aatttatgcc tcttccgacc atcaagcatt   3120
ttatccgtac tcctgatgat gcatggttac tcaccactgc gatccccgga aaaacagcat   3180
tccaggtatt agaagaatat cctgattcag gtgaaaatat tgttgatgcg ctggcagtgt   3240
tcctgcgccg gttgcattcg attcctgttt gtaattgtcc ttttaacagc gatcgcgtat   3300
ttcgtctcgc tcaggcgcaa tcacgaatga ataacggttt ggttgatgcg agtgattttg   3360
atgacgagcg taatggctgg cctgttgaac aagtctggaa agaaatgcat aaacttttgc   3420
cattctcacc ggattcagtc gtcactcatg gtgatttctc acttgataac cttatttttg   3480
acgaggggaa attaataggt tgtattgatg ttggacgagt cggaatcgca gaccgatacc   3540
aggatcttgc catcctatgg aactgcctcg gtgagttttc tccttcatta cagaaacggc   3600
tttttcaaaa atatggtatt gataatcctg atatgaataa attgcagttt catttgatgc   3660
tcgatgagtt tttctaatca gaattggtta attggttgta acactggcag agcattacgc   3720
tgacttgacg ggacggcgca agctcatgac caaaatccct taacgtgagt tttcgttcca   3780
ctgagcgtca gaccccgtag aaaagatcaa aggatcttct tgagatcctt tttttctgcg   3840
cgtaatctgc tgcttgcaaa caaaaaaacc accgctacca gcggtggttt gtttgccgga   3900
tcaagagcta ccaactcttt ttccgaaggt aactggcttc agcagagcgc agataccaaa   3960
tactgtcctt ctagtgtagc cgtagttagg ccaccacttc aagaactctg tagcaccgcc   4020
tacatacctc gctctgctaa tcctgttacc agtggctgct gccagtggcg ataagtcgtg   4080
tcttaccggg ttggactcaa gacgatagtt accggataag gcgcagcggt cgggctgaac   4140
ggggggttcg tgcacacagc ccagcttgga gcgaacgacc tacaccgaac tgagatacct   4200
acagcgtgag ctatgagaaa gcgccacgct tcccgaaggg agaaaggcgg acaggtatcc   4260
ggtaagcggc agggtcggaa caggagagcg cacgagggag cttccagggg gaaacgcctg   4320
gtatctttat agtcctgtcg ggtttcgcca cctctgactt gagcgtcgat ttttgtgatg   4380
ctcgtcaggg gggcggagcc tatggaaaaa cgccagcaac gcggcctttt tacggttcct   4440
ggccttttgc tggccttttg ctcacatgtt                                    4470
```

<210> 117
<211> 13599
<212> DNA
<213> Artificial

<220>
<223> Cloning vector pK7GWIWG2(I)

<400> 117

```
ctagagggcc cgacgtcgca tgcctgcagg tcactggatt ttggttttag gaattagaaa     60
ttttattgat agaagtattt tacaaataca aatacatact aagggtttct tatatgctca    120
acacatgagc gaaaccctat aagaacccta attcccttat ctgggaacta ctcacacatt    180
attctggaga aaaatagaga gagatagatt tgtagagaga gactggtgat tttgcggac    240
tctagcatgg ccgcgggata tcacaagttt gtacaaaaaa gctgaacgag aaacgtaaaa    300
tgatataaat atcaatatat taaattagat tttgcataaa aaacagacta cataatactg    360
taaaacacaa catatccagt cactatggcg gccgcattag gcaccccagg ctttacactt    420
tatgcttccg gctcgtataa tgtgtggatt ttgagttagg atccggcgag attttcagga    480
gctaaggaag ctaaaatgga gaaaaaaatc actggatata ccaccgttga tatatcccaa    540
tggcatcgta aagaacattt tgaggcattt cagtcagttg ctcaatgtac ctataaccag    600
accgttcagc tggatattac ggccttttta aagaccgtaa agaaaaataa gcacaagttt    660
tatccggcct ttattcacat tcttgcccgc ctgatgaatg ctcatccgga attccgtatg    720
gcaatgaaag acggtgagct ggtgatatgg gatagtgttc acccttgtta caccgttttc    780
catgagcaaa ctgaaacgtt ttcatcgctc tggagtgaat accacgacga tttccggcag    840
tttctacaca tatattcgca agatgtggcg tgttacggtg aaaacctggc ctatttccct    900
aaagggttta ttgagaatat gtttttcgtc tcagccaatc cctgggtgag tttcaccagt    960
tttgatttaa acgtggccaa tatggacaac ttcttcgccc ccgttttcac catgggcaaa   1020
tattatacgc aaggcgacaa ggtgctgatg ccgctggcga ttcaggttca tcatgccgtc   1080
tgtgatggct tccatgtcgg cagaatgctt aatgaattac aacagtactg cgatgagtgg   1140
cagggcgggg cgtaaacgcg tggatccggc ttactaaaag ccagataaca gtatgcgtat   1200
ttgcgcgctg atttttgcgg tataagaata tatactgata tgtatacccg aagtatgtca   1260
aaaagaggtg tgctatgaag cagcgtatta cagtgacagt tgacagcgac agctatcagt   1320
tgctcaaggc atatatgatg tcaatatctc cggtctggta agcacaacca tgcagaatga   1380
agcccgtcgt ctgcgtgccg aacgctggaa agcggaaaat caggaaggga tggctgaggt   1440
cgcccggttt attgaaatga acggctcttt tgctgacgag aacaggggact ggtgaaatgc   1500
agtttaaggt ttacacctat aaaagagaga gccgttatcg tctgtttgtg gatgtacaga   1560
gtgatattat tgacacgccc gggcgacgga tggtgatccc cctggccagt gcacgtctgc   1620
tgtcagataa agtctcccgt gaactttacc cggtggtgca tatcggggat gaaagctggc   1680
gcatgatgac caccgatatg gccagtgtgc cggtctccgt tatcggggaa gaagtggctg   1740
```

atctcagcca ccgcgaaaat gacatcaaaa acgccattaa cctgatgttc tggggaatat 1800
aaatgtcagg ctcccttata cacagccagt ctgcaggtcg accatagtga ctggatatgt 1860
tgtgttttac agtattatgt agtctgtttt ttatgcaaaa tctaatttaa tatattgata 1920
tttatatcat tttacgtttc tcgttcagct ttcttgtaca aagtggtgat atcactagtg 1980
cggccgcctg caggtcgacc atatggtcga cctgcaggcg gccgcactag tgatgctgtt 2040
atgttcagtg tcaagctgac ctgcaaacac gttaaatgct aagaagttag aatatatgag 2100
acacgttaac tggtatatga ataagctgta aataaccgag tataaactca ttaactaata 2160
tcacctctag agtataatat aatcaaattc gacaatttga ctttcaagag taggctaatg 2220
taaaatcttt atatatttct acaatgttca aagaaacagt tgcatctaaa cccctatggc 2280
catcaaattc aatgaacgct aagcttaata tgactctcaa taaagtctca taccaacaag 2340
tgccacctta ttcaaccatc aagaaaaaag ccaaaattta tgctactcta aggaaaactt 2400
cactaaagaa gacgatttag agtgttttac caagaatttc tgtcatctta ctaaacaact 2460
aaagatcggt gtgatacaaa acctaatctc attaaagttt atgctaaaat aagcataatt 2520
ttacccacta agcgtgacca gataaacata actcagcaca ccagagcata tatattggtg 2580
gctcaaatca tagaaactta cagtgaagac acagaaagcc gtaagaagag gcaagagtat 2640
gaaaccttac ctcatcattt ccatgaggtt gcttctgatc ccgcgggata tcaccacttt 2700
gtacaagaaa gctgaacgag aaacgtaaaa tgatataaat atcaatatat taaattagat 2760
tttgcataaa aaacagacta cataatactg taaaacacaa catatccagt cactatggtc 2820
gacctgcaga ctggctgtgt ataagggagc ctgacattta tattccccag aacatcaggt 2880
taatggcgtt tttgatgtca ttttcgcggt ggctgagatc agccacttct tccccgataa 2940
cggagaccgg cacactggcc atatcggtgg tcatcatgcg ccagctttca tccccgatat 3000
gcaccaccgg gtaaagttca cgggagactt tatctgacag cagacgtgca ctggccaggg 3060
ggatcaccat ccgtcgcccg ggcgtgtcaa taatatcact ctgtacatcc acaaacagac 3120
gataacggct ctctctttta taggtgtaaa ccttaaactg catttcacca gtccctgttc 3180
tcgtcagcaa aagagccgtt catttcaata aaccgggcga cctcagccat cccttcctga 3240
ttttccgctt tccagcgttc ggcacgcaga cgacgggctt cattctgcat ggttgtgctt 3300
accagaccgg agatattgac atcatatatg ccttgagcaa ctgatagctg tcgctgtcaa 3360
ctgtcactgt aatacgctgc ttcatagcac acctcttttt gacatacttc gggtatacat 3420
atcagtatat attcttatac cgcaaaaatc agcgcgcaaa tacgcatact gttatctggc 3480
ttttagtaag ccggatccac gcgtttacgc cccgccctgc cactcatcgc agtactgttg 3540
taattcatta agcattctgc cgacatggaa gccatcacag acggcatgat gaacctgaat 3600
cgccagcggc atcagcacct tgtcgccttg cgtataatat ttgcccatgg tgaaaacggg 3660

ggcgaagaag ttgtccatat tggccacgtt taaatcaaaa ctggtgaaac tcacccaggg 3720
attggctgag acgaaaaaca tattctcaat aaaccctta gggaaatagg ccaggttttc 3780
accgtaacac gccacatctt gcgaatatat gtgtagaaac tgccggaaat cgtcgtggta 3840
ttcactccag agcgatgaaa acgtttcagt ttgctcatgg aaaacggtgt aacaagggtg 3900
aacactatcc catatcacca gctcaccgtc tttcattgcc atacggaatt ccggatgagc 3960
attcatcagg cgggcaagaa tgtgaataaa ggccggataa aacttgtgct tatttttctt 4020
tacggtcttt aaaaaggccg taatatccag ctgaacggtc tggttatagg tacattgagc 4080
aactgactga aatgcctcaa aatgttcttt acgatgccat tgggatatat caacggtggt 4140
atatccagtg atttttttct ccattttagc ttccttagct cctgaaaatc tcgccggatc 4200
ctaactcaaa atccacacat tatacgagcc ggaagcataa agtgtaaagc ctggggtgcc 4260
taatgcggcc gccatagtga ctggatatgt tgtgttttac agtattatgt agtctgtttt 4320
ttatgcaaaa tctaatttaa tatattgata tttatatcat tttacgtttc tcgttcagct 4380
tttttgtaca aacttgtgat atcactagtg cggccgcctg caggtcgact agaatagtaa 4440
attgtaatgt tgtttgttgt ttgttttgtt gtggtaattg ttgtaaaaat acggatcgtc 4500
ctgcagtcct ctccaaatga aatgaacttc cttatataga ggaagggtct tgcgaaggat 4560
agtgggattg tgcgtcatcc cttacgtcag tggagatatc acatcaatcc acttgctttg 4620
aagacgtggt tggaacgtct tctttttcca cgatgctcct cgtgggtggg ggtccatctt 4680
tgggaccact gtcggcagag gcatcttgaa cgatagcctt tcctttatcg caatgatggc 4740
atttgtaggt gccaccttcc ttttctactg tccttttgat gaagtgacag atagctgggc 4800
aatggaatcc gaggaggttt cccgatatta ccctttgttg aaaagtctca atagcccttt 4860
ggtcttctga gactgtatct ttgatattct tggagtagac gagagtgtcg tgctccacca 4920
tgttgacgaa gattttcttc ttgtcattga gtcgtaaaag actctgtatg aactgttcgc 4980
cagtcttcac ggcgagttct gttagatcct cgatctgaat ttttgactcc atggcctttg 5040
attcagtagg aactactttc ttagagactc caatctctat tacttgcctt ggtttatgaa 5100
gcaagccttg aatcgtccat actggaatag tacttctgat cttgagaaat atatctttct 5160
ctgtgttctt gatgcagtta gtcctgaatc ttttgactgc atctttaacc ttcttgggaa 5220
ggtatttgat ctcctggaga ttattactcg ggtagatcgt cttgatgaga cctgccgcgt 5280
aggcctctct aaccatctgt gggtcagcat tctttctgaa attgaagagg ctaatcttct 5340
cattatcggt ggtgaacatg gtatcgtcac cttctccgtc gaactttctt cctagatcgt 5400
agagatagag aaagtcgtcc atggtgatct ccggggcaaa ggagatcagc ttggctctag 5460
tcgaccatat gggagagctc aagcttagct tgagcttgga tcagattgtc gtttcccgcc 5520
ttcagtttaa actatcagtg tttgacagga tatattggcg ggtaaaccta agagaaaaga 5580
gcgtttatta gaataacgga tatttaaaag ggcgtgaaaa ggtttatccg ttcgtccatt 5640

```
tgtatgtgca tgccaaccac agggttcccc tcgggatcaa agtactttga tccaacccct   5700
ccgctgctat agtgcagtcg gcttctgacg ttcagtgcag ccgtcttctg aaaacgacat   5760
gtcgcacaag tcctaagtta cgcgacaggc tgccgccctg cccttttcct ggcgttttct   5820
tgtcgcgtgt tttagtcgca taaagtagaa tacttgcgac tagaaccgga gacattacgc   5880
catgaacaag agcgccgccg ctggcctgct gggctatgcc cgcgtcagca ccgacgacca   5940
ggacttgacc aaccaacggg ccgaactgca cgcggccggc tgcaccaagc tgttttccga   6000
gaagatcacc ggcaccaggc gcgaccgccc ggagctggcc aggatgcttg accacctacg   6060
ccctggcgac gttgtgacag tgaccaggct agaccgcctg gcccgcagca cccgcgacct   6120
actggacatt gccgagcgca tccaggaggc cggcgcgggc ctgcgtagcc tggcagagcc   6180
gtgggccgac accaccacgc cggccggccg catggtgttg accgtgttcg ccggcattgc   6240
cgagttcgag cgttccctaa tcatcgaccg cacccggagc gggcgcgagg ccgccaaggc   6300
ccgaggcgtg aagtttggcc cccgccctac cctcaccccg gcacagatcg cgcacgcccg   6360
cgagctgatc gaccaggaag gccgcaccgt gaaagaggcg gctgcactgc ttggcgtgca   6420
tcgctcgacc ctgtaccgcg cacttgagcg cagcgaggaa gtgacgccca ccgaggccag   6480
gcggcgcggt gccttccgtg aggacgcatt gaccgaggcc gacgccctgg cggccgccga   6540
gaatgaacgc caagaggaac aagcatgaaa ccgcaccagg acggccagga cgaaccgttt   6600
ttcattaccg aagagatcga ggcggagatg atcgcggccg ggtacgtgtt cgagccgccc   6660
gcgcacgtct caaccgtgcg gctgcatgaa atcctggccg gtttgtctga tgccaagctg   6720
gcggcctggc cggccagctt ggccgctgaa gaaaccgagc gccgccgtct aaaaaggtga   6780
tgtgtatttg agtaaaacag cttgcgtcat gcggtcgctg cgtatatgat gcgatgagta   6840
aataaacaaa tacgcaaggg gaacgcatga aggttatcgc tgtacttaac cagaaaggcg   6900
ggtcaggcaa gacgaccatc gcaacccatc tagcccgcgc cctgcaactc gccggggccg   6960
atgttctgtt agtcgattcc gatccccagg gcagtgcccg cgattgggcg gccgtgcggg   7020
aagatcaacc gctaaccgtt gtcggcatcg accgcccgac gattgaccgc gacgtgaagg   7080
ccatcggccg gcgcgacttc gtagtgatcg acggagcgcc ccaggcggcg gacttggctg   7140
tgtccgcgat caaggcagcc gacttcgtgc tgattccggt gcagccaagc ccttacgaca   7200
tatgggccac cgccgacctg gtggagctgg ttaagcagcg cattgaggtc acggatggaa   7260
ggctacaagc ggcctttgtc gtgtcgcggg cgatcaaagg cacgcgcatc ggcggtgagg   7320
ttgccgaggc gctggccggg tacgagctgc ccattcttga gtcccgtatc acgcagcgcg   7380
tgagctaccc aggcactgcc gccgccggca caaccgttct tgaatcagaa cccgagggcg   7440
acgctgcccg cgaggtccag gcgctggccg ctgaaattaa atcaaaactc atttgagtta   7500
atgaggtaaa gagaaaatga gcaaaagcac aaacacgcta agtgccggcc gtccgagcgc   7560
```

acgcagcagc aaggctgcaa cgttggccag cctggcagac acgccagcca tgaagcgggt 7620
caactttcag ttgccggcgg aggatcacac caagctgaag atgtacgcgg tacgccaagg 7680
caagaccatt accgagctgc tatctgaata catcgcgcag ctaccagagt aaatgagcaa 7740
atgaataaat gagtagatga attttagcgg ctaaaggagg cggcatggaa aatcaagaac 7800
aaccaggcac cgacgccgtg gaatgcccca tgtgtggagg aacgggcggt tggccaggcg 7860
taagcggctg ggttgtctgc cggccctgca atggcactgg aacccccaag cccgaggaat 7920
cggcgtgacg gtcgcaaacc atccggcccg gtacaaatcg gcgcggcgct gggtgatgac 7980
ctggtggaga agttgaaggc cgcgcaggcc gcccagcggc aacgcatcga ggcagaagca 8040
cgccccggtg aatcgtggca agcggccgct gatcgaatcc gcaaagaatc ccggcaaccg 8100
ccggcagccg gtgcgccgtc gattaggaag ccgcccaagg gcgacgagca accagatttt 8160
ttcgttccga tgctctatga cgtgggcacc cgcgatagtc gcagcatcat ggacgtggcc 8220
gttttccgtc tgtcgaagcg tgaccgacga gctggcgagg tgatccgcta cgagcttcca 8280
gacgggcacg tagaggtttc cgcagggccg gccggcatgg ccagtgtgtg ggattacgac 8340
ctggtactga tggcggtttc ccatctaacc gaatccatga accgataccg ggaagggaag 8400
ggagacaagc ccggccgcgt gttccgtcca cacgttgcgg acgtactcaa gttctgccgg 8460
cgagccgatg gcggaaagca gaaagacgac ctggtagaaa cctgcattcg gttaaacacc 8520
acgcacgttg ccatgcagcg tacgaagaag gccaagaacg gccgcctggt gacggtatcc 8580
gagggtgaag ccttgattag ccgctacaag atcgtaaaga gcgaaaccgg gcggccggag 8640
tacatcgaga tcgagctagc tgattggatg taccgcgaga tcacagaagg caagaacccg 8700
gacgtgctga cggttcaccc cgattacttt ttgatcgatc ccggcatcgg ccgttttctc 8760
taccgcctgg cacgccgcgc cgcaggcaag gcagaagcca gatggttgtt caagacgatc 8820
tacgaacgca gtggcagcgc cggagagttc aagaagttct gtttcaccgt gcgcaagctg 8880
atcgggtcaa atgacctgcc ggagtacgat ttgaaggagg aggcggggca ggctggcccg 8940
atcctagtca tgcgctaccg caacctgatc gagggcgaag catccgccgg ttcctaatgt 9000
acggagcaga tgctagggca aattgcccta gcaggggaaa aaggtcgaaa aggtctcttt 9060
cctgtggata gcacgtacat tgggaaccca aagccgtaca ttgggaaccg gaacccgtac 9120
attgggaacc caaagccgta cattgggaac cggtcacaca tgtaagtgac tgatataaaa 9180
gagaaaaaag gcgatttttc cgcctaaaac tctttaaaac ttattaaaac tcttaaaacc 9240
cgcctggcct gtgcataact gtctggccag cgcacagccg aagagctgca aaaagcgcct 9300
acccttcggt cgctgcgctc cctacgcccc gccgcttcgc gtcggcctat cgcggccgct 9360
ggccgctcaa aaatggctgg cctacggcca ggcaatctac cagggcgcgg acaagccgcg 9420
ccgtcgccac tcgaccgccg gcgcccacat caaggcaccc tgcctcgcgc gtttcggtga 9480
tgacggtgaa aacctctgac acatgcagct cccggagacg gtcacagctt gtctgtaagc 9540

ggatgccggg agcagacaag cccgtcaggg cgcgtcagcg ggtgttggcg ggtgtcgggg 9600
cgcagccatg acccagtcac gtagcgatag cggagtgtat actggcttaa ctatgcggca 9660
tcagagcaga ttgtactgag agtgcaccat atgcggtgtg aaataccgca cagatgcgta 9720
aggagaaaat accgcatcag gcgctcttcc gcttcctcgc tcactgactc gctgcgctcg 9780
gtcgttcggc tgcggcgagc ggtatcagct cactcaaagg cggtaatacg gttatccaca 9840
gaatcagggg ataacgcagg aaagaacatg tgagcaaaag gccagcaaaa ggccaggaac 9900
cgtaaaaagg ccgcgttgct ggcgtttttc cataggctcc gcccccctga cgagcatcac 9960
aaaaatcgac gctcaagtca gaggtggcga aacccgacag gactataaag ataccaggcg 10020
tttccccctg gaagctccct cgtgcgctct cctgttccga ccctgccgct taccggatac 10080
ctgtccgcct ttctcccttc gggaagcgtg gcgctttctc atagctcacg ctgtaggtat 10140
ctcagttcgg tgtaggtcgt tcgctccaag ctgggctgtg tgcacgaacc ccccgttcag 10200
cccgaccgct gcgccttatc cggtaactat cgtcttgagt ccaacccggt aagacacgac 10260
ttatcgccac tggcagcagc cactggtaac aggattagca gagcgaggta tgtaggcggt 10320
gctacagagt tcttgaagtg gtggcctaac tacggctaca ctagaaggac agtatttggt 10380
atctgcgctc tgctgaagcc agttaccttc ggaaaaagag ttggtagctc ttgatccggc 10440
aaacaaacca ccgctggtag cggtggtttt tttgtttgca agcagcagat tacgcgcaga 10500
aaaaaaggat ctcaagaaga tcctttgatc ttttctacgg ggtctgacgc tcagtggaac 10560
gaaaactcac gttaagggat tttggtcatg catgatatat ctcccaattt gtgtagggct 10620
tattatgcac gcttaaaaat aataaaagca gacttgacct gatagtttgg ctgtgagcaa 10680
ttatgtgctt agtgcatcta atcgcttgag ttaacgccgg cgaagcggcg tcggcttgaa 10740
cgaatttcta gctagacatt atttgccgac taccttggtg atctcgcctt tcacgtagtg 10800
gacaaattct tccaactgat ctgcgcgcga ggccaagcga tcttcttctt gtccaagata 10860
agcctgtcta gcttcaagta tgacgggctg atactgggcc ggcaggcgct ccattgccca 10920
gtcggcagcg acatccttcg gcgcgatttt gccggttact gcgctgtacc aaatgcggga 10980
caacgtaagc actacatttc gctcatcgcc agcccagtcg ggcggcgagt tccatagcgt 11040
taaggtttca tttagcgcct caaatagatc ctgttcagga accggatcaa agagttcctc 11100
cgccgctgga cctaccaagg caacgctatg ttctcttgct tttgtcagca agatagccag 11160
atcaatgtcg atcgtggctg gctcgaagat acctgcaaga atgtcattgc gctgccattc 11220
tccaaattgc agttcgcgct tagctggata acgccacgga atgatgtcgt cgtgcacaac 11280
aatggtgact tctacagcgc ggagaatctc gctctctcca ggggaagccg aagtttccaa 11340
aaggtcgttg atcaaagctc gccgcgttgt ttcatcaagc cttacggtca ccgtaaccag 11400
caaatcaata tcactgtgtg gcttcaggcc gccatccact gcggagccgt acaaatgtac 11460

ggccagcaac gtcggttcga gatggcgctc gatgacgcca actacctctg atagttgagt 11520
cgatacttcg gcgatcaccg cttcccccat gatgtttaac tttgttttag ggcgactgcc 11580
ctgctgcgta acatcgttgc tgctccataa catcaaacat cgacccacgg cgtaacgcgc 11640
ttgctgcttg gatgcccgag gcatagactg taccccaaaa aaacatgtca taacaagaag 11700
ccatgaaaac cgccactgcg ccgttaccac cgctgcgttc ggtcaaggtt ctggaccagt 11760
tgcgtgacgg cagttacgct acttgcatta cagcttacga accgaacgag gcttatgtcc 11820
actgggttcg tgcccgaatt gatcacaggc agcaacgctc tgtcatcgtt acaatcaaca 11880
tgctaccctc cgcgagatca tccgtgtttc aaacccggca gcttagttgc cgttcttccg 11940
aatagcatcg gtaacatgag caaagtctgc cgccttacaa cggctctccc gctgacgccg 12000
tcccggactg atgggctgcc tgtatcgagt ggtgattttg tgccgagctg ccggtcgggg 12060
agctgttggc tggctggtgg caggatatat tgtggtgtaa acaaattgac gcttagacaa 12120
cttaataaca cattgcggac gtttttaatg tactgaatta acgccgaatt gaattatcag 12180
cttgcatgcc ggtcgatcta gtaacataga tgacaccgcg cgcgataatt tatcctagtt 12240
tgcgcgctat attttgtttt ctatcgcgta ttaaatgtat aattgcggga ctctaatcat 12300
aaaaacccat ctcataaata acgtcatgca ttacatgtta attattacat gcttaacgta 12360
attcaacaga aattatatga taatcatcgc aagaccggca acaggattca atcttaagaa 12420
actttattgc caaatgtttg aacgatctgc ttgactctag ctagagtccg aaccccagag 12480
tcccgctcag aagaactcgt caagaaggcg atagaaggcg atgcgctgcg aatcgggagc 12540
ggcgataccg taaagcacga ggaagcggtc agcccattcg ccgccaagct cttcagcaat 12600
atcacgggta gccaacgcta tgtcctgata gcggtccgcc acacccagcc ggccacagtc 12660
gatgaatcca gaaaagcggc cattttccac catgatattc ggcaagcagg catcgccgtg 12720
ggtcacgacg agatcctcgc cgtcgggcat ccgcgccttg agcctggcga acagttcggc 12780
tggcgcgagc ccctgatgct cttcgtccag atcatcctga tcgacaagac cggcttccat 12840
ccgagtacgt gctcgctcga tgcgatgttt cgcttggtgg tcgaatgggc aggtagccgg 12900
atcaagcgta tgcagccgcc gcattgcatc agccatgatg gatactttct cggcaggagc 12960
aaggtgagat gacaggagat cctgccccgg cacttcgccc aatagcagcc agtcccttcc 13020
cgcttcagtg acaacgtcga gcacagctgc gcaaggaacg cccgtcgtgg ccagccacga 13080
tagccgcgct gcctcgtctt ggagttcatt cagggcaccg gacaggtcgg tcttgacaaa 13140
aagaaccggg cgcccctgcg ctgacagccg gaacacggcg gcatcagagc agccgattgt 13200
ctgttgtgcc cagtcatagc cgaatagcct ctccacccaa gcggccggag aacctgcgtg 13260
caatccatct tgttcaatca tgcctcgatc gagttgagag tgaatatgag actctaattg 13320
gataccgagg ggaatttatg gaacgtcagt ggagcatttt tgacaagaaa tatttgctag 13380
ctgatagtga ccttaggcga cttttgaacg cgcaataatg gtttctgacg tatgtgctta 13440

gctcattaaa ctccagaaac ccgcggctga gtggctcctt caacgttgcg gttctgtcag 13500

ttccaaacgt aaaacggctt gtcccgcgtc atcggcgggg gtcataacgt gactccctta 13560

attctcatgt atgataattc gagggtaccc ggggatcct 13599

<210> 118
<211> 11135
<212> DNA
<213> Artificial

<220>
<223> Cloning vector pKSGW7

<400> 118

ctcccatatg gtcgactaga gccaagctga tctcctttgc cccggagatc accatggacg 60
actttctcta tctctacgat ctaggaagaa agttcgacgg agaaggtgac gataccatgt 120
tcaccaccga taatgagaag attagcctct tcaatttcag aaagaatgct gacccacaga 180
tggttagaga ggcctacgcg gcaggtctca tcaagacgat ctacccgagt aataatctcc 240
aggagatcaa ataccttccc aagaaggtta aagatgcagt caaaagattc aggactaact 300
gcatcaagaa cacagagaaa gatatatttc tcaagatcag aagtactatt ccagtatgga 360
cgattcaagg cttgcttcat aaaccaaggc aagtaataga gattggagtc tctaagaaag 420
tagttcctac tgaatcaaag gccatggagt caaaaattca gatcgaggat ctaacagaac 480
tcgccgtgaa gactggcgaa cagttcatac agagtctttt acgactcaat gacaagaaga 540
aaatcttcgt caacatggtg gagcacgaca ctctcgtcta ctccaagaat atcaaagata 600
cagtctcaga agaccaaagg gctattgaga cttttcaaca aagggtaata tcgggaaacc 660
tcctcggatt ccattgccca gctatctgtc acttcatcaa aaggacagta gaaaaggaag 720
gtggcaccta caaatgccat cattgcgata aaggaaaggc tatcgttcaa gatgcctctg 780
ccgacagtgg tcccaaagat ggacccccac ccacgaggag catcgtggaa aaagaagacg 840
ttccaaccac gtcttcaaag caagtggatt gatgtgatat ctccactgac gtaagggatg 900
acgcacaatc ccactatcct tcgcaagacc cttcctctat ataaggaagt tcatttcatt 960
tggagaggac tccggtattt ttacaacaat accacaacaa aacaaacaac aaacaacatt 1020
acaatttact attctagtcg acctgcaggc ggccgcacta gtgatatcac aagtttgtac 1080
aaaaaagctg aacgagaaac gtaaaatgat ataaatatca atatattaaa ttagattttg 1140
cataaaaaac agactacata atactgtaaa acacaacata tccagtcact atggcggccg 1200
cattaggcac cccaggcttt acactttatg cttccggctc gtataatgtg tggattttga 1260
gttaggatcc ggcgagattt tcaggagcta aggaagctaa aatggagaaa aaaatcactg 1320
gatataccac cgttgatata tcccaatggc atcgtaaaga acattttgag gcatttcagt 1380
cagttgctca atgtacctat aaccagaccg ttcagctgga tattacggcc tttttaaaga 1440

ccgtaaagaa aaataagcac aagttttatc cggcctttat tcacattctt gcccgcctga 1500
tgaatgctca tccggaattc cgtatggcaa tgaaagacgg tgagctggtg atatgggata 1560
gtgttcaccc ttgttacacc gttttccatg agcaaactga aacgttttca tcgctctgga 1620
gtgaatacca cgacgatttc cggcagtttc tacacatata ttcgcaagat gtggcgtgtt 1680
acggtgaaaa cctggcctat ttccctaaag ggtttattga gaatatgttt ttcgtctcag 1740
ccaatccctg ggtgagtttc accagttttg atttaaacgt ggccaatatg gacaacttct 1800
tcgcccccgt tttcaccatg ggcaaatatt atacgcaagg cgacaaggtg ctgatgccgc 1860
tggcgattca ggttcatcat gccgtctgtg atggcttcca tgtcggcaga atgcttaatg 1920
aattacaaca gtactgcgat gagtggcagg gcggggcgta aacgcgtgga tccggcttac 1980
taaaagccag ataacagtat gcgtatttgc gcgctgattt ttgcggtata agaatatata 2040
ctgatatgta tacccgaagt atgtcaaaaa gaggtgtgct atgaagcagc gtattacagt 2100
gacagttgac agcgacagct atcagttgct caaggcatat atgatgtcaa tatctccggt 2160
ctggtaagca caaccatgca gaatgaagcc cgtcgtctgc gtgccgaacg ctggaaagcg 2220
gaaaatcagg aagggatggc tgaggtcgcc cggtttattg aaatgaacgg ctcttttgct 2280
gacgagaaca gggactggtg aaatgcagtt taaggtttac acctataaaa gagagagccg 2340
ttatcgtctg tttgtggatg tacagagtga tattattgac acgcccgggc gacggatggt 2400
gatccccctg gccagtgcac gtctgctgtc agataaagtc tcccgtgaac tttacccggt 2460
ggtgcatatc ggggatgaaa gctggcgcat gatgaccacc gatatggcca gtgtgccggt 2520
ctccgttatc ggggaagaag tggctgatct cagccaccgc gaaaatgaca tcaaaaacgc 2580
cattaacctg atgttctggg gaatataaat gtcaggctcc cttatacaca gccagtctgc 2640
aggtcgacca tagtgactgg atatgttgtg ttttacagta ttatgtagtc tgttttttat 2700
gcaaaatcta atttaatata ttgatattta tatcatttta cgtttctcgt tcagctttct 2760
tgtacaaagt ggtgatatcc cgcggccatg ctagagtccg caaaaatcac cagtctctct 2820
ctacaaatct atctctctct atttttctcc agaataatgt gtgagtagtt cccagataag 2880
ggaattaggg ttcttatagg gtttcgctca tgtgttgagc atataagaaa cccttagtat 2940
gtatttgtat ttgtaaaata cttctatcaa taaaatttct aattcctaaa accaaaatcc 3000
agtgacctgc aggcatgcga cgtcgggccc aagcttagct tgagcttgga tcagattgtc 3060
gtttcccgcc ttcagtttaa actatcagtg tttgacagga tatattggcg ggtaaaccta 3120
agagaaaaga gcgtttatta gaataacgga tatttaaaag ggcgtgaaaa ggtttatccg 3180
ttcgtccatt tgtatgtgca tgccaaccac agggttcccc tcgggatcaa agtactttga 3240
tccaacccct ccgctgctat agtgcagtcg gcttctgacg ttcagtgcag ccgtcttctg 3300
aaaacgacat gtcgcacaag tcctaagtta cgcgacaggc tgccgccctg ccctttttcct 3360
ggcgttttct tgtcgcgtgt tttagtcgca taaagtagaa tacttgcgac tagaaccgga 3420

gacattacgc catgaacaag agcgccgccg ctggcctgct gggctatgcc cgcgtcagca 3480
ccgacgacca ggacttgacc aaccaacggg ccgaactgca cgcggccggc tgcaccaagc 3540
tgttttccga gaagatcacc ggcaccaggc gcgaccgccc ggagctggcc aggatgcttg 3600
accacctacg ccctggcgac gttgtgacag tgaccaggct agaccgcctg gcccgcagca 3660
cccgcgacct actggacatt gccgagcgca tccaggaggc cggcgcgggc ctgcgtagcc 3720
tggcagagcc gtgggccgac accaccacgc cggccggccg catggtgttg accgtgttcg 3780
ccggcattgc cgagttcgag cgttccctaa tcatcgaccg cacccggagc gggcgcgagg 3840
ccgccaaggc ccgaggcgtg aagtttggcc cccgccctac cctcaccccg gcacagatcg 3900
cgcacgcccg cgagctgatc gaccaggaag gccgcaccgt gaaagaggcg gctgcactgc 3960
ttggcgtgca tcgctcgacc ctgtaccgcg cacttgagcg cagcgaggaa gtgacgccca 4020
ccgaggccag gcggcgcggt gccttccgtg aggacgcatt gaccgaggcc gacgccctgg 4080
cggccgccga gaatgaacgc caagaggaac aagcatgaaa ccgcaccagg acggccagga 4140
cgaaccgttt ttcattaccg aagagatcga ggcggagatg atcgcggccg ggtacgtgtt 4200
cgagccgccc gcgcacgtct caaccgtgcg gctgcatgaa atcctggccg gtttgtctga 4260
tgccaagctg gcggcctggc cggccagctt ggccgctgaa gaaaccgagc gccgccgtct 4320
aaaaaggtga tgtgtatttg agtaaaacag cttgcgtcat gcggtcgctg cgtatatgat 4380
gcgatgagta aataaacaaa tacgcaaggg gaacgcatga aggttatcgc tgtacttaac 4440
cagaaaggcg ggtcaggcaa gacgaccatc gcaacccatc tagcccgcgc cctgcaactc 4500
gccggggccg atgttctgtt agtcgattcc gatccccagg gcagtgcccg cgattgggcg 4560
gccgtgcggg aagatcaacc gctaaccgtt gtcggcatcg accgcccgac gattgaccgc 4620
gacgtgaagg ccatcggccg gcgcgacttc gtagtgatcg acggagcgcc ccaggcggcg 4680
gacttggctg tgtccgcgat caaggcagcc gacttcgtgc tgattccggt gcagccaagc 4740
ccttacgaca tatgggccac cgccgacctg gtggagctgg ttaagcagcg cattgaggtc 4800
acggatggaa ggctacaagc ggcctttgtc gtgtcgcggg cgatcaaagg cacgcgcatc 4860
ggcggtgagg ttgccgaggc gctggccggg tacgagctgc ccattcttga gtcccgtatc 4920
acgcagcgcg tgagctaccc aggcactgcc gccgccggca caaccgttct tgaatcagaa 4980
cccgagggcg acgctgcccg cgaggtccag gcgctggccg ctgaaattaa atcaaaactc 5040
atttgagtta atgaggtaaa gagaaaatga gcaaaagcac aaacacgcta agtgccggcc 5100
gtccgagcgc acgcagcagc aaggctgcaa cgttggccag cctggcagac acgccagcca 5160
tgaagcgggt caactttcag ttgccggcgg aggatcacac caagctgaag atgtacgcgg 5220
tacgccaagg caagaccatt accgagctgc tatctgaata catcgcgcag ctaccagagt 5280
aaatgagcaa atgaataaat gagtagatga attttagcgg ctaaaggagg cggcatggaa 5340

aatcaagaac aaccaggcac cgacgccgtg gaatgcccca tgtgtggagg aacgggcggt 5400
tggccaggcg taagcggctg ggttgtctgc cggccctgca atggcactgg aacccccaag 5460
cccgaggaat cggcgtgacg gtcgcaaacc atccggcccg gtacaaatcg gcgcggcgct 5520
gggtgatgac ctggtggaga agttgaaggc cgcgcaggcc gcccagcggc aacgcatcga 5580
ggcagaagca cgccccggtg aatcgtggca agcggccgct gatcgaatcc gcaaagaatc 5640
ccggcaaccg ccggcagccg gtgcgccgtc gattaggaag ccgcccaagg gcgacgagca 5700
accagatttt ttcgttccga tgctctatga cgtgggcacc cgcgatagtc gcagcatcat 5760
ggacgtggcc gttttccgtc tgtcgaagcg tgaccgacga gctggcgagg tgatccgcta 5820
cgagcttcca gacgggcacg tagaggtttc cgcagggccg gccggcatgg ccagtgtgtg 5880
ggattacgac ctggtactga tggcggtttc ccatctaacc gaatccatga accgataccg 5940
ggaagggaag ggagacaagc ccggccgcgt gttccgtcca cacgttgcgg acgtactcaa 6000
gttctgccgg cgagccgatg gcggaaagca gaaagacgac ctggtagaaa cctgcattcg 6060
gttaaacacc acgcacgttg ccatgcagcg tacgaagaag gccaagaacg gccgcctggt 6120
gacggtatcc gagggtgaag ccttgattag ccgctacaag atcgtaaaga gcgaaaccgg 6180
gcggccggag tacatcgaga tcgagctagc tgattggatg taccgcgaga tcacagaagg 6240
caagaacccg gacgtgctga cggttcaccc cgattacttt ttgatcgatc ccggcatcgg 6300
ccgttttctc taccgcctgg cacgccgcgc cgcaggcaag gcagaagcca gatggttgtt 6360
caagacgatc tacgaacgca gtggcagcgc cggagagttc aagaagttct gtttcaccgt 6420
gcgcaagctg atcgggtcaa atgacctgcc ggagtacgat ttgaaggagg aggcggggca 6480
ggctggcccg atcctagtca tgcgctaccg caacctgatc gagggcgaag catccgccgg 6540
ttcctaatgt acggagcaga tgctagggca aattgcccta gcaggggaaa aaggtcgaaa 6600
aggtctcttt cctgtggata gcacgtacat tgggaaccca aagccgtaca ttgggaaccg 6660
gaacccgtac attgggaacc caaagccgta cattgggaac cggtcacaca tgtaagtgac 6720
tgatataaaa gagaaaaaag gcgatttttc cgcctaaaac tctttaaaac ttattaaaac 6780
tcttaaaacc cgcctggcct gtgcataact gtctggccag cgcacagccg aagagctgca 6840
aaaagcgcct acccttcggt cgctgcgctc cctacgcccc gccgcttcgc gtcggcctat 6900
cgcggccgct ggccgctcaa aaatggctgg cctacggcca ggcaatctac cagggcgcgg 6960
acaagccgcg ccgtcgccac tcgaccgccg gcgcccacat caaggcaccc tgcctcgcgc 7020
gtttcggtga tgacggtgaa aacctctgac acatgcagct cccggagacg gtcacagctt 7080
gtctgtaagc ggatgccggg agcagacaag cccgtcaggg cgcgtcagcg ggtgttggcg 7140
ggtgtcgggg cgcagccatg acccagtcac gtagcgatag cggagtgtat actggcttaa 7200
ctatgcggca tcagagcaga ttgtactgag agtgcaccat atgcggtgtg aaataccgca 7260
cagatgcgta aggagaaaat accgcatcag gcgctcttcc gcttcctcgc tcactgactc 7320

gctgcgctcg gtcgttcggc tgcggcgagc ggtatcagct cactcaaagg cggtaatacg 7380
gttatccaca gaatcagggg ataacgcagg aaagaacatg tgagcaaaag gccagcaaaa 7440
ggccaggaac cgtaaaaagg ccgcgttgct ggcgtttttc cataggctcc gcccccctga 7500
cgagcatcac aaaaatcgac gctcaagtca gaggtggcga aacccgacag gactataaag 7560
ataccaggcg tttccccctg gaagctccct cgtgcgctct cctgttccga ccctgccgct 7620
taccggatac ctgtccgcct ttctcccttc gggaagcgtg gcgctttctc atagctcacg 7680
ctgtaggtat ctcagttcgg tgtaggtcgt tcgctccaag ctgggctgtg tgcacgaacc 7740
ccccgttcag cccgaccgct gcgccttatc cggtaactat cgtcttgagt ccaacccggt 7800
aagacacgac ttatcgccac tggcagcagc cactggtaac aggattagca gagcgaggta 7860
tgtaggcggt gctacagagt tcttgaagtg gtggcctaac tacggctaca ctagaaggac 7920
agtatttggt atctgcgctc tgctgaagcc agttaccttc ggaaaaagag ttggtagctc 7980
ttgatccggc aaacaaacca ccgctggtag cggtggtttt tttgtttgca agcagcagat 8040
tacgcgcaga aaaaaaggat ctcaagaaga tcctttgatc ttttctacgg ggtctgacgc 8100
tcagtggaac gaaaactcac gttaagggat tttggtcatg catgatatat ctcccaattt 8160
gtgtagggct tattatgcac gcttaaaaat aataaaagca gacttgacct gatagtttgg 8220
ctgtgagcaa ttatgtgctt agtgcatcta atcgcttgag ttaacgccgg cgaagcggcg 8280
tcggcttgaa cgaatttcta gctagacatt atttgccgac taccttggtg atctcgcctt 8340
tcacgtagtg gacaaattct tccaactgat ctgcgcgcga ggccaagcga tcttcttctt 8400
gtccaagata agcctgtcta gcttcaagta tgacgggctg atactgggcc ggcaggcgct 8460
ccattgccca gtcggcagcg acatccttcg gcgcgatttt gccggttact gcgctgtacc 8520
aaatgcggga caacgtaagc actacatttc gctcatcgcc agcccagtcg ggcggcgagt 8580
tccatagcgt taaggtttca tttagcgcct caaatagatc ctgttcagga accggatcaa 8640
agagttcctc cgccgctgga cctaccaagg caacgctatg ttctcttgct tttgtcagca 8700
agatagccag atcaatgtcg atcgtggctg gctcgaagat acctgcaaga atgtcattgc 8760
gctgccattc tccaaattgc agttcgcgct tagctggata acgccacgga atgatgtcgt 8820
cgtgcacaac aatggtgact tctacagcgc ggagaatctc gctctctcca ggggaagccg 8880
aagtttccaa aaggtcgttg atcaaagctc gccgcgttgt ttcatcaagc cttacggtca 8940
ccgtaaccag caaatcaata tcactgtgtg gcttcaggcc gccatccact gcggagccgt 9000
acaaatgtac ggccagcaac gtcggttcga gatggcgctc gatgacgcca actacctctg 9060
atagttgagt cgatacttcg gcgatcaccg cttcccccat gatgtttaac tttgttttag 9120
ggcgactgcc ctgctgcgta acatcgttgc tgctccataa catcaaacat cgacccacgg 9180
cgtaacgcgc ttgctgcttg gatgcccgag gcatagactg taccccaaaa aaacatgtca 9240

taacaagaag ccatgaaaac cgccactgcg ccgttaccac cgctgcgttc ggtcaaggtt 9300
ctggaccagt tgcgtgacgg cagttacgct acttgcatta cagcttacga accgaacgag 9360
gcttatgtcc actgggttcg tgcccgaatt gatcacaggc agcaacgctc tgtcatcgtt 9420
acaatcaaca tgctaccctc cgcgagatca tccgtgtttc aaacccggca gcttagttgc 9480
cgttcttccg aatagcatcg gtaacatgag caaagtctgc cgccttacaa cggctctccc 9540
gctgacgccg tcccggactg atgggctgcc tgtatcgagt ggtgattttg tgccgagctg 9600
ccggtcgggg agctgttggc tggctggtgg caggatatat tgtggtgtaa acaaattgac 9660
gcttagacaa cttaataaca cattgcggac gtttttaatg tactgaatta acgccgaatt 9720
gaattatcag cttgcatgcc ggtcgatcta gtaacataga tgacaccgcg cgcgataatt 9780
tatcctagtt tgcgcgctat attttgtttt ctatcgcgta ttaaatgtat aattgcggga 9840
ctctaatcat aaaaacccat ctcataaata acgtcatgca ttacatgtta attattacat 9900
gcttaacgta attcaacaga aattatatga taatcatcgc aagaccggca acaggattca 9960
atcttaagaa actttattgc caaatgtttg aacgatctgc ttgactctag ctagagtccg 10020
aaccccagag tcccgctcag aagaactcgt caagaaggcg atagaaggcg atgcgctgcg 10080
aatcgggagc ggcgataccg taaagcacga ggaagcggtc agcccattcg ccgccaagct 10140
cttcagcaat atcacgggta gccaacgcta tgtcctgata gcggtccgcc acacccagcc 10200
ggccacagtc gatgaatcca gaaaagcggc cattttccac catgatattc ggcaagcagg 10260
catcgccgtg ggtcacgacg agatcctcgc cgtcgggcat ccgcgccttg agcctggcga 10320
acagttcggc tggcgcgagc ccctgatgct cttcgtccag atcatcctga tcgacaagac 10380
cggcttccat ccgagtacgt gctcgctcga tgcgatgttt cgcttggtgg tcgaatgggc 10440
aggtagccgg atcaagcgta tgcagccgcc gcattgcatc agccatgatg gatactttct 10500
cggcaggagc aaggtgagat gacaggagat cctgccccgg cacttcgccc aatagcagcc 10560
agtcccttcc cgcttcagtg acaacgtcga gcacagctgc gcaaggaacg cccgtcgtgg 10620
ccagccacga tagccgcgct gcctcgtctt ggagttcatt cagggcaccg gacaggtcgg 10680
tcttgacaaa aagaaccggg cgcccctgcg ctgacagccg gaacacggcg gcatcagagc 10740
agccgattgt ctgttgtgcc cagtcatagc cgaatagcct ctccacccaa gcggccggag 10800
aacctgcgtg caatccatct tgttcaatca tgcctcgatc gagttgagag tgaatatgag 10860
actctaattg gataccgagg ggaatttatg gaacgtcagt ggagcatttt tgacaagaaa 10920
tatttgctag ctgatagtga ccttaggcga cttttgaacg cgcaataatg gtttctgacg 10980
tatgtgctta gctcattaaa ctccagaaac ccgcggctga gtggctcctt caacgttgcg 11040
gttctgtcag ttccaaacgt aaaacggctt gtcccgcgtc atcggcgggg gtcataacgt 11100
gactccctta attctcatgt atgataattc gagct 11135

<210> 119
<211> 20
<212> DNA
<213> Artificial

<220>
<223> Construct specific primer

<400> 119
gggtaagatc aacttgaagg 20

<210> 120
<211> 20
<212> DNA
<213> Artificial

<220>
<223> Construct gene specific primer

<400> 120
acttctccac aagagcattc 20

<210> 121
<211> 22
<212> DNA
<213> Artificial

<220>
<223> Construct-specific forward primer

<400> 121
gtctatggat cggagcattg tg 22

<210> 122
<211> 20
<212> DNA
<213> Artificial

<220>
<223> Construct-specific reverse primer

<400> 122
cagcagcaac atcctcagtg 20

<210> 123
<211> 22
<212> DNA
<213> Artificial

<220>
<223> PCR primer

<400> 123
cactatgtcc tctacttcct tg 22

<210> 124
<211> 19
<212> DNA

<213> Artificial

<220>
<223> PCR primer

<400> 124
atggtcttgg gtcttcctg 19

<210> 125
<211> 20
<212> DNA
<213> Artificial

<220>
<223> PCR primer

<400> 125
cactggtacg tgtagctgat 20

<210> 126
<211> 20
<212> DNA
<213> Artificial

<220>
<223> PCR primer

<400> 126
gtaagaggtc cacctttacc 20

<210> 127
<211> 52
<212> DNA
<213> Artificial

<220>
<223> Forward primer

<400> 127
ggggaccact ttgtacaaga aagctgggtt ccgagaaaga aaatggtgac cg 52

<210> 128
<211> 53
<212> DNA
<213> Artificial

<220>
<223> Forward primer

<400> 128
ggggaccact ttgtacaaga aagctgggta accaaccctg tacaaatacc acg 53

<210> 129
<211> 52
<212> DNA
<213> Artificial

<220>

<223> Forward Primer

<400> 129
ggggaccact ttgtacaaga aagctgggtg ctgcagaaca gatgcttact cc 52

<210> 130
<211> 52
<212> DNA
<213> Artificial

<220>
<223> Forward primer

<400> 130
ggggaccact ttgtacaaga aagctgggtg aagaattagc aatgggcaaa cc 52

<210> 131
<211> 52
<212> DNA
<213> Artificial

<220>
<223> Forward primer

<400> 131
ggggaccact ttgtacaaga aagctgggta ttcacatggg aaaggtagtg gg 52

<210> 132
<211> 53
<212> DNA
<213> Artificial

<220>
<223> Forward primer

<400> 132
ggggaccact ttgtacaaga aagctgggtt ttctctaggt cccagacagt tgg 53

<210> 133
<211> 52
<212> DNA
<213> Artificial

<220>
<223> Forward primer

<400> 133
ggggaccact ttgtacaaga aagctgggtt aagtggtcca cgaaacagtt gg 52

<210> 134
<211> 52
<212> DNA
<213> Artificial

<220>
<223> Reverse primer

<400> 134

ggggacaagt ttgtacaaaa aagcaggctt ctttgcctaa ctttgggacc tc 52

<210> 135
<211> 50
<212> DNA
<213> Artificial

<220>
<223> Reverse primer

<400> 135
ggggacaagt ttgtacaaaa aagcaggcta atcggaaccg tctgtcttgg 50

<210> 136
<211> 50
<212> DNA
<213> Artificial

<220>
<223> Reverse primer

<400> 136
ggggacaagt ttgtacaaaa aagcaggctt tacaagatgc tccgaatggg 50

<210> 137
<211> 53
<212> DNA
<213> Artificial

<220>
<223> Reverse primer

<400> 137
ggggacaagt ttgtacaaaa aagcaggctc agaaaacaga taatcaacga gcg 53

<210> 138
<211> 51
<212> DNA
<213> Artificial

<220>
<223> Reverse primer

<400> 138
ggggacaagt ttgtacaaaa aagcaggctg aagcacaacg gaagactgag g 51

<210> 139
<211> 54
<212> DNA
<213> Artificial

<220>
<223> Reverse primer

<400> 139
ggggacaagt ttgtacaaaa aagcaggctt tgataaggtt tgtctaatct cccg 54

<210> 140

<211> 54
<212> DNA
<213> Artificial

<220>
<223> Reverse primer

<400> 140
ggggacaagt ttgtacaaaa aagcaggctc gcttttgttc ttattatagc tggc 54

<210> 141
<211> 54
<212> DNA
<213> Artificial

<220>
<223> Forward primer

<400> 141
ggggacaagt ttgtacaaaa aagcaggctg agttcttgag ctaaacgaga ggag 54

<210> 142
<211> 23
<212> DNA
<213> Artificial

<220>
<223> Forward primer

<400> 142
cggtcttatt tgtattcgcc tgc 23

<210> 143
<211> 19
<212> DNA
<213> Artificial

<220>
<223> Forward primer

<400> 143
atgcaaccag gcttggatg 19

<210> 144
<211> 27
<212> DNA
<213> Artificial

<220>
<223> Forward primer

<400> 144
atggtggaga agatcatcaa tatgaac 27

<210> 145
<211> 20
<212> DNA
<213> Artificial

<220>
<223> Forward primer

<400> 145
atggagtttg atgggctggc 20

<210> 146
<211> 23
<212> DNA
<213> Artificial

<220>
<223> Forward primer

<400> 146
atgttgatgg ctttaaaatc ccg 23

<210> 147
<211> 23
<212> DNA
<213> Artificial

<220>
<223> Forward primer

<400> 147
atggacaaca atacctctcg ctc 23

<210> 148
<211> 25
<212> DNA
<213> Artificial

<220>
<223> Forward primer

<400> 148
atggaaacaa agtgtccagt agatg 25

<210> 149
<211> 55
<212> DNA
<213> Artificial

<220>
<223> Reverse primer

<400> 149
ggggaccact ttgtacaaga aagctgggtt aactcaccaa attaagatca aagcc 55

<210> 150
<211> 26
<212> DNA
<213> Artificial

<220>
<223> Reverse primer

<400> 150
gcaaattagc aacacatgaa cctacc 26

<210> 151
<211> 22
<212> DNA
<213> Artificial

<220>
<223> Reverse primer

<400> 151
taaatgcagt aaatgatgga gc 22

<210> 152
<211> 28
<212> DNA
<213> Artificial

<220>
<223> Reverse primer

<400> 152
attacacaaa tattcacact agaaaggg 28

<210> 153
<211> 22
<212> DNA
<213> Artificial

<220>
<223> Reverse primer

<400> 153
caggggatat ctagcttagg gc 22

<210> 154
<211> 22
<212> DNA
<213> Artificial

<220>
<223> Reverse primer

<400> 154
gaagttaacg ccaagcagat gc 22

<210> 155
<211> 27
<212> DNA
<213> Artificial

<220>
<223> Reverse primer

<400> 155
gacaaactta tggacccgtt aataatc 27

<210> 156
<211> 24
<212> DNA
<213> Artificial

<220>
<223> Reverse primer

<400> 156
ctactttttg ttggggtctt gctc 24

<210> 157
<211> 336
<212> DNA
<213> Artificial

<220>
<223> Construct sequence

<400> 157

```
tccgagaaag aaaatggtga ccgttgatga aattcggaag gcgcagaggg cagagggccc     60
tgccaccatc ttggccatcg gaacatcaac ccctccgaac tgtgttgatc aaagcaccta    120
tcctgactat tattttcgta tcacaaacag cgagcataag gtggagctta aagaaaaatt    180
caagcgaatg tgcgagaaat ccatgatcaa gaagagatac atgcacttga cggaggagat    240
cttgaaagaa aatcctagtg tatgcgaata catggcacct tcattggatg ctaggcaaga    300
catggtggtg gtggaggtcc caaagttagg caaaga                              336
```

<210> 158
<211> 322
<212> DNA
<213> Artificial

<220>
<223> Construct seqeunce

<400> 158

```
aaccaaccct gtacaaatac cacgggttcc aacacagtac caggcactga gccgcagttc     60
cccaattacc cgagccttct aaccaaatgc catttgaatg tctccaacac aagggatgga    120
gctgtgatgc ctgcttgtgt tcccaacctg gatccaagtc ttgtgctcac taacttcaca    180
cggactacat actccgataa ccttaatgat actaagttta caggagctgg gattggttct    240
gatggtaact ggatagttgt tgttctgacc acaagcacac ccgagggaag ttttgtgact    300
tccaagacag acggttccga tt                                             322
```

<210> 159
<211> 349
<212> DNA

<213> Artificial

<220>
<223> Construct sequence

<400> 159

```
gctgcagaac agatgcttac tccagcttct ccagctctcc cattctctct atgattgccc      60
tcttgcttgt ttcttgaagc tccccagcga gaatgaattc atcaagtatc aaataaacct     120
tgtgaaagtt aaacaccaaa tctagctcac acacattgct gaagaaatga tccaatatct     180
ccacaaataa atgaatgcac tccaaataag ccaattcgtt atccgttatg tcgacacaca     240
gcgaaaaaaa caatccagca taccgcctgt gtttttgaaa ggcaatcttg gaattttttt     300
ttcctttgtt ttttttccct gtggtctccc cattcggagc atcttgtaa                 349
```

<210> 160
<211> 326
<212> DNA
<213> Artificial

<220>
<223> Construct sequence

<400> 160

```
gaagaattag caatgggcaa accagatggt aaacgagctt ctcctggaag tcaggtcagt      60
gtgcactata ttggtaagct gaagaacgga aaagtatttg actctaatgt cggaagggct     120
ccctttaaat tccgcctagg tgttggacaa gtcataaaag gatgggatgt tggagttaat     180
ggtatgcgag ttggggacaa aagaagactc acagttccac catcgatggg ttacggagat     240
cggggtgctg gcgggaagat acctccaaac tcatggcttg tgtttgatgt tgagttggtg     300
aacgctcgtt gattatctgt tttctg                                          326
```

<210> 161
<211> 296
<212> DNA
<213> Artificial

<220>
<223> Construct sequence

<400> 161

attcacatgg gaaaggtagt gggtccctta tcagtggggc gatgggaggg ttaagtttgg 60

aagaagggag taactacgag aaagagaaag ttgctactga taaggagagt cacagctacc 120

atgattatcg tcgcgaatat tgaaggaata gagagactgt gtgcgtgtgt gtgtgaaggt 180

atatttgagc tttttatact cgcattgtgg ctttgactga tggatatact tttgtatgta 240

catgcaaaaa attatcataa ctgctagatg ctgccctcag tcttccgttg tgcttc 296

<210> 162
<211> 248
<212> DNA
<213> Artificial

<220>
<223> Construct sequence

<400> 162

tttctctagg tcccagacag ttgggaacca acagcatcaa ttctgcaaat caaggaatat 60

cttacataaa ggaaggaaga aaaattcagg ggcaaaagat gtttcttcga cctgacctct 120

cttctcatct tcgagatgaa gcagcttcag ttgtctcatc attttctggg ccattatctc 180

cctgaattcc aattcggaca tttgaatcga aaatcacatg tcacgggaga ttagacaaac 240

cttatcaa 248

<210> 163
<211> 234
<212> DNA
<213> Artificial

<220>
<223> Constsruct sequence

<400> 163

taagtggtcc acgaaacagt tggtgatttg cactccaact aggttatgct ctttaattgg 60

ttggatttct tttgacaggc aaacttgcct gttttagtgt gctctataat aacagtcttg 120

aaaaataagt gtacttttaa gacaacaatg catatggtat ttcatcaatt aagtgaagtt 180

tctgtatttg aatggaaagc tgatgagagg ccagctataa taagaacaaa agcg 234

<210> 164
<211> 1176
<212> DNA
<213> Populus trichocarpa

<400> 164

atggtgaccg ttgatgaaat tcggaaggcg cagagggcag agggccctgc caccatcttg 60

gccattggaa catcaacccc tccgaattgt gttgatcaaa gcacctatcc tgactattat 120

tttcgtatca ccaacagcga gcataaggtg gagcttaaag aaaagttcaa gcgaatgtgc 180

gagaaatcca tgatcaagaa gaggtacatg cacttgactg aggagatctt gaaagaaaat 240

cctagtgtgt gcgaatacat ggcaccttca ttggatgcta ggcaggacat ggtggtggtc 300

gagataccaa agttgggcaa agaagcagct gccaaggcca tcaaggaatg gggccagccc 360

aagtccaaaa taacccattt ggtcttttgc acaaccagtg gtgttgacat gcccggggct 420

gactatcaac tcactaagct cttgggtctt cgctcatcag taaaacgttt catgatgtac 480

cagcaaggtt gttttgctgg tgggacggtg ctccgcctag ctaaagacct tgctgaaaac 540

aacaaaggtg ctcgcgtgct agtcgtgtgc tcagaaataa cagcagtgac atttcgtggc 600

cctagtgaca cccacctcga tagtcttgtg ggtcaagcgt tgtttggtga cggcgcagct 660

gctatcataa taggctcgga tcctgtgctg gggtcgaga agcctttgtt tgagctggtg 720

tctgccgccc agactattct acccgatagt gaggggcta tagatggaca tcttcgcgaa 780

gttgggctta catttcacct tctcaaggat gtgcccgggc ttatttcaaa aaacgtcgaa 840

aagagtctaa ccgaggcttt caagcccttg ggcatctcgg attggaactc ccttttctgg 900

attgcgcacc ctggtgggcc cgcaattctg gaccaggtgg aggctaagtt ggagcttaaa 960

cctgaaaaac tgcgagccac tcgacaagta ctggctgact atggtaacat gtccagtgca 1020

tgtgtgctat ttattttgga cgagatgagg aagaaatctg ccaaggatgg gcttaaatct 1080

actggagaag ggttggaatg gggcgtcctt ttcgggttcg ggcctgggct tactgttgag 1140

acagtggtgc tccacagtct gcctgccaca atttag 1176

<210> 165
<211> 618
<212> DNA
<213> Populus trichocarpa

<400> 165

atggcttctt caagattttc tcttctcttc ccattctttg ttttcttcat tattctctgc 60
ttgatcagtc accctgtcat ttgtgacggt gatcaggagg atgcccttct tcaagggatt 120
aacaactaca ggacatcctt caatttgact accctcacaa aaaatgataa cgcagagtgc 180
cttgctgagg agatagctga ccaatttaag aaccaacctt gtacaaatac cacgggttcc 240
aacacagtac caggcactga gccgcagttc cccaattacc cgagccttct agccaaatgc 300
catttgaatg tctccaacac aagggatgga gctgtgatgc ctgcttgtgt tccccacctg 360
gatccaagtc ttgtgctcac taacttcaca cggactccat actccgataa ccttaatgat 420
actaagttta caggagctgg gattggttct gatggtaact ggatcgttgt tgttctgacc 480
acaagcacac ctgagggaag ttatgtgact tccaagacag acggttcgga ttacaatgca 540
gccaatttga ccgccaagaa tactggtcta atctatcact tgctgttttt gctgataggt 600
tctctcttct tgttgtga 618

<210> 166
<211> 429
<212> DNA
<213> Populus trichocarpa

<400> 166

atgatccgat ttatactcct gcaaaacaga caaggcaaga ctcgtctcgc taaatactat 60
gttcctcttg aggattccga gaagcacaaa gtcgaatacg aggttcaccg attggttgtt 120
aatagagatg ctaaattcac gaattttgtt gagtttagga cacacaaggt gatatacagg 180
cggtatgctg gattgttttt ttcgctgtgt gtcgacataa cggataacga attggcttat 240
ttggagtgca ttcatttatt tgtggagata ttggatcatt tcttcagcaa tgtgtgtgag 300
ctagatttgg tgtttaactt tcacaaggtt tatttgatac ttgatgaatt cattctcgct 360
ggggagcttc aagaaacaag caagagggca atcatagaga gaatgggaga gctggagaag 420
ctggagtaa 429

<210> 167
<211> 1503
<212> DNA
<213> Populus tricocharpa

<400> 167

atgagcttct ggggtattga agtgaaacca ggcaagcctc atccttatca ctctgatgat 60
gtgcaaggaa agcttcgggt aactcaggca actttgggcc ttggttcgtc aaaagaaagg 120
agcatactcc agtgttcagt tggacacagg agtccaattt ttgtatgttc actgttgcca 180
gggaaagctg aaagttgctc tctgaatctt gaatttacgg atgaattagt agcgttctca 240

```
gttattggac cacaaagcat ccatctttgt ggttactttg attctgtaga aggagatcac     300
ctccgagatg aatatgaata tgattcggga gaggatattg ttgatacgga gtcggatgag     360
tccagtgaat atgattatga tgatgaatat gatgacgacg atgatgatct tcaaatgtat     420
tccccttcgc ctgttcccaa aagtggagtt gtaattgagg agataactga agatgacaaa     480
cctacaaaag aaaatggcaa gtctaaacgg ctaaagaaga agaataatca atcaagtgac     540
aaggaagacc aaaacaactc tcaacaacaa attgttctta agagggatag aggtatttct     600
gttctggaaa gtgaagatga agatggtttt ccaatttctt cctctgcaaa aagcaaagat     660
acagttcagg agcagcaagc agaactagat ggacaaaaag ataaggaaac aacccaagag     720
actaagaaaa agatggcaag tgaggataat gatgacacta gaaaaaagag aaaagttaaa     780
agcgttgatc aagatggtca accagaaagg aaaacaaaga aaaagaagaa gcagagagaa     840
cgaggtacag aggcaaaggt tgatgaaatg gatgacaagg aagagatcaa caatgcctct     900
agagatgaaa tcgagtccaa gcaagtgaaa aagcaggatt ctaccaaccg aaacaaacat     960
gaacagaggg accttgacac tgatgcagat agtatgcccg gtgaagattc atcggataag    1020
aaaaagaaga agaataagaa gaagaaaaag acccaggata gtggagcaac aactaatgat    1080
caagctgttt cagctgcggg aggtgaagcc aagtcttcat tggagtctga ggataagcaa    1140
tctacagcca agtcctcaca agttagaacc ttttcaaatg gattggttat tgaagaatta    1200
gcaatgggca aaccagatgg taaacgagct tctcctggaa gtcaggtcag tgtgcactat    1260
attggtaagc tgaagaacgg aaaaatattt gactctaatg tcgggagggc tccctttaaa    1320
ttccgcctag gtgttggaca agtcataaaa ggatgggatg ttggagttaa tggtatgcga    1380
gttggggaca aaagaagact cacaattcca ccatcgatgg gttacggaga tcggggtgct    1440
ggcgggaaga tacctccaaa ctcatggctt gtgtttgatg ttgagttggt gaacgctcgt    1500
tga                                                                  1503
```

<210> 168
<211> 927
<212> DNA
<213> Populus trichocarpa

<400> 168

```
atggcttcct cgaagtctca cgatctcgag atcacaataa tctcagctaa acacctcaaa     60
aacgttaatt ggcgaaacgg cgatctcaaa ccgtacgcaa ccttctatct cgacaactcg    120
gaccgccgac tcgcaactca cgctgacgac tcactctcaa ctcgcccagt ctggaacgaa    180
cgatttactc tccccataac tcgccagatc tacgattcag tcctcaccct cgagatcttc    240
cactccaagc catcggaaac cccccaacct ctcgtaggca ctgtgaaatt cccactgagt    300
aatctcatgg tctcggatga gtcgctgtcc tgcgaagtcc tcacgctcga gctcctacgt    360
ccctctggtc gtccacaggg taaagtccgg gtgaaactag aggtgaaaga acggcctttg    420

cctccgccag tgcaggatta ccatactgcc cctaattata gtcattatta caaccctgcc    480
cctgcccccc ctcctcctcc tccaccacct cctgcacgtg actacaggga atactctcca    540
tctccatacg gctacgccga tccgtacggt tactaccctg cctattatcc tccacaacca    600
ccccgacctc tctacaatcg agcatccaat tacagcttgc caggtgggcc ttctgctcca    660
gttgatctat ctgcccaatc atcaccatcg ccctacgatc acaagccgcc gccgccgccg    720
ccgggattgt tccaaaagac gtccaattat ggtgtgccaa gtggaccgtc ggctcccgtc    780
gattattcac atgggaaagg tagtgggtcc cttatcagtg gggcaatggg agggttaagt    840
ttggaagaag ggagtaacta cgagaaagag aaagttgcta ctgataagga gagtcacagc    900
taccatgatt atcgtcgcga atattga                                        927
```

<210> 169
<211> 1716
<212> DNA
<213> Populus trichocarpa

<400> 169

```
atgtggatat ctacttcagg aattgcaggc aagactgcct cttggaaatc aatgatatta      60
atttatcaat caataacagc attgcttttt tcttcacgat ctctttccct tcctcttaat     120
atgaaacaga tgatatggga agaagttggg gaagatcagt ttgaacgaga gaaggtcctg     180
ctggatttag aacaagagtg cttagaggtt tataggagaa aagttgacaa tgcaaacata     240
tcaagagctc gcctgcatca agagctggcg gaatctgaag ctgaattcac ccatcttctt     300
ctgtcccttg gtgaacgatc actcccagga cggccagaaa gaaagtcagg aacactgaag     360
gagcagctag atgcgatcac cccagctcta cgggagatgc gtttgagaaa agaagagagg     420
gtgaatcaat ttagatctgt gcaaggtcaa attcaaaaaa tttctgcaga aattgcaggt     480
caatcagtgt acgatgaccc gataacaaat gtcaaggtaa atgagaatga tctttcatta     540
aagaaacttg aggaatacca aattgagcta caaaggctgc acaatgagaa gaatgacagg     600
ctccagctag tggacacata tattgacaca atccatgatt tgtcctcaac attaggaatg     660
gaatcctcca tgatcataac aaaggtgcat ccaactttga atgaattgtg tggaatatca     720
aaaaatataa gtgacagtat tctggataaa ctcaacagca ctgtggaatc tctcaaagaa     780
gaaaaacaaa agcggcttga gaagcttcat cagcttggaa aggcactgaa aaacttgtgg     840
aatctaatgg acacgcccta taaagattgt cactcatttt ccaatgtcac ggacttatta     900
tttctctcat cagatgaagt atcaggtcct ggaagccttg ctctaaatat aatccagcag     960
gctaaaaaag gggagattga ccatgccgat ctcctcatga gcttggatga gcagatatca    1020
agagcaaaag aggaagcttc tagcaggaag gctataatgg aaaaggttga aaagtggata    1080
ttagcacgtg atgaagagcg ctggttagaa gaatacagca tggacgagaa tcgatattca    1140
gttcgcagag gtgctcataa gaatctgcaa cgtgcagaac gtgcccgggt aatagtcaac    1200

aagatcccag ttctagtggc atcgctcgta gcaaagactg agagttggga agaggaaaga    1260
aacaaagtat tcctgtatga tggagtaccc ctgctggcaa tgctggaaga gtataacata    1320
tcgaggcagg agatggaaga ggaaaagcaa agacaaaggg ctagttcatc caacactcta    1380
caggcaaagg aaaagaaggt cccaagccat gtagaagttg agcaagaaaa tttgatcggg    1440
tcaagctcaa ggccaagcac cagcagtcga cgtctttcaa acaagagctt gaatggagga    1500
tttagcaatg caacgccttt aaacagaagg ctttctcttg gtcccagaca gctgggaacc    1560
aacagcatca attctgcaaa tcaaggaata tcttacataa aggaaggaag aaaaattcag    1620
gggcaaaaga tgtttcttcg acctgacctc tcttctcatc ttcgagatga agcagcttca    1680
gttgtctcat cattttctgg gccattatct ccctga                              1716
```

<210> 170
<211> 2763

<212> DNA
<213> Populus trichocarpa

<400> 170

```
atgcaagcag cctccggaac taagcgtggt tctagtggtt ccccggtgat tgataagcaa     60
gggagggcag tggccttgaa cgctgggggc agtgtatcat cttcatcagc cttctattta    120
catttagaga gagttgttag ggcattggca tttctccaga agtccaaaga tgcctgtaaa    180
aacaaatggg aagcagtttc tatacctcgt ggcacccttc aggtaacgtt tctccataaa    240
ggatttgatg agacacgccg gcttggtctt ccgaatgaaa cagagcagat ggtgcggcaa    300
gcctctgcac caggtgaaac tggaatgttg gttgttgact ctgtggtgcc atgtgggcca    360
gctgataggc aattggagtc aggggatgtg cttgtgcgtg tgaatgggga agtgactact    420
cagtttttaa aattggaggc attgcttgat gacagtgttg acaaaaagat cgaattacag    480
attgaaaggg gtggcacatc attaaccgtt gatatagtgg ttcaggattt gcacttgata    540
actcctgact gcttcttaga agtaagtggt gcagtgatac accgtttgtc ttatcaacag    600
gccagaaact tctgcttcca atgtggtctt gtatatgttt cggaccccgg atacatgcta    660
tctagggctg gcattcctcg tcatgccatc attaagaagt ttgccgatga tgagatatca    720
caacttgaag atgtaatctc tgttctgtct aagctatgta agggtgatag agtgccattg    780
gagtatataa gctacaggga tcgccatcga agaaagtcta ccctggtcac aattgatcgc    840
catgaatggt acgatgctcc gaagatatac acacgggatg atagttctgg tttatggatg    900
gcaaggcctg ctattcaacc gaccaccctg cagttgtcac cttgtagtag caatgtcact    960
caaggtctaa acagccaagc atcttcattg aatagtgaat caactcctgc tgaaggtacg   1020
gatcaagcta acaaccagga gttgacacat gatattttaa gaacagaagc cggttatgaa   1080
catatttctg aggaggtcca ttccagggaa gagtgtgatg ttaaaactaa taagcaacag   1140
gtacagggga acttgtcctc tgatgaaatt gcagttgctg accattcctc gcttgaaatt   1200
```

ggagaaatga aattggagac tccaggtacc actgaaatta cagtttcgaa tggctatgaa 1260
ggtgcaatag cagcagcaac taatgcttca tttgcggaat gtgtggtgga gcccactctt 1320
gtaacattgg aggtcaatgt gccaccatca tgtttgcttg atggcatcca ttcagtgcat 1380
gcttctggga ctggtgttgt tgtacatcat tctcaagaca tgggattggt tgctattgac 1440
aagaacaccg ttgaaacatc tgcatgtgat gtgatgctgt catttgctgc ttttcctatc 1500
gagattccag gagaggttgt ctttcttcat cctgtttaca attatgctct tgttggttat 1560
gatccctctg ctctgggagc tgttggtgct tctatggttc gtgctgctga gttacttcct 1620
gagcctgcat tatgtcgtgg agatccaata tatctgattg ggttaagtaa aaatcaacga 1680
gcaaagtcta ggaaatcaat tgtgacaaac ccttatgtta cattaaattt tggctatgct 1740
gatcgtccac ggtatagagc aataaatatg gaagtgattg agcttgacac tgattttggt 1800
aatgcattta ctggtgtgct atgtaatgag catggaaagg ttcaggctat atgggggagc 1860
ttttcaaata agccaaaatc tagtcacact acatcaaagg atcatcaatt tgttcgaggt 1920
gtcccaatct acatgattag ccaagtactt gacaaaatca tatctggtgc aaatggggct 1980
tctattctca taaacggtat caaaaggtca atgccacttg ttaggacttt agaggtggaa 2040
ctttgctcta gattgctttc aaaggcccgg agttttggtc ttggtgatga atggatccaa 2100
agacttgtaa agaaagaccc aatgagacga caagttttgc gtgttaaagg ttgtttggct 2160
ggatctaaca ctgagaatct attaaaacaa ggcgatatgt tgttggcaat caacaaagag 2220
ccagttactt gcttccaaga tgtagaaaat gcatgtcaag cattagagaa ctgtgttgac 2280
agtgatggga agcttaaaat taccatttgt cggcagggag gtgaagttga tctacttgtt 2340
ggaacagaca ttagggatgg taatggtaca acacgagcag taaattggtg tggctgtctt 2400
gttcaggatc ctcatccagc agttcgtgct cttggatttc ttcctggtga aggtcatggt 2460
gtctatgcag caatgtgctg ccgtggaagt cctgcagata gatatgctct aggtgctctt 2520
agatggattg ttcgagtcaa tggaaaaccc actcctgact tggatgcttt tgtaaatgtg 2580
acaaagggat tacgatatga tgagttcgtt cgtgtaaaga ccatcaatct ggatgggaag 2640
ccacgagtgc taacgttgaa gcaggacttg cactactggc ctacatggga gttgaggttt 2700
gatccaaaca ccgctaggtg gcggagagag acaataaagg ctttagattg caatattgaa 2760
taa 2763

<210> 171
<211> 651
<212> DNA
<213> Populus tremula x Populus tremuloides

<400> 171

```
agaaaacaac agtagcttca attagcagca gcagcaatgg cttcttcaag attttctctt     60
ctcttcccat tctttgtttt cttcattatt ctctgcttga tcagtcaccc tgtcatttgt    120
gacggtgatg aggaggatgc tcttcttcta gggattaaca agtataggac atccttcaat    180
ttgagtaccc tcacaaaaaa tgataacgca gagtgccttg cagaggagat agctgaccaa    240
tttaagaacc aaccctgtac aaataccacg ggttccaaca cagtaccagg cactgagccg    300
cagttcccca attacccgag ccttctaacc aaatgccatt tgaatgtctc caacacaagg    360
gatggagctg tgatgcctgc ttgtgttccc aacctggatc caagtcttgt gctcactaac    420
ttcacacgga ctacatactc cgataacctt aatgatacta agtttacagg agctgggatt    480
ggttctgatg gtaactggat cgttgttgtt ctgaccacaa gcacacctga gggaagtttt    540
gtgacttcca agacagacgg ttccgattcc aatgcagcca atttgaccgc caagaaaact    600
ggtctaatct atcacttact gttttgctga taggttctct cttcatgttg t             651
```

<210> 172
<211> 826
<212> DNA
<213> Populus tremula x Populus tremuloides

<400> 172

```
cccctcggag gtcgacccca cgcgtgccgc taatgcagat aatatacccg gtgaagattc     60
atcggataag aaaaagaaga agaagaagaa aaagaaaaag acccaggata gtggagcaac    120
aactaatgat caagctgttt cagctgtggg aggtgaagcc aagtcttcat tggagtctga    180
ggataagcaa tctacagcca agtcctcaca agttagaacc ttttcaaatg gattggttat    240
tgaagaatta gcaatgggca aaccagatgg taaacgagct tctcctggaa gtcaggtcag    300
tgtgcactat attggtaagc tgaagaacgg aaaagtattt gactctaatg tcggaagggc    360
tccctttaaa ttccgcctag gtgttggaca agtcataaaa ggatgggatg ttggagttaa    420
tggtatgcga gttggggaca aaagaagact cacagttcca ccatcgatgg gttacggaga    480
tcggggtgct ggcgggaaga tacctccaaa ctcatggctt gtgtttgatg ttgagttggt    540
gaacgctcgt tgattatctg ttttctgtgc tccttttctg cttttgaact caatggctgc    600
ttttacagac agtgggtata gatggatctc gaaaattggt gttttaactg taggcttgtt    660
ttacaatact gcaagttcac ccccatcgca taacttaagt gtcaagatgg atggtgtgaa    720
taaaattttg gttaattcaa ttgtagcaag actagtgtgc tctgcaaaaa ttttggttaa    780
aactagctgt tctgtttcag cagaatgtaa aaaaaaaaaa aaaaaa                   826
```

<210> 173
<211> 731

<212> DNA
<213> Populus tremula x Populus tremuloides

<400> 173

```
gggaatacgc tccatctcca tacggctacg ccgatccgta cggttactac cctgcctatt     60
atcctccaca accaccccga cctctctaca atcgagcatc caattacagc ttgccaggtg    120
ggccttctgc tccagttgat ctatctgccc aatcatcacc atcgccctac gatcacaagc    180
cgccgccgcc gccgcgggat tgttccaaaa gacgtccaat tatggtgtgc caagtggacc    240
gtcggctccc gtcgattatt cacatgggaa aggtagtggg tcccttatca gtggggcgat    300
gggagggtta agtttggaag aagggagtaa ctacgagaaa gagaaagttg ctactgataa    360
ggagagtcac agctaccatg attatcgtcg cgaatattga aggaatagag agactgtgtg    420
cgtgtgtgtg tgaaggtata tttgagcttt ttatactcgc attgtggctt tgactgatgg    480
atatactttt gtatgtacat gcaaaaaatt atcataactg ctagatgctg ccctcagtct    540
tccgttgtgc ttcaattatt tcttatctag tgtgtctgtt tctcttactt ctgtaatgga    600
tgtttggttc tatacttgct tctttgtttc aagtcttaat tcgagtttgc tagcttatac    660
acttgggtac tccattgatt gttaattgta gtataaatag tgaatgatta gagaatgatg    720
ggggaactta a                                                         731
```

<210> 174
<211> 822
<212> DNA
<213> Populus tremula x Populus tremuloides

<400> 174

aaaaaaaaaa aaaaaaaact cgagactaga tcgcttgtaa caaagactga gagttgggaa 60

gaggaaagaa acaaagtatt cctgtatgat ggggtacccc tgctggcaat gctggaagag 120

tacaacatat cgaggcagga gatggaagag gaaaagcaaa gccaatgggg tagttcatcc 180

aacactttac aggcaaagga aaagaaggtc ccaagccatg tagtagttga gcaagaaaat 240

ttgatcgggt caaggccaag caccagcagt cgacgtcttt caaacaagag cttgaatgga 300

ggatttagca atgcaacgcc tttaaacaga aggctttctc taggtcccag acagttggga 360

accaacagca tcaattctgc aaatcaagga atatcttaca taaaggaagg aagaaaaatt 420

caggggcaaa agatgtttct tcgacctgac ctctcttctc atcttcgaga tgaagcagct 480

tcagttgtct catcattttc tgggccatta tctccctgaa ttccaattcg gacatttgaa 540

tcgaaaatca catgtcacgg gagattagac aaaccttatc aaggaccata atagtaactg 600

ttatttctgc aaaacaataa tttacctatt tgcatcaatt tgataagaaa taacttgtgt 660

atctatgcct tgtaccaaca cccgtattta gaacagaacc ccaaagaact gtcattgcaa 720

tgaaaaataa ttattcaggt ttaaaactat gtagagctat gttttgtaat caagaagctt 780

tgattttcct attctattga aattatgaaa catgataatt tc 822

<210> 175
<211> 829
<212> DNA
<213> Populus tremula x Populus tremuloides

<400> 175

ctgtgttgac agtgatggga agcttaaaat taccatttgt cggcagggag gtgaagttga 60

tctacttctt ggaacagaca ttagggatgg taatggtaca acacgagcaa taaattggtg 120

cggctgtctt gttcaggatc ctcatccagc agttcgtgct cttggatttc ttcctggtga 180

aggtcatggt gtctatgcag caatgtgctg tcgaggaagt cccgcagata gatatgctct 240

aggtgctctt agatggattg ttcaagtcaa tggaaaaccc actcctgact tggatgcttt 300

tgtaaatgtg acgaagggat tacgatatga tgagttcgtt cgtgtaaaga ccatcaatct 360

ggatgggaag ccacgagtgc taacgttgaa gcaggacttg cactactggc ctacatggga 420

gttgaggttt gatccaaaca ccgctaggtg gcggagagag acaataaagg ctttagattg 480

caataatgaa taaacagatt gttctgatct cacttcgtgt tctttcctgg aatggttatt 540

gcacgttcaa cactgcttca agagcgtaag tggtccacga aacagttggt gatttgcact 600

ccaactaggt tatgcccttt aattggttgg atttcttttg acaggcaaac ttgcctgttt 660

tagtgtgctc tataataacg gtcttgaaaa ataagtgtac ttttaagaca acaatgcata 720

tggtatttca tcaattaagt gaagtttctg tatttgaatg gaaagctgat gagaggccag 780

ctataataag aacaaaagcg aatgctttat tttgtgaatg taaaaaaaa 829

<210> 176
<211> 487
<212> DNA
<213> Artificial

<220>
<223> Construct sequence

<400> 176

gagttcttga gctaaacgag aggaggagga gaattgtttt tcgtttttca gatatggagg 60

agaggcatgt tatttttggg aagtatgaaa tggggaggct tttagggaag ggaacttttg 120

ctaaagtttt ctatgggaaa cacttggtga caggggagag tgtggcaatc aaagtcataa 180

gcaaagatca agtcaagaaa gaagggatga tggagcaaat ccagagagaa atctcagtca 240

tgcgtcttgt tcgtcatccc aacattgtag agctcaagga agtcatggct accaagacta 300

agatcttctt catcatggag tatgttcgag gaggagagtt gtttgccaaa gtagccaaag 360

gaaggctgaa agaagaagct gctcgaaaat atttccagca actaatcagc gcaattgatt 420

attgccatag cagaggtgtt taccatagag atttgaagcc agagaatttg ttgcttgatg 480

aagatga 487

<210> 177
<211> 452
<212> DNA
<213> Artificial

<220>
<223> Construct sequence

<400> 177

```
taactcacca aattaagatc aaagcctttc tacaacattt gaatctcagg atcttctaca     60

tgactattgt tgttatcttg attacaaaca ttgtctcctt gccatgtcca aacgatgtct    120
tttagtgcag gcctaacatc ttcctcgcag aacctagtat actccaaggt atctccggaa    180
gacttggaga actccaccac cgcaacctcc ggtgccacct cgaacacctc cgccgtcacc    240
gccagcttcc cctttctccc ctcacatgga ccttgtaacg tcactttgaa atctttgact    300
tttgccacct tataactcag ccccttgca accccttcaa ttttttccat gatcgcactc    360
gctgaaaatt ttgaagtgaa catagagctt gatttcctct tagtctcaaa aagaccagac    420
agatcaaagc cggatgacat cgaggaaata aa                                 452
```

<210> 178
<211> 1560
<212> DNA
<213> Artificial

<220>
<223> Construct sequence

<400> 178

```
cggtcttatt tgtattcgcc tgcaatatcg tacggcctgg gatctctctt ctcttcttac      60
tgagcaagtt gcatctcata agcactggcg atcatggagc tgaatggtca aacaagagtg     120
agaagaaagg accattttgc tcatacaaac ggtgatttag cattagcaag tgttggtgat     180
gtagatccct ggactgcatg ggcatataag cctcgaacta tttcgctgtt acttattggt     240
gcttgctttc taatatgggc aagtggagcc ctagatccag agagctgtac atctggtgat     300
gttgttacat ctgtgaaaag gggtatatgg gcaatgactg cagtttttct tggttattgc     360
ttgctacaag ccccttcaac ggttctaata aggccacatc cagcaatttg gcgcttagtt     420
catggattgg ccattgttta tcttgttgcc ctcacatttc tgctttttca gaagcgtgat     480
gatgcgcggc aatttatgaa gtttctccat cctgaccttg gaattgaact acctgaaaga     540
tcatatggtg ctgattgtcg catttatgtg cctgaaaatc ctacaagcaa gtttaagaac     600
gttttggaca ctctttttga tgaatttgtt ctagcacata tctttggatg gtggggcaag     660
gctatattaa tccgtaatca gccacttcta tgggtattgt caattggttt tgagctgatg     720
gagtttacct tccgccacat gctaccaaac ttcaatgaat gctggtggga cagtatcatt     780
cttgatattt tgatatgcaa ttggtttggc atttgggctg gaatgcatac agtcgggtat     840
tttgatggaa aaacatacga gtgggttggt ataagtcgcc aacctaatat tatgagcaag     900
gtcaaacgaa cattggaaca atttacacct gcacagtggg acaaagatga atggcatccc     960
ttgcttggtc catggcgatt tatccaagtt cttagtctct gtattgtctt cctgactgta    1020
gagctcaaca cattcttttt gaagttttgt ctatgggttc ctcctcggaa ccctgtaata    1080
gtgtacaggc tgatcttgtg gtggctaatt gccataccta caacacgtga atacaattca    1140
tatctccaag accgaaagcc tgtgaaaaag gttggtgctt tttgttggct ttcccttgct    1200
atttgcattg tggaacttct catttgcatc aagtttggac atggtcttta tcccaaacca    1260
atgcctgcat ggttggtcat cttctggaca tctgttggag ttagccttgt aatattcctg    1320
attatgtggt catggaaaag tttgggaaga aagagacgat gattttgcta gcatggtaca    1380
ctattctttt attccttcat gttcttatgg gtatacatct tgtaaatgcc ctatagcttc    1440
atggaattgt aaatgcttat tctcttaggt tagtggatca cagttttaga gtattcaatt    1500
cttttatgat aggtagttgg aaaatgcttc taatggtagg ttcatgtgtt gctaatttgc    1560
```

<210> 179
<211> 561
<212> DNA
<213> Artificial

<220>
<223> Construct sequence

<400> 179

```
atgcaaccag gcttggatga gatcacaatg acggggtgga agaatctgaa gcaacggctg     60
tcattcaagg gcctgggtag ttgctgcggg agcacaagct ggatttccag aagtaccacc    120
caaaccatgc cctttatcga tatagaggaa gaagaagagg aagagaccac catgcaaaac    180
caagctcaaa gaggaggagg aggaggagca gcagcagcgc caggtgctgg gatgaatctg    240
gcaatggcat tagctgctga gcgcgattta ggggattcaa atgtcaagac attgatgagt    300
ttgatcgaag aaacggacgg tgttgattgg aggaagaaga ataacagtaa taacaaaagc    360
aggagggaca aggaacagga acagaagcag gaagaagaga aggattgggt atgctgcgtg    420
tgcatggaga gaaataaagg agcagctttt attccatgtg gacacacctt ttgtagggtt    480
tgttcaagag aaatgtgggt taatcgaggg tgctgtcctg tctgcaaccg ttccattctc    540
gacatccttg atatcttcta g                                              561
```

<210> 180
<211> 369
<212> DNA
<213> Artificial

<220>
<223> Construct sequence

<400> 180

```
atggtggaga agatcatcaa tatgaacagt caagatcatc tgaggagtac taattataaa     60
gatgatgatg atgaagaaga agaagaagtt caacttccag ggtttcgatt tcacccaaca    120
gatgaagaac ttgttgggtt ctatcttcgt agaatggttg acaagaagcc tctcagaatt    180
gaactcatca aacaagtcga gatctacaaa tatgatccat gggatctacc aattaagtca    240
agctgtgtgg gagataagga agggtacttc ttttgcaaac gaggaagaaa gtataggaat    300
agcataagac ctaacagggt gacaggttct ggattttgga aagcaaccgg cattgataag    360
cctgtgtat                                                            369
```

<210> 181
<211> 410
<212> DNA
<213> Artificial

<220>
<223> Construct sequence

<400> 181

attacacaaa tattcacact agaaagggat caaaagcaca atctacaact gatgtaagct 60
catcccagtc cccatgcgta agaatctgat ttccaaatgg gctggaaatg ttcaaagatg 120
aagccataga tggtggttga gctacgtagc tcaactcgtc cacatgcagt ggtttcctct 180
ccttgacatg atgaactgga ggcttattat cgtaatgttg aatgagtgga gcaccaaaat 240
tgacgtagct ttcttgattg taattagatt ccacttgaca gcatgtcttg gagctcgcgt 300
cgattggtgc ttgttgaggt gtagttgata attgcttcaa atctggtgtg tattgtctgc 360
atgacacatt tcttttgaaa attctacaca gtgtccatac ctcagcttct 410

<210> 182
<211> 787
<212> DNA
<213> Artificial

<220>
<223> Construct sequence

<400> 182

atggagtttg atgggctccc aggttttcga ttccacccaa cagaagaaga actcttagat 60
ttctacctta caaagactgt cttaggtcaa gacacagctg atatcattgg tttcctcaac 120
atatacaatc acgaaccttg ggacttgcca ggattatcga agataggga gagggagtgg 180
tatttccttg tgcacaggga gagcgttctt gggagaccca ggaggacaac cgagaaaggt 240
tactggaagg caactggttc ggacaggccc atccgatgct tgatggatcc gaaaaggttg 300
ttgggtcata gaaaaactat ggttttctac agaggaagag ctccacgagg gagcaaaacg 360
gattgggtca tgaatgagta cagattgcct agcaactgct atttatcgaa ggagatcgtg 420
ctttgcaaag tatatagaaa ggcaacatca ttgaaggtct tggagcaaag ggcagccata 480
gaagaaattg ttagagcacc taacgtcaca tctttatctc ctccaatgcg agggaatttc 540
tctttttatg accaccaaaa gagcttcaac aagttcttgt tagaacaaaa taatgttgtt 600
cacaaagtag agggagtgac agtaatagaa gaagaagaag acaacaaaat cggttaccca 660
tcccaaataa tgggagttgg acccatcaaa gagcactact ccgagctcct gtcaccaaag 720
ttagtcctgg attggaccat ggattcactt tggcctaact aaggagccct aagctagata 780
tcccctg 787

<210> 183
<211> 276
<212> DNA
<213> Artificial

<220>
<223> Construct sequence

<400> 183

```
atgttgatgg ctttaaaatc ccgggttaat tctgctgaaa agacaccttc tgtaagggag      60
ttgtacagca aggaacatgt agatgcaacc cagtccttat atgacttgtc acctttttc      120
aagcttggtt tcatggctgc caatcttgcc atcattgagg ccacaagaga gcaaagccga     180
gagatgaata gctgcagcaa tggcttccat gttgttgatt ttgatatcgg tcaaggaggg     240
caatacatga accttctaca cgcgctttct gggctt                               276
```

<210> 184
<211> 364
<212> DNA
<213> Artificial

<220>
<223> Construct sequence

<400> 184

```
gaagttaacg ccaagcagat gcgacagtga gagtttttcc cttccaacca aaacataccc      60
caccgttctc ttctttaaca gtaaatcctg ggttaactcg gtttgttaaa ctcaatctcg     120
ctttcattga ctcagcaacg atttgactca tcggcttcaa ctcgaatcct gccattccca     180
tccgggcccg ccatttccca aagacctcgc atctttcaat ccggtccctg ccttcgcaag     240
caaccgagtt tactatcgtc cgacccagtc ccccctcgac ccttgctcgc tttgagctat     300
cccctttcca ttgttgagtc gattgagtcc gaatagcgcc tccgtaatat gaacatgatt     360
cgtt                                                                  364
```

<210> 185
<211> 1681
<212> DNA
<213> Artificial

<220>
<223> Construct sequence

<400> 185

```
atggacaaca atacctctcg ctctgaggtt tccgactgcg gcgatccaac gaggccggag      60
tctggcggcg gcggtgctag gtacaagttg atgtcgccgg ccaagcttcc gatctcgagg     120
tcggcttgca tcacgatccc tcctggactc agtccgacct cgtttcttga gtctccagtt     180
cttctctcta atgttaaggc agagccttcc cccactactg gtacgtttac taagcctcga     240
acagttcttg gctctcttag ctccactcca tattctgcta caactgtttc atccacagcg     300
tgtggagaaa gaaaatccga cagctttgag tttagaccat atgctaggtc aaacatggtt     360
tctgcagata ttaaccatca gaggagcaca caatgtgctc aagttcaaag ccagtgccat     420

tcacaatcat ttgcctcacc acctttggta aaaggtgaga tggaagtatc cacaaatgag     480
ttgagtctgt cagcacccct tcacatggtt acttcagttg ctagtgcacc tgctgaagtt     540
gattcagatg aactaaacca aacagggctc tctggcagtg ggcttcaggc atcacagtct     600
gatcatagag taggaagtgc gccttcaatg tcatctgatg atggatataa ctggagaaaa     660
tatggacaga aacatgttaa aggaagtgag ttccctcgca gctattacaa atgtacgcat     720
cctaactgtg aagtgaaaaa gttatttgag cgctcccatg atggacaaat aacagagatt     780
atctacaagg gtacacatga tcatcctaaa ccacaaccca gccgccgata tgcctctggt     840
tctgttttgt ccatgcagga agatagattt gacaagtctt catctctgcc taaccaagat     900
gacaagtcac ctggtgctta tggacaggtg cctcatgcta ttgagccaaa tggtgccctt     960
gaactgtcta ctggagcaaa tgatgatact ggagaaggtg ctgaagacga tgatgatccc    1020
ttctcgaaaa gaaggaggtt ggatgctgga ggttttgatg ttactccagt agtcaaacct    1080
atccgggaac cacgtgttgt cgtgcaaact ctgagcgagg ttgatatact ggatgatggg    1140
tacaggtggc gcaaatatgg ccagaaagtg gtgagaggaa atcctaatcc aaggagttac    1200
tacaaatgca caaatgctgg atgcccagtt agaaagcacg ttgagagggc atcacatgat    1260
ccaaaagcag ttataaccac atatgagggg aaacacaatc atgatgtacc tacagctagg    1320
acaaacagtc atgacatggc gggaccatca gctgtgaatg gaccctcaag gatcagacca    1380
gatgaaaatg agaccataag ccttgatctt ggggtcggga tcagttctac aactgaaaac    1440
cagtccggcg atcagcaaca agctttccgt gcggaactta tcaaacatga aaaccaagca    1500
agtggttcta gtttcagagt agttcatgca accccaatta cggcttacta cggtgtttta    1560
aatggtggca tgaatcagta tggatctagg caaattccag gtgaaagccg tagcattgaa    1620
attccacctt atccatatcc gcagaacatg ggaagattat taacgggtcc ataagtttgt    1680
c                                                                    1681
```

<210> 186
<211> 312

<212> DNA
<213> Artificial

<220>
<223> Construct sequence

<400> 186

```
atggaaacaa agtgtccagt agatgcaagg tgcgcagctg aaatatctgc catgctcacc      60
cctccttctc ctcttcaact ccaggagtat tttgaggaga ttatatctga aaggaaatgc     120
catggcattg aagtcaaacg agatggaaac ttgagcaaag ggtttatgc aactatggaa      180
cttaaagaag gagaacttat actgaaggac aagatacttg tgggtttaca gcatgttcca     240
aataagcttg actgtctggt gtgcagctat tgtttccaat ttattgagtc agttgaatat     300

cagattggga gg                                                         312
```

<210> 187
<211> 235
<212> DNA
<213> Artificial

<220>
<223> Construct sequence

<400> 187

```
ctactttttg ttggggtctt gctctaagca agcgttgcac ctgcatttga atccataatc      60
tgctagcaat gcctgtctgt cttcaaatgg aaggtcctcg tctatgtatg aaacggtaac     120
ctcttctccc tttagaattg gtttcagggt gatgattgct gcctggccat ctctgtcctc     180
atctcttttg aaggcgtgtg catttgggca gcaggagtga ttcatgcagc tctgt          235
```

<210> 188
<211> 1356
<212> DNA
<213> Populus trichocarpa

<400> 188

```
atggaggaga ggcatgttat ttttgggaag tatgaaatgg gtaggctttt agggaaggga      60
acttttgcta aagtttacta tgggaaacac ttggtgacag gagagagtgt ggcaatcaaa     120
gtcataagca aagatcaagt caagaaagaa gggatgatgg agcaaatcca gagagaaatc     180
tcagtcatgc gtcttgttcg tcatcccaac attgtagagc tcaaggaagt catggctacc     240
aagacaaaaa tcttcttcat catggagtat gttcgaggag gagagttgtt tgccaaagta     300
gccaaaggaa ggctgaaaga agaagctgct cgaaaatatt tccagcaact aatcagcgca     360
attgattatt gtcatagcag aggtgtttat catagagatt tgaagccaga gaatatgttg     420
cttgatgaag atgaaaactt gaaaatctct gattttggct tgtcagcctt gcctgaacaa     480
tttcggcaag atgggctttt gcatactcag tgtggaactc ctgcttatgt tgccccagaa     540
gtcttgagaa agaaaggcta tgatggatca aaagctgata tatggtcatg tggggtgatt     600
ctttatgtgc ttcttgcagg attcttgcca tttcaagatg agaatgttat gaagatgtac     660
aggaaagtct tcaaggctga atatcagttt ccgccttggt tttcaacaga ttctaagagg     720
ttgatttcaa gacttcttgt tgctgatcct gaaaagagaa tcacaattcc tgccataatg     780
agaaatcatt ggtttcttaa aggttttca agaccagtgg ccttttctat ccaagaatca     840
agcatggatc aaacaggaca agaacaagat cttgattctt gttctgttgt caacaccaag     900
gtatcatcac cagaattctt caatgcattc gagtttattt cctcaatgtc atccggcttt     960
gatctgtcta gtctttttga gactaagagg aaatcaggct ctatgttcac ttcaaaattt    1020
tcagcgagtg ctatcatgga aaaaattgaa ggggttgcaa aagggctgag ttataaggtg    1080
gcaaaagtca aagatttcaa agtgacgtta caaggtccat gcgagggtag aaaggggaag    1140
ctggcagtga cggcggaggt gttcgaggtg gcaccggagg ttgcggtggt ggagttctcc    1200
aagtcttccg gagatacctt ggagtatact aagttctgtg aggaagatgt taggcctgca    1260
ctaaaagaca tcgtttggac atggcaagga gacaatgttt gtaataaaga taacaacaat    1320
agtcatgtag aagatcgtga aattcaaatg ttgtag                              1356
```

<210> 189
<211> 1272
<212> DNA
<213> Populus trichocarpa

<400> 189

```
atggagctga atggtcaaac aagagtgaga agaaaggacc attttgctca tacaaacggt      60
gatttagcat taccaagtgt tggtgatgta gatccctgga ctgcatgggc atataagcct     120
cgaactattt cgttgttact tattggtgct tgctttctaa tatgggcaag tggagcccta     180
gatccagaga gctgcacatc tggtgatgtt gttacatctg tgaaaagggg tatatgggca     240
atgactgcag tttttcttgg ttattgcttg ctacaggccc cttcaacggt tctaataagg     300
ccacatccag caatttggcg cttagttcat ggattggcca ttgtttatct tgttgccctc     360
acatttctgc tttttcagaa gcgtgatgat gcgcggcaat ttatgaagtt tctccatcct     420
gaccttggaa ttgcagaact acctgaaaga tcatatggtg ctgattgtcg catttatgtg     480
cctgaaaatc ctacaagcaa gtttaagaac gttttggaca ctctttttga tgaatttgtt     540
ctagcacata tctttggatg gtggggcaag gctatattaa tccgtaatca gccacttcta     600
tgggtattgt caattggttt tgagctgatg gagtttacct tccgccacat gctaccaaac     660
ttcaatgaat gctggtggga cagtatcatt cttgatattt tgatatgcaa ttggtttggc     720
atttggctg gaatgcatac agtcgggtat tttgatggaa aaacatacga gtgggttggt      780
ataagtcgcc aacctaatat tatgagcaag gtcaaacgaa cattggaaca atttacacct     840
gcacagtggg acaaagatga atggcatccc ttgcttggtc catggcgatt tatccaagtt     900
cttagtctct gtattgtctt cctgactgta gagctcaaca cattcttttt gaagttttgt     960
ctatgggttc ctcctcggaa ccctgtaata gtgtacaggt tgatcttgtg gtggctaatt    1020
gccataccta caacacgtga atacaattca tatctccaag accgaaagcc tgtgaaaaag    1080
gtaggcgctt tttgttggct ttcccttgct atttgcatcg tggaacttct catttgcatc    1140
aagtttggac atggtcttta tcccaaacca atgcctgcat ggttggtcat cttctggaca    1200
tctgttggag ttagccttgt aatattcctg attatgtggt catggaaaag tttgggaaga    1260
aagagacgat ga                                                         1272
```

<210> 190
<211> 564
<212> DNA
<213> Populus trichocarpa

<400> 190

```
atgcaaccag gcttggatga gatcacaatg acggcgtgga agaatctaaa gcaacggctc      60
tcattcaagg gcctgggtag ttgctgcggg agcacaagct ggagttccag aagtgccacc     120
ccaaccatgc cctttatcga tatagagcaa gaagaagagg aagagcccat catgcaaaac     180
caagctcaaa gaggaggagg agctgcagca gcagcagcag cgccaggtgc tgggatgaat     240
ctggcaatgg cattagctgc tgagcgcaat ttaggggatt caaatgtcaa gacattgatg     300
agtttgatcg aagaaacgga cggtgttgat tggaggaaga agaataacag taatgataaa     360
agtaggaggg acaaggaaca ggaacagaag caggaagaag agaaggattg ggtatgctgc     420
gtttgcatgg agagaaataa aggcgcagct tttattccat gtggacacac cttttgtagg     480
gtttgttcaa gagaaatgtg ggttaatcga gggtgctgtc ctatctgcaa ccgttccatt     540
ctcgacatcc ttgatatctt ctag                                            564
```

<210> 191
<211> 927
<212> DNA
<213> Populus trichocarpa

<400> 191

```
atggtggaga agatcatcaa tatgaacagt caagatcatc tgaggagtac taattataaa      60
gatgatgatg atgaagaaga agttcaactt ccagggtttc gatttcaccc aacagatgaa     120
gaacttgttg ggttctatct tcgtagaatg gttgacaaga agcctctcag aattgaactc     180
atcaaacaag tcgagatcta caaatatgac ccttgggatc taccaaagtc aagctgtgtg     240
ggagataagg aagggtactt cttttgcaaa cgaggaagaa agtataggaa tagcataaga     300
cctaacaggg tgacagggtc tggattttgg aaagcaactg gcattgataa gccagtgttt     360
tcacttggag gagaaggccg tgacagcatt ggactcaaga aaacacttgt ttactaccgc     420
ggaagtgcag gaaaaggtac aaaaaccgat tggatgatgc acgagtttcg ccttcccacg     480
aaagataaca gcacctccac ggccactgtc aaagctaaaa tctctgacca agaagctgag     540
gtatggacac tgtgtagaat tttcaaaaga aatgtgtcat gcagaaaata cacaccagat     600
ttgaagcaat tatcaactac acctcaacaa ccaccaatcg acacaagctc caagttatgc     660
tgtcaagtgg aatctaatta cactcaagaa agctacgtca attttggtgc tccactcatt     720
caacattacg ataataagcc tccagttcat catgtcaagg aaaggaaacc actgcatgtg     780
gatcagttga gctacgtagc tcaaccacca tctatggctt catctttaaa catttccagc     840
ccatatggaa atcagattct tacgcatggg gactgggatg agcttacatc agttgtagat     900
tgtgcttttg atccctttct tgtgtga                                         927
```

<210> 192

<211> 762
<212> DNA
<213> Populus trichocarpa

<400> 192

```
atggagtttg atgggctccc aggttttcga ttccacccaa cagaagaaga actcttagat      60
ttctacctta caaagactgt cttaggtcaa gacacagctg atatcattgg tttcctcaac     120
atatacaatc acgaaccttg ggacttgcca ggattatcga agataggga gagggagtgg     180
tatttccttg tgcacaggga gagcgttctt gggagaccca ggaggacaac cgagaaaggt     240
tactggaagg caactggttc ggacaggccc atccgatgct tgatggatcc gaaaaggttg     300
ttgggtcata gaaaaactat ggttttctac agaggaagag ctccacgagg gagcaaaacg     360
gattgggtca tgaatgagta cagattgcct agcaactgct atttatcgaa ggagattgtg     420
ctttgcaaag tatatagaaa ggcaacatca ttgaaggtct tggagcaaag ggcagccata     480
gaagaaattg ttagagcacc taacgtcaca tctttatctc ctccaatgca agggaatttc     540
tctttttatg acccccaaaa gagcttcaac aagttcttgt tagaacaaaa taatgttgtt     600
cacaaagtag agggagtgac agtaatagaa gaagaagaag acaacaaaat tggttaccca     660
tcccaaataa tgggagttgg acccatcaaa gagcactact ccgagctcct gtcaccaaag     720
ttagtcctgg attggaccat ggattcactt tggcctaact aa                        762
```

<210> 193
<211> 1005
<212> DNA
<213> Populus trichocarpa

<400> 193

```
atgttgatgg ctttaaaatc ccggcttaat tcttctgaaa acacaacttc tgtaatggag      60
ctgtatagca aggaacatgt ggatgcaacc cagttgttat acgacctgtc tccttgtttc     120
aaacttggtt tcatggctgc caatcttgcc atcattgacg ctacaagaga gcaagaacaa     180
gaggcgaaca cttccagcaa tggcttccat gttgttgatt ttgatattgg tcatggaggg     240
caatacaaga accttctaca cgcgctttct gggcttcaga attcgaagcc agccattgtt     300
aagatcacag ccgttgctgc tgatagcaat ggcgtagagg aggagaggct gaggctagtt     360
ggtgaaacgc ttacccaact cgctcgtcga gttggtctga atttgtgttt caacgttgtg     420
agctgcaaac tcagtgagtt gactcgggag tccctcgggt gcgagccgga cgaggcattg     480
gctgtcaatt ttgcgttcaa gctgtataga atgccggacg agagcgtgtc ctccaccgag     540
aatcccaggg atgagctttt gaggcgcgtg aaggggctgg cgccgcgcgt ggtgactgta     600
gtggagcaag aaatgaatac caacacggcc ccattcatgg cgcgcgtgaa cgaatcctgt     660
tcatattacg gagcgctatt cgactcgatt gagtcaacgg ttaaagggga taactcggag     720
cgagccaagg tcgaggaggg actggggcgg aggatggtaa actcggttgc ttgtgaaggg     780
agggaccgtg ttgaaagatg cgaggtgttt ggaaaatggc gggcccggat gggcatggca     840
gggttcgagt tgaagcctct gagtcacaac atagccgagt cgatgaaaac gagattgagt     900

ttagcaaaca gagttaaccc aggatttagt gttaaagaag agaatggtgg ggtatgtttc     960
ggttggatgg gaaaaactct cacagtcgca tctgcttggc gttaa                    1005
```

<210> 194
<211> 1674
<212> DNA
<213> Populus trichocarpa

<400> 194

```
atggacaaca atacctctcg ctctgaggtt tccgactgcg gcgatccaac gaggccggag      60
tccggcggcg gcggtgctag gtacaagctg atgtcgccgg ccaagcttcc gatctcgagg     120
tcggcttgca tcacgatccc tcctggactc agtccgacct cgtttcttga gtctccagtt     180
cttctctcta atgttaaggc agagccttcc cccactactg gtacgtttac taagcctcga     240
acagctcttg gctctcttag ctccactcca tattctgcta caactgtttc atccacagcg     300
tgcggagaaa gaaaatccga ctactttgag tttagaccat atgctaggtc aaacatggtt     360
tctgcagata taaaccatca gaggagcaca caatgtgctc aagtccaaag ccagtgccat     420
tcacaatcat ttgcctcacc acctttggta aaaggtgaga tggaagtatc cacaaatgag     480
ttgagtctgt cagcatccct tcacatggtt acttcagttg ctagtgcacc tgctgaagtt     540
gattcagatg aactaaacca aacggggctc tctagcagtg ggcttcaggc atcacagtct     600
gatcatagag caggaactgc gccttcaatg tcatctgatg atggatataa ctggagaaaa     660
tatggacaga aacatgttaa gggaagtgag ttccctcgaa gctattacaa atgtacacat     720
cctaactgtg aagtgaaaaa gttatttgag cgctcccatg atggacaaat aacagagatt     780
atctacaagg gtacacatga tcatcctaaa ccacaaccca gccgccgata tgcctctggt     840
tctgttttgt ccatgcagga agatagattt gacaagtctt catctctgcc taaccaaggt     900
gacaagtcac ctggtgctta tggacaggtg cctcatgcta tcgagccaaa tggtgccctt     960
gaactgtcta ctggagcaaa tgatgatact ggagaaggtg ccgaagacga tgatgatccc    1020
ttctcaaaaa gaaggaggtt ggatgctgga ggttttgatg ttactccagt ggtcaaacct    1080
atccgggaac cacgtgttgt cgtgcaaact ctgagtgagg ttgatatact ggatgatggg    1140
tacaggtggc gcaaatatgg ccagaaagtg gtgagaggaa atcctaatcc aaggagttac    1200
tacaaatgca caaatgctgg atgcccagtt agaaagcacg ttgagagggc atcgcatgat    1260
ccaaaagcag ttataaccac atatgagggg aaacacaatc atgatgtacc tacagctagg    1320
acaaacagtc atgacatggc gggaccatca gctgtgaatg gaccctcaag gattagacca    1380
gatgaaaacg agacaattag ccttgatctt ggggtcggga tcagttctac aactgaaaac    1440
cagtccagcg atcagcaaca agctttgcat gctgaactta tcaaacacga gaaccaagca    1500
agtggttcta gtttcagagt agttcatgca accccaatta cggcttacta tggtgtttta    1560
aatggtggca tgaatcagta tggatctagg caaattccag gtgaaagccg tagcattgaa    1620

attccacctt atccatatcc gcagaacatg ggaagattat taacgggtcc ataa          1674
```

<210> 195
<211> 1371

<212> DNA
<213> Populus trichocarpa

<400> 195

```
atggaaacaa agtgtccagt agatgcaaag tgcgcagctg aaatatctgc catgctcacc     60
cctccttctc ctcttcaact ccaggagtat ttcgaggaga ttatatctga aaggaaatgc    120
catggcattg aagtcaaaca agatggaaac ttgagcaaag gggtttatgc aactatggaa    180
cttaaagaag gagaacttat actgaaggac aagatacttg tgggtttaca gcatgttcca    240
aataagcttg actgtctggt gtgtggctat tgtttccaat ttattgagtc agttgaatat    300
cagattggga ggaaacttta tttgcaaagt ctaggcgtgc ccagctgcaa tggatgtgat    360
gagggggaat gtagttccag cagctcatac aataaagctt gtttgcctga aggagttata    420
gaagcattaa tgaatggtga attggtattg ccttactctg acaaatttcc cttgccttca    480
actgttcctt gtcctggggg gtgtcaagaa gcttattatt gcagcaaatc ttgtgcgcag    540
actgattggg aatcttcaca ttctttactt tgcaccgggg agaggtcaga atcattatct    600
atagaggcac tttcaaaatt tatacagcat gctactgaaa caaatgatat tttcctccta    660
gcagccaaga caatttcttt caccatttta aggtatagga agctgaaagc agctaatgca    720
gatcgttctg aactttcctt actttggag gcatggaagc caatatcaat gggatacaag    780
agaaggtggt gggagtgcat ttcatttcca gatgatgttg atcgttctga tgacactgca    840
tttaggatgc aaatacagca gcttgcattc aagtcactgc agcttctcaa ggctgccata    900
tttgatgagg aatgcgagcc attattctcc cttgaaatct atgggaatat tattggcatg    960
tttgagctga ataatcttga tctggtcgta gcatctccag ttgaggatta ttttctgtac   1020
attgatgatc ttccagatcc tgaaaaggaa aaggccgaaa aaattgcacg gcaacttcta   1080
gatgctcttg gagatgacta ttcaatttgt tgccaaggta ctgcgttcta tcctttacag   1140
agctgcatga atcactcctg ctgcccaaat gcacacgcct tcaaaagaga tgaggacaga   1200
gatggccagg cagcaatcat caccctgaaa ccaattcgaa agggagaaga ggttaccgtt   1260
tcatacatag acgaggacct tccatttgaa gacagacagg cattgctagc agattatgga   1320
ttcaaatgca ggtgcaacgc ttgcttagag caagacccca acaaaaagta g            1371
```

## Description

## Technical field

The present invention is related to a set of genes, which when modified in plants gives altered lignin properties. More specifically, the invention relates to methods for phenotypically modifying plants and transgenic plants and plants obtained by a specific crossing method having altered expression of a gene resulting in a modified lignin expression. The invention also provides DNA construct such as a vector useful in the method of the invention. Further, the invention relates to a plant cell or plant progeny of the plants and wood produced by the plants according to the invention.

Lower lignin levels will result in improved saccharification for bio-refining and ethanol production and improved pulp and paper. Increased lignin levels will utilise lignin properties for energy production.

The genes and DNA constructs may be used for the identification of plants having altered lignin characteristics as compared to the wild-type.

According to the invention genes and DNA constructs may also be used as candidate genes in marker assisted breeding.

## Background

Wood from forest trees provides the biopolymers cellulose, hemicellulose and lignin for industrial uses. Major products produced from wood include pulp and paper, energy and building materials. However, we now see a shift where industry looks for novel production possibilities as well as for improved bio-refining of the wood raw material to more valuable products. This is driven by the aim to replace the use of fossil materials with renewable resources. Wood is such a resource of major importance, and it is therefore important to supply the industry with large amount of wood with designed properties for variable uses.

Lignin is a major wood biopolymer that is highly important for industrial processing of most wood based materials. Lignin biosynthesis has been well described by biochemical and genetic research tools (Baucher, Critical Reviews in Biochemistry and Molecular Biology, 38:305-350, 2003). All major enzymes in the biosynthetic pathway have been identified, and reverse genetics has been helpful in highlighting the major route of the pathway(s). Lignin is a highly complex biopolymer. It is composed of p-hydroxycinnamyl alcohol monomers and related compounds. This are polymerized oxidised and coupled in a chemical process, and the lignin is encrusting the cellulose and hemicellulose matrix in the wood cell walls. It links in complex, and hitherto, poorly described ways to the hemicellulose network with so-called "lignin carbon complexes". It has recently also been obvious that the lignin structure is very plastic and variable. Both lignin content and its composition are naturally variable between organs and cell types, and this variability can be mimicked and extended by genetic engineering (Vanholme et al. Current Opinion in Plant Biology 2008, 11:1-8). Not surprisingly in transgenic trees with modified lignin, pulping properties such as yield and kappa number has been modified (Bauhcer, Critical Reviews in Biochemistry and Molecular Biology, 38:305-350, 2003). More recent research has also demonstrated that saccharification needed for wood bio-refining and for example ethanol production is also modified when lignin is modified (Chen and Dixon. Nature Biotechnology Vol. 25 no 7: pp759-761, 2007). This paper shows that lower lignin levels results in improved saccharification. Saccharification is a process were the poly carbohydrates are hydrolysed into sugar monomers such as glucose. This process is expensive and advanced for lignocellulosic raw materials (Breaking the Biological Barriers to Cellulosic Ethanol: A Joint Research Agenda. A Research Roadmap Resulting from the Biomass to Biofuels Workshop, December 7-9, 2005, Rockville, Maryland).

To access the cellulose and hemicellulose for the saccharification, the cell wall structure has to be loosened, thereby enabling it for enzymatic degradation or hydrolysis.

Furthermore, this pretreatment may increase the surface area of the cellulose thereby enhancing its reactivity with the enzyme and the transformation to monosaccharides.

Despite the rather well researched lignin biosynthetic pathway recent observations point to the fact that lignin content can also be modified through perturbing genes related to other metabolic and cell wall biopolymer pathways in a rather unexplored, and not yet understood, fashion This is an unexplored potential to modify wood properties and lignin chemistry in trees.

The commercial value of engineering lignin content in wood is obvious, but the usefulness of more or less lignin depends upon the utilization of wood and the industrial process used. For energy production through burning or other combustion technology high lignin content is valuable because lignin has a high-energy value compared to other wood biopolymers.

Also, in chemical pulping, forage digestibility and processing of plant biomass to bio fuels lignin represent a major obstacle. This is because lignin is covalently bound to the carbon matrix, and energy is required to isolate or degrade the lignin. In pulp and paper processing, for example, it becomes a rest product ending up in the rest liquor, and separation of lignin from the cellulose is an energy consuming process. Therefore low lignin content has been viewed as a valuable property.

One example of genes involved in the biosynthetic pathway of lignin is the genes involved in the monolignol synthesis. Forster and colleagues describe in the US patent 7402428 that they can reduce or modulate the lignin content in plants by RNAi knockout of a gene in the monolignol biosynthetic pathway. This is one gene of many genes involved in the lignin synthesis pathway, due to different growth and environment condition very little is know how the this gene construct will function in field growth conditions and which genes will be best for different applications. Thus there is still a need for additional genes that can be used for modulation of lignin content in plants.

But ongoing research trying to establish wood bio refining, where all wood components are processed may change this view. The use of lignin as a dispergent, emulsion, binding agent or stabilizer has already been developed. If novel lignin based products are becoming more attractive, the lignin content of the wood may be looked at in different views. It is therefore of value to be able to modify lignin content in wood in a desirable way, e.g. up or down depending on the intended use.

Problems remaining are how to identify the potentially most important genes involved in regulation of lignin and expression of lignin related genes, in order to utilise lignin properties for energy production or remove unwanted lignin properties in paper production. In this present invention we examined a number of genes affecting the lignin levels in living trees.

Brief description of the invention

The present invention pertains to genes, and DNA constructs SEQ ID No: 29 that can be used to modify lignin level in plants. This is done by modification of the expression level of these genes.

The invention relates to methods for phenotypically modifying plants and transgenic plants and plants obtained by a specific crossing method having altered expression of a gene resulting in a modified lignin expression. The invention also provides DNA constructs useful in the method of the invention. Further, the invention relates to a plant cell or plant progeny of the plants and wood produced by the plants according to the invention and uses thereof in applications where increased or decreased lignin content is an advantage.

The inventors have found that the down regulation and/or over expression of certain genes, gives changed wood chemistry in the produced transgenic trees. Especially it was realized that certain genes can be used to modify lignin levels. These genes have been identified from a program where gene have been transferred to trees and been down regulated by the use of RNAi and over expressed by high level expression promoters.

These genes were indentified to affect the lignin level after a two step analysis, firstly the wood was analysed with FT-IR analysis (general reference Griffiths, P. R. and De Haseth, J. A. Fourier Transform Infrared Spectrometry. New York: Wiley, 1986 and the text below) and secondly the wood was analysed with the Klason-lignin analysis (see below in text.).

The genes (SEQ ID No: 29) have changed chemical wood properties especially changed lignin levels.

The genes (SEQ ID 29) can be expressed in plants for further use as energy crops, for example if the lignin content is increased a high-energy crop will be generated.

Furthermore, these genes (SEQ ID r 29 can when expressed in plants further be used to increase and improve saccharification potentials and thereby enhance the economics in producing fuels and chemicals from ligno-cellulose.

**Detailed description of the invention**

***Definitions***

Prior to discussing the present invention in further details, the following terms and conventions will first be defined:

Definition of "saccharification" is the process of converting a complex carbohydrate, such as starch, cellulose or hemicellulose, into sugars such as fermentable sugars. It is essentially a hydrolysis. The process may for example be accomplished by the use of enzymes or acids or other chemicals.

The term "transgenic plant" refers to a plant that contains genetic material, not found in a wild type plant of the same species, variety or cultivar.

"Over expression" refers to the increased expression of an mRNA or polypeptide compared to control plants, wherein said mRNA or polypeptide or protein is either not normally present in the host cell, or wherein said mRNA or polypeptide or protein is present in said host cell at a higher level than that normally expressed.

Over expression of the proteins of the instant invention may be accomplished by first constructing a chimeric gene in which the coding region is operably linked to a promoter capable of directing expression of a gene in the desired tissues at the desired stage of development. The chimeric gene may comprise promoter sequences and translation leader sequences derived from the same genes. 3' Non-coding sequences encoding transcription termination signals may also be provided. The instant chimeric gene may also comprise one or more introns in order to facilitate gene expression. A suitable promoter may be the CaMV 35 S promoter which may be used with *Agrobacterium* as a vector.

The term "RNA interference" or "RNAi" may refer to a process in which a double-stranded RNA molecule or a short hairpin RNA changes the expression of a nucleic acid sequence with which they share substantial or total homology.

The term "RNAi down-regulation" refers to the reduction in the expression of a nucleic acid sequence which may be mediated by one or more RNAi species. The term "RNAi species" refers to a distinct RNA Sequence that elicits RNAi.

The term "RNAi construction group" refers to different transgenic trees emanating from one RNAi construct.

The term "photoperiod" refers to the daily cycle of light and darkness.

The term "recombinant" when used with reference, e.g., to a cell, nucleotide, vector, protein, or polypeptide typically indicates that the cell, nucleotide, or vector has been modified by the introduction of a heterologous (or foreign) nucleic acid or the alteration of a native nucleic acid, or that the protein or polypeptide has been modified by the introduction of a heterologous amino acid, or that the cell is derived from a cell so modified. Recombinant cells express nucleic acid sequences (e.g., genes) that are not found in the native (non-recombinant) form of the cell or express native nucleic acid sequences (e.g. genes) that would be abnormally expressed under-expressed, or not expressed at all. The term "recombinant" when used with reference to a cell indicates that the cell replicates a heterologous nucleic acid, or expresses a peptide or protein encoded by a heterologous nucleic acid. Recombinant cells can contain genes that are not found within the native (non-recombinant) form of the cell. Recombinant cells can also contain genes found in the native form of the cell wherein the genes are modified and re-introduced into the cell by artificial means. The term also encompasses cells that contain a nucleic acid endogenous to the cell that has been modified without removing the nucleic acid from the cell; such modifications include those obtained by gene replacement, site-specific mutation, and related techniques.

In the context of the present invention "complementary" refers to the capacity for precise pairing between two nucleotides sequences with one another. For example, if a nucleotide at a certain position of an oligonucleotide is capable of hydrogen bonding with a nucleotide at the corresponding position of a DNA or RNA molecule, then the oligonucleotide and the DNA or RNA are considered to be complementary to each other at that position. The DNA or RNA strand are considered complementary to each other when a sufficient number of nucleotides in the oligonucleotide can form hydrogen bonds with corresponding nucleotides in the target DNA or RNA to enable the formation of a stable complex.

In the present context the expressions "complementary sequence" or "complement" therefore also refer to nucleotide sequences which will anneal to a nucleic acid molecule of the invention under stringent conditions.

The term "stringent conditions" refers to general conditions of high, weak or low stringency.

The term "stringency" is well known in the art and is used in reference to the conditions (temperature, ionic strength and the presence of other compounds such as organic solvents) under which nucleic acid hybridisations are conducted.

Hybridisation means matching one nucleic acid with another e.g. base pairing between single-stranded nucleic acid molecules with each other to form a duplex.

A "sub sequence" or a "fragment" is any portion of an entire sequence. Thus, a fragment or sub sequence refers to a sequence of amino acids or nucleic acids that comprises a part of a longer sequence of amino acids (e.g. polypeptide) or nucleic acids (e.g. polynucleotide), respectively.

In the present context, the term "homology" indicates similarities between two amino acid sequences or between two nucleotide sequences is described by the parameter "sequence identity".

The term "sequence identity" indicates a quantitative measure of the degree of homology between two amino acid sequences or between two nucleic acid sequences of equal length. If the two sequences to be compared are not of equal length, they must be aligned to give the best possible fit, allowing the insertion of gaps or, alternatively, truncation at the ends of the polypeptide Sequences or nucleotide Sequences. The sequence identity can be calculated as $\frac{(N_{ref}-N_{dif})100}{N_{ref}}$, wherein $N_{dif}$ is the total number of non-identical residues in the two Sequences when aligned and wherein $N_{ref}$ is the number of residues in one of the sequences. Hence, the DNA sequence AGTCAGTC will have a sequence identity of 75% with the sequence AATCAATC ($N_{dif}$=2 and $N_{ref}$=8). A gap is counted as non-identity of the specific residue(s), i.e. the DNA sequence AGTGTC will have a sequence identity of 75% with the DNA sequence AGTCAGTC ($N_{dif}$=2 and $N_{ref}$=8).

With respect to all embodiments of the invention relating to nucleotide sequences, the percentage of sequence identity between one or more sequences may also be based on alignments using the clustalW software (http:/www.ebi.ac.uk/clustalW/index.html) with default settings. For nucleotide Sequence alignments these settings are: Alignment=3Dfull, Gap Open 10.00, Gap Ext. 0.20, Gap separation Dist. 4, DNA weight matrix: identity (IUB). Alternatively, the sequences may be analysed using the program DNASIS Max and the comparison of the sequences may be done at http://www.paralign.org/. This service is based on the two comparison algorithms called Smith-Waterman (SW) and ParAlign. The first algorithm was published by Smith and Waterman (1981) and is a well established method that finds the optimal local alignment of two sequences The other algorithm, ParAlign, is a heuristic method for sequence alignment; details on the method is published in Rognes (2001). Default settings for score matrix and Gap penalties as well as E-values were used.

The phrase "substantially identical" or "substantial identity" in the context of two nucleic acids or polypeptides, may refer to two or more sequences or sub-sequences that have at least about 60%, 65%, 70%, 75%, preferably 80% or 85%, more preferably 90%, 91%, 92%, 93%, 94%, 95%, 96%, 97%, 98%, 99%, 99.5%, or greater nucleotide or amino acid residue percent identity, respectively, when compared and aligned for maximum correspondence, as measured using a sequence comparison algorithm or by visual inspection. The substantially identity may refer to one gene, two or a group of genes separately and not to the other genes in the present invention, for example one gene might be 75% homologous and another might be 60 % homologous and further another might be 93 % homologous to the gene of interests. In certain aspects, the substantial identity exists over a region of amino acid sequences of at least about 50 residues in length, such as, at least about 100, 110, 120, 125, 130, 135, 140, 145, 150, 155, 160, or 165 amino acid residues. In certain aspects, substantial identity exists over a region of nucleic acid sequences of at least about 150 nucleic acid residues, such as at least about 200, 250, 300, 330, 360, 375, 400, 425, 450, 460, 480, 500, 600, 700, 800 such as at least about 900 nucleotides or such as at least about 1 kb, 2 kb, or such as at least about 3 kb. In some aspects, the amino acid or nucleic acid sequences are substantially identical over the entire length of the polypeptide sequence or the corresponding coding region.

Examples of "conservative substitutions" are within the group of basic amino acids (arginine, lysine and histidine), acidic amino acids (glutamic acid and aspartic acid), polar amino acids (glutamine and asparagine), hydrophobic amino acids (leucine, isoleucine, valine and methionine), aromatic amino acids (phenylalanine, tryptophan and tyrosine), and small amino acids (glycine, alanine, serine and threonine). Amino acid substitutions which do not generally alter the specific activity are known in the art and are described, for example, by Neurath and Hill, 1979. The most commonly occurring exchanges are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly as well as these in reverse.

The term "conservatively substituted variant" as used herein refers to a variant of a nucleotide sequence comprising one or more conservative substitutions.

Generally and in the present context, the term "silent substitution" refers to a base substitution which does not affect the sense of a codon and thus has no effect on polypeptide structure. As the skilled person will know silent substitutions are possible because of the degeneracy of the genetic code.

The term "conserved domain" may be a sequence of amino acids in a polypeptide or a sequence of nucleotides in DNA or RNA that is similar across multiple species. A known set of conserved sequences is represented by a consensus sequence. Amino acid motifs are often composed of conserved sequences. Additionally, the term "conserved sequence" refers to a base sequence in a nucleic acid sequence molecule or an amino acid sequence in a protein that has remained essentially unchanged throughout evolution. A "consensus sequence" may be defined in terms of an idealized sequence that represents the base most often present at each position in a nucleic acid sequence or the amino acid most often present at each position in a protein. A "consensus sequence" is identified by aligning all known examples of a nucleic acid Sequence or a protein so as to maximise their sequence identity. For a sequence to be accepted as a consensus sequence each particular base or amino acid must be reasonably predominant at its position and most of the sequences must be related to the consensus by only few substitutions, such as 1 or 2.

A homologue may also be in the form of an "insertional variant" of a protein, i.e. where one or more amino acid residues are introduced into a predetermined site in a protein. Insertions may comprise N-terminal and/or C-terminal fusions as well as intra-sequence insertions of single or multiple amino acids.

Homologues in the form of "deletion variants" of a protein are characterised by the removal of one or more amino acids from a protein.

Homologues in the form of "addition variants" of a protein are characterised by the addition of one or more amino acids from a protein, whereby the addition may be at the end of the sequence.

Substitution is another variant of a protein wherein one or more amino acids have been changed for another (other) amino acid(s).

The terms "Orthologs" and "Paralogs" -sequences are also a type of homologous sequences as described above. Several different methods are known by those of skill in the art for identifying and defining these functionally homologous sequences. Three general methods for defining orthologs and paralogs are described; an ortholog, paralog or homolog may be identified by one or more of the methods described below.

Orthologs and paralogs are evolutionarily related genes that have similar sequence and similar functions. Orthologs are structurally related genes in different species that are derived by a speciation event. Paralogs are structurally related genes within a single species that are derived by a duplication event.

Within a single plant species, gene duplication may cause two copies of a particular gene, giving rise to two or more genes with similar sequence and often similar function known as paralogs. A paralog is therefore a similar gene formed by duplication within the same species. Paralogs typically cluster together or in the same clade (a group of similar genes) when a gene family phylogeny is analyzed using programs such as CLUSTAL (Thompson et al.; Higgins et al.) Groups of similar genes can also be identified with pair-wise BLAST analysis (Feng and Doolittle). For example, a clade of very similar MADS domain transcription factors from *Arabidopsis* all share a common function in flowering time (Ratcliffe et al.), and a group of very similar AP2 domain transcription factors from *Arabidopsis* are involved in tolerance of plants to freezing (Gilmour et al.). Analysis of groups of similar genes with similar function that fall within one clade can yield sub-Sequences that are particular to the clade. These sub-sequences, known as consensus sequences, can not only be used to define the sequences within each clade, but define the functions of these genes; genes within a clade may contain paralogous sequences, or orthologous sequences that share the same function (see also, for example, Mount (2001), in Bioinformatics: sequence and Genome Analysis Cold Spring Harbor Laboratory Press, Cold Spring Harbor, N.Y., page 543.)

Speciation, the production of new species from a parental species, can also give rise to two or more genes with similar sequence and similar function. These genes, termed orthologs, often have an identical function within their host plants and are often interchangeable between species without losing function. Because plants have common ancestors, many genes in any plant species will have a corresponding orthologous gene in another plant species. Once a phylogenic tree for a gene family of one species has been constructed using a program such as CLUSTAL potential orthologous sequences can be placed into the phylogenetic tree and their relationship to genes from the species of interest can be determined. Orthologous sequences can also be identified by a reciprocal BLAST strategy. Once an orthologous sequence has been identified, the function of the ortholog can be deduced from the identified function of the reference sequence.

Orthologous genes from different organisms may have highly conserved functions, and very often essentially identical functions (Lee et al. and Remm et al.). Paralogous genes, which have diverged through gene duplication, may retain similar functions of the encoded proteins. In such cases, paralogs can be used interchangeably with respect to certain embodiments of the instant invention (for example, transgenic expression of a coding sequence). An example of such highly related paralogs is the CBF family, with three well-defined members in *Arabidopsis* and at least one ortholog in *Brassica napus*, all of which control pathways involved in both freezing and drought stress (Gilmour et al. and Jaglo et al.)

Hybrid aspen is a hybrid of the two species *Populus tremuloides* and *Populus tremula*.

The following references represent a small sampling of the many studies that demonstrate that orthologous transcription factor genes from diverse species are likely to function similarly (i.e., regulate similar target sequences and control the same traits), and that transcription factors may be transformed into diverse species to confer or improve traits.

(1) The *Arabidopsis* NPR1 gene regulates systemic acquired resistance (SAR); over-expression of NPR1 leads to enhanced resistance in *Arabidopsis.* When either *Arabidopsis* NPR1 or the rice NPR1 ortholog was overexpressed in rice (which, as a monocot, is diverse from *Arabidopsis),* challenge with the rice bacterial blight pathogen *Xanthomonas oryzae* pv. *Oryzae*, the transgenic plants displayed enhanced resistance (Chern et al.). NPR1 acts through activation of expression of transcription factor genes, such as TGA2 (Fan and Dong).

(2) E2F genes are involved in transcription of plant genes for proliferating cell nuclear antigen (PCNA). Plant E2Fs share a high degree of similarity in amino acid Sequence between monocots and dicots, and are even similar to the conserved domains of the animal E2Fs. Such conservation indicates a functional similarity between plant and animal E2Fs. E2F transcription factors that regulate meristem development act through common cis-elements, and regulate related (PCNA) genes (Kosugi and Ohashi).

The term "closely related" genes is used for genes that are orthologous or paralogous.

The term "promoter," as used herein, refers to a region of sequence determinants located upstream from the start of transcription of a gene and which are involved in recognition and binding of RNA polymerase and other proteins to initiate and modulate transcription. Promoters useful in plants need not be of plant origin. A "basal promoter" is the minimal sequence necessary for assembly of a transcription complex required for transcription initiation. Basal promoters frequently include a TATA box" element usually located between 15 and 35 nucleotides upstream from the site of initiation of transcription. Basal promoters also sometimes include a CCAAT box" element (typically a Sequence CCAAT) and/or a GGGCG Sequence, usually located between 40 and 200 nucleotides, preferably 60 to 120 nucleotides, upstream from the start site of transcription.

Promoters referred to herein as "constitutive promoters" may actively promote transcription under most, but not necessarily all, environmental conditions and states of development or cell differentiation. Examples of constitutive promoters include the cauliflower mosaic virus (CaMV) 35S transcript initiation region and the 1’ or 2’ promoter derived from T-DNA of *Agrobacterium tumefaciens*, and other transcription initiation regions from various plant genes, such as the maize ubiquitin-1 promoter, known to those of skill. Organ-specific promoters may be, for example, a promoter from storage sink tissues such as seeds, potato tubers, and fruits, or from metabolic sink tissues such as meristems. Furthermore, the promoter may be a leaf specific promoter such as the *rbcs* promoter from rice or tomato.

An "inducible promoter" in the context of the present invention refers to a promoter which is regulated under certain conditions, such as light, chemical concentration, protein concentration, conditions in an organism, cell, or organelle, etc. An example of an inducible promoter is the HSP promoter and the PARSK1, the promoter from the *Arabidopsis* gene encoding a serine-threonine kinase enzyme and which is induced by dehydration, abscissic acid and sodium chloride. In essence, expression under the control of an inducible promoter is "switched on" or increased in response to an applied stimulus. The nature of the stimulus varies between promoters and may include the above environmental factors. Whatever the level of expression is in the absence of the stimulus, expression from any inducible promoter is increased in the presence of the correct stimulus.

As used herein, the term "tissue specific" refers to a characteristic of a particular tissue that is not generally found in all tissues, or may be exclusive found in a tissue of interest. In the present application, "tissue specific" is used in reference to a gene regulatory element (promoter or promoter plus enhancer and/or silencer), the gene it encodes, or the polypeptide product of such a gene. In the context of a gene regulatory element or a "tissue specific promoter", the term means that the promoter (and also other regulatory elements such as enhancer and/or silencer elements) directs the transcription of a linked sequence in a cell of a particular lineage, tissue, or cell type, but is substantially inactive in cells or tissues not of that lineage, tissue, or cell type. A tissue specific promoter useful according to the invention is at least 5-fold, 10-fold, 25-fold, 50-fold, 100-fold, 500-fold or even 1,000 times more active in terms of transcript production in the particular tissue than it is in cells of other tissues or in transformed or malignant cells of the same lineage. In the context of a gene or the polypeptide product of a gene, the term tissue specific means that the polypeptide product of the gene is detectable in cells of that particular tissue or cell type, but not substantially detectable in certain other cell types. Particularly relevant tissue specific promoters include promoter sequences specifically expressed or active in the xylem forming tissue in a plant. Examples of such promoters are the Lmp1, Lmx2, Lmx3, Lmx4 and Lmx5 promoters, described in WO2004097024.

A "terminator sequence" refers to a section of genetic sequence that marks the end of gene or operon on genomic DNA for transcription. Terminator sequences are recognized by protein factors that co-transcriptionally cleave the nascent RNA at a polyadenylation signal, halting further elongation of the transcript by RNA polymerase. A nucleic acid is "operably linked" when it is placed into a functional relationship with another nucleic acid sequence. For instance, a promoter or enhancer is operably linked to a coding sequence if it increases the transcription of the coding sequence. Operably linked means that the DNA sequences being linked are typically contiguous and, where necessary to join two protein coding regions, contiguous and in reading frame. However, since enhancers generally function when separated from the promoter by several kilobases and intronic sequences may be of variable lengths, some polynucleotide elements may be operably linked but not contiguous.

In the context of the present invention the terms "transformation" and "transforming" are used interchangeably and as synonyms to "transfecting" and "transfection", respectively, to refer to the process of introducing DNA into a cell. The DNA constructs, including at least a portion of the gene or promoter of interest, can be introduced into host cells, which as stated previously, can be individual cells, cells in culture, cells as part of a host organism, a fertilized oocyte orgametophyte or an embryonic cell. By the term "introduction" when used in reference to a host cell is meant to refer to standard procedures known in the art for introducing recombinant vector DNA into the target host cell. Such procedures include, but are not limited to, transfection, infection, transformation, natural uptake, electroporation, biolistics and *Agrobacterium* mediated.

By "regenerable cell" is meant a plant cell from which a whole plant can be regenerated. It will be understood that the regenerable cell is a cell that has maintained its genetic potential, also known in the art as "totipotency". It will further be understood that the regenerable cells, when grown in culture, may need the appropriate stimuli to express the total genetic potential of the parent plant.

A number of genes analyzed using the analytical platform show interesting and most often unexpected commercial features.

One aspect of the present invention with intentionally changed (increased or decreased expression) levels of one gene's SEQ ID No: 23 to 115, 157-195 provide a method of producing a plant having modified lignin levels.

***Method of producing a plant having altered lignin properties***

In specific embodiments of the invention advantageous plant phenotypes are generated by modifying, relative to the corresponding wild-type plant, the expression level of candidate genes that have been evaluated and selected according to the above criteria to modify lignin quantity, content and/or quality. According to these aspects a method is provided which comprises altering in the plant the level of a gene product of at least one gene comprising a nucleotide sequence selected from the group consisting of:

a) a nucleotide sequence from SEQ ID No: 29 or;

b) a nucleotide sequence being at least 80% identical to a nucleotide sequence from SEQ ID NO 29

Lower lignin content, may be obtained by down regulating the gene with SEQ ID No: 29

All these definitions regarding SEQ ID numbers mentioned above relating to the higher and lower lignin content are also combined with the above made definition of the homology language "substantially identical" or "substantial identity".

According to one aspect of the invention a method is provided comprising the following steps:

(i) providing an expression vector comprising a nucleotide sequence selected from the group consisting of

a) a nucleotide sequence from SEQ ID NO 29 or

b) a nucleotide sequence being at least 80% identical to a nucleotide sequence shown as SEQ ID NO: 29 and

c) at least one regulatory element operable linked to the polynucleotide sequence, wherein said at least one regulatory element controls expression of the polynucleotide sequence in a target plant;

(ii) introducing the expression vector into at least one plant; and

(iii) selecting at least one transgenic plant that has a modulated lignin quantity compared to its wild type.

The sequences specified by Sequence ID numbers 59 to 93, 164-169, 170 and 188-195 represent sequences of the candidate genes as predicted from Populus t[eta]chocarpa, the sequences specified by sequence ID numbers 94 to 112, 157, 171 -175, represent sequences of the candidate genes from hybrid aspen and SEQ ID numbers 23 to 58 113 to 1 15 and 157-163, 176-187 as cloned from hybrid aspen., Additional sequence from these genes 5' as well as 3' to the sequence described in SEQ ID No: 23 to 1 15, and 157-195 is readily achievable using conventional cloning techniques, as the skilled person will understand, such as those described in Sambrook et al.

In further embodiments of the invention the nucleic acid sequences in a)-d) above are substantially identical to SEQ ID NO 23 to 115 and 157-195 as defined herein.

The modulated expression may be effected by introducing a genetic modification preferably in the locus of a gene comprising SEQ ID NO 29 encoding a polypeptide or a homologue of such polypeptide.

The modification may also for example be effected by one of: T-DNA activation, TILLING, homologous recombination, site-directed mutagenesis or directed breeding using one or more of SEQ ID NO 23 to 115 and 157-195 or a fragment thereof as markers in any step of the process of producing the desirable plants.

According to the invention the modulation is decreased yield in lignin.

According to the invention, the method comprises the step of providing a nucleic acid construct, such as a recombinant DNA construct, comprising a nucleotide sequence selected from the group consisting of:

a) a nucleotide sequence comprising a sequence selected from SEQ ID No: 29;

b) a complementary nucleotide sequence of a nucleotide sequence of a);

c) a nucleic acid sequence being at least 80% identical to a nucleotide sequence shown as SEQ ID NO: 29.

d) a nucleotide sequence which hybridizes under stringent conditions to a nucleotide sequence of a), b) or c).

In further embodiments of the invention the nucleic acid sequence in d) is substantially identical as herein defined to any one of the sequences in a), b), or c),

In preferred embodiments of this aspect of the invention the nucleotide sequence of a) is selected from the group consisting of SEQ ID NOs: 29.

A variety of methods exist in the art for producing the nucleic acid sequences and nucleic acid/DNA constructs of the invention. Procedures for identifying and isolating DNA clones are well known to those of skill in the art, and are described in, e. g. Sambrook et al., Molecular Cloning-A Laboratory Manual (3rd Ed.), Vol. 1-3, Cold Spring Harbor Laboratory, Cold Spring Harbor, New York, 2001.

Nucleic acid sequences of the present invention can be produced by a variety of *in vitro* amplification methods adapted to the present invention by appropriate selection of specific or degenerate primers. Examples of protocols sufficient to direct persons of skill through *in vitro* amplification methods, including the polymerase chain reaction (PCR) the ligase chain reaction (LCR), Qbeta-replicase amplification and other RNA polymerase mediated techniques (e. g., NASBA), e. g., for the production of the homologous nucleic acids of the invention are found in Sambrook, *supra.*

Alternatively, nucleic acid constructs of the present invention can be assembled from fragments made by solid-phase synthesis methods. Typically, fragments of up to approximately 100 bases are individually synthesized and then enzymatically or chemically ligated to produce a desired sequence, e. g., a polynucletotide encoding all or part of a transcription factor. For example, chemical synthesis using the phosphoramidite method is well known to the skilled person. According to such methods, oligonucleotides are synthesized, purified, annealed to their complementary strand, ligated and then optionally cloned into suitable vectors.

As mentioned, the above described sequences are from hybrid aspen and *Populus trichocarpa*. As the skilled person will understand, homologues of the described Sequences may be isolated from other species, non-limiting examples of which include acacia, eucalyptus, hornbeam, beech, mahogany, walnut, oak, ash, hickory, birch, chestnut, alder, maple, sycamore, ginkgo, palm tree, sweet gum, cypress, Douglas fir, fir, sequoia, hemlock, cedar, juniper, larch, pine, redwood, spruce and yew, apple, plum, pear, banana, orange, kiwi, lemon, cherry, grapevine, fig, cotton, bamboo, switch grass, red canary grass, *Miscantus species* and rubber plants. Useful homologues of the described sequences may also be isolated from hardwood plants from the *Salicaceae* family, e.g. from the *Salix* and *Populus* genus. Members of this genus are known by their common names: willow, poplar and aspen.

In particular, the method according to the present invention may comprise a step of providing a nucleic acid construct, such as a recombinant DNA construct, comprising a nucleotide sequence which relative to the particular sequences described, comprises conservative variations altering only one, or a few amino acids in the encoded polypeptide may also be provided and used according to the present invention. Accordingly, it is within the scope of the invention to provide and use a recombinant DNA construct comprising a nucleotide sequence which encodes a polypeptide comprising a conservatively substituted variant of a polypeptide encoded from a nucleotide sequence from SEQ ID NO 29.

Sequence alterations that do not change the amino acid sequence encoded by the polynucleotide are termed "silent" substitutions. With the exception of the codons ATG and TGG, encoding methionine and tryptophan, respectively, any of the possible codons for the same amino acid can be substituted by a variety of techniques, e. g., site-directed mutagenesis, available in the art. Accordingly, the present invention may also provide a recombinant nucleic acid construct, wherein the nucleotide sequence comprises a silent substitution in a nucleotide sequence.

In certain further embodiments of the invention, the sub-sequences or fragments of c) or d) have substantial sequence identity as herein defined to a conserved domain of a nucleotide sequence as described above under item a) or b), .

Thus, there are methods for identifying a sequence similar or paralogous or orthologous or homologous to one or more polynucleotides as noted herein, or one or more target polypeptides encoded by the polynucleotides, or otherwise noted herein and may include linking or associating a given plant phenotype or gene function with a Sequence.

***DNA construct***

According to a second main aspect of the invention a DNA construct, such as a recombinant DNA construct, is provided comprising at least one sequence as described above. The DNA construct may be a vector. In particular, the recombinant DNA construct may comprise a nucleotide sequence selected from the group consisting of:

a) a nucleotide sequence comprising a sequence selected from SEQ ID NO:29 or;
b) a complementary nucleotide sequence of a nucleotide sequence of a) or;
c) a nucleic acid sequence being at least 80% identical to a nucleotide sequence shown as SEQ ID NO: 29 or; d) a nucleotide sequence which hybridizes under stringent conditions to a nucleotide sequence of a), b) or c).

In selected embodiments of the invention the nucleic acid sequence in d) is substantially identical as herein defined to any one of the sequences in a), b) and c).

Variants of DNA constructs of a), b), c) an d) above that encode polypeptides having additions, deletions, substitutions or insertions of one or more amino acids are also within the scope of the invention as long as lignin properties are affected.

Also, in accordance with the discussion above, the nucleotide Sequence encodes a polypeptide comprising a conservatively substituted variant of a polypeptide of (a). Further, the nucleotide sequence comprises a silent substitution in a nucleotide sequence.

In additional embodiments of the pertaining to this aspect of the invention, the sub-sequences or fragments have substantial sequence identity as herein defined to a conserved domain of a nucleotide sequence as described above under item a).

In further embodiments and in accordance with the description above, the recombinant DNA construct further comprising a promoter, that may be constitutive, inducible, or tissue specific, operably linked to said nucleotide sequence. In particular, the recombinant DNA construct may further comprise a strong constitutive promoter in front of a transcribed cassette consisting of the full open reading frame of the gene followed by a terminator sequence.

In a particular embodiment of the present invention the nucleic acid construct, or recombinant DNA construct, further comprises a strong constitutive promoter in front of a transcribed cassette consisting of part of the target gene followed by a plant functional intron followed by the same part of the target gene in reverse orientation, the transcribed cassette is followed by an terminator sequence. The preferred vector is of such type with one of the nucleotide sequence of the invention is inserted in inverted repeat orientation.

Thus, according to one embodiment the invention relates to the method according to any one of claims 1 -13, wherein the recombinant DNA construct further comprises a strong constitutive promoter in front of a transcribed cassette consisting comprising a nucleotide sequence as defined in claim 1 followed by a plant functional intron followed by the nucleotide sequence as defined in claim 1 in reverse orientation The recombinant DNA construct may be used for transforming regenerable cells of a plant and regenerating a transgenic plant from said transformed cell. In the presently exemplified embodiments of the invention the recombinant DNA construct comprises the sequence of SEQ ID NO: 29.

### *Construction of vectors*

In general, those skilled in the art are well able to construct vectors of the present invention and design protocols for recombinant gene expression. For further details on general protocols for preparation of vectors reference is made to: Molecular Cloning: a Laboratory Manual: 3rd edition, Sambrook et al, 2001, Cold Spring Harbor Laboratory Press. Further information about plant vector and cloning in plants can be found in Gelvin SB and Schilperoort RA (eds) (2000) Plant Molecular Biology Manual. Dordrecht: Kluwer Academic Publishers. The promoter used for the gene may influence the level, timing, tissue, specificity, or inducibility of the over expression.

Generally, over expression of a gene can be achieved using a recombinant DNA construct having a promoter operably linked to a DNA element comprising a sense element of a segment of genomic DNA or cDNA of the gene, e.g., the segment should contain enough of the open reading frame to produce a functional protein and preferably the full open reading frame.

In pertinent embodiments of the invention the nucleic acid construct, or recombinant DNA construct, further comprising a constitutive, inducible, or tissue specific promoter operably linked to said nucleotide sequence.

In a presently preferred embodiment of the invention, the nucleic acid construct, or recombinant DNA construct, used for RNAi comprises the sequence of SEQ ID NO: 116 the vector named pK7GWIWG2(I), Karimi et al 2002.

In another preferred embodiment of the invention, the nucleic acid construct, or recombinant DNA construct, used for over expression comprises the sequence of SEQ ID NO: 117 the vector named pK2GW7, Karimi et al 2002.

### Approaches to obtaining altering the level of a gene product

Description of each gene and what is known about them and how it might be related to lignin is found in the experimental section below.

One aspect of the invention is to increase the expression of certain genes, non limiting examples how this can be done are presented here. Up regulation or over expression of a gene can for example be achieved by placing the full open reading frame of the gene behind a suitable promoter, which are described elsewhere, and usually placing terminator and poly-adenylation signal sequence 3’ of the gene to be over expressed.

One other aspect of the invention is to decrease the expression of certain genes, non limiting examples how this can be done are presented here. Suppression of gene expression can for instance be achieved using a ribozyme or an antisense sequence, or down-regulated by an RNAi species. Several extensive descriptions on how genes can be down-regulated can be found in the literature and in patent literature as well, one example is the WO 2008/067841.

One of the genes SEQ ID No: 23-115 and 157-195 could be used as targets for marker assisted breeding because changes in the gene regulatory sequences can give changes in the expression patterns and changes in the coding sequences can give changes in the gene function, and we have shown that manipulating these genes gives changes in the desired traits. This is usually referred to that the genes SEQ ID No: 23-115 and 157-195 can be used as candidate genes in marker assisted breeding Brady and Provart 2007, Varshney et al 2005, and Burke et al. 2007. This can be used to achieve both up and down regulation of gene activity.

One particular way to use this invention is to measure the expression of one or more of the genes SEQ ID No 23 to 115 and 157-195 using for example quantitative RT-PCR in natural populations and select for unusual high or low expression of the measured gene and use such plants as parents in a breeding program, this could be repeated for each breeding cycle. Many methods to quantify gene expression are known to the art, an efficient method is real time PCR, described in Bustin 2000.

In addition, the nucleic acid construct or recombinant DNA construct according to the invention may be used for the purpose of gene replacement in order to modify the plant lignin properties phenotype.

Suppression of endogenous gene expression can for instance be achieved using a ribozyme. Ribozymes are RNA molecules that possess highly specific endoribonuclease activity. The production and use of ribozymes are disclosed in US 4987071 and US 5543508. While antisense techniques are discussed below, it should be mentioned that synthetic ribozyme sequences including antisense RNAs can be used to confer RNA cleaving activity on the antisense RNA, such that endogenous mRNA molecules that hybridize to the antisense RNA are cleaved, which in turn leads to an enhanced antisense inhibition of endogenous gene expression.

Vectors in which RNA encoded by a relevant gene homologue is over-expressed can also be used to obtain co-suppression of a corresponding endogenous gene, e. g., in the manner described in US 5231020 to Jorgensen. Such co-suppression (also termed sense suppression) does not require that the entire gene sequence be introduced into the plant cells, nor does it require that the introduced sequence be exactly identical to the endogenous sequence of interest. However, the suppressive efficiency will be enhanced as specificity of hybridization is increased, e. g., as the introduced sequence is lengthened, and/or as the sequence similarity between the introduced sequence and the endogenous transcription factor gene is increased.

Vectors expressing an untranslatable form of gene, e. g., sequences comprising one or more stop codons, or nonsense mutation, can also be used to suppress expression of an endogenous gene, thereby reducing or eliminating its activity and modifying one or more traits. Methods for producing such constructs are described in US 5583021. In particular, such constructs can be made by introducing a premature stop codon into the gene.

One way of performing targeted DNA insertion is by use of the retrovirus DNA integration machinery as described in WO2006/078431. This technology is based on the possibility of altering the integration site specificity of retroviruses and retrotransposons integrase by operatively coupling the integrase to a DNA-binding protein (tethering protein). Engineering of the integrase is preferably carried out on the nucleic acid level, via modification of the wild type coding sequence of the integrase by PCR. The integrase complex may thus be directed to a desired portion or be directed away from an undesired portion of genomic DNA thereby producing a desired integration site characteristic.

Another technology that can be used to alter gene expression and gene activity is the TILLING, "Targeting Induced Local Lesions in Genomes", which is a non-transgenic way to alter gene function in a targeted way. This approach involves mutating a plant with for example ethyl methanesulfonate (EMS) and later locating the individuals in which a particular desired gene has been modified. The technology is described for instance in Slade and Knauf, 2005 and Henikoff, et al.

A method for abolishing the expression of a gene is by insertion mutagenesis using the T-DNA of *Agrobacterium tumefaciens.* After generating the insertion mutants, the mutants can be screened to identify those containing the insertion in an appropriate gene. Plants containing a single transgene insertion event at the desired gene can be crossed to generate homozygous plants for the mutation.

The polynucleotides and polypeptides of this invention can also be expressed in a plant in the absence of an expression cassette by manipulating the activity or expression level of the endogenous gene by other means, for example, by ectopically expressing a gene by T-DNA activation tagging, Ichikawa et al. (1997); Kakimoto et al. (1996). This method entails transforming a plant with a gene tag containing multiple transcriptional enhancers and once the tag has inserted into the genome, expression of a flanking gene coding sequence becomes deregulated, Ichikawa et al. (1997); Kakimoto et al. (1996). In another example, the transcriptional machinery in a plant can be modified so as to increase transcription levels of a polynucleotide of the invention (See, e. g., PCT Publications WO 96/06166 and WO 98/53057 which describe the modification of the DNA binding specificity of zinc finger proteins by changing particular amino acids in the DNA binding motif).

However, the recombinant DNA construct, comprising a nucleotide sequence as described above is particularly useful for sense and anti-sense suppression of expression, e. g., to down-regulate expression of a particular gene, in order to obtain a plant phenotype with altered lignin properties. That is, the nucleotide sequence of the invention, or subsequences or anti-sense sequences thereof, can be used to block expression of naturally occurring homologous nucleic acids. Varieties of traditional sense and antisense technologies are known in the art, e. g., as set forth in Lichtenstein and Nellen (1997). The objective of the antisense approach is to use a sequence complementary to the target gene to block its expression and create a mutant cell line or organism in which the level of a single chosen protein is selectively reduced or abolished.

For more elaborate descriptions of anti-sense regulation of gene expression as applied in plant cells reference is made to US 51 07065.

Gene silencing that is induced by double-stranded RNA is commonly called RNA interference or RNAi. RNA interference is a molecular mechanism in which fragments of double-stranded ribonucleic acid (dsRNA) interfere with the expression of a particular gene that shares a homologous sequence with the dsRNA. The process that is mediated by the same cellular machinery that processes microRNA, known as the RNA-induced silencing complex (RISC). The process is initiated by the ribonuclease protein Dicer, which binds and cleaves exogenous double-stranded RNA molecules to produce double-stranded fragments of 20-25 base pairs with a few unpaired overhang bases on each end. The short double-stranded fragments produced by Dicer, called small interfering RNAs (siRNAs), are separated and integrated into the active RISC complex. If one part of an RNA transcript is targeted by an RNAi molecule or construct, the whole transcript is down-regulated.

For more elaborate descriptions of RNAi gene suppression in plants by transcription of a dsRNA reference is made to US 6506559, US 2002/0168707, and WO 98/53083, WO 99/53050 and WO 99/61 631.

The presently preferred nucleic acid construct for RNAi is the pDONR vector (Invitrogen USA) and a destination vector pK2GW7 using Gateway technology (Invitrogen USA).

***Transformation of plant cells***

In accordance with the present invention, the method comprise the further step of transforming regenerable cells of a plant with said nucleic acid construct or recombinant DNA construct and regenerating a transgenic plant from said transformed cell. When introducing the above DNA construct or vector into a plant cell, certain considerations must be taken into account, well known to those skilled in the art. The nucleic acid to be inserted should be assembled within a construct that contains effective regulatory elements that will drive transcription, as described above. There must be available a method of transporting the construct into the cell. Once the construct is within the cell, integration into the endogenous chromosomal material either will or will not occur.

Transformation techniques, well known to those skilled in the art, may be used to introduce the DNA constructs and vectors into plant cells to produce transgenic plants, in particular transgenic trees, with altered lignin properties.

A person of skills in the art will realise that a wide variety of host cells may be employed as recipients for the DNA constructs and vectors according to the invention. Non-limiting examples of host cells include cells in embryonic tissue, callus tissue type I, II, and III, hypocotyls, meristem, root tissue, tissues for expression in phloem, leaf discs, petioles and stem internodes.

As listed above, *Agrobacterium* transformation is one method widely used by those skilled in the art to transform tree species, in particular hardwood species such as poplar. Production of stable, fertile transgenic plants is now a routine in the art. Other methods, such as microprojectile or particle bombardment, electroporation, microinjection, direct DNA uptake, liposome mediated DNA uptake, or the vortexing method may be used where *Agrobacterium* transformation is inefficient or ineffective, for example in some gymnosperm species.

Alternatively, a combination of different techniques may be employed to enhance the efficiency of the transformation process, e.g. bombardment with *Agrobacterium* coated microparticles or microprojectile bombardment to induce wounding followed by co-cultivation with *Agrobacterium.*

It will be understood, that the particular choice of a transformation technology will be determined by its efficiency to transform certain plant species as well as the experience and preference of the person practising the invention with a particular methodology of choice. It will be apparent to the skilled person that the particular choice of a transformation system to introduce nucleic acid into plant cells is not essential to or a limitation of the invention, nor is the choice of technique for plant regeneration.

Following transformation, transgenic plants are preferably selected using a dominant selectable marker incorporated into the transformation vector. Typically, such a marker will confer antibiotic or herbicide resistance on the transformed plants and selection of transformants can be accomplished by exposing the plants to appropriate concentrations of the antibiotic or herbicide. A novel selection marker using the D-form of amino acids and based on the fact that plants can only tolerate the L-form offers a fast, efficient and environmentally friendly selection system. An interesting feature of this selection system is that it enables both selection and counter-selection.

Subsequently, a plant may be regenerated, e.g. from single cells, callus tissue or leaf discs, as is standard in the art. Almost any plant can be entirely regenerated from cells, tissues and organs of the plant, Gelvin SB and Schilperoort RA (eds) (2000) Plant Molecular Biology Manual. Dordrecht: Kluwer Academic Publishers. After transformed plants are selected and grown to maturity, those plants showing an altered lignin properties phenotype are identified. Additionally, to confirm that the phenotype is due to changes in expression levels or activity of the polypeptide or polynucleotide disclosed herein can be determined by analyzing mRNA expression using Northern blots, RT-PCR or microarrays, or protein expression using immunoblots or Western blots or gel shift assays.

***Plants***

According to another aspect the invention provides a plant having altered lignin properties compared to its wild type. The plant may be transgenic

A transgenic plant may comprise a recombinant polynucleotide (DNA construct) comprising a nucleotide sequence capable of altering in the plant the level of a gene product of at least one of the genes SEQ ID 23-115 giving altered lignin content when comparing said group of transgenic plants grown for 8 -9 weeks in a greenhouse under a photoperiod of 18 hours, a temperature of 22°C/15°C (day/night) and a weekly were fertilized weekly Weibulls Rika S NPK 7-1-5 diluted 1 to 100 (final concentrations $NO_3$, 55 g/l; $NH_4$, 29 g/l; P, 12 g/l; K, 56 g/l; Mg 7,2 g/l; S, 7,2 g/l; B, 0,18 g/l; Cu, 0,02 g/l; Fe, 0,84 g/l; Mn, 0,42 g/l; Mo, 0,03 g/l; Zn, 0,13 g/l) with a group of wild-type plants grown under identical conditions. Growth conditions must be adapted to the particular species transform, since they have different growth optima. The alteration may vary between a decrease in lignin content from -6% to -20% and an increase in lignin content from +5% to +16%.

According to particular embodiments of the invention the level of a gene product of at least one gene comprising a nucleotide sequence selected from the group consisting of:

a) a nucleotide sequence from SEQ ID NO: 29 or;
b) a nucleotide sequence being at r 80% identical to a nucleotide sequence shown as SEQ ID NO: 29 has been altered relative to the level found in the respective corresponding wild-type plant.

The invention especially relates to plants having lower lignin content, comprising the genes as mentioned under the heading "Method of producing a plant having altered lignin properties".

According to yet another embodiment of the invention, the transgenic plant comprises a recombinant polynucleotide (DNA construct) comprising a nucleotide sequence selected from the group consisting of:

a)a nucleotide sequence comprising a sequence selected from SEQ ID NO: 29 or
b)a complementary nucleotide sequence of a nucleotide sequence of a) or
c) a nucleic acid sequence being at 80% identical to a nucleotide sequence shown as SEQ ID NO: 29 or
d) a nucleotide sequence which hybridizes under stringent conditions to a nucleotide sequence of a), b) or c).

In further embodiments of this aspect of the invention the nucleic acid sequence in d) is substantially identical as herein defined to any one of the sequences in a), b) or c).

As mentioned above the skilled person will realize that a variety of methods exist in the art for producing the nucleic acid sequences and polynucleotide constructs of the invention, e.g. by cloning techniques, assembly of fragments generated by solid phase synthesis.

In particular, the transgenic plant according to the present invention may comprise a recombinant DNA construct comprising a nucleotide sequence which relative to the particular sequences described, comprises conservative variations altering only one, or a few amino acids in the encoded polypeptide may also be provided and used according to the present invention. Accordingly, it is within the scope of the invention to provide a transgenic plant comprising a recombinant DNA construct comprising a nucleotide sequence which encodes a polypeptide comprising a conservatively substituted variant of a polypeptide of a) or d).

Accordingly, the present invention may also provide a recombinant DNA construct, wherein the nucleotide sequence comprises a silent substitution in a nucleotide sequence, that is, the recombinant DNA construct may comprise a sequence alteration that does not change the amino acid sequence encoded by the polynucleotide.

In certain further embodiments of the invention, the sub-sequences or fragments substantial sequence identity to a conserved domain of a nucleotide sequence as described above under item a) or d).

In further embodiments the transgenic plant provided according to the invention comprises a recombinant polynucleotide construct which further comprises a constitutive, inducible, or tissue specific promoter operably linked to said nucleotide sequence.

In still further embodiments the recombinant polynucleotide construct further comprises a strong constitutive promoter in front of a transcribed cassette.

In a presently preferred embodiment of the invention, the transgenic plant according to the invention comprises a recombinant DNA construct comprising the sequence of SEQ ID NO: 29.

Another aspect of the invention provides a plant cell, plant progeny or any vegetable material of a transgenic plant or a plant with intentionally changed (increased or decreased expression) levels of one or more gene's SEQ ID No: 29 according to the invention or the above mentioned variants thereof and comprising a recombinant polynucleotide.

A further aspect of the invention provides a plant cell, plant progeny or any vegetable material of a transgenic plant or a plant with intentionally changed (increased or decreased expression) levels of one or more gene's SEQ ID: 29 according to the invention and comprising a recombinant polynucleotide, where the lignin content measured by the Klason method is modified.

The invention also relates to wood produced by a plant, which may be transgenic as defined above. It may comprise the nucleic acids of one or more of SEQ ID: 29 or the variants thereof defined above.

Selections of plants may be done by measuring the lignin level according to the Klason method.

***Plant species***

In accordance with the present invention, the transgenic plant may be a perennial plant which preferable is a woody plant or a woody species. In a useful embodiment, the woody plant is a hardwood plant which may be selected from the group consisting of acacia, eucalyptus, hornbeam, beech, mahogany, walnut, oak, ash, willow, hickory, birch, chestnut, poplar, alder, maple, sycamore, ginkgo, a palm tree and sweet gum. Hardwood plants from the *Salicaceae* family, such as willow, poplar and aspen including variants thereof, are of particular interest, as these two groups include fast-growing species of tree or woody shrub which are grown specifically to provide timber and biofuel.

In further embodiments, the woody plant is a conifer which may be selected from the group consisting of cypress, Douglas fir, fir, sequoia, hemlock, cedar, juniper, larch, pine, redwood, spruce and yew.

In useful embodiments, the woody plant is a fruit bearing plant which may be selected from the group consisting of apple, plum, pear, banana, orange, kiwi, lemon, cherry, grapevine and fig.

Other woody plants which may be useful in the present method may also be selected from the group consisting of cotton, bamboo and rubber plants.

Other plants which may be useful is grasses grown for biomass production, for example *Miscanthus* and Switchgrass.

The present invention extends to any plant cell of the above transgenic plants obtained by the methods described herein, and to all plant parts, including harvestable parts of a plant, seeds and propagules thereof, and plant explant or plant tissue. The present invention also encompasses a plant, a part thereof, a plant cell or a plant progeny comprising a DNA construct according to the invention. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced in the parent by the methods according to the invention.

**Improved lignin quality and amount**

In addition, the nucleic acid construct or recombinant DNA construct according to the invention may be used for the purpose of gene replacement in order to modify the lignin level in the plant.

This invention relates to the problem of changing wood chemistry especially for changing lignin quality and amounts.

Such trees can be used in many applications. For example for these usages

1. Changing surface chemistry for e.g. composite material usage.
2. Saccharification potential with emphasis of changing lignin levels and/or composition and/or lignin binding to the carbohydrates of the cell wall.
3. Changing lignin levels for energy content.
4. Changing lignin levels for improved pulping properties.

The inventors have found that the down regulation and/or over expression of certain genes, gives changed wood chemistry in the produced transgenic trees. Especially we have found that certain genes can be used to modify lignin levels. These genes have been found within an RNAi gene mining program, with a gene selection a basic set up identical to the one described in the patent applications WO 2008/067840 and WO 2008/067841, but where the first level of analysis were done with FT-IR analysis of wood and the second level of analysis were done with Klason Lignin analysis.

Thus, according to one aspect the invention regards the use of a nucleotide sequence selected from the group consisting of

a) one down regulated gene with SEQ ID NO 29 or,
b) a nucleotide sequence being at least 80% identical to a nucleotide sequence shown as SEQ ID NO: 29, in a plant for lowering lignin levels improving saccharification for bio-refining and ethanol production and for lowering lignin levels in pulp and paper processing.

The preferred nucleotide sequences are selected from the group consisting of 29 in a plant for lowering lignin levels improving saccharification for bio-refining and ethanol production and for lowering lignin levels in pulp and paper processing. See table 1 for lignin levels.

The invention also relates to the use of a nucleotide sequence selected from the group consisting of

a) at least one down regulated gene chosen from of the genes with SEQ ID NO 29
b) a nucleotide sequence being at least 80% identical to a nucleotide sequence shown as SEQ ID NO: 29. in a plant for lowering lignin levels improving saccharification for bio-refining and ethanol production and for lowering lignin levels in pulp and paper processing.

The effect of the preferred gene is shown in the table 1 below.

**Table 1** Down regulated genes with lower lignin content vs wildtype

| Gene | Construct | T-test | Anova | <T int | >T Int | < Min | >Max | Ratio | Modulation | SEQ ID | +/- vs wt T89 |
|---|---|---|---|---|---|---|---|---|---|---|---|
| STT 23 | KR080B | | | 1 | 0 | 1 | 0 | 0,97 | Down regulated RNAi | Seq ID No: 29 | -10% |

**Further uses of the invention**

Moreover, the invention relates to the use of a nucleotide sequence selected from the group consisting of

a) a nucleotide sequence from SEQ ID NO 29 or
b) a nucleotide sequence being at least 80% identical to a nucleotide sequence shown as SEQ ID NO: 29 for the identification of plants having altered lignin characteristics as compared to the wild-type.

According to the invention these sequences may also be used as candidate genes in marker assisted breeding.
It should be noted that embodiments and features described in the context of one of the aspects of the present invention also apply mutatis mutandis to the all other aspects of the invention. For example the variant and homology language for defining genes and expressed polypeptides regards all aspects of the inventions such as DNA constructs, expressions products of the genes, vectors, plant cells, plant progeny, wood and the methods for producing and identification of plants according to the invention.
The invention will now be described in further details in the following non-limiting examples.

**Examples**

In order to find and elucidate the function of genes involved in wood formation and wood growth, an extensive gene mining program was performed, resulting in the identification of genes useful in wood industrial applications.

**Gene Selection**

The genes indentified for changing wood chemistry especially for changing lignin quality and amounts was done in a gene mining program, which is described in detail in the patent applications WO 2008/067840, WO 2008/067841 and WO 2009/084999.

**Cloning of the selected genes**

Selected genes were subsequently cloned into a RNAi vector under the control of the CaMV 35S promoter (RNA interference vector, pK7GWIWG2(I)) using Gateway technology (Invitrogen USA), SEQ ID NO: 116. Two principal sets of cloning primers were used, one set was a universal primer pair binding to the vector and the poly-A tail, and the other set were gene-specific primers. The PCR product was first transferred into the pDONR vector and subsequently transferred into the destination vector pK7GWIWG2 according to manufacturers' recommendations (Invitrogen USA). The sequences of the genes and the corresponding construct, and cloning primer are listed in Table 2 and 3.
Full Sequence from *Populus trichocarpa* means sequences of the candidate genes as predicted from *Populus trichocarpa* genome sequence at JGI (G.A. Tuskan et al. Science 15 September 2006:Vol. 313. no. 5793, pp. 1596 - 1604). The predicted sequence corresponds to the sequences that best maps the actual transcript sequence from hybrid aspen or the corresponding gene model at JGI. Full Sequence from *Populus tremula x tremuloides* means the best sequence of the candidate genes resulting from resequencing of hybrid aspen EST's, sequencing of fragments cloned in the RNAi construct and EST sequences present in the Pop-db, sequence used for RNAi construct means the sequence of the actually cloned fragment in the RNAi constructs.

Table 2. Summary of the cloning primers used

| Gene | Construct | Cloning forward primer | Cloning reverse primer |
|---|---|---|---|
| STT23 | KR080B | SEQ ID No:01 | SEQ ID No:09 |

Table 3. Genes and the corresponding RNAi construct sequences

| Gene | Construct | Sequence used for RNAi construct | Full Sequence from *Populus trichocarpa* | Full Sequence from *Populus tremula x tremuloides* |
|---|---|---|---|---|
| STT23 | KR080B | SEQ ID No:29 | SEQ ID No:65 | SEQ ID No:29 |

***Plant transformation***

CaMV 35S: Inverted repeat DNA constructs were transformed into *Agrobacterium* and subSEQuent into Hybrid aspen, *Populus tremula* L. x *P. tremuloides* Minch. Clone T89, hereafter called "poplar", was transformed and regenerated essentially as described in Nilsson et al. (1992). Approximately 3-8 independent lines were generated for each construct. One such group of transgenic trees produced using one construct is hereafter called a "construction group", e.g. different transgenic trees emanating from one construct. Each transgenic line within each construction group, e.g. KR555-2B KR555-3A, KR555-2B and so on, are different transformation events and therefore most probably have the recombinant DNA inserted into different locations in the plant genome. This makes the different lines within one construction group partly different. For example it is known that different transformation events will produce plants with different levels of gene down-regulation when using RNAi constructs of the type used here.

***Plant growth***

The transgenic poplar lines were grown together with their wild type control (wt) trees, in a greenhouse under a photoperiod of 18 h and a temperature of 22°C/15°C (day/night). The plants were fertilized weekly Weibulls Rika S NPK 7-1-5 diluted 1 to 100 (final concentrations $NO_3$, 55 g/l; $NH_4$, 29 g/l; P, 12 g/l; K, 56 g/l; Mg 7,2 g/l; S, 7,2 g/l; B, 0,18 g/l; Cu, 0,02 g/l; Fe, 0,84 g/l; Mn, 0,42 g/l; Mo, 0,03 g/l; Zn, 0,13 g/l). The plants were grown for 8-9 weeks before harvest. During this time their height and diameter was measured 1 to 2 times per week. A number of wild type trees (typically 15-25 trees) and a number of transgenic trees comprising several construction groups (typically 6-20 construction groups) were grown in parallel in the greenhouse under the same above conditions. All comparisons between the wild type trees and construction groups are made within each growth group.

***Sampling***

Two principal types of harvest and sampling were performed. One general type was for example for chemical analysis, wood morphological analysis, gene expression analysis, wood density analysis and metabolomics analysis. And another type for dry weight measurements of bark, wood, leafs and roots.

***Selection of Construction Groups***

In the first round of growth for each group of trees with a specific gene down regulated using RNAi, i.e. a construction group, a number of the following analyses was performed: FT-IR analysis. These data were analysed in order to single out the Construction Groups that showed a phenotypic variation compared to wild type control trees.

***Selection of construction groups using FT-IR and Multivariate Analysis***

FT-IR Analysis

All samples were freeze dried and milled to a fine powder using a ball mill (Retch NM 301) prior to FT-IR analysis. The measuring procedure has been shifted over time; samples have been measured as pure samples or as 2.5% sample in potassium bromide (KBr). Different instruments (Bruker IFS 66v and Bruker 55 Equinox, Bruker) have been used hence the wavelength range changed from 900-1800nm to 400-5200nm in the latter case only the fingerprint region 800-1860nm have been used for multivariate analysis.

Baseline correction and normalization

Two different approaches have been used to normalize the samples prior to multivariate analysis in order to reduce effects such as baseline and concentration variations.

When the samples are measured using the instrument with the larger wavelength range a baseline is fitted for each sample using one point on each side of the fingerprint region (~800nm and ~1860nm) the baseline is subtracted and the area under the spectrum normalized to a constant sum.[ Orthogonal Projections to Latent Structures Discriminant Analysis Modeling on in Situ FT-IR Spectral Imaging of Liver Tissue for Identifying Sources of Variability Stenlund, Hans, Gorzsás, András, Persson, Per, Sundberg, Björn, and Trygg, Johan Anal. Chem., 2008, 10.1021/ac8005318 Web Release Date: August 20, 2008]

When samples are measured using the other instrument that only measures between 900-1800nm the first two principal components has been removed from data since they were considered to only contain variation related to baseline and concentration variations.

Multivariate analysis

The goal with the multivariate analysis has been to detect constructions (genes) that in some means deviate from the wild type population. Principal component analysis (PCA) has been utilized to reduce the dimensionality from -500 variables (recorded wavelengths) into ~5 principal components. All wild type samples in the same cultivation round are modelled using PCA and the different "construction samples" belonging to the same cultivation round has been predicted into that model to detect deviations in principal component space (score space) as well as in the residual. A construction that has at least 2 samples deviating in the same direction in the score space or in the residual has been scored as a changed phenotype and then been further analyzed by other methods.

A construction group that scored positive in the FT-IR analysis was in most cases analyzed for lignin content.

Lignin measurements

Lignin content was measured at MoRe Research, Örnsköldsvik, using their internal method MoRe KA 10.219, which is based upon the Tappi method T 222 om-88 (http://www.tappi.org) with some modifications. The method is known as Klason-lignin and uses the fact that lignin is insoluble in acids.

The dried milled wood samples are pre hydrolyzed in 72% sulfuric acid which then is diluted to 2% and further hydrolyzed during refluxing for 9hr. The dispersed lignin is filtered of and then washed and dried. The weight of the dried lignin is measured and compared with the original weight to decide the lignin content.

Statistical Evaluation

Each construction is subjected to 4 different tests;

1) T-test Blom ISBN 91-44-05592-7; the mean value for the samples belonging to each construction is compared with the mean value for the corresponding T89 samples, using a 2-tailed T-test assuming unequal variance. If the probability that the mean values are equal is less that 5% the construction is considered to have changed lignin content.
2) Anova; the between and within group (construction and wild type) variation is compared using an F-test Blom ISBN 91-44-05592-7. If the probability that the between and the within group variation is equal is lower than 5% the construction is considered to have changed lignin content. This is equivalent to a 2-tailed T-test assuming equal variance.
3) A 95 % confidence interval is calculated around the mean value for the wild type samples using T statistics. One or more lines of a construction have lignin content outside this confidence interval the construction is considered to affect the lignin content.
4) A non statistical measure, were the samples for each construction is compared with the lowest and highest value for the corresponding T89 samples. If one or more samples from one construction have higher lignin content than the highest or lower than the lowest lignin content for a T89 sample, it the construction is assumed to affect the lignin content.

Construction groups meeting one or more of these criteria were selected.

A number of the construction groups that showed differences in lignin levels were tested with RT-PCR in order to verify the down regulation of the targeted gene.

Real-time RT-PCR (Bustin 2000) was used to compare construct gene expression levels of the construction group with corresponding wild type group. The expression level of 26S ribosome regulatory subunit S2 was used as a reference to which construct gene expression was normalized. The comparative CT method was used for calculation of relative construct gene expression levels, where the ratio between construction and reference gene expression levels is described by $(1 + E_{target})^{-CTtarget} / (1 + E_{reference})^{-CTreferenc}e$ where $E_{target}$ and $E_{reference}$ are the efficiencies of construct and reference gene PCR amplification respectively and $CT_{target}$ and $CT_{reference}$ are the threshold cycles as calculated for construct and reference gene amplification respectively.

For total RNA extraction, stem samples (approx. 50mg) were harvested from greenhouse grown plants and flash frozen in liquid nitrogen. Frozen samples were ground in a bead mill. Total RNA was extracted using Aurum Total RNA Mini kit according to manufacturers' recommendations (Bio-Rad). cDNA synthesis was performed using iScript cDNA synthesis kit according to manufacturers recommendations (Bio-Rad).

For real-time PCR, cDNA template was mixed with SYBR Green Supermix according to manufacturers recommendations (Bio-Rad) and corresponding construct gene specific primers or internal reference gene specific primers, forward PCR primer SEQ ID NO: 123 and reverse PCR primer sequence SEQ ID NO: 124. Real-time PCR was run on a MyiQ PCR thermocycler (Bio-Rad) and analysed using included software iQ5. For each sample, reactions were set up in three to six replicates, with an equal number of replicates using construct gene specific primers and reference gene specific primers, and the average threshold cycle for the reaction replicates was subsequently used for calculation of relative construct gene expression levels.

The 96 well plate was covered with microfilm and set in the thermocycler to start the reaction cycle. By way of illustration, the reaction cycle may include the following steps: Initial denaturation at 95°C for 3 minutes 30 seconds followed by 40 rounds of amplification comprising the following steps 95°C for 10 seconds, 55°C for 30 seconds and 72°C for 40 seconds.

Selected genes were in a number of cases tested in over expression experiments were the full length open reading frame (ORF) for the gene corresponding to the RNAi construct were cloned and put under the CaMV 35S promoter and tested in transgenic trees. The RNAi constructs were from Hybrid aspen i.e. a hybrid of the two species *Populus tremuloides* and *Populus tremula.*

These plants were in many cases tested for lignin content and a number of these gene constructs also modified the cell wall chemistry.

Cloning of full length genes for over expression analysis using the 35S promoter

For over expression analysis of a corresponding full length gene, previously down regulated using RNAi, the gene coding sequence (CDS) was predicted from the genome sequence of *Populus trichocarpa* (Tuskan et al., 2006). The gene models were compared to, and in some instances corrected based on, information published for homologous genes in Arabidopsis thaliana and other plant species. This was done using databases such as http://www.ncbi.nlm.nih.gov/ and http://www.arabidopsis.org/. Selected genes were subsequently cloned into an over-expression vector under the control of the CaMV 35S promoter. Cloning primers were designed to be gene specific and to include the full CDS and in some instances parts of the untranslated transcript region (UTR). The full length gene was amplified from hybrid aspen cDNA using Phusion high fidelity DNA polymerase (Finnzymes) and first transferred into the pDONR vector (Invitrogen USA) and subsequently transferred into the destination vector pK2GW7 (Karimi et al., 2002) using Gateway technology (Invitrogen USA). Constructs thus cloned have construct names starting with 35s. The sequences of the selected full length genes, PCR primers etc. are listed in Table 3.

Cloning of full length genes for over expression analysis using the LMP1 promoter

For over expression analysis of a corresponding full length gene, previously down regulated using RNAi, the gene coding sequence (CDS) was predicted from the genome sequence of *Populus trichocarpa* (Tuskan et al., 2006). The gene models were compared to, and in some instances corrected based on, information published for homologous genes in Arabidopsis thaliana and other plant species. This was done using databases such as http://www.ncbi.nlm.nih.gov/ and http://www.arabidopsis.org/. Selected genes were subsequently cloned into an over-expression vector under the control of the LMP1 promoter, described in patent application WO 2004/097024. Cloning primers were designed to be gene specific and to include the full CDS and in some instances parts of the untranslated transcript region (UTR). The full length gene was amplified from hybrid aspen cDNA using Phusion high fidelity DNA polymerase (Finnzymes) and first transferred into the pDONR vector (Invitrogen USA) and subsequently transferred into the destination vector pPCV812-LMP1-Gateway using Gateway technology (Invitrogen USA). Constructs thus cloned have construct names starting with LMP1. The pPCV812 plant binary vector is described in Koncz et al., 1994. The pPCV812 vector has been further modified to create the pPCV812-LMP1-Gateway vector by insertion of the LMP1 promoter between the BglII and SalI sites and by replacing the GUS reporter gene *uidA* with a Gateway recombination cassette (attR1-ccdB-attR2, Invitrogen Life Technologies) using BamHI and SacI sites. The sequences of the selected full length genes, PCR primers etc. are listed in Table 3.

Cloning of full length transcription factor genes for over expression analysis using the 35S promoter

The corresponding gene models for the selected transcription factor genes were extracted from data derived from the genome sequencing of *Populus trichocarpa* (Tuskan et al., 2006) using BLAST analysis. The gene models were compared to, and in some instances corrected based on, information published for homologous genes in Arabidopsis thaliana and other plant species. This was done using databases such as http://www.ncbi.nlm.nih.gov/ and http://www.arabidopsis.org/. Selected genes were subsequently cloned into an over-expression vector under the control of the CaMV 35S promoter. For isolation of cDNA, total RNA was isolated from stem, leaf and bark tissue sampled from hybrid aspen clone T89 plants and reverse transcribed to cDNA using Superscript III First Strand Synthesis System (Invitrogen). cDNA were then amplified by PCR with gene specific forward and reverse primers using Phusion high fidelity DNA polymerase (Finnzymes). PCR primers were selected as follows, the 5'-primer was placed at the start codon and the 3' reverse primer was placed 3' of the translational stop site. Forward primers were modified by the introduction of a Kozak sequence (5'-AGAACC-3') upstream and next to the start codon of each target gene. The amplified cDNAs were inserted into a Gateway entry vector pENTR/D-TOPO (Invitrogen), followed by transfer of the genes into the expression vector pK2GW7 (Karimi et al., 2002) using the Gateway LR recombination reaction (Invitrogen). The cloned genes were control sequenced and compared to the selected genes using standard techniques before sub cloning into the plant vector pK2GW7.

The gene STT 23 was not over expressed.

**Results**

The isolated genes and the corresponding constructions that has been used is shown below, which passed the selection criteria according to the invention, table 4.

Table 4: Summary of modulated lignin results

| Gene | Construct | T-test | Anova | <T int | >T Int | < Min | >Max | Ratio | Modulation | SEQ ID | +/- vs wk T89 |
|---|---|---|---|---|---|---|---|---|---|---|---|
| STT 23 | KR080B | | | 1 | 0 | 1 | 0 | 0,97 | Down reg. RNAi | No: 29 | -10% |

The first column "Gene" denotes the gene number of the construction, which was used in transformation. The following two columns contain the result of a paired t-test and ANOVA respectively. "<T inv" and ">T inv" denotes the number of samples outside the T (95%) distribution for T89 on the lower and upper side respectively. ">Max" and "<Min" denotes the number of samples higher the highest T89 and lower than the lowest T89 sample respectively. Ratio is the ratio between construction average and T89 average.

Gene STT 23

This gene is a UTP--glucose-1-phosphate uridylyltransferase family protein. The RNAi construction group KR080 shows a maximum decrease of lignin of 10% compared to the wild type average see Table 5 below.

Table 5

| Individual Name | Lignin Content (%) | Individual Name | Lignin Content (%) |
|---|---|---|---|
| T89-152 | 19,1 | KR080B-1A-A | 16,6 |
| T89-153 | 18,1 | KR080B-1A-B | 17,7 |
| T89-154 | 19 | KR080B-1B-A | 17,4 |
| T89-155 | 18,5 | KR080B-1B-B | 18,8 |
| T89-158 | 18,8 | KR080B-2A-A | 18,6 |
| T89-159 | 17,4 | KR080B-2A-B | 18,3 |
| T89-160 | 17,9 | KR080B-2B-A | 17,8 |
| | | KR080B-2B-B | 17,6 |

The down regulation of the gene STT 23 in the KR080B construction group were assessed with RT-PCR, see RT-PCR Table 6 below for results. These results shows that the lines tested have a weakly down-regulated expression of the STT 23 gene. Construct gene specific primer sequences used were forward SEQ ID NO: 125 and reverse SEQ ID NO: 126.

Table 6: RT-PCR Relative expression level.

| The table shows the relative expression levels for the different construction group lines and control trees for the gene targeted. | | | |
|---|---|---|---|
| **Line** | **Relative expression** | **Line** | **Relative expression** |
| KR080 1AA-1 | 1,1 | T89-1 | 0,7 |
| KR080 1AA-2 | 0,7 | T89-15 | 0,7 |
| KR080 1AA-3 | 1,0 | T89-16 | 0,6 |
| KR080 1BA-1 | 0,4 | T8923 | 1,0 |
| KR080 1BA-2 | 0,6 | T89-24 | 0,9 |
| KR080 1BA-3 | 0,4 | T89-30 | 0,9 |
| KR080 1BB-1 | 0,3 | T89-39 | 1,3 |
| KR080 1BB-2 | 0,5 | T89-37 | 0,7 |
| KR080 1BB-3 | 0,8 | T89-33 | 0,7 |
| KR080 2AA-1 | 0,5 | T89-12 | 1,0 |
| KR080 2AA-2 | 0,7 | T89-41 | 0,8 |
| KR080 2AA-3 | 0,7 | T89-4 | 1,0 |
| KR080 2BA-1 | 1,6 | T89-26 | 0,7 |
| KR080 2BA-2 | 0,4 | T89-27 | 1,0 |
| KR080 2BA-3 | 0,6 | T89-45 | 0,7 |
| KR080 2BB-1 | 0,5 | | |
| KR080 2BB-2 | 0,8 | | |
| KR080 2BB-3 | 0,3 | | |
| KR080 AB-1 | 0,3 | | |
| KR080 AB-2 | 0,6 | | |

| | |
|---|---|
| Number (KR080B) | 8 |
| Average (KR080B) | 17,85 |
| Standard deviation (KR080B) | 0,71 |
| Number (T89) | 7 |
| Average (T89) | 18,40 |
| Standard deviation (T89) | 0,63 |
| Max (T89) | 19,10 |
| Min (T89) | 17,40 |
| Confidence Interval (T89) (95%) | 18,4 +/- 1,53 |
| Ratio (Number (KR080B)/Average (T89)) | 0,97 |
| Ratio (Max(KR080B)/Average(T89)) | 1,02 |
| Ratio (Min(KR080B)/Average(T89)) | 0,90 |
| T-test | 0,1349 |
| Anova | 0,1381 |

(continued)

| The table shows the relative expression levels for the different construction group lines and control trees for the gene targeted. | | | | |
|---|---|---|---|---|
| **Line** | **Relative expression** | **Line** | **Relative expression** | |
| Num KR080B >Confidence Interval (T89) (95%) (19,93) | | | | 0 |
| Num KR080B <Confidence Interval (T89) (95%) (16,87) | | | | 1 |
| Num KR080B >Max (T89) (19,1) | | | | 0 |
| Num KR080B <Min (T89) (17,4) | | | | 1 |

**References:**

Aspeborg et al., Plant Physiology (2005), 137(3), 983-997

Baucher, Critical Reviews in Biochemistry and Molecular Biology, 38:305-350, 2003

Blom Statistikteori med tillämpningar ISBN 91-44-05592-7

Brady and Provart 2007. Journal of the Science of Food and Agriculture Vol 87, Pp 925 - 929,

Breaking the Biological Barriers to Cellulosic Ethanol: A Joint Research Agenda . A Research Roadmap Resulting from the Biomass to Biofuels Workshop, December 7-9, 2005, Rockville, Maryland

Burke et al 2007. Current opinion in genetics and development vol17, 525-532.

Bustin S A. Journal of Molecular Endocrinology ,2000. 25, 169-193.

Chen and Dixon. Nature Biotechnology Vol. 25 no 7: pp759-761, 2007

Chern et al. 2001) Plant J. 27: 101 113

Falco et al. US2005034176

Fan and Dong (2002) Plant Cell 14: 1377 1389

Feng and Doolittle (1987) J. Mol. Evol. 25: 351 360

Flinn et al in. US6,451,604

Gilmour et al. (1998) Plant J. 16: 433 442.

Griffiths, P. R. and De Haseth, J. A. Fourier Transform Infrared Spectrometry. New York: Wiley, 1986

Henikoff, Till, and Comai, Plant Physiol. 2004 Jun;135(2):630-6..

Heo et al., Plant and Cell Physiology (2005), 46(12), 2005-2018

Ichikawa et al. 1997 Nature 390 698-701;

Jaglo et al. (1998) Plant Physiol. 127: 910 917.

Kakimoto et al. 1996 Science 274: 982-985.

Karimi M, Inze D and Depicker A. Treands in plant Sciences. (2002) vol7 no5 pp193-195

Kang et al., Cell. 2001 Jun 1;105(5):625-36

Klason-lignin analysis Tappi method T 222 om-88, http://www.tappi.org

Koncz et al. (1994) Plant Molecular Biology Manual B2:1-22

Kosugi and Ohashi, (2002) Plant J. 29: 45 59

Lee et al. (2002) Genome Res. 12: 493 502

Lichtenstein and Nellen (1997), Antisense Technology: A Practical Approach IRL Press at Oxford University, Oxford, England.

Mount (2001), in Bioinformatics: Sequence and Genome Analysis Cold Spring Harbor Laboratory Press, Cold Spring Harbor, N.Y., page 543.

Nilsson et al. (1992) Transgenic Research 1, pp209-220

Ratcliffe et al. (2001) Plant Physiol. 126: 122 132.

Remm et al. (2001) J. Mol. Biol. 314: 1041 1052

Rognes T, 2001, Nucleic Acids Research, 29, 1647-1652, 1989

Sambrook et al., Molecular Cloning-A Laboratory Manual (3rd Ed.), Vol. 1-3, Cold Spring Harbor Laboratory, Cold Spring Harbor, New York, 2001.

Slade and Knauf, Transgenic Res. 2005 Apr;14(2):109-15

Smith and Waterman (1981). Hellene! DNA aligning

Thompson et al. (1994) Nucleic Acids Res. 22: 4673 4680.
Tuskan et al. Science 15 September 2006:Vol. 313. no. 5793, pp. 1596 - 1604
Vanholme et al. Current Opinion in Plant Biology 2008, 11:1-8
Varshney et al 2005. Trends in Plant Science, Vol 10, 621-630
Vasil et al. 1984, Cell Culture and Somatic Cell Genetics of Plants, Vol I, II and III, Laboratory Procedures.
Zhou et al. Plant and Cell Physiology (2006), 47(9), 1229-1240
US 2002/0168707
US 2005/034176
US 4987071
US 5107065
US 5231020
US 5543508
US 5583021
US 6451604
US 6506559
US 7402428
WO 91/14772
WO 96/06166
WO 98/53057
WO 98/53083
WO 99/53050
WO 99/61631
WO2004/097024
WO2006/078431
WO2008/067840
WO2008/067841
WO2008/006033

## Claims

**1.** A method for producing a tree having lower lignin content compared to its wild type, comprising down-regulating expression of a gene product expressed in the xylem forming tissue of a tree, said gene product being encoded by a nucleotide sequence selected from the group consisting of:

a) the nucleotide sequence shown as SEQ ID NO: 29; and
b) a nucleotide sequence being at least 80% identical to a nucleotide sequence shown as SEQ ID NO: 29.

**2.** The method according to claim 1, comprising:

i) providing an expression vector comprising a nucleotide sequence selected from the group consisting of:

a) the nucleotide sequence shown as SEQ ID NO: 29; and
b) a nucleotide sequence being at least 80% identical to a nucleotide sequence shown as SEQ ID NO: 29; and

at least one regulatory element operable linked to the polynucleotide sequence, wherein said at least one regulatory element controls expression of the polynucleotide sequence in a target plant;
ii) introducing the expression vector into at least one plant; and
iii) selecting at least one transgenic plant that has a modulated lignin quantity compared to its wild type.

**3.** The method according to claim 1 or 2, wherein said down-regulated expression is effected by introducing a genetic modification preferably in the locus of a gene comprising the nucleotide sequence shown as SEQ ID NO: 29 encoding a polypeptide or a homologue of such polypeptide or wherein said modification is effected by one of: T-DNA activation, TILLING, homologous recombination, site-directed mutagenesis using the nucleotide sequence shown as SEQ ID NO: 29 as markers in any step of the process of producing the desirable plants.

**4.** The method according to any one of claims 1-3, comprising the step of providing a recombinant DNA construct comprising a nucleotide sequence selected from the group consisting of:

a) the nucleotide sequence shown as SEQ ID NO: 29;
b) a complementary nucleotide sequence of a nucleotide sequence of a);
c) a nucleic acid sequence being at least 80% identical to any one of the sequences in a) and b); and
d) a nucleotide sequence which hybridizes under stringent conditions to a nucleotide sequence of a) or b).

**5.** The method according to any one of claims 1-4, wherein the nucleotide sequence encodes a polypeptide comprising a conservatively substituted variant of a polypeptide of a) or wherein the nucleotide sequence comprises a silent substitution in a nucleotide sequence.

**6.** The method according to claim 4 or 5, wherein the recombinant DNA construct further comprises a constitutive, inducible, or tissue specific promoter operably linked to said nucleotide sequence or wherein the recombinant DNA construct further comprises a strong constitutive promoter in front of a transcribed cassette consisting comprising a nucleotide sequence as defined in claim 4 followed by a plant functional intron followed by the nucleotide sequence as defined in claim 1 in reverse orientation.

**7.** The method according to any one of claims 4 to 6, wherein the method comprises the further step of transforming regenerable cells of a plant with said recombinant DNA construct and regenerating a transgenic plant from said transformed cell.

**Patentansprüche**

**1.** Verfahren zur Herstellung eines Baums mit einem niedrigeren Gehalt an Lignin verglichen mit seinem Wildtyp, umfassend Herunterregelung der Expression eines in dem Xylem-bildenden Gewebe eines Baums exprimierten Genprodukts, welches Genprodukt von einer Nukleotidsequenz kodiert wird, ausgewählt aus der Gruppe bestehend aus:

a) der als SEQ ID NO:29 gezeigten Nukleotidsequenz; und
b) einer Nukleotidsequenz, die zumindest 80 % mit einer als SEQ ID NO: 29 gezeigten Nukleotidsequenz identisch ist.

**2.** Verfahren nach Anspruch 1, umfassend:

i) Bereitstellen eines Expressionsvektors umfassend eine Nukleotidsequenz, ausgewählt aus der Gruppe bestehend aus:

a) der als SEQ ID NO:29 gezeigten Nukleotidsequenz; und

b) einer Nukleotidsequenz, die zumindest 80 % mit einer als SEQ ID NO:29 gezeigten Nukleotidsequenz identisch ist; und zumindest einem regulatorischen mit der Polynukleotidsequenz operabel verbundenen Element, wobei das zumindest eine regulatorische Element die Expression der Polynukleotidsequenz in einer Zielpflanze kontrolliert;

ii) Einführen des Expressionsvektors in zumindest eine Pflanze; und
iii) Auswählen zumindest einer transgenen Pflanze, die verglichen mit ihrem Wildtyp eine modulierte Menge an Lignin aufweist.

**3.** Verfahren nach Anspruch 1 oder 2, wobei die herunterregulierte Expression durch Einführen einer genetischen Modifikation, vorzugsweise in den Lokus eines Gens, umfassend die als SEQ ID NO:29 gezeigte Nukleotidsequenz, kodierend ein Polypeptid oder ein Homolog eines solchen Polypeptids, erfolgt, oder wobei die Modifikation durch eine von: T-DNA-Aktivierung, TILLING, homologer Rekombination, ortsgerichteter Mutagenese unter Anwendung der als SEQ ID NO:29 gezeigten Nukleotidsequenz als die Marker in jedem Schritt des Prozesses zur Herstellung der erwünschten Pflanzen erfolgt.

**4.** Verfahren nach einem der Ansprüche 1 bis 3, umfassend den Schritt des Bereitstellens eines rekombinanten DNA-Konstrukts umfassend eine Nukleotidsequenz, ausgewählt aus der Gruppe bestehend aus:

a) der als SEQ ID NO: 29 gezeigten Nukleotidsequenz;
b) einer komplementären Nukleotidsequenz einer Nukleotidsequenz von a);
c) einer Nukleinsäuresequenz, die zumindest 80 % mit einer der Sequenzen in a) und b) identisch ist; und
d) einer Nukleotidsequenz, die unter stringenten Bedingungen zu einer Nukleotidsequenz von a) oder b) hybridisiert.

**5.** Verfahren nach einem der Ansprüche 1 bis 4, wobei die Nukleotidsequenz ein Polypeptid kodiert, umfassend eine konservativ substituierte Variante eines Polypeptids von a), oder wobei die Nukleotidsequenz eine stille Substitution in einer Nukleotidsequenz umfasst.

**6.** Verfahren nach Anspruch 4 oder 5, wobei das rekombinante DNA-Konstrukt weiter einen konstitutiven, induzierbaren oder gewebespezifischen Promoter umfasst, der mit der Nukleotidsequenz operabel verbunden ist, oder wobei das rekombinante DNA-Konstrukt weiter einen starken konstitutiven Promoter vor einer transkribierten Kassette, umfassend eine Nukleotidsequenz nach Anspruch 4 gefolgt von einem funktionellen Pflanzenintron gefolgt von der Nukleotidsequenz nach Anspruch 1 in umgekehrter Orientierung, umfasst.

**7.** Verfahren nach einem der Ansprüche 4 bis 6, wobei das Verfahren den weiteren Schritt des Transformierens von regenerierbaren Zellen einer Pflanze mit dem rekombinanten DNA-Konstrukt und des Regenerierens einer transgenen Pflanze von der transformierten Zelle umfasst.

## Revendications

**1.** Procédé de production d'un arbre ayant une teneur en lignine inférieure par rapport à son type sauvage, comprenant la régulation négative de l'expression d'un produit génique exprimé dans le tissu formant le xylème d'un arbre, ledit produit génique étant codé par une séquence nucléotidique choisie dans le groupe consistant en:

a) la séquence nucléotidique représentée par SEQ ID NO: 29; et
b) une séquence nucléotidique étant au moins 80% identique à une séquence nucléotidique représentée par SEQ ID NO: 29.

**2.** Procédé selon la revendication 1, comprenant les étapes consistant à:

i) fournir un vecteur d'expression comprenant une séquence nucléotidique choisie dans le groupe constitué par:

a) la séquence nucléotidique représentée par SEQ ID NO: 29; et
b) une séquence nucléotidique étant au moins 80% identique à une séquence nucléotidique représentée par SEQ ID NO: 29; et au moins un élément de régulation lié de manière fonctionnelle à la séquence poly-nucléotidique, ledit au moins un élément de régulation contrôlant l'expression de la séquence poly-nucléotidique dans une plante cible;

ii) introduire le vecteur d'expression dans au moins une plante; et
iii) sélectionner au moins une plante transgénique qui présente une quantité de lignine modulée par rapport à son type sauvage.

**3.** Procédé selon la revendication 1 ou 2, dans lequel la régulation négative de l'expression est effectuée en introduisant une modification génétique de préférence dans le locus d'un gène comprenant la séquence nucléotidique représentée par SEQ ID NO: 29 codant pour un polypeptide ou un homologue d'un tel polypeptide, ou dans lequel ladite modification est effectuée par l'un de: l'activation d'ADN-T, le TILLING, la recombinaison homologue, une mutagenèse dirigée en utilisant la séquence nucléotidique représentée par SEQ ID NO: 29 en tant que marqueurs dans une étape quelconque du processus de production des plantes désirables.

**4.** Procédé selon l'une quelconque des revendications 1 à 3, comprenant l'étape consistant à fournir une construction d'ADN recombinant comprenant une séquence nucléotidique choisie dans le groupe constitué par:

a) la séquence nucléotidique représentée par SEQ ID NO: 29;
b) une séquence nucléotidique complémentaire d'une séquence nucléotidique de a);
c) une séquence d'acide nucléique étant d'au moins 80% identique à l'une quelconque des séquences en a) et b); et
d) une séquence nucléotidique qui hybride dans des conditions stringentes à une séquence nucléotidique de a) ou b).

**5.** Procédé selon l'une quelconque des revendications 1 à 4, dans lequel la séquence nucléotidique code pour un polypeptide comprenant un variant substitué de façon conservatrice d'un polypeptide de a), ou dans lequel la séquence nucléotidique comprend une substitution silencieuse dans une séquence nucléotidique.

**6.** Procédé selon la revendication 4 ou 5, dans lequel la construction d'ADN recombinant comprend en outre un promoteur constitutif, inductible, ou spécifique d'un tissu lié de manière fonctionnelle à ladite séquence nucléotidique ou dans lequel la construction d'ADN recombinant comprend en outre un promoteur constitutif fort, devant une cassette transcrite constituée comprenant une séquence nucléotidique telle que définie dans la revendication 4, suivie d'un intron fonctionnel de plante suivi par la séquence nucléotidique telle que définie dans la revendication 1 dans une orientation inversée.

**7.** Procédé selon l'une quelconque des revendications 4 à 6, dans lequel le procédé comprend l'étape supplémentaire consistant à transformer des cellules régénérables d'une plante avec ladite construction d'ADN recombinant et la régénération d'une plante transgénique à partir de ladite cellule transformée.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7402428 B
- WO 2004097024 A
- WO 2008067841 A
- US 4987071 A
- US 5543508 A
- US 5231020 A
- US 5583021 A
- WO 2006078431 A
- WO 9606166 A
- WO 9853057 A
- US 5107065 A
- US 6506559 B
- US 20020168707 A
- WO 9853083 A
- WO 9953050 A
- WO 9961631 A
- WO 2008067840 A
- WO 2009084999 A
- US 2005034176 A, Falco
- US 6451604 B, Flinn
- WO 9114772 A
- WO 2008006033 A


### Non-patent literature cited in the description


- **BAUCHER.** *Critical Reviews in Biochemistry and Molecular Biology,* 2003, vol. 38, 305-350
- **VANHOLME et al.** *Current Opinion in Plant Biology,* 2008, vol. 11, 1-8
- **BAUHCER.** *Critical Reviews in Biochemistry and Molecular Biology,* 2003, vol. 38, 305-350
- **CHEN ; DIXON.** *Nature Biotechnology,* 2007, vol. 25 (7), 759-761
- Breaking the Biological Barriers to Cellulosic Ethanol: A Joint Research Agenda. *A Research Roadmap Resulting from the Biomass to Biofuels Workshop,* 07 December 2005
- **GRIFFITHS, P. R. ; DE HASETH, J. A.** Fourier Transform Infrared Spectrometry. Wiley, 1986
- **MOUNT.** Bioinformatics: sequence and Genome Analysis. Cold Spring Harbor Laboratory Press, 2001, 543
- **SAMBROOK et al.** Molecular Cloning-A Laboratory Manual. Cold Spring Harbor Laboratory, 2001, vol. 1-3
- **SAMBROOK et al.** Molecular Cloning: a Laboratory Manual. Cold Spring Harbor Laboratory Press, 2001
- Plant Molecular Biology Manual. Kluwer Academic Publishers, 2000
- **G.A. TUSKAN et al.** *Science,* 15 September 2006, vol. 313 (5793), 1596-1604
- **HANS ; GORZSÁS ; ANDRÁS ; PERSSON ; PER ; SUNDBERG ; BJÖRN ; TRYGG.** Orthogonal Projections to Latent Structures Discriminant Analysis Modeling on in Situ FT-IR Spectral Imaging of Liver Tissue for Identifying Sources of Variability Stenlund. *Johan Anal. Chem., 2008,* 20 August 2008
- **ASPEBORG et al.** *Plant Physiology,* 2005, vol. 137 (3), 983-997
- *Blom Statistikteori med tillämpningar,* ISBN 91-44-05592-7
- **BRADY ; PROVART.** *Journal of the Science of Food and Agriculture,* 2007, vol. 87, 925-929
- *Roadmap Resulting from the Biomass to Biofuels Workshop,* 07 December 2005
- **BURKE et al.** *Current opinion in genetics and development,* 2007, vol. 17, 525-532
- **BUSTIN S A.** *Journal of Molecular Endocrinology,* 2000, vol. 25, 169-193
- **CHERN et al.** *Plant J.,* 2001, vol. 27, 101-113
- **FAN ; DONG.** *Plant Cell,* 2002, vol. 14, 1377-1389
- **FENG ; DOOLITTLE.** *J. Mol. Evol.,* 1987, vol. 25, 351-360
- **GILMOUR et al.** *Plant J.,* 1998, vol. 16, 433-442
- **HENIKOFF ; TILL ; COMAI.** *Plant Physiol.,* June 2004, vol. 135 (2), 630-6
- **HEO et al.** *Plant and Cell Physiology,* 2005, vol. 46 (12), 2005-2018
- **ICHIKAWA et al.** *Nature,* 1997, vol. 390, 698-701
- **JAGLO et al.** *Plant Physiol.,* 1998, vol. 127, 910-917
- **KAKIMOTO et al.** *Science,* 1996, vol. 274, 982-985
- **KARIMI M ; INZE D ; DEPICKER A.** *Treands in plant Sciences,* 2002, vol. 7 (5), 193-195
- **KANG et al.** *Cell.,* 01 June 2001, vol. 105 (5), 625-36

- *Klason-lignin analysis Tappi method T 222 om-88, http://www.tappi.org*
- **KONCZ et al.** Plant Molecular Biology Manual. 1994, vol. B2, 1-22
- **KOSUGI ; OHASHI.** *Plant J.,* 2002, vol. 29, 45-59
- **LEE et al.** *Genome Res.,* 2002, vol. 12, 493-502
- **LICHTENSTEIN ; NELLEN.** Antisense Technology: A Practical Approach. IRL Press at Oxford University, 1997
- **MOUNT.** Bioinformatics: Sequence and Genome Analysis. Cold Spring Harbor Laboratory Press, 2001, 543
- **NILSSON et al.** *Transgenic Research,* 1992, vol. 1, 209-220
- **RATCLIFFE et al.** *Plant Physiol.,* 2001, vol. 126, 122-132
- **REMM et al.** *J. Mol. Biol.,* 2001, vol. 314, 1041-1052
- **ROGNES T.** *Nucleic Acids Research,* 1989, vol. 29, 1647-1652
- **SLADE ; KNAUF.** *Transgenic Res.,* April 2005, vol. 14 (2), 109-15
- **SMITH ; WATERMAN.** *Hellene! DNA aligning,* 1981
- **THOMPSON et al.** *Nucleic Acids Res.,* 1994, vol. 22, 4673-4680
- **TUSKAN et al.** *Science,* 15 September 2006, vol. 313 (5793), 1596-1604
- **VARSHNEY et al.** *Trends in Plant Science,* 2005, vol. 10, 621-630
- **VASIL et al.** Cell Culture and Somatic Cell Genetics of Plants. Laboratory Procedures, 1984, vol. I, II, III
- **ZHOU et al.** *Plant and Cell Physiology,* 2006, vol. 47 (9), 1229-1240